# EUROPEAN PATENT APPLICATION

(11) **EP 0 885 890 A1**
(43) Date of publication of application: **23.12.1998**
(21) Application number: 97904634.9
(22) Date of filing: 25.02.1997
(51) Int. Cl.: C07D 231/40, C07D 405/12, C07D 407/12, C07D 409/12, C07D 417/12, A61K 31/415, A61K 31/425, A61K 31/445, A61K 31/47, A61K 31/535

(54) **SULFONYLUREIDOPYRAZOLE DERIVATIVES**

(30) Priority: 26.02.1996 JP 65498/96
(71) Applicant: SUMITOMO PHARMACEUTICALS COMPANY, LIMITED, Osaka-shi, Osaka-fu 541 (JP)
(72) Inventor: MATSUSHITA, Kayo, Izumisano-shi, Osaka 598 (JP); HASEGAWA, Hirohiko, Ibaraki-shi, Osaka 567 (JP); KURIBAYASHI, Yoshikazu, Takatsuki-shi, Osaka 569 (JP); OHASHI, Naohito, Takatsuki-shi, Osaka 569-11 (JP)
(74) Representative: VOSSIUS & PARTNER
(86) International application number: JP9700532
(87) International publication number: WO9730978

(57) **Abstract**

A sulfonylureidopyrazole derivative of formula (1) or (2) is disclosed. The derivative has an inhibitory activity on endothelin converting enzyme, and is useful for treating or preventing various cardiac failures, tracheal constrictions, nervous disorders, parasecretion, vascular disorders, various ulcers and the like.

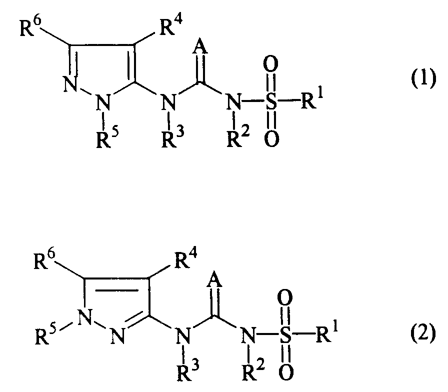

## Description

### FIELD OF THE INVENTION

The present invention relates to an inhibitor of endothelin converting enzyme, which comprises a sulfonyureidopyrazole derivative or a pharmaceutically acceptable salt thereof, and to a composition which contains such endothelin converting enzyme inhibitor as an active agent for treating or preventing various diseases such as a cardiovascular disease.

### PRIOR ART

Endothelin (hereinafter, it is abbreviated to as "ET") is a strong vasoconstrictive peptide composed of 21 amino acid residues, which was isolated from a culture supernatant of vascular endothelial cells (Yanagisawa et. al. Nature, 322, 411-415, 1988). It is understood that ET plays an important role physiologically since it exhibits a vasoconstrictive effect and a cell proliferative effect in vivo, and is produced by various organs such as blood vessels. On the basis of its action, ET is believed to be responsible for pathogenesis of diseases such as hypertension, cerebral vasospasm after subarachnoid hemorrhage, myocardial infarction, arteriosclerosis, renal failure, cardiac failure, asthma, and the like. Further, it is known that ET level in blood of patients of Raynaud's disease, Buerger's disease, Takayasu's disease, Kawasaki's disease, cisplatin-induced renal damage, and the like, is significantly elevated when it is compared with that of normal adults.

ET is formed in the course of its biosynthesis from big endothelin (hereinafter, it is abbreviated to as big ET) which is a precursor having low activity, by a specific protease, ET converting enzyme (hereinafter, it is abbreviated to as ECE). Accordingly, it is understood that reduction of the ET biosynthesis by inhibiting ECE is effective to treat and prevent the various diseases as mentioned above. So far, phosphoramidon, which is produced by Actinomycetes such as Streptomyces tanashiensis has been known as an inhibitor of ECE.

Sulfonylureidopyrazole derivatives are described in the treatise of the department of agriculture in Meijo university (1992), vol. 28, pp 49-59; Japanese Patent Publication (kokai) No. 47757/1989; Japanese Patent Publication (kokai) No. 148482/1987; Indian J. Chem., sect. B (1986), 25B (9) 934-938; Pol. J. Pharmacol. Pharm. (1974), 26 (4), 479-482; WO92/10480. However, the activity of these derivatives as an endothelin converting enzyme inhibitor has not been yet known.

### DISCLOSURE OF THE INVENTION

Under the circumstances, there is a demand of the exploration for an inhibitor of ECE, said exploration may lead to development of new agents which are useful in treating and preventing various diseases induced or suspected to be induced by ET, for example, cardiac failure such as myocardial ischemia, congestive heart failure, arrhythmia, unstable angina, cardiac hypertrophy, hypertension; tracheal constriction such as pulmonary hypertension, asthma; nervous disorder such as cerebral vasospasm, subarachnoid hemorrhage, stroke, cerebral infarction, Alzheimer's disease; parasecretion such as eclampsia; vascular disorder such as arteriosclerosis, Buerger's disease, Takayasu's arteritis, Raynaud's disease, complication of diabetes mellitus; ulcer such as gastric ulcer; cancer such as lung cancer; damage of gastric mucosa; endotoxin shock; sepsis; renal damage such as acute and chronic renal failure; and the like. Thus, the aim of the present invention includes the exploration for the inhibitors of ECE and the development of agents which are based on such inhibitory action against ECE so as to treat and prevent the various diseases as described above.

The inventors have studied new inhibitors of ET converting enzyme, and the inventors accomplished the present invention by finding the fact that compounds of the following formula have the much better inhibitory activities than the known compounds as shown above. Specifically, the present invention provides pharmaceutical compositions and active compounds of the embodiments (I) to (VI):
(I) a composition for inhibiting endothelin converting enzyme which comprises a compound of formula (1) or (2): 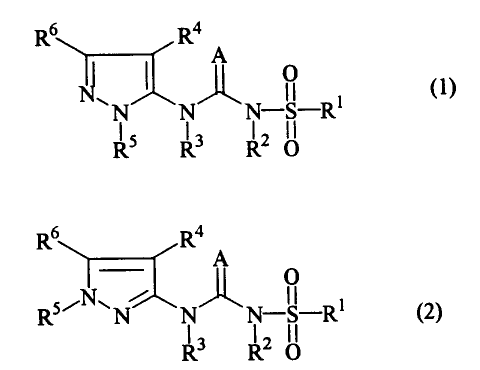 wherein:
   A is an oxygen atom or a sulfur atom;
   R¹ is an alkyl group, an alkenyl group, an alkynyl group, a cycloalkyl group, a cycloalkenyl group, an aryl group, a heteroarylalkyl group, a heterocyclic group, -OR⁷, -SR⁷, -N(R⁷)R⁷¹, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heteroarylalkyl group, or a substituted heterocyclic group, or a group of formula (a): 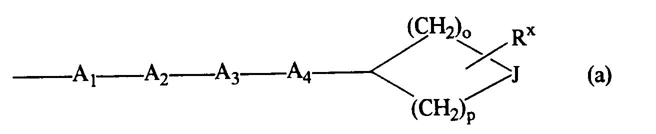 or formula (b): 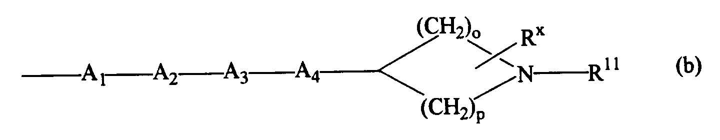
   R² and R³, which may be the same or different, are each a hydrogen atom, an alkyl group, an alkenyl group, an alkynyl group, a cycloalkyl group, a cycloalkenyl group, an aryl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (a) or (b) as shown above;
   R⁴ and R⁶, which may be the same or different, are each a hydrogen atom, a halogen atom, a cyano group, a nitro group, an alkyl group, an alkenyl group, an alkynyl group, a cycloalkyl group, a cycloalkenyl group, an aryl group, a heterocyclic group, a heteroarylalkyl group, -OR¹², -N(R¹²)R¹³, -CO-R¹², -CS-R¹², -CO₂-R¹², -CO-S-R¹², -CS₂-R¹², -CS-O-R¹², -O-CO-R¹², -O-CS-R¹², -S-CO-R¹², -S-CS-R¹², -CON(R¹²)R¹³, -CSN(R¹²)R¹³, -S(O)ₗ-R¹², -SO₂-N(R¹²)R¹³, -N(R¹²)-CO-R¹³, -OSO₂-R¹², a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heteroarylalkyl group, or a substituted heterocyclic group, or a group of formula (a) or (b) as shown above;
   R⁵ is a hydrogen atom, an alkyl group, an alkenyl group, an alkynyl group, a cycloalkyl group, a cycloalkenyl group, an aryl group, a heterocyclic group, a heteroarylalkyl group, -CO-R¹², -CS-R¹², -CO₂-R¹², -CO-S-R¹², -CS₂-R¹², -CS-O-R¹², -CON(R¹²)R¹³, -CSN(R¹²)R¹³, -S(O)ₗ-R¹², or -SO₂-N(R¹²)R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (a) or (b) as shown above;
   R⁷ and R⁷¹ are the same or different and are each a hydrogen atom, an alkyl group, an alkenyl group, an alkynyl group, a cycloalkyl group, a cycloalkenyl group, an aryl group, a heteroarylalkyl group, a heterocyclic group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group,
      provided that in the case of -N(R⁷)R⁷¹, then R⁷ and R⁷¹ may be combined together with the nitrogen atom to which they are attached to form a saturated 3- to 8-membered ring which optionally contains other heteroatoms in the ring;
   A₁, A₂, A₃, and A₄ are the same or different and are each a single bond or -CH₂-, or any two adjacent groups of them are combined together to form -CH=CH-, or -C≡C-;
   R^{X} may be absent, or one or more groups which replaces a hydrogen atom attached to a ring carbon atom, and they are the same or different and are each a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, a heterocyclic group, or -A₅-A₆-A₇-A₈-R^{Y};
   o and p are independently 0 or an integer of any of 1 to 3, provided that o and p are not simultaneously 0;
   J is an oxygen atom, or -S(O)_{q}- in which q is 0, 1, or 2;
   R¹¹ is a hydrogen atom, an alkyl group, an alkenyl group, an alkynyl group, a cycloalkyl group, a cycloalkenyl group, an aryl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
   A₅, A₆, A₇, and A₈ are the same or different and are each a single bond or -CH₂-, or any two adjacent groups of them are combined together to form -CH=CH-, or -C≡C-;
   R^{Y} is -OR⁸, -N(R⁸)R⁹, -CO-R⁸, -CS-R⁸, -CO₂-R⁸, -CO-S-R⁸, -CS₂-R⁸, -CS-O-R⁸, -O-CO-R⁸, -O-CS-R⁸, -S-CO-R⁸, -S-CS-R⁸, -CON(R⁸)R⁹, -CSN(R⁸)R⁹, -S(O)ₗ-R⁸, -SO₂-N(R⁸)R⁹, -O-CO₂-R⁸, or -N(R⁸)-CO-R⁹;
   l is 0, 1, or 2;
   R⁸ and R⁹ are the same or different and are independently a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, or a heteroarylalkyl group,
      provided that in the case of -N(R⁸)R⁹, -CON(R⁸)R⁹, -CSN(R⁸)R⁹, -SO₂-N(R⁸)R⁹, or -N(R⁸)-CO-R⁹, then R⁸ and R⁹ may be combined together with the nitrogen atom (and carbon atom) to which they are attached to form a saturated 3- to 8-membered ring which optionally contains other heteroatoms in the ring,
      provided that R⁸ is not a hydrogen atom in the case of -O-CO-R⁸, -O-CS-R⁸, -S-CO-R⁸, -S-CS-R⁸, -SO-R⁸, or -SO₂-R⁸;
   R¹² and R¹³ are the same or different and are each a hydrogen atom, an alkyl group, an alkenyl group, an alkynyl group, a cycloalkyl group, a cycloalkenyl group, an aryl group, a heteroarylalkyl group, a heterocyclic group, a substituted alkyl group, a substituted alkenyl group, a substituted alkynyl group, a substituted cycloalkyl group, a substituted cycloalkylalkyl group, a substituted cycloalkenyl group, a substituted cycloalkenylalkyl group, a substituted aryl group, a substituted aralkyl group, a substituted heteroarylalkyl group, or a substituted heterocyclic group,
      provided that R¹² is not a hydrogen atom, when R⁴ or R⁶ is -O-CO-R¹², -O-CS-R¹², -S-CO-R¹², -S-CS-R¹², -SO-R¹², or -SO₂-R¹²;
   in the above R¹ to R¹³, such substituents on the substituted alkyl group, the substituted alkenyl group, and the substituted alkynyl group are the same or different and are each one or more selected from the group consisting of a halogen atom, a nitro group, a cyano group, a cycloalkyl group, a cycloalkenyl group, an aryl group, -A₅-A₆-A₇-A₈-R^{Y}, and a group of the formula: 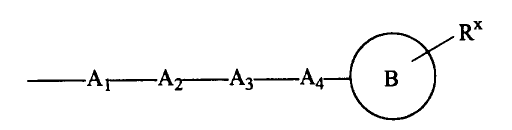 wherein A₁, A₂, A₃, A₄ and R^{X} are as defined above, B ring is a cycloalkyl group, a cycloalkenyl group, an aryl group, or a heterocyclic group; and
   such substituents on the substituted cycloalkyl group, the substituted cycloalkenyl group, the substituted cycloalkylalkyl group, the substituted cycloalkenylalkyl group, the substituted aryl group, the substituted aralkyl group, the substituted heterocyclic group, or the substituted heteroarylalkyl group are the same or different and are each one or more selected from the group consisting of a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -A₅-A₆-A₇-A₈-R^{Y}, and a group of the formula: 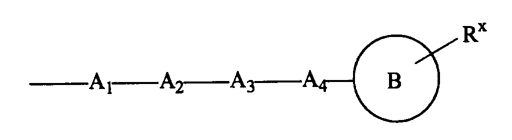 wherein A₁, A₂, A₃, A₄ and R^{X} are as defined above, B ring is a cycloalkyl group, a cycloalkenyl group, an aryl group, or a heterocyclic group; or
   any substituents attached to the adjacent carbon atoms may be optionally combined together with the carbon atoms with which they are substituted to form a 4- to 8-membered ring optionally containing other heteroatoms;
   or a pharmaceutically acceptable acid addition salt or alkali salt thereof;
(II) a pharmaceutical composition for treating or preventing a cardiac failure, a tracheal constriction, a nervous disorder, a dyschylia, a vascular disorder, an ulcer, a cancer, a damage of gastric mucosa, an endotoxic shock, sepsis, or a renal damage, which comprises the compound of formula (1) or (2) as shown above, or a pharmaceutically acceptable acid addition salt or alkali salt thereof;
(III) a 5-sulfonylureido-(1H)-pyrazole derivative of formula (1) as shown above, or a pharmaceutically acceptable acid addition salt or alkali salt thereof;
   provided that the following compounds are excluded:
   i) a compound of the formula: 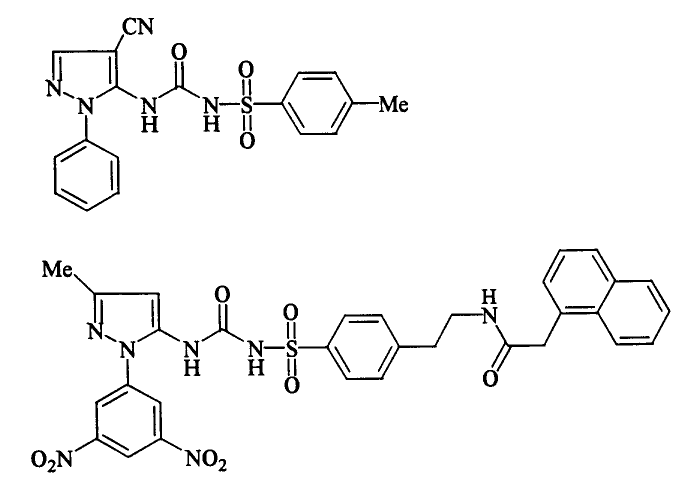 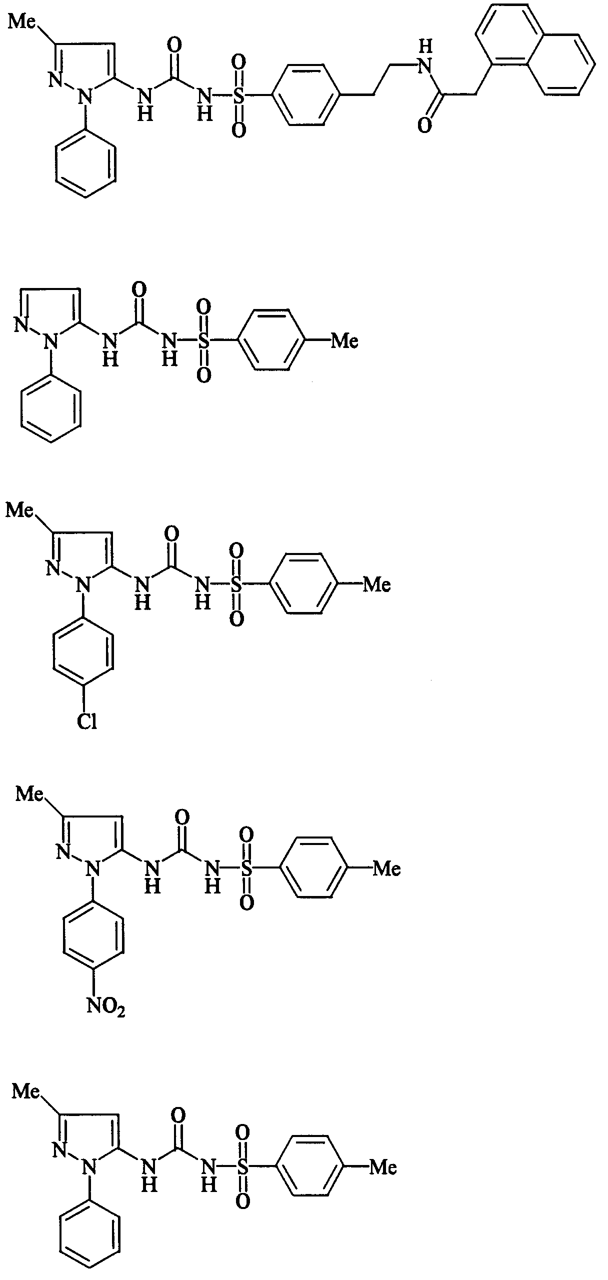 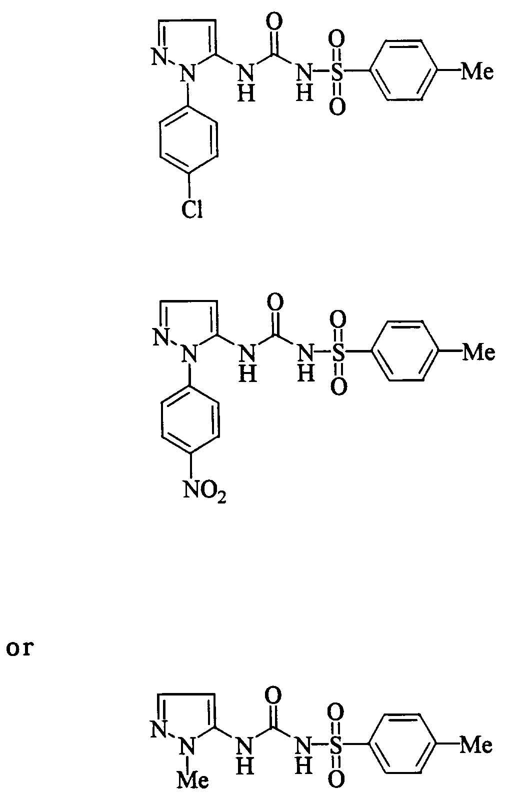
   ii) a compound wherein R¹ is 2-methylphenyl group, and R⁵ is hydrogen or an alkyl group,
   iii) a compound wherein R¹ is -NR⁷R⁷¹, and
   iv) a compound wherein R¹ is a substituted heteroarylalkyl group;
(IV) a 3-sulfonylureido-(1H)-pyrazole derivative of formula (2) as shown above, or a pharmaceutically acceptable acid addition salt or alkali salt thereof;
   provided that the following compounds are excluded:
   i) a compound of the formula: 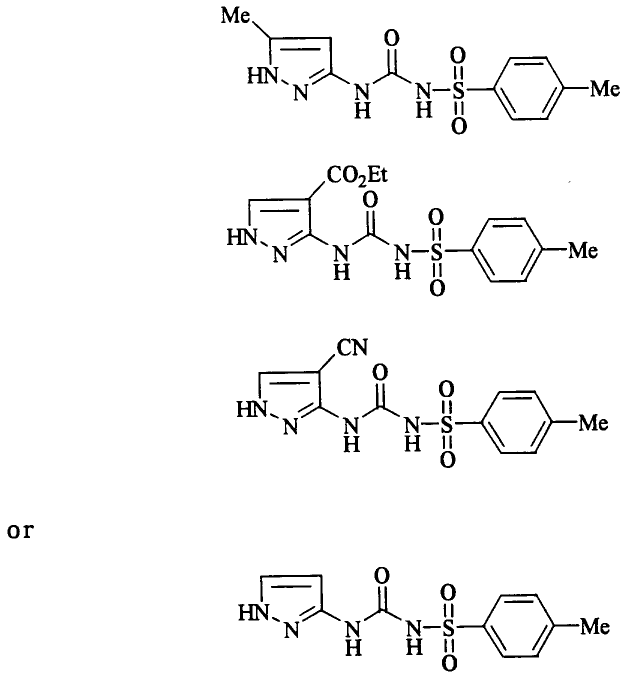
   ii) a compound wherein R¹ is 2-methylphenyl group, and R⁵ is hydrogen,
   iii) a compound wherein R¹ is a substituted or unsubstituted pyrazolyl,
   iv) R¹ is a substituted heteroarylalkyl group,
(V) a compound of formula (1) as shown above,
   wherein:
   A is an oxygen atom or a sulfur atom;
   R¹ is an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁷, -SR⁷, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, or a substituted heteroarylalkyl group, or a group of formula (c): 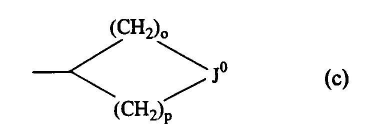 or formula (d): 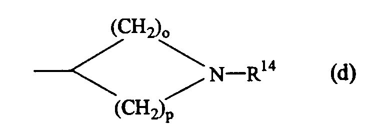
   R² and R³, which may be the same or different, are each a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (c) or (d) as shown above;
   R⁴ is a hydrogen atom, a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -OSO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (c) or (d) as shown above;
   R⁵ is an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -S(O)ₗ-R¹², -CO₂-R¹², -CO-R¹², -CS-R¹², -CON(R¹²)R¹³, -SO₂-N(R¹²)R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
   R⁶ is a hydrogen atom, a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -O-SO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
   R⁷ is a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
   o and p are independently 0 or an integer of 1 to 3, provided that o and p are not simultaneously 0;
   J^{O} is an oxygen atom, or a sulfur atom;
   R¹⁴ is a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
   l is 0, 1, or 2;
   R⁸ and R⁹ are the same or different and are independently a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, or a heteroarylalkyl group;
   R¹² and R¹³ are the same or different and are each a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a substituted alkyl group, a substituted cycloalkyl group, a substituted cycloalkenyl group, a substituted aryl group, a substituted aralkyl group, a substituted heterocyclic group, or a substituted heteroarylalkyl group;
   in the above R¹ to R¹³, such substituents on the substituted alkyl group, the substituted alkenyl group, and the substituted alkynyl group are the same or different and are each one or more selected from the group consisting of a halogen atom, a nitro group, a cyano group, a cycloalkyl group, a cycloalkenyl group, an aryl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹; and
   such substituents on the substituted cycloalkyl group, the substituted cycloalkenyl group, the substituted cycloalkylalkyl group, the substituted cycloalkenylalkyl group, the substituted aryl group, the substituted aralkyl group, the substituted heterocyclic group, and the substituted heteroarylalkyl group are the same or different and are each one or more selected from the group consisting of a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹,
      provided that i) when R⁴ is the hydrogen atom, then R¹ is 4-chlorophenyl or 2-methylphenyl, ii) when R⁵ is the alkyl group, then R⁴ is the cyano group, and iii) the compound of the formula: 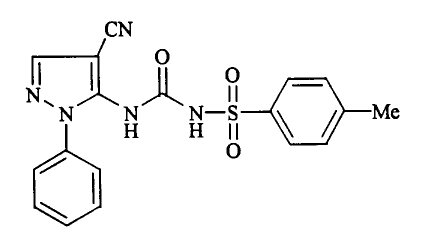 is excluded;
   or a pharmaceutically acceptable acid addition salt or alkali salt thereof; and
(VI) a compound of formula (2) as shown above,
   wherein:
   A is an oxygen atom or a sulfur atom;
   R¹ is an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁷, -SR⁷, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, or a substituted heteroarylalkyl group, or a group of formula (c) or (d) as shown above;
   R² and R³, which may be the same or different, are each a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (c) or (d) as shown above;
   R⁴ is a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -OSO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
   R⁵ is an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -S(O)ₗ-R¹², -CO₂-R¹², -CO-R¹², -CS-R¹², -CON(R¹²)R¹³, -SO₂-N(R¹²)R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
   R⁶ is a hydrogen atom, a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -O-SO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
   R⁷ is a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
   o and p are independently 0 or an integer of 1 to 3, provided that o and p are not simultaneously 0;
   J^{O} is an oxygen atom, or a sulfur atom;
   R¹⁴ is a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
   l is 0, 1, or 2;
   R⁸ and R⁹ are the same or different and are independently a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, or a heteroarylalkyl group;
   R¹² and R¹³ are the same or different and are each a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a substituted alkyl group, a substituted cycloalkyl group, a substituted cycloalkenyl group, a substituted aryl group, a substituted aralkyl group, a substituted heterocyclic group, or a substituted heteroarylalkyl group;
   in the above R¹ to R¹³, such substituents on the substituted alkyl group, the substituted alkenyl group, and the substituted alkynyl group are the same or different and are each one or more selected from the group consisting of a halogen atom, a nitro group, a cyano group, a cycloalkyl group, a cycloalkenyl group, an aryl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹; and
   such substituents on the substituted cycloalkyl group, the substituted cycloalkenyl group, the substituted cycloalkynyl group, the substituted cycloalkenylalkyl group, the substituted aryl group, the substituted aralkyl group, the substituted heterocyclic group, and the substituted heteroarylalkyl group are the same or different and are each one or more selected from the group consisting of a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹;
   or a pharmaceutically acceptable acid addition salt or alkali salt thereof.

Various groups on the compounds of the present invention are illustrated by the following descriptions.

The term "alkyl group" includes a straight or branched chain alkyl group having one to eight carbon atoms, such as methyl, ethyl, propyl, 2-propyl, butyl, 2-butyl, 2-methylpropyl, 1,1-dimethylethyl, pentyl, hexyl, heptyl, or octyl.

The preferable substituted alkyl groups include the cycloalkylalkyl group, the cycloalkenylalkyl group, the aralkyl group, and the like.

The term "alkenyl group" includes a straight or branched chain alkenyl group having two to eight carbon atoms, such as vinyl, allyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-heptenyl, 2-heptenyl, 3-heptenyl, 4-heptenyl, 5-heptenyl, 6-heptenyl, 1-octenyl, 2-octenyl, 3-octenyl, 4-octenyl, 5-octenyl, 6-octenyl, or 7-octenyl.

The term "alkynyl group" includes a straight or branched chain alkynyl group having two to eight carbon atoms, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 2-heptynyl, 3-heptynyl, 4-heptynyl, 5-heptynyl, 6-heptynyl, 1-octynyl, 2-octynyl, 3-octynyl, 4-octynyl, 5-octynyl, 6-octynyl, or 7-octynyl.

The term "cycloalkyl group" includes a cycloalkyl group having 3 to 12 carbon atoms, such as cyclopropyl, cyclobutyl, cyclopentyl, cyclohexyl, cycloheptyl, cyclooctyl, *cis*-decalin-1-yl, *cis*-decalin-2-yl, or *trans*-decalin-1-yl.

The term "cycloalkylalkyl group" includes a cycloalkylalkyl group having 4 to 14 carbon atoms, such as cyclopropylmethyl, cyclobutylmethyl, cyclopentylmethyl, cyclohexylmethyl, cycloheptylmethyl, cyclooctylmethyl, cyclopropylethyl, cyclobutylethyl, cyclopentylethyl, cyclohexylethyl, cycloheptylethyl, or cyclooctylethyl.

The term "cycloalkenyl group" includes a cycloalkenyl group having 3 to 8 carbon atoms, such as 1-cyclobutenyl, 1-cyclopentenyl, 2-cyclopentenyl, 3-cyclopentenyl, 1-cyclohexenyl, 2-cyclohexenyl, 3-cyclohexenyl, 1-heptenyl, 2-heptenyl, 3-heptenyl, 4-heptenyl, 1-octenyl, 2-octenyl, 3-octenyl, or 4-octenyl.

The term "cycloalkenylalkyl group" includes a cycloalkenylalkyl group having 4 to 14 carbon atoms, such as 1-cyclobutenylmethyl, 1-cyclopentenylmethyl, 2-cyclopentenylmethyl, 3-cyclopentenylmethyl, 1-cyclohexenylmethyl, 2-cyclohexenylmethyl, 3-cyclohexenylmethyl, 1-cycloheptenylmethyl, 2-cycloheptenylmethyl, 3-cycloheptenylmethyl, 4-cycloheptenylmethyl, 1-cyclooctenylmethyl, 2-cyclooctenylmethyl, 3-cyclooctenylmethyl, 4-cyclooctenylmethyl, 1-cyclobutenylethyl, 1-cyclopentenylethyl, 2-cyclopentenylethyl, 3-cyclopentenylethyl, 1-cyclohexenylethyl, 2-cyclohexenylethyl, 3-cyclohexenylethyl, 1-cycloheptenylethyl, 2-cycloheptenylethyl, 3-cycloheptenylethyl, 4-cycloheptenylethyl, 1-cyclooctenylethyl, 2-cyclooctenylethyl, 3-cyclooctenylethyl, or 4-cyclooctenylethyl.

The term "aryl group" includes an aryl group having 6 to 10 carbon atoms, such as phenyl, or naphthyl.

The term "aralkyl group" includes an aralkyl group having 7 to 14 carbon atoms, such as benzyl, 1-phenylethyl, 2-phenylethyl, 3-phenylpropyl, 2-phenylpropyl, 1-phenylpropyl, 4-phenylbutyl, 3-phenylbutyl, 2-phenylbutyl, 1-phenylbutyl, 1-naphthylmethyl, 2-naphthylmethyl, 2-(1-naphthyl)-ethyl, 2-(2-naphthyl)-ethyl, 3-(1-naphthyl)-propyl, 2-(2-naphthyl)-propyl, 4-(1-naphthyl)-butyl, 3-(2-naphthyl)-butyl.

The term "heterocyclic group" includes a heteroaryl group, or an unsaturated or saturated 5- or 6-membered heterocyclic group containing carbon atoms and two or three heteroatoms selected from the group consisting of nitrogen, oxygen, and sulfur atoms.

The saturated heterocyclic groups include 2-piperazinyl, 1-morpholinyl, 2-morpholinyl, 3-morpholinyl, morpholino, and the like.

The unsaturated heterocyclic groups include imidazoline-2-yl and the like.

The term "heteroaryl group" includes a 5- or 6-membered cyclic ring system containing one to four nitrogen atoms, a 5-or 6-membered cyclic ring system containing one or two nitrogen atoms as well as one oxygen atom or one sulfur atom a 5-membered cyclic ring system containing one oxygen atom or one sulfur atom, or a fused ring system which is formed by fusing any cyclic ring systems as shown above each other, or by fusing any of the cyclic ring systems as shown above with a benzene or naphthalene ring. Specific groups include 2-pyridyl, 3-pyridyl, 4-pyridyl, 2-thienyl, 3-thienyl, 2-furyl, 3-furyl, imidazolyl, triazolyl, tetrazolyl, pyrazolyl, pyrrolyl, 2-thiazolyl, 3-isothiazolyl, 2-oxazolyl, 3-isoxazolyl, 2-benzofuryl, 2-benzothienyl, 2-quinolyl, 3-quinolyl, 4-quinolyl, 2-indolinyl, 3-(1H)-indazolyl, 8-purinyl, 2-quinazolinyl, 3-cinnolinyl, 2-naphthyridinyl, and the like.

The term "heteroarylalkyl group" includes a straight or branched chain alkyl group having one to eight carbon atoms attached to a 5- or 6-membered cyclic ring system containing one to four nitrogen atoms, a 5- or 6-membered cyclic ring system containing one or two nitrogen atoms as well as one oxygen atom or one sulfur atom, or a 5- or 6-membered cyclic ring system containing one oxygen atom or one sulfur atom. Specific groups includes 2-pyridylmethyl, 2-pyridylethyl, 3-pyridylmethyl, 3-pyridylethyl, 3-pyridylpropyl, 4-pyridylmethyl, 2-thienylmethyl, 3-(2-thienyl)-2-methylpropyl, 3-thienylmethyl, 4-(2-thienyl)-3-methylbutyl, 2-furylethyl, 2-furylpentyl, 3-furylmethyl, 5-(3-furyl)-3-methylpentyl, 2-imidazolylmethyl, triazolylethyl, tetrazolylmethyl, 3-(1-pyrazolyl)propyl, 3-(3-pyrazolyl)propyl, 1-pyrrolylmethyl, 3-(1-pyrrolyl)butyl, 2-pyrrolylmethyl, 2-thiazolylmethyl, 4-(2-thiazolyl)pentyl, 3-isothiazolylmethyl, 3-(2-oxazolyl)pentyl, 3-isoxazolylmethyl, and the like.

The term "saturated 3- to 8-membered ring which optionally contains other heteroatoms in the ring, which is formed by combining "R⁷ and R⁷¹" or "R⁸ and R⁹", in the case of -N(R⁷)R⁷¹, -N(R⁸)R⁹, -CON(R⁸)R⁹, -CSN(R⁸)R⁹, -SO₂-N(R⁸)R⁹, or -N(R⁸)-CO-R⁹, includes an unsaturated or saturated 3- to 8-membered cyclic ring system containing one nitrogen atom as well as 0-2 heteroatoms selected from the group consisting of a nitrogen, oxygen and sulfur atoms, and carbon atoms. For example, such rings include piperidin-1-yl, pyrrolidin-1-yl, 4-morpholino, piperazin-1-yl, in the case of -N(R⁸)R⁹ or -N(R⁷)R⁷¹; piperidin-1-ylcarbonyl, pyrrolidin-1-ylcarbonyl, 4-morpholinocarbonyl, piperazin-1-ylcarbonyl in the case of -CON(R⁸)R⁹; piperidin-1-ylthiocarbonyl, pyrrolidin-1-ylthiocarbonyl, 4-morpholinothiocarbonyl, piperazin-1-ylthiocarbonyl in the case of -CSN(R⁸)R⁹; piperidin-1-ylsulfonyl, pyrrolidin-1-ylsulfonyl, 4-morpholinosulfonyl, piperazin-1-ylsulfonyl, in the case of -SO₂-N(R⁸)R⁹; 2-pyrrolidinon-1-yl, 3-oxo-4-morpholino, in the case of -N(R⁸)-CO-R⁹; and the like.

The term "halogen atom" includes a fluorine, chlorine, bromine, or iodine atom.

The term "4- to 8-membered ring optionally containing other heteroatoms, which is formed by combining any substituents attached to the adjacent carbon atoms together with the carbon atoms to which they attached" in the case of the substituted cycloalkyl group, the substituted cycloalkenyl group, the substituted cycloalkylalkyl group, the substituted cycloalkenylalkyl group, the substituted aryl group, the substituted aralkyl group, the substituted heterocyclic group, or the substituted heteroarylalkyl group, includes a 4- to 8-membered, unsaturated or saturated cyclic ring system containing carbon atoms and 0-2 heteroatoms selected from the group consisting of nitrogen, oxygen, and sulfur atoms, which ring system is fused to a cycloalkyl group, a cycloalkenyl group, an aryl group, a heterocyclic group, or the like to form a fused ring. Such rings include perhydroindol-5-yl, perhydrobenzofuran-5-yl as the substituted cycloalkyl group; 2, 3, 4, 5, 6, 7-hexahydro-(1H)-indol-5-yl, 5, 6, 7, 8-tetrahydroquinolin-7-yl as the substituted cycloalkenyl group; perhydroindol-5-ylethyl, perhydrobenzofuran-5-ylethyl as the substituted cycloalkylalkyl group; 2, 3, 4, 5, 6, 7-hexahydro-(1H)-indol-5-ylethyl, 5, 6, 7, 8-tetrahydroquinolin-7-ylethyl as the substituted cycloalkenylalkyl group; 2, 3-dihydro-(1H)-indol-5-yl, 2, 3-dihydrobenzofuran-6-yl, 1, 3-dioxaindan-4-yl as the substituted aryl group; 2, 3-dihydro-(1H)-indol-5-ylmethyl, chroman-6-ylmethyl as the substituted aralkyl group; 5, 6, 7, 8-tetrahydroquinazolin-6-yl as the substituted heterocyclic group; 5, 6, 7, 8-tetrahydroquinazolin-6-ylethyl as the substituted heteroarylalkyl group; and the like.

Preferred groups in R¹ include cyclohexyl, phenyl, 2-naphthyl, 3-naphthyl, 3-tolyl, 4-tolyl, 3-ethylphenyl, 4-ethylphenyl, 3-isopropylphenyl, 4-isopropylphenyl, 3-isobutylphenyl, 4-isobutylphenyl, 3-n-butylphenyl, 4-n-butylphenyl, 3-methoxyphenyl, 4-methoxyphenyl, 3-n-propylphenyl, 4-n-propylphenyl, 3-chlorophenyl, 4-chlorophenyl, 3-bromophenyl, 4-bromophenyl, benzyl, and the like.

Preferred groups in R² include hydrogen, methyl, benzyl, and the like.

Preferred groups in R³ include hydrogen, methyl, 2-methoxyethyl, benzyl, and the like.

Preferred groups in R⁴ include hydrogen, cyano, and the like.

Preferred groups in R⁵ include vinyl, ethyl, n-propyl, isopropyl, cyclohexyl, cyclopentyl, phenyl, a substituted phenyl, thiophen-2-yl, thiophen-3-yl, furan-2-yl, furan-3-yl, tetrahydro-(4H)-pyran-4-yl, and the like.

Preferred groups in R⁶ include hydrogen; an alkyl such as methyl, ethyl, n-propyl, isopropyl, or n-butyl; and a substituted alkyl such as cyanomethyl, methoxycarbonylmethyl, or ethoxycarbonylmethyl; and the like.

Some compounds of the present invention have one or several asymmetric carbon atoms, and can thus exist in the form of stereoisomers. The compounds of the invention encompass a mixture of individual stereoisomer, and an isolated one.

The compounds of formula (I) may be prepared according to the procedure, for example, as illustrated in the following schemes:
(A) 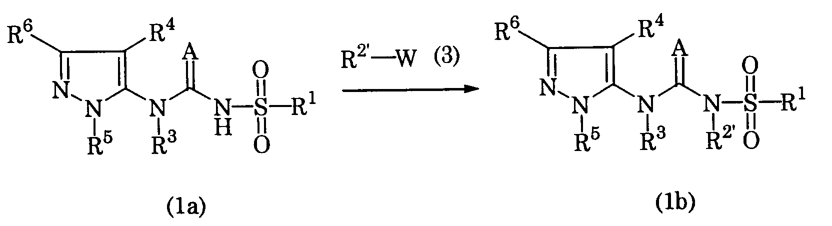 wherein A, R¹, R³, R⁴, R⁵, and R⁶ are as defined above, R²' has the same meaning as R² provided that R²' does not denote a hydrogen atom, W is a leaving group which may be easily replaced via nucleophilic reaction.
   Compound (1b) may be synthesized by reacting compound (1a) with one to five equivalents of compound (3) in a conventional solvent under cooling, at room temperature or under heating in the presence of an appropriate base.
   In the above reaction, the base includes an inorganic base such as lithium hydroxide, sodium hydroxide, potassium hydroxide, sodium carbonate, sodium bicarbonate, potassium carbonate, potassium bicarbonate; a metal hydride such as sodium hydride, lithium hydride, potassium hydride, calcium hydride; an organometallic base such as butyl lithium, phenyl lithium, sodium ethoxide, sodium methoxide, sodium *tert*-butoxide, potassium *tert*-butoxide, lithium amide, lithium (di-isopropyl)amide; an organic base such as triethylamine, pyridine, di-isopropylethylamine. The solvent includes aromatic hydrocarbons such as benzene, toluene; halogenated hydrocarbons such as dichloromethane, chloroform, 1, 2-dichloroethane; amides such as dimethylformamide, dimethylacetamide; ethers such as tetrahydrofuran, ether, 1, 4-dioxane, 1, 2-dimethoxyethane; basic solvents such as pyridine; and mixtures thereof.
   The leaving group W includes a halogen atom such as a chlorine atom, bromine atom and iodine atom, methanesulfonyloxy, benzenesulfonyloxy, toluenesulfonyloxy, substituted benzenesulfonyloxy, trifluoromethanesulfonyloxy, and the like.
   The compounds of formula (1a) of the present invention can be prepared according to the procedures, for example, as illustrated in any of the following schemes (B) to (E):
(B) The compounds of formula (1a) may be obtained by reacting compound (4) with one to five equivalents of compound (5) in a conventional solvent under cooling, at room temperature or under heating in the presence or absence of a base, as shown in the following scheme. 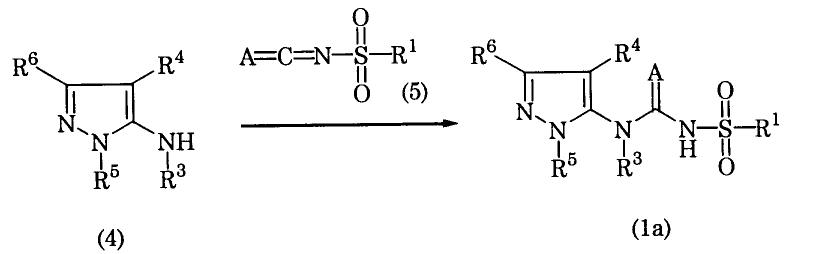 wherein A, R¹, R³, R⁴, R⁵, and R⁶ are as defined above.
   In the above reaction, the base includes an inorganic base such as lithium hydroxide, sodium hydroxide, potassium hydroxide, sodium carbonate, sodium bicarbonate, potassium carbonate, potassium bicarbonate; a metal hydride such as sodium hydride, lithium hydride, potassium hydride, calcium hydride; an organometallic base such as butyl lithium, phenyl lithium, sodium ethoxide, sodium methoxide, sodium *tert*-butoxide, potassium *tert*-butoxide, lithium amide, lithium (di-isopropyl)amide; an organic base such as triethylamine, pyridine, di-isopropylethylamine. The solvent includes aromatic hydrocarbons such as benzene, toluene; halogenated hydrocarbons such as dichloromethane, chloroform, 1, 2-dichloroethane; amides such as dimethylformamide, dimethylacetamide; ethers such as tetrahydrofuran, ether, 1, 4-dioxane, 1, 2-dimethoxyethane; basic solvents such as pyridine; and mixtures thereof.
   The starting compounds of formula (5) are commercially available, or may be synthesized according to the procedures described in a literature, for example, Japanese Patent Publication (kokai) No. 26816/1976, Tetrahedron Letters, 34, 2839, (1993).
(C) The compounds of formula (1a) may also be obtained by reacting compound (4) with one to five equivalents of compound (6) in an inert solvent under cooling, at room temperature or under heating in the presence of a base and one to five equivalents of compound (7), as shown in the following scheme. 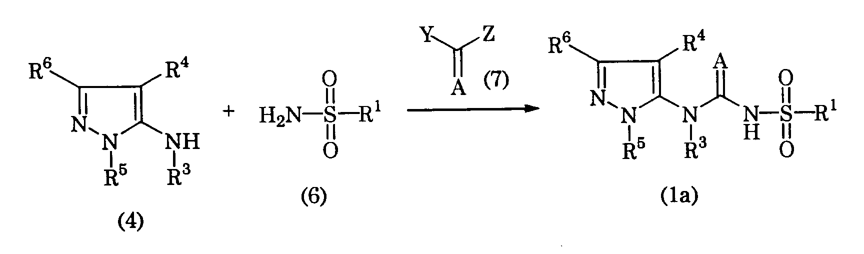 wherein A, R¹, R³, R⁴, R⁵, and R⁶ are as defined above, Y and Z are a leaving group which may be easily replaced via nucleophilic reaction.
   The base and the solvent as used in the reaction are similar to those as described in the above item (B).
(D) Alternatively, the compounds of formula (1a) may also be obtained by reacting compound (4) with one to five equivalents of compound (7) in an inert solvent under cooling, at room temperature or under heating in the presence of an appropriate base, to yield compound (8), which is then reacted with one to five equivalents of compound (6) in an inert solvent under cooling, at room temperature or under heating in the presence of an appropriate base, as shown in the following scheme. 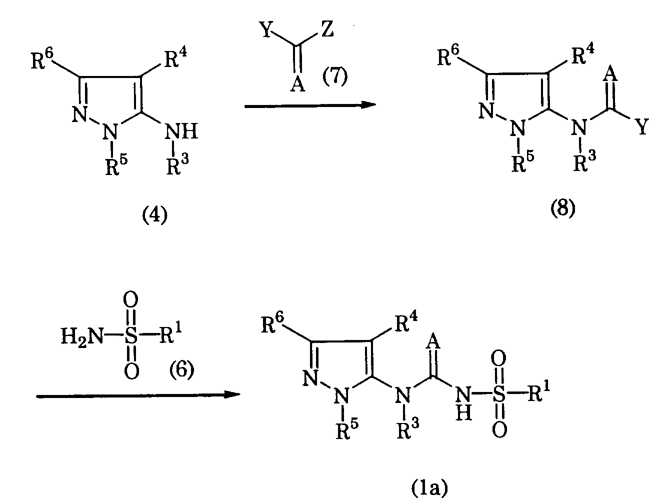 wherein A, R¹, R³, R⁴, R⁵, R⁶, Y and Z are as defined above.
   The base and the solvent as used in the reaction are similar to those as described in the above item (B).
(E) Alternatively, the compounds of formula (1a) may also be obtained by reacting compound (6) with one to five equivalents of compound (7) in an inert solvent under cooling, at room temperature or under heating in the presence of an appropriate base, to yield compound (9), which can then be reacted with one to five equivalents of compound (4) in an inert solvent under cooling, at room temperature or under heating in the presence of an appropriate base, as shown in the following scheme. 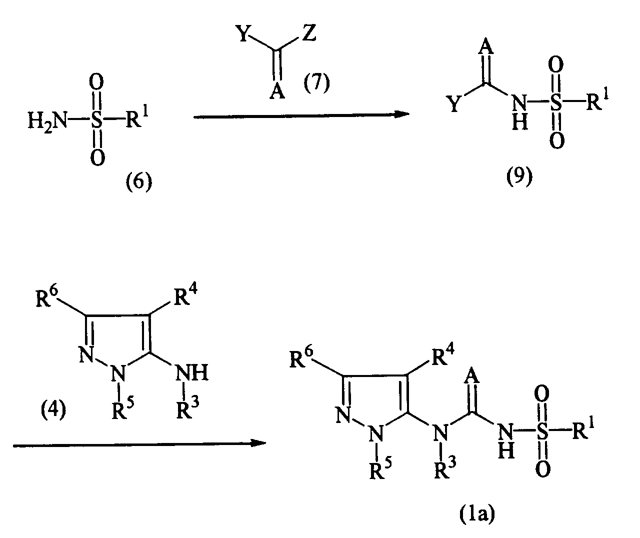 wherein A, R¹, R³, R⁴, R⁵, R⁶, Y and Z are as defined above.
   The base and the solvent as used in the reaction are similar to those as described in the above item (B).
   The leaving groups Y and Z on compounds (7), (8), and (9) are the same or different, and are independently exemplified by a lower alkoxy group, an aralkyloxy group, an aryloxy group, a substituted aryloxy group, 1-imidazolyl group, a trihalomethyl group such as trifluoromethyl, trichloromethyl, tribromomethyl, triiodomethyl, a halogen atom, and the like.
(F) Starting compounds (4) may be prepared according to the procedure as shown in the following scheme. 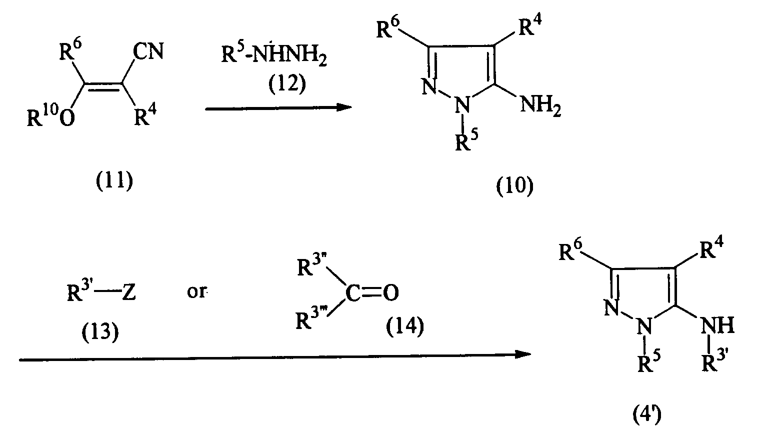 wherein R⁴, R⁵, R⁶, and Z are as defined above, R³' has the same meaning as R³ provided that R³' does not denote a hydrogen atom R³'' and R³''' are each a group that forms -CH(R³'')R³''', which has the same meaning as R³ in which R³ is limited to a group having a hydrogen atom at its α position, and R¹⁰ is an alkyl group.
   The intermediates of formula (10) are commercially available, or may be obtained by reacting compound (11) with one to five equivalents of compound (12) in a conventional solvent under cooling, at room temperature or under heating in the presence of an acid or a base. Intermediate (10) may also be synthesized according to the procedures described in a literature, for example, J. Org. Chem., 21, 1240, (1956), Japanese Patent Publication (kokai) No. 195376/1987, Aust. J. Chem., 42, 747, (1989), J. Med. Chem., 3263, 35, (1992), Chemical Abstract 56, 1459, (1962), U.S. Patent No. 4622330, Japanese Patent Publication (kokai) No. 115581/1985, J. Med. Chem. 34, 2892, (1991), Japanese Patent Publication (kohyo) No. 503069/1994, J. Am. Chem. Soc., 81, 2456, (1959), Chemical abstract. 79, 146518, Heterocycles. 26, 613, (1987), and J. Org. Chem. 58, 6155, (1993).
   Starting compounds (4') can be obtained by reaction of compound (10) with one to five equivalents of compound (13) in a conventional solvent under cooling, at room temperature or under heating in the presence of an appropriate base, or by reductive N-alkylation of one to five equivalents of compound (10) with compound (14).
   In the reaction between compounds (10) and (13), the base includes an inorganic base such as lithium hydroxide, sodium hydroxide, potassium hydroxide, sodium carbonate, sodium bicarbonate, potassium carbonate, potassium bicarbonate; a metal hydride such as sodium hydride, lithium hydride, potassium hydride, calcium hydride; an organometallic base such as butyl lithium, phenyl lithium, sodium ethoxide, sodium methoxide, sodium *tert*-butoxide, potassium *tert*-butoxide, lithium amide, lithium (di-isopropyl)amide; an organic base such as triethylamine, pyridine, di-isopropylethylamine. The solvent includes aromatic hydrocarbons such as benzene, toluene; halogenated hydrocarbons such as dichloromethane, chloroform, 1, 2-dichloroethane; amides such as dimethylformamide, dimethylacetamide; ethers such as tetrahydrofuran, ether, 1, 4-dioxane, 1, 2-dimethoxyethane; basic solvents such as pyridine; and mixtures thereof.
   Reductive N-alkylation of compound (10) with compound (14) may be accomplished by reaction of compounds (10) and (14) in a conventional solvent under cooling, at room temperature or under heating in the presence of sodium cyanoborohydride and an appropriate acid, or by catalytic reduction of compound (10) with compound (14) in a hydrogen atmosphere in the presence of an appropriate acid and catalyst.
(G) Compounds (2b) of the present invention may be synthesized by reacting compound (2a) with one to five equivalents of compound (3) in a conventional solvent under cooling, at room temperature or under heating in the presence of an appropriate base, as shown in the following scheme. 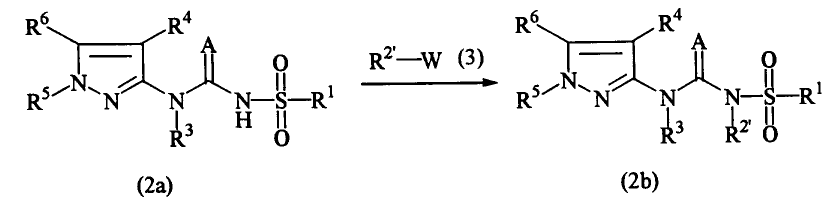 wherein A, R¹, R²', R³, R⁴, R⁵, R⁶, and W are as defined above.
   The base and the solvent as used in the reaction are similar to those as described in the above item (A).
   The compounds of formula (2a) of the present invention may be prepared according to the procedures, for example, as illustrated in any of the following schemes (H) to (K).
(H) Compounds (2a) of the present invention may be obtained by reacting compound (54) with one to five equivalents of compound (5) in a conventional solvent under cooling, at room temperature or under heating in the presence or absence of a base, as shown in the following scheme. 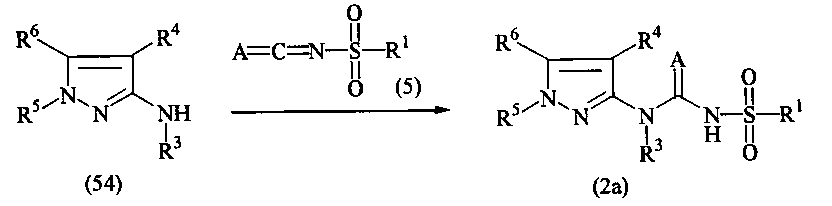 wherein A, R¹, R³, R⁴, R⁵, and R⁶ are as defined above.
   The base and the solvent as used in the reaction are similar to those as described in the above item (B).
(I) Alternatively, compounds (2a) of the present invention may also be obtained by reacting compound (54) with one to five equivalents of compound (6) in an inert solvent under cooling, at room temperature or under heating in the presence of a base and compound (7), as shown in the following scheme. 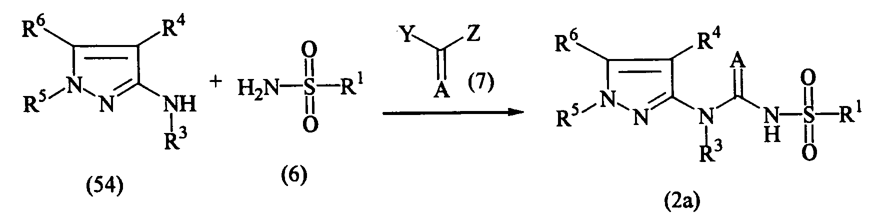 wherein Y, Z, A, R¹, R³, R⁴, R⁵, and R⁶ are as defined above.
   The base and the solvent as used in the reaction are similar to those as described in the above item (B).
(J) Alternatively, compounds (2a) of the present invention may also be obtained by reacting compound (18) with one to five equivalents of compound (7) in an inert solvent under cooling, at room temperature or under heating in the presence of an appropriate base, to yield compound (19), which is then reacted with one to five equivalents of compound (6) in an inert solvent under cooling , at room temperature or under heating in the presence of an appropriate base, as shown in the following scheme. 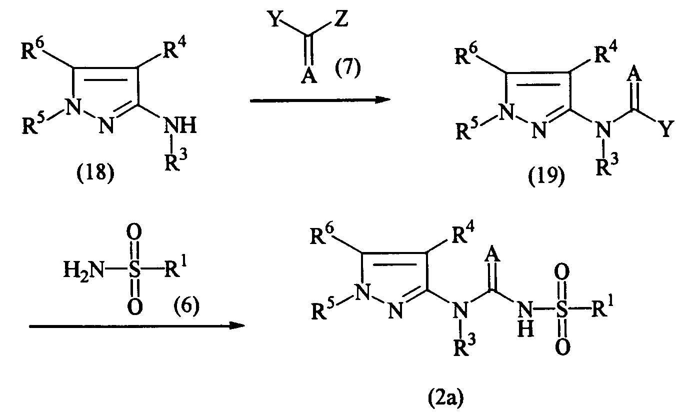 wherein Y, Z, A, R¹, R³, R⁴, R⁵, and R⁶ are as defined above.
   The base and the solvent as used in the reaction are similar to those as described in the above item (B).
(K) Alternatively, compounds (2a) of the present invention may also be obtained by reacting compound (6) with one to five equivalents of compound (7) in an inert solvent under cooling, at room temperature or under heating in the presence of an appropriate base, to yield compound (9), which is then reacted with compound (18) in an inert solvent under cooling , at room temperature or under heating in the presence of an appropriate base, as shown in the following scheme. 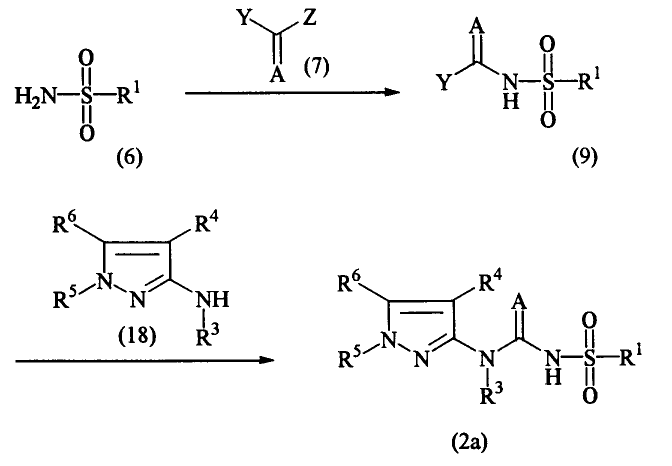 wherein Y, Z, A, R¹, R³, R⁴, R⁵, and R⁶ are as defined above.
   The base and the solvent as used in the reaction are similar to those as described in the above item (B).
(L) Compounds of formula (18) can be obtained according to the procedure as illustrated in the following scheme. 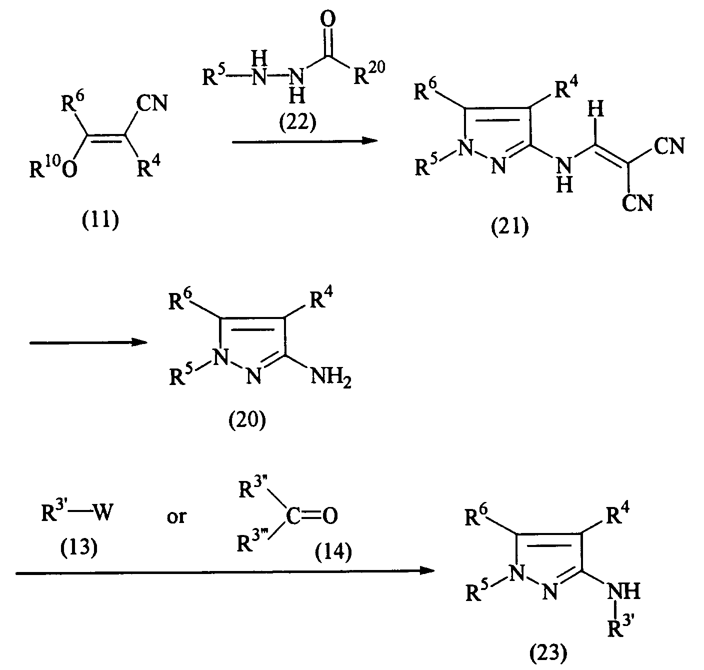 wherein R³', R³'', R³''', R⁴, R⁵, R⁶, R¹⁰, and W are as defined above, and R²⁰ is an alkyl group.
   The intermediates of formula (20) are commercially available, or may be obtained by reacting compound (11) with one to five equivalents of compound (22) in a conventional solvent under cooling, at room temperature or under heating in the presence of an acid or a base, to yield compound (21), which is then hydrolyzed in a conventional solvent in the presence of an acid or a base, as shown in the above scheme. The intermediates (20) may also be synthesized according to the procedure described previously, for example, Aust. J. Chem., 44, 1795, (1991).
   Starting compounds (23) can be obtained by reaction of compound (20) with one to five equivalents of compound (13) in a conventional solvent under cooling, at room temperature or under heating in the presence of an appropriate base, or by reductive N-alkylation of one to five equivalents of compound (20) with compound (14).
   In the reaction between compounds (13) and (20), the base includes an inorganic base such as lithium hydroxide, sodium hydroxide, potassium hydroxide, sodium carbonate, sodium bicarbonate, potassium carbonate, potassium bicarbonate; a metal hydride such as sodium hydride, lithium hydride, potassium hydride, calcium hydride; an organometallic base such as butyl lithium, phenyl lithium, sodium ethoxide, sodium methoxide, sodium *tert*-butoxide, potassium *tert*-butoxide, lithium amide, lithium (di-isopropyl)amide; an organic base such as triethylamine, pyridine, di-isopropylethylamine. The solvent includes aromatic hydrocarbons such as benzene, toluene; halogenated hydrocarbons such as dichloromethane, chloroform, 1, 2-dichloroethane; amides such as dimethylformamide, dimethylacetamide; ethers such as tetrahydrofuran, ether, 1, 4-dioxane, 1, 2-dimethoxyethane; basic solvents such as pyridine; and mixtures thereof.
   Reductive N-alkylation of compound (20) with compound (14) may be accomplished by reaction of compounds (14) and (20) in a conventional solvent under cooling, at room temperature or under heating in the presence of sodium cyanoborohydride and an appropriate acid, or by catalytic reduction of compound (20) with compound (14) in a hydrogen atmosphere in the presence of an appropriate acid and catalyst.

During the reactions (A) to (L) as mentioned above, compounds which have a reactive group such as hydroxyl, carboxyl, amino, thiol, and the like may be previously protected by an appropriate protecting group, so that after performing the reactions, removal of such protecting group yields a desired compound.

Such protecting group as used herein may be a protecting group conventionally used in the field of organic chemistry, and incorporation and removal of such protecting group can be performed according to the conventional procedures. See, for example, Protective Groups in Organic Synthesis, JOHN WILLEY & SONS, 1991.

Protecting groups for the hydroxyl group include methoxymethyl group, tetrahydropyranyl group, benzyl group, acetyl group, benzoyl group, benzyl group, 4-methoxybenzyl group, and the like. Protecting groups for the carboxyl group include methyl group, ethyl group, propyl group, n-butyl group, iso-butyl group, *tert*-butyl group, benzyl group, and the like. Protecting groups for the amino group include *tert*-butyloxycarbonyl group, benzyloxycarbonyl, group, acetyl group, benzoyl group, benzyl group, and the like. Protecting groups for the thiol group include benzyl group, diphenylmethyl group, methoxymethyl group, acetyl group, benzoyl group, *tert*-butoxycarbonyl group, benzyloxycarbonyl group, and the like.

The intermediates and the final compounds in the processes as shown above may be isolated and purified by purification procedures commonly used in organic chemistry, including, for example, filtration, extraction, washing, drying, concentration, recrystallization, various chromatography. The intermediates may also be used in the next step without further purification. If desired, a salt of compound (1) or (2) may be formed by dissolving or suspending such compound in an appropriate solvent, and by adding an acid or a base thereto.

Compound (1) or (2) and a pharmaceutically acceptable salt can exist in the form of additives such as hydrate or solvate, which additives are also included in the scope of the present invention.

The compounds of formula (1) or (2) prepared as shown above include those listed in the following tables, as well as those prepared in the following preparations and examples.

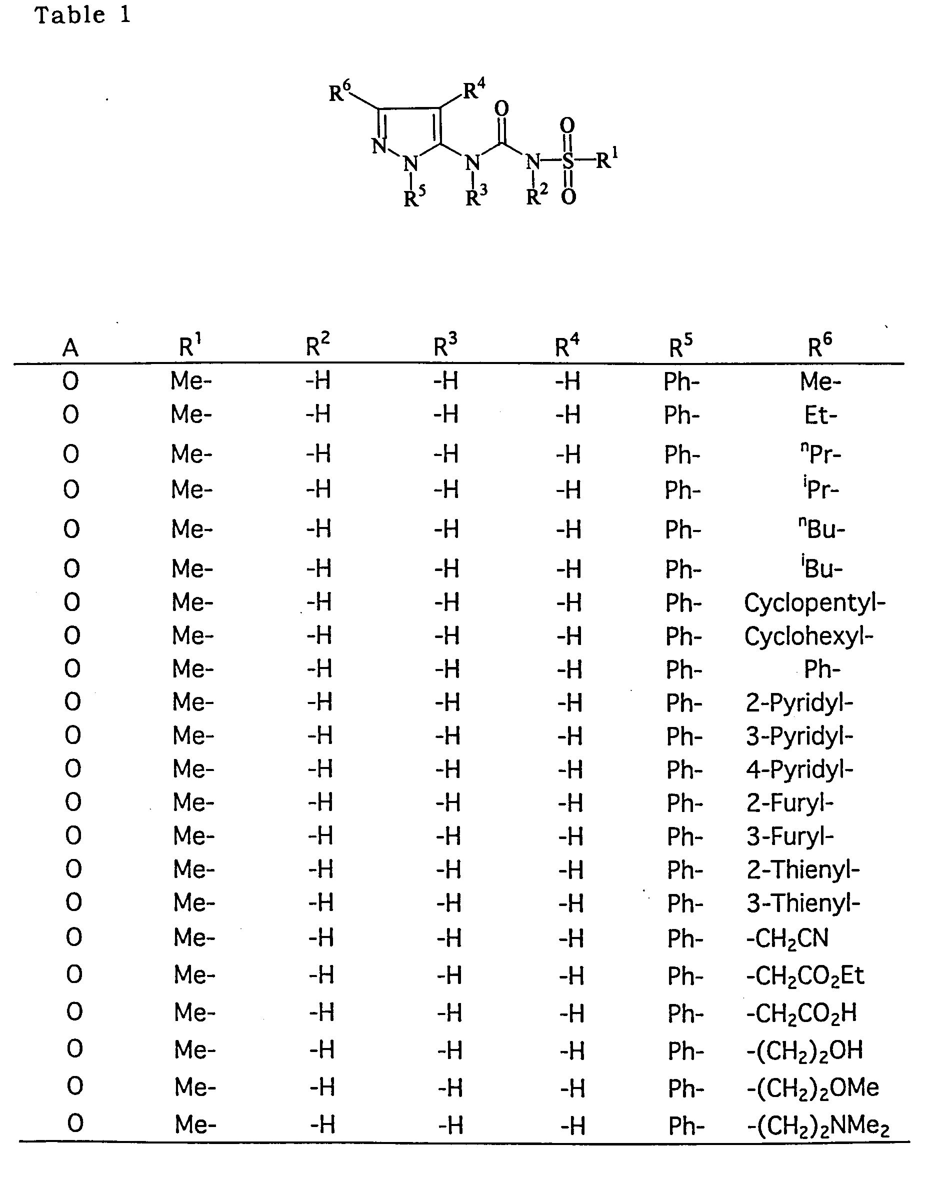

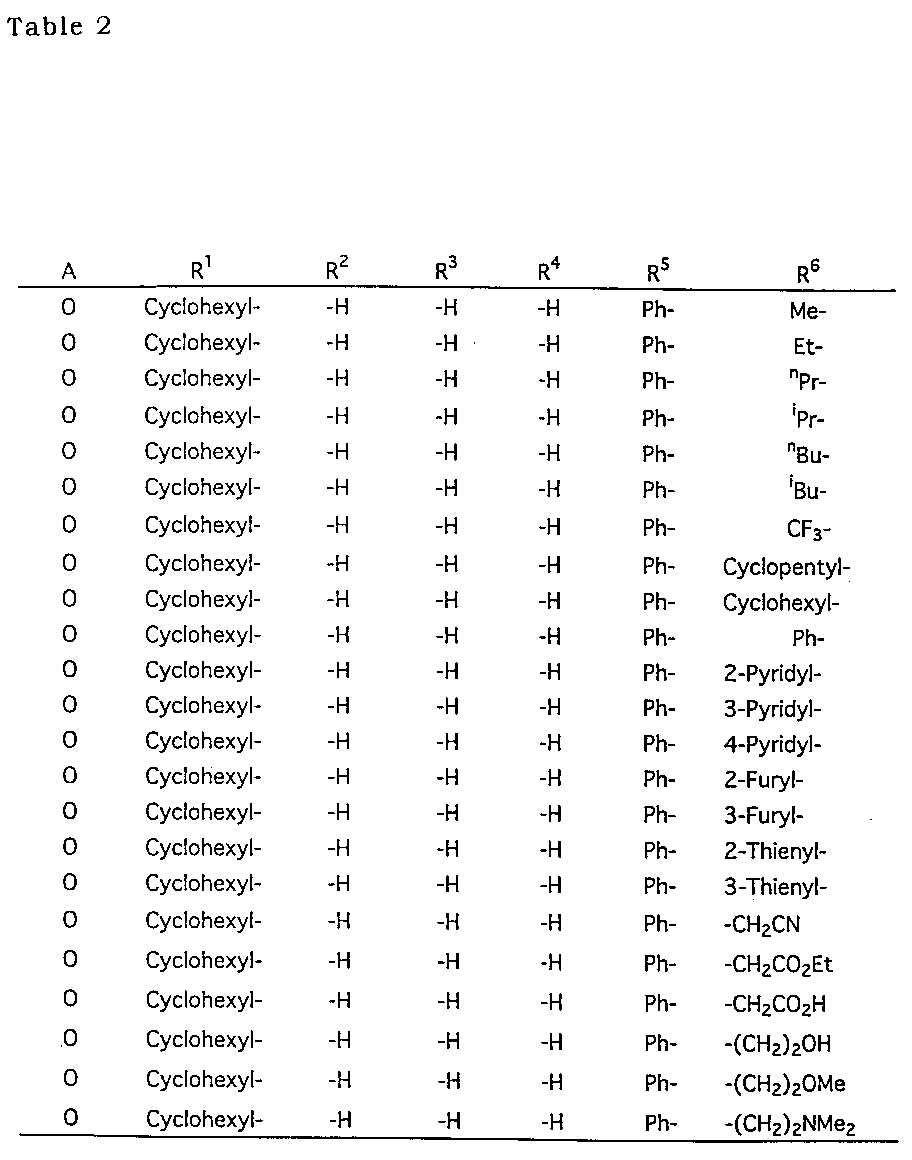

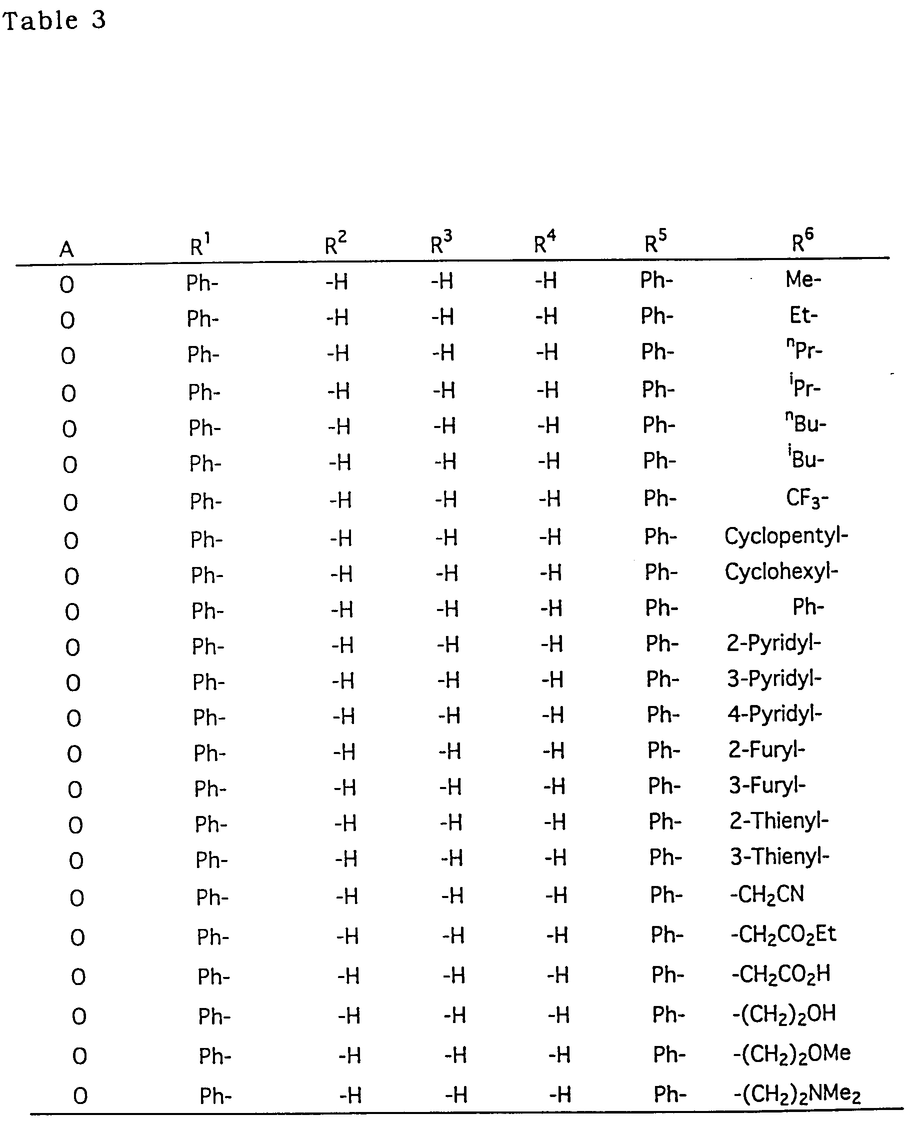

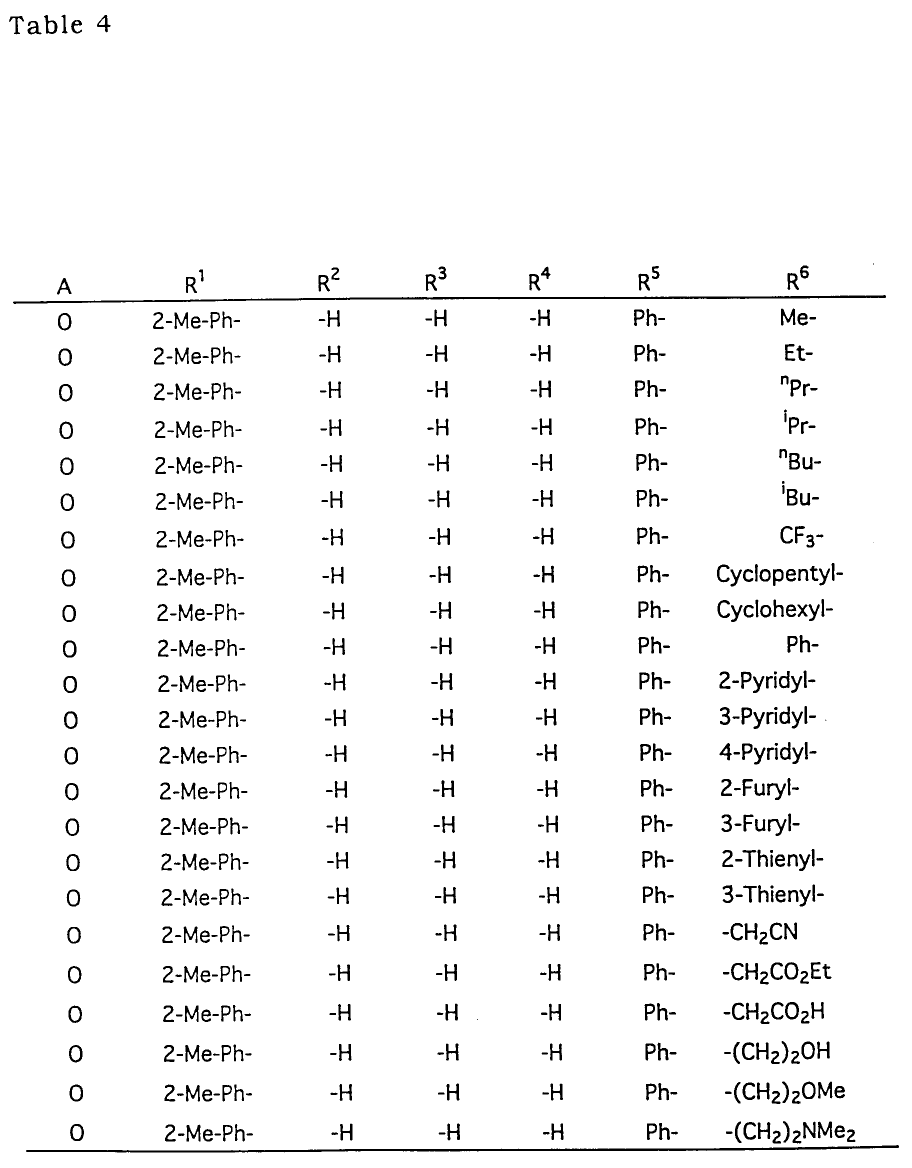

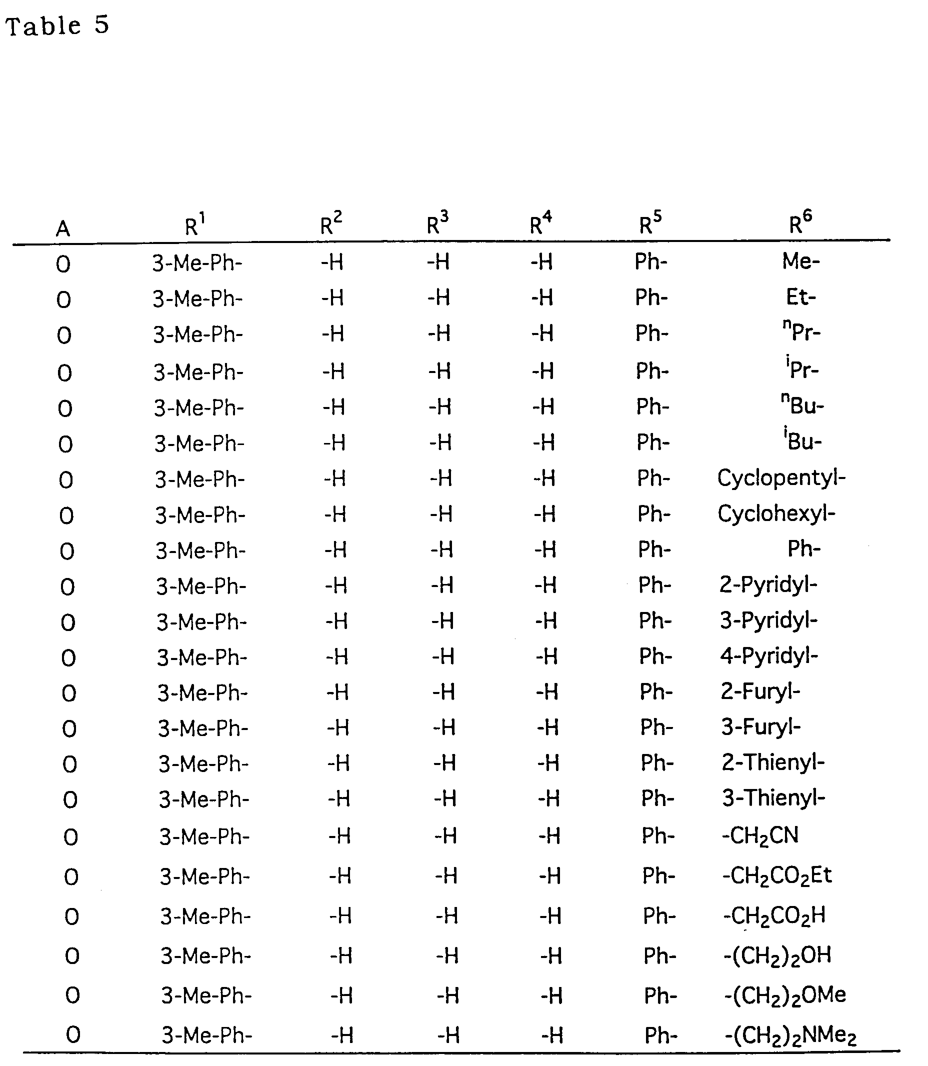

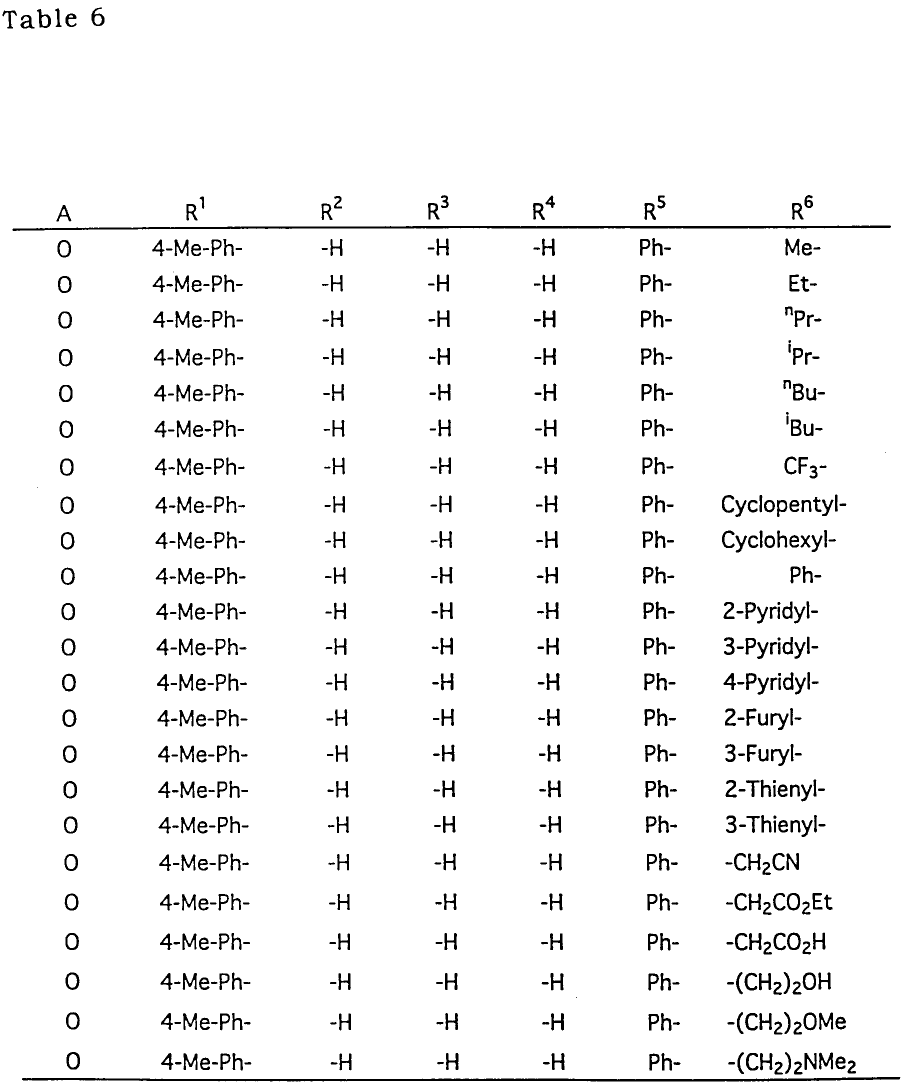

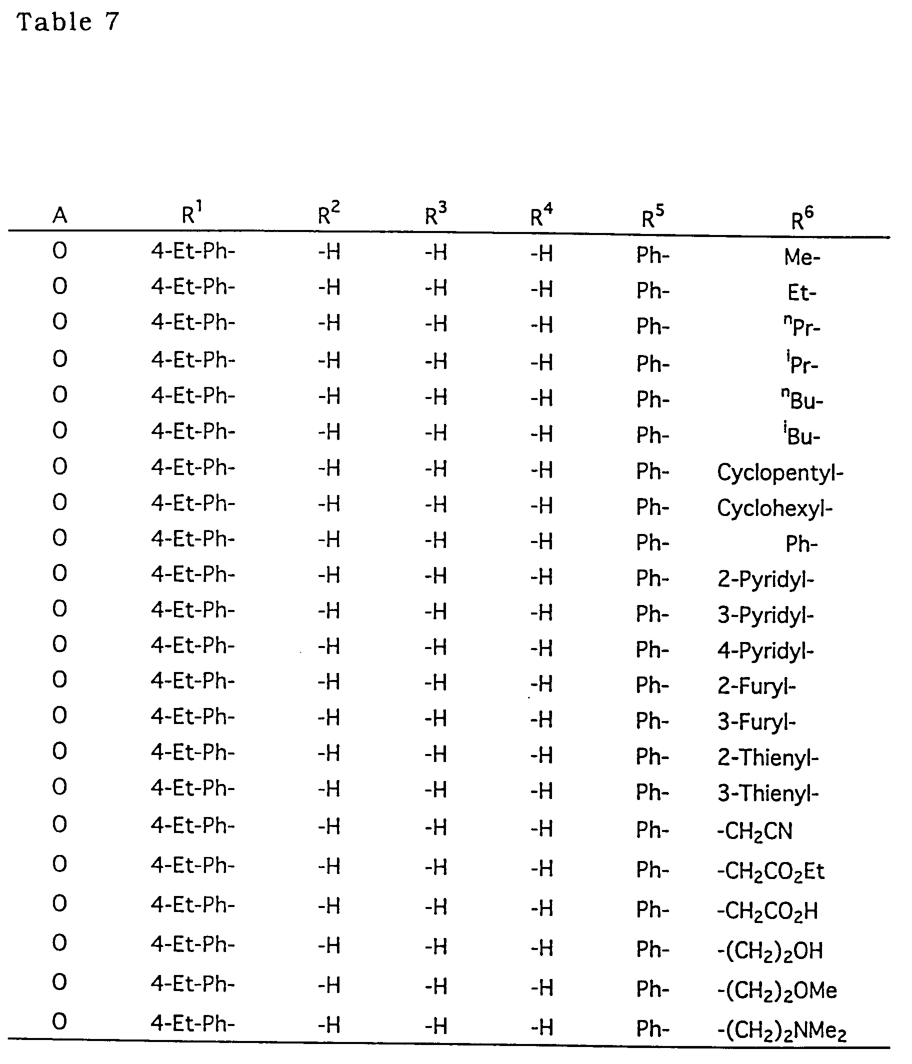

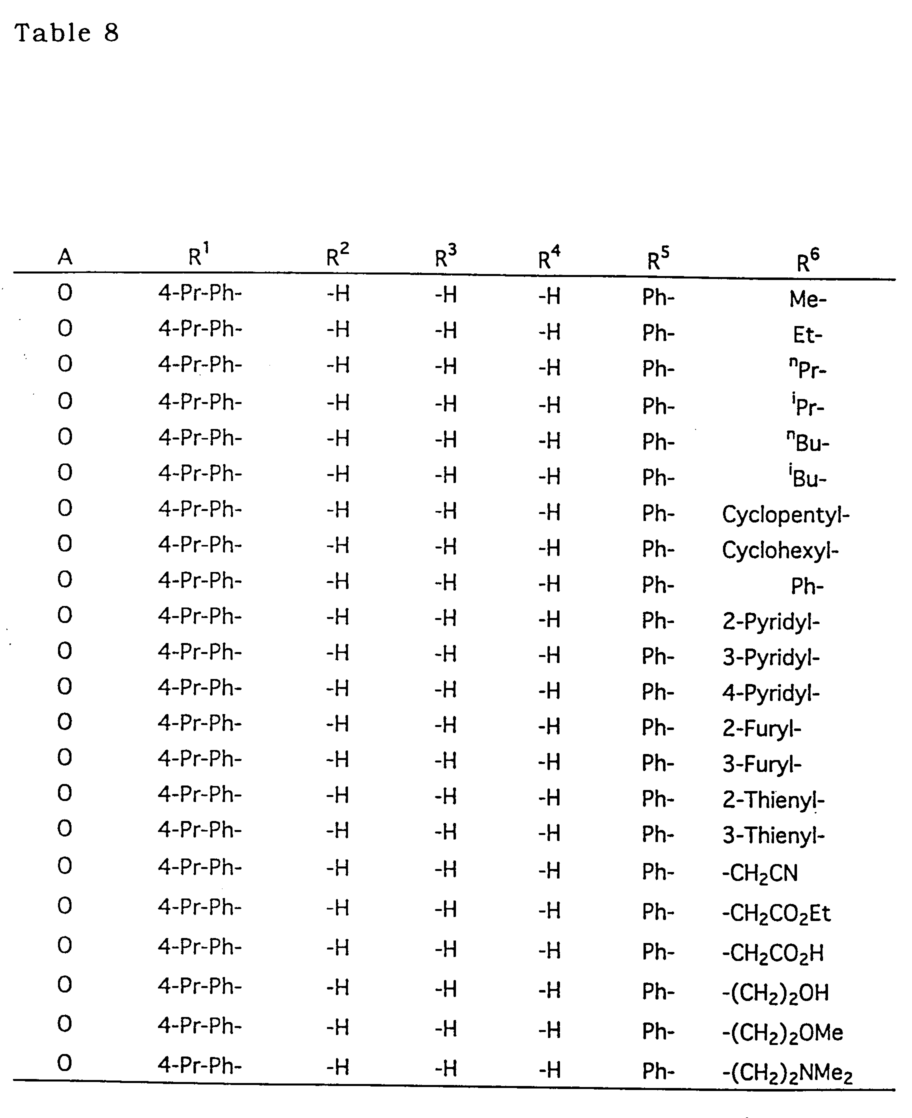

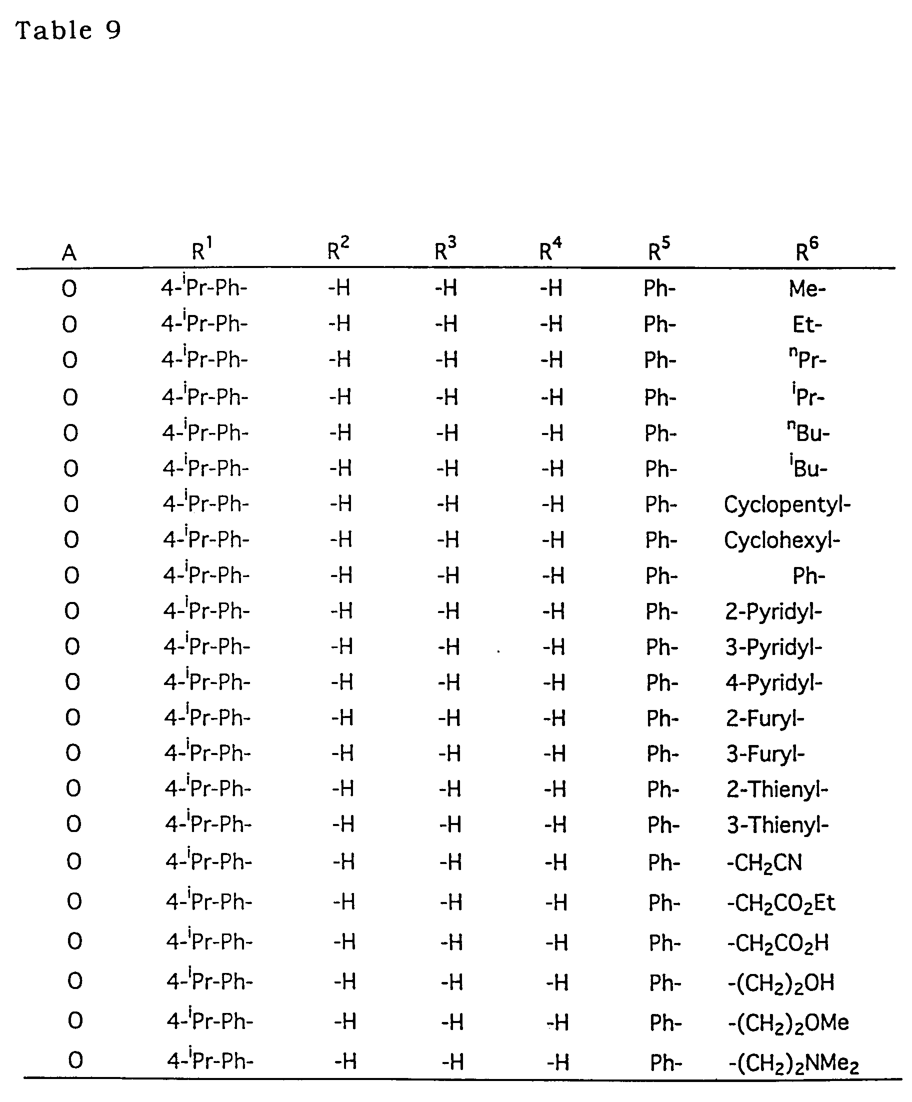

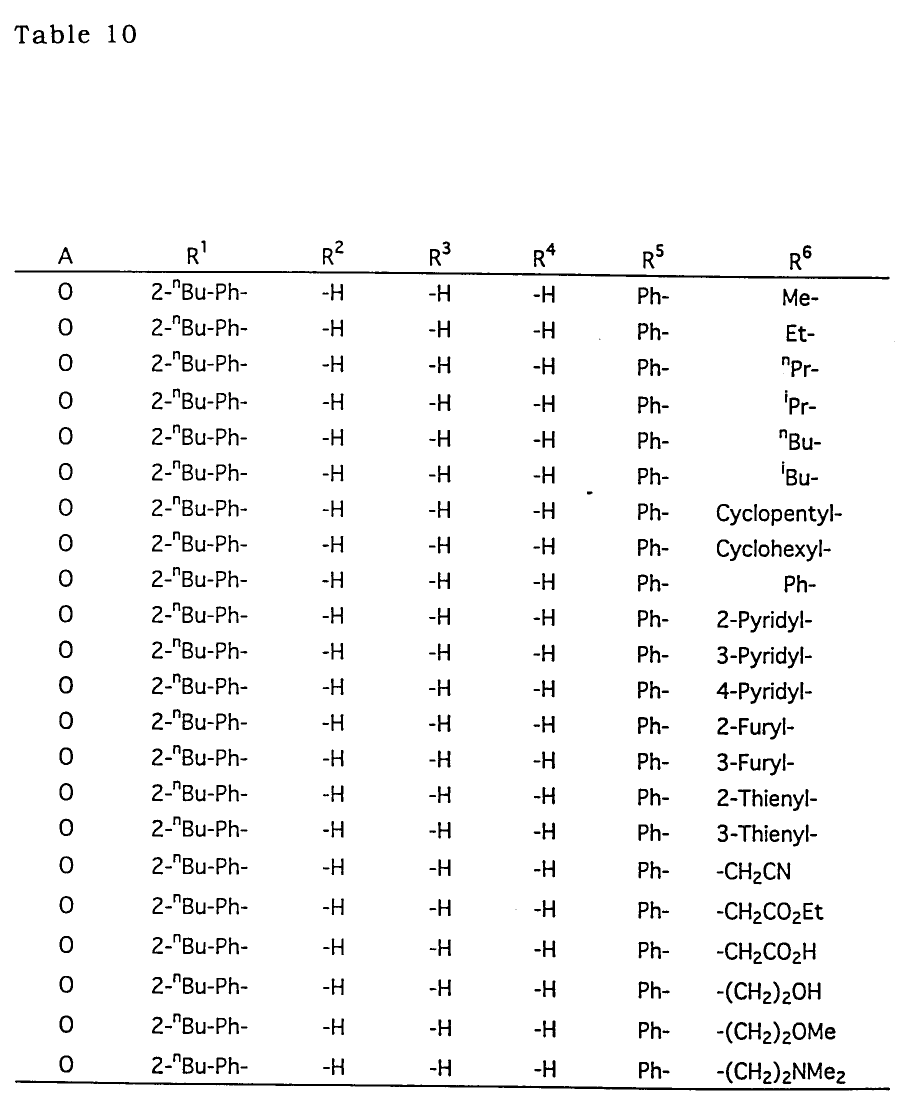

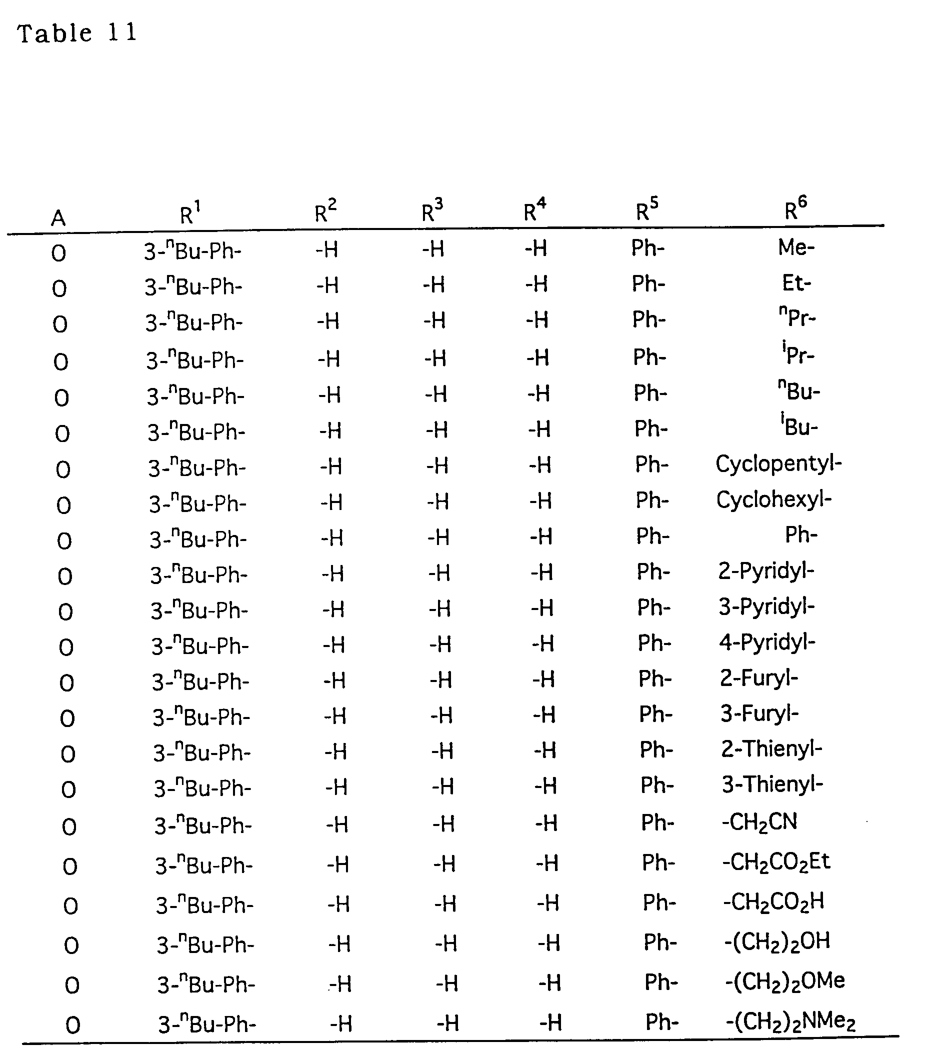

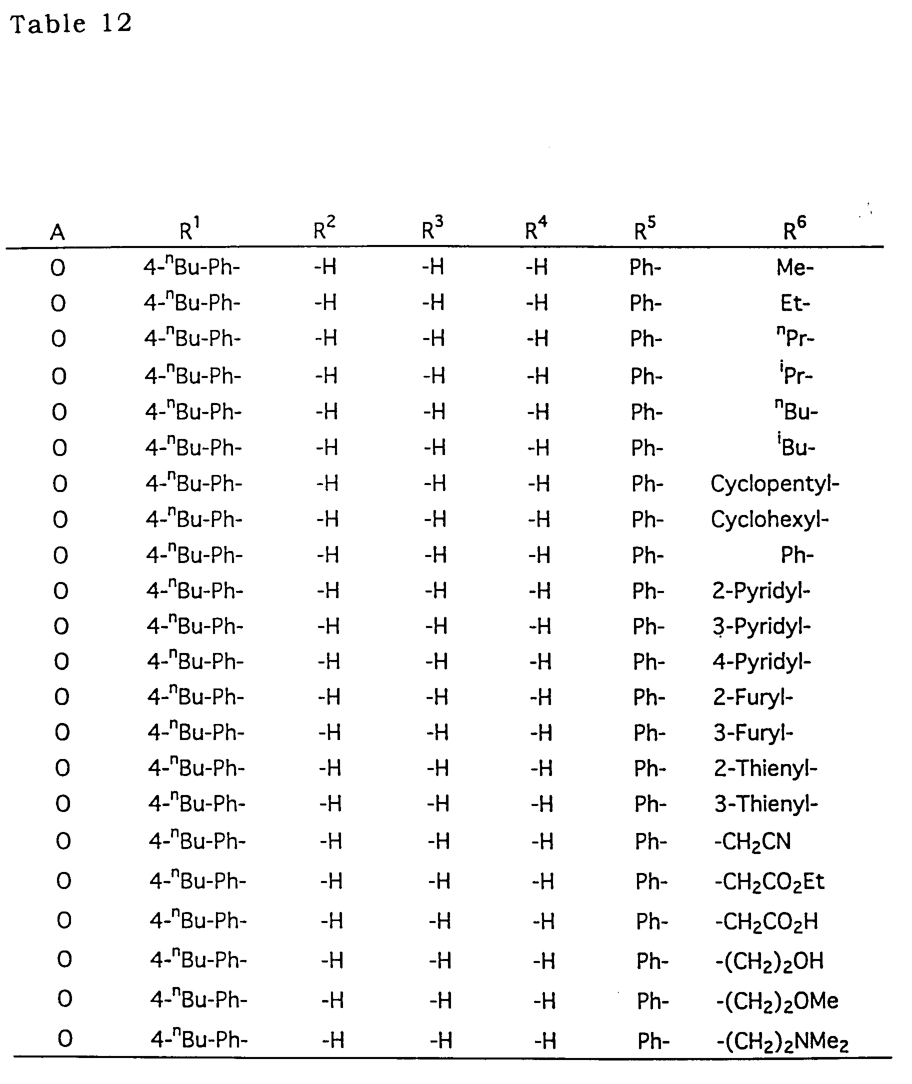

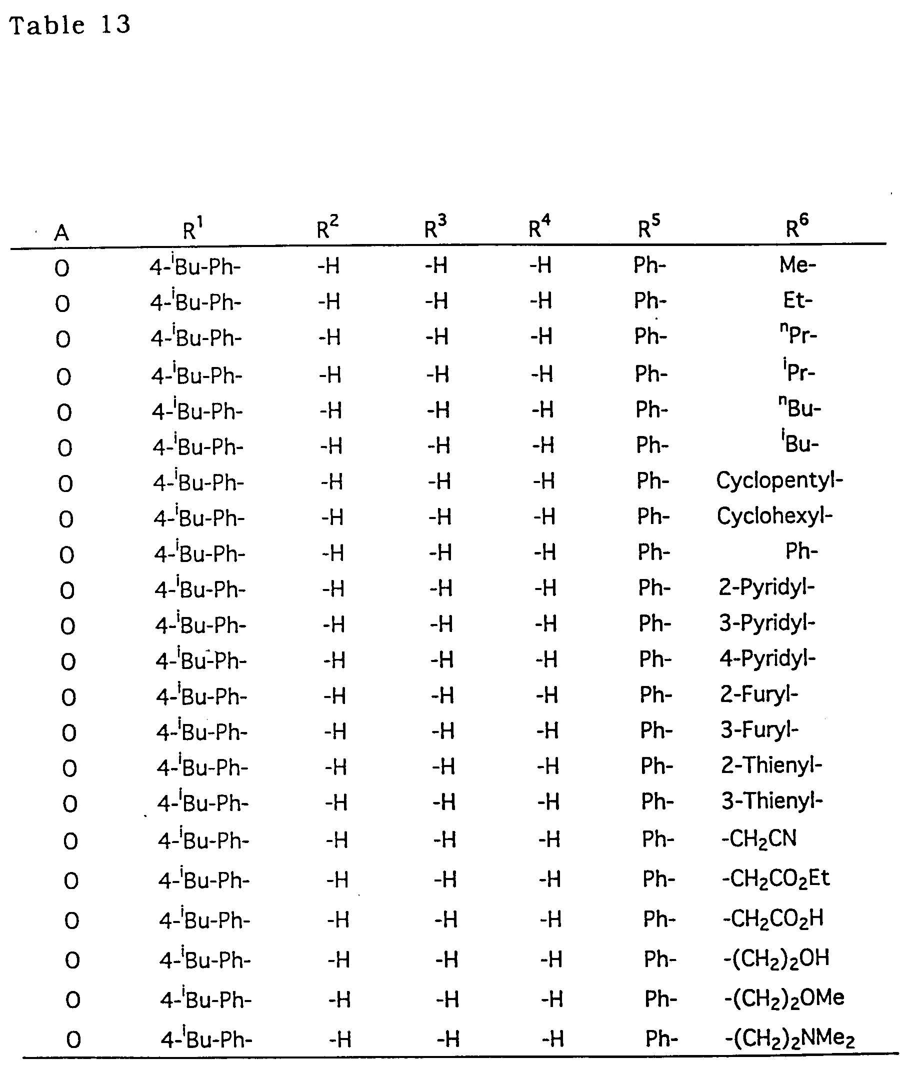

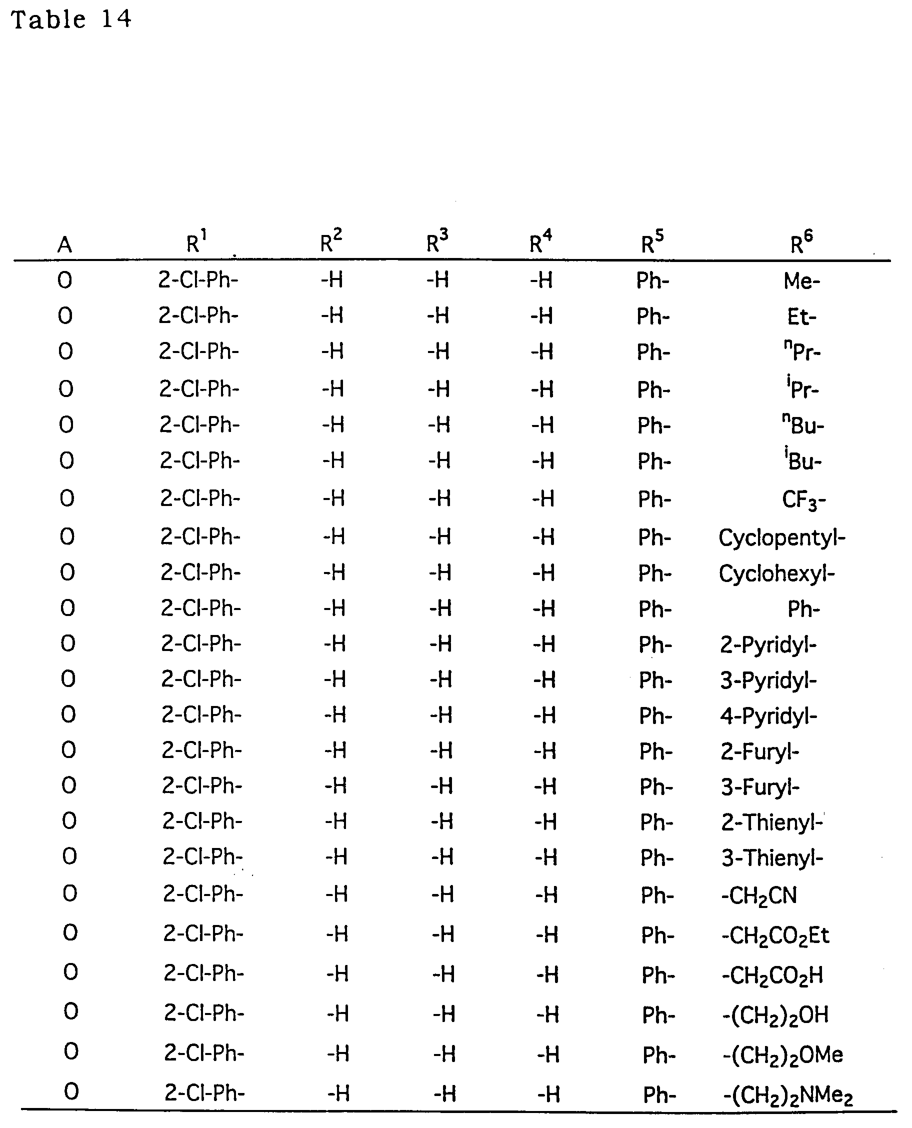

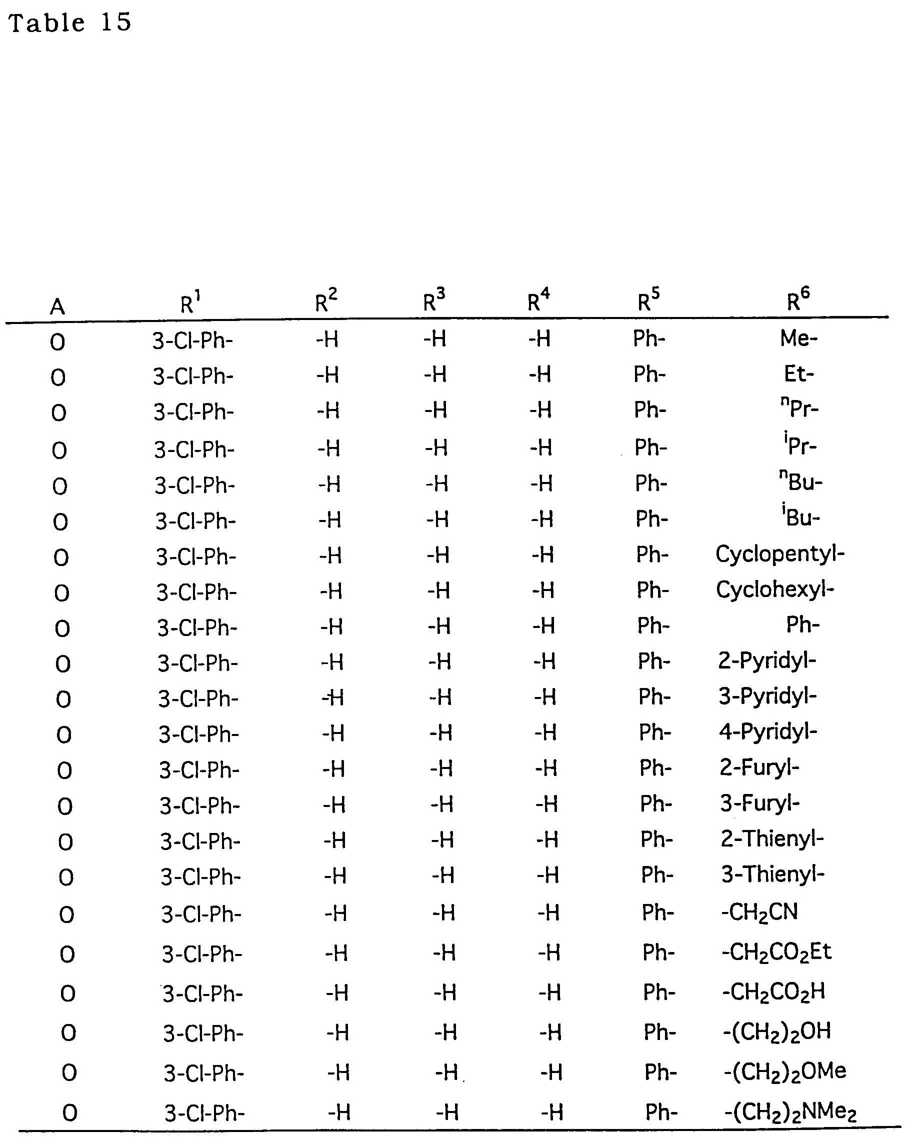

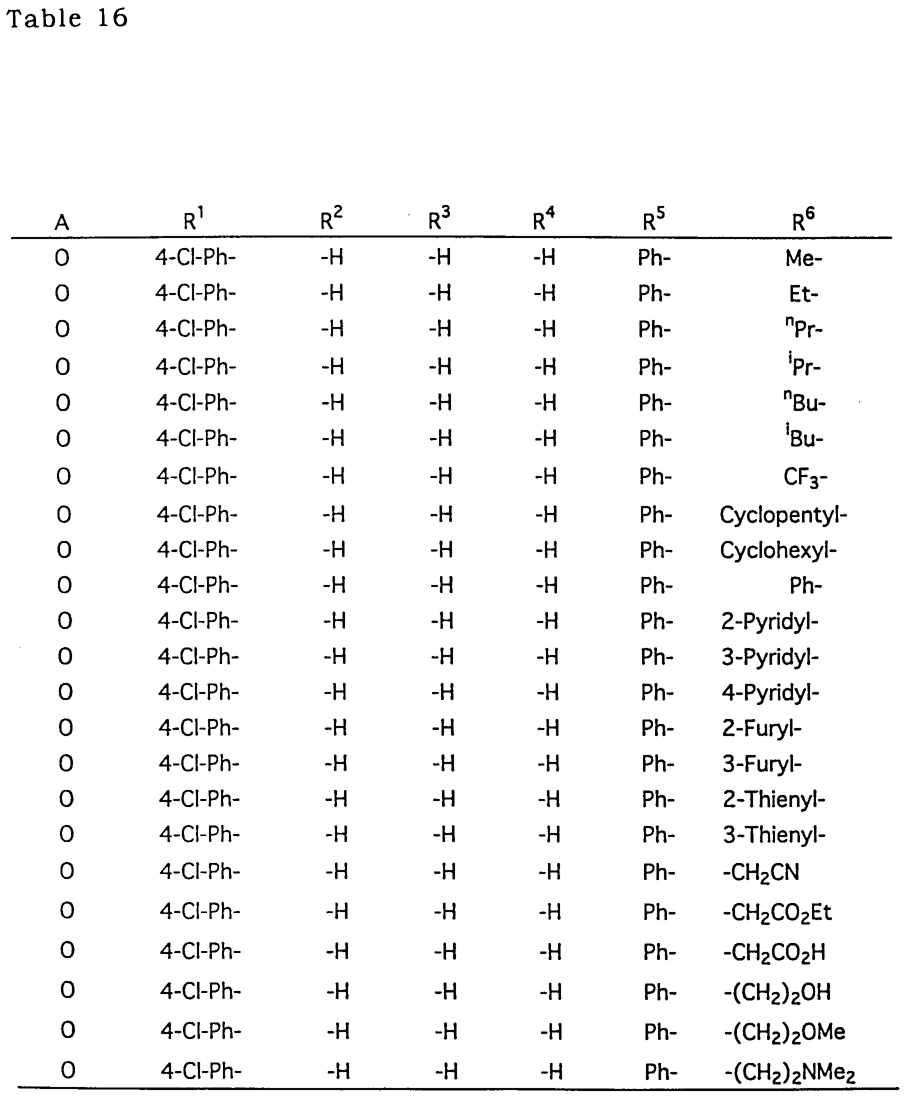

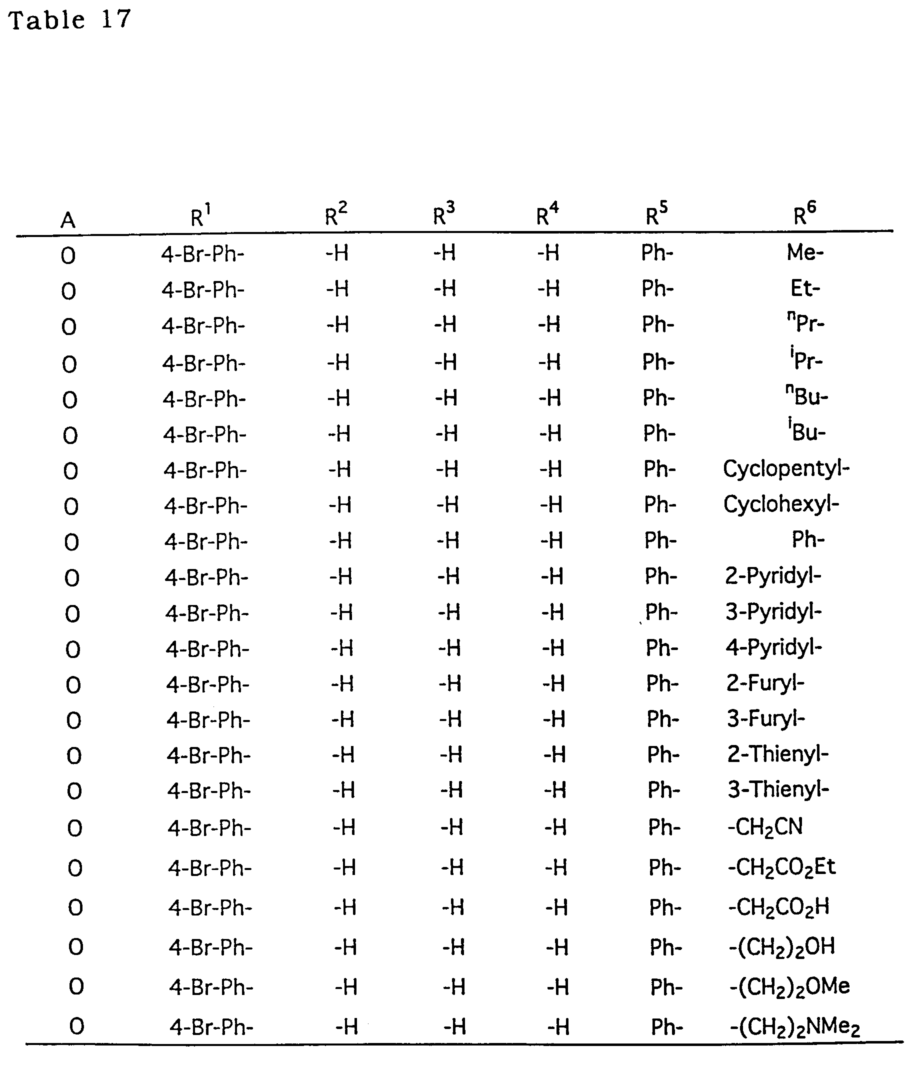

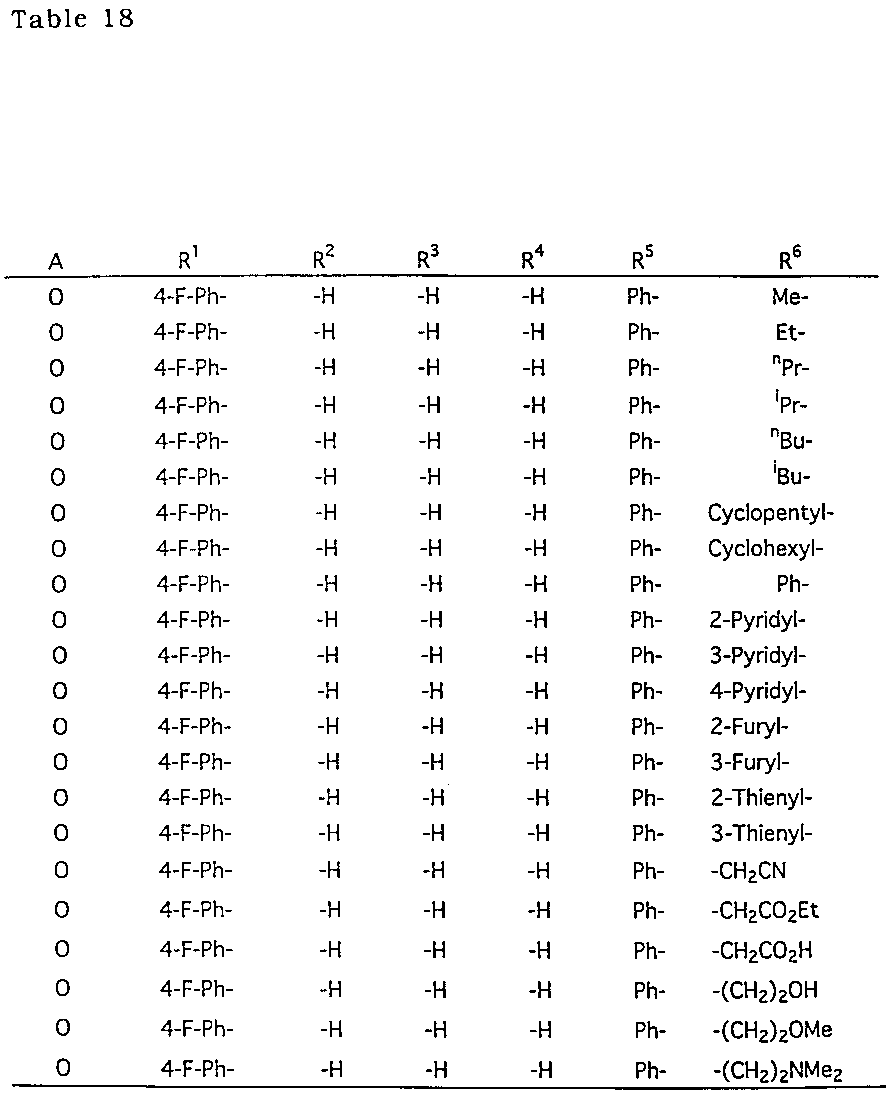

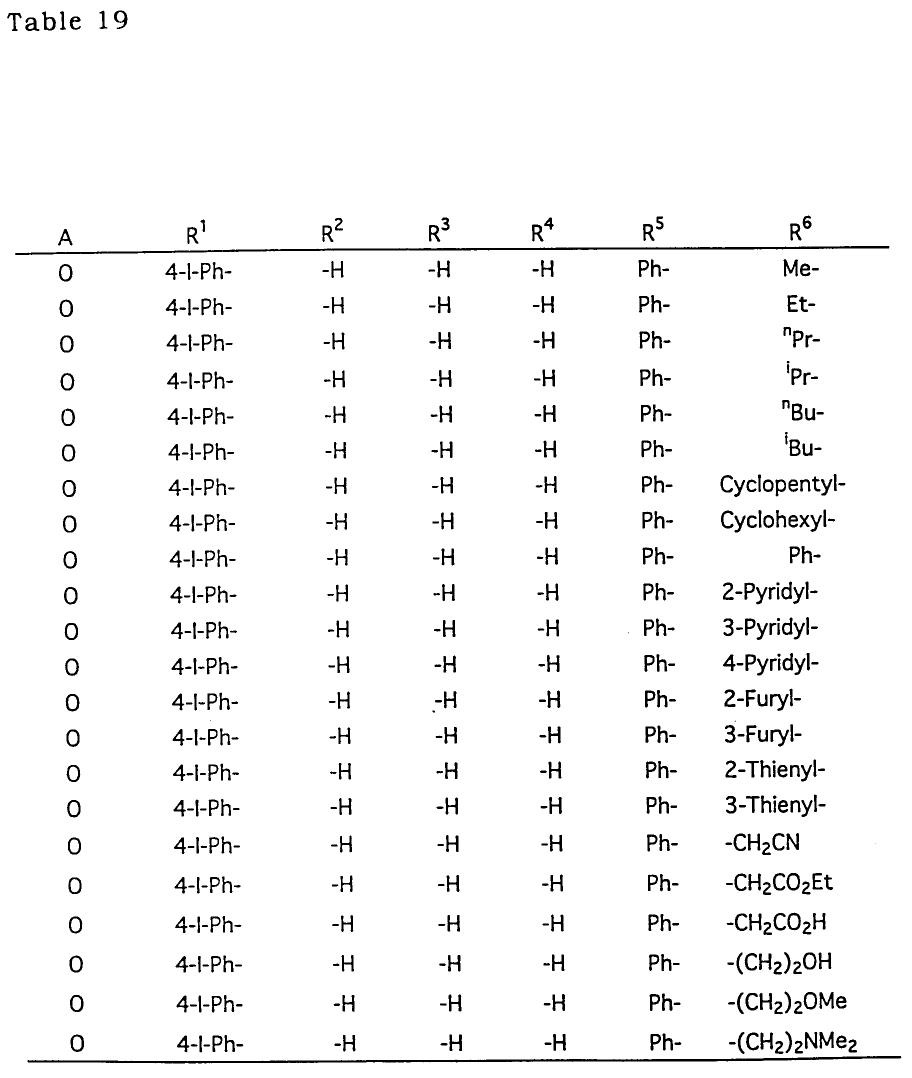

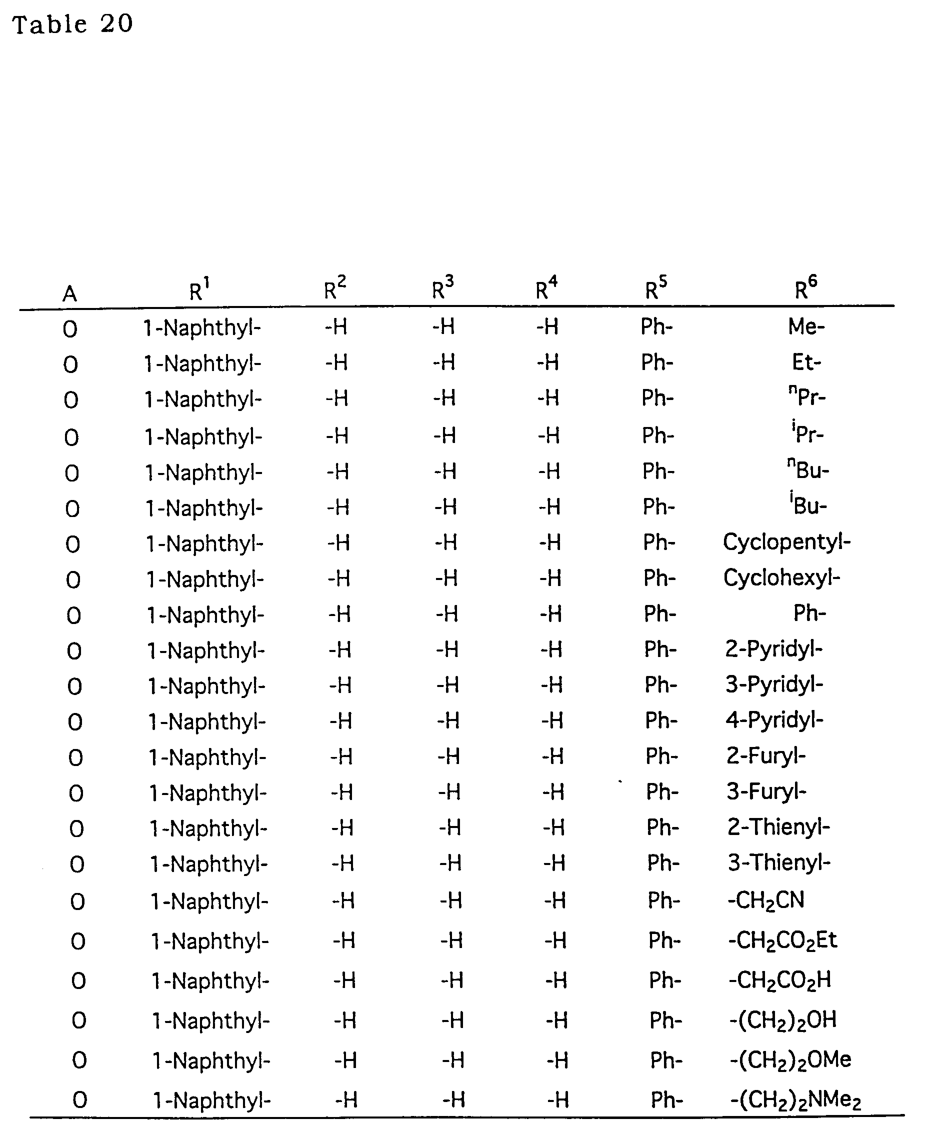

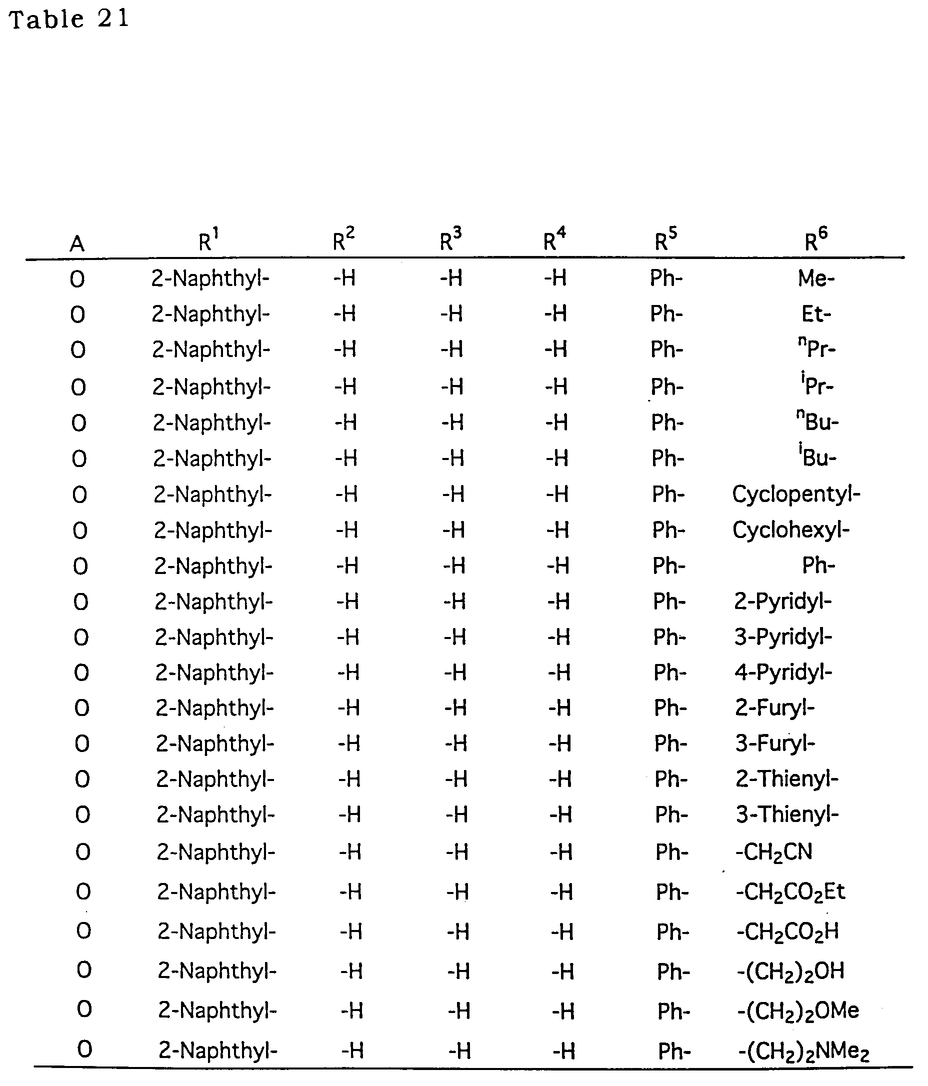

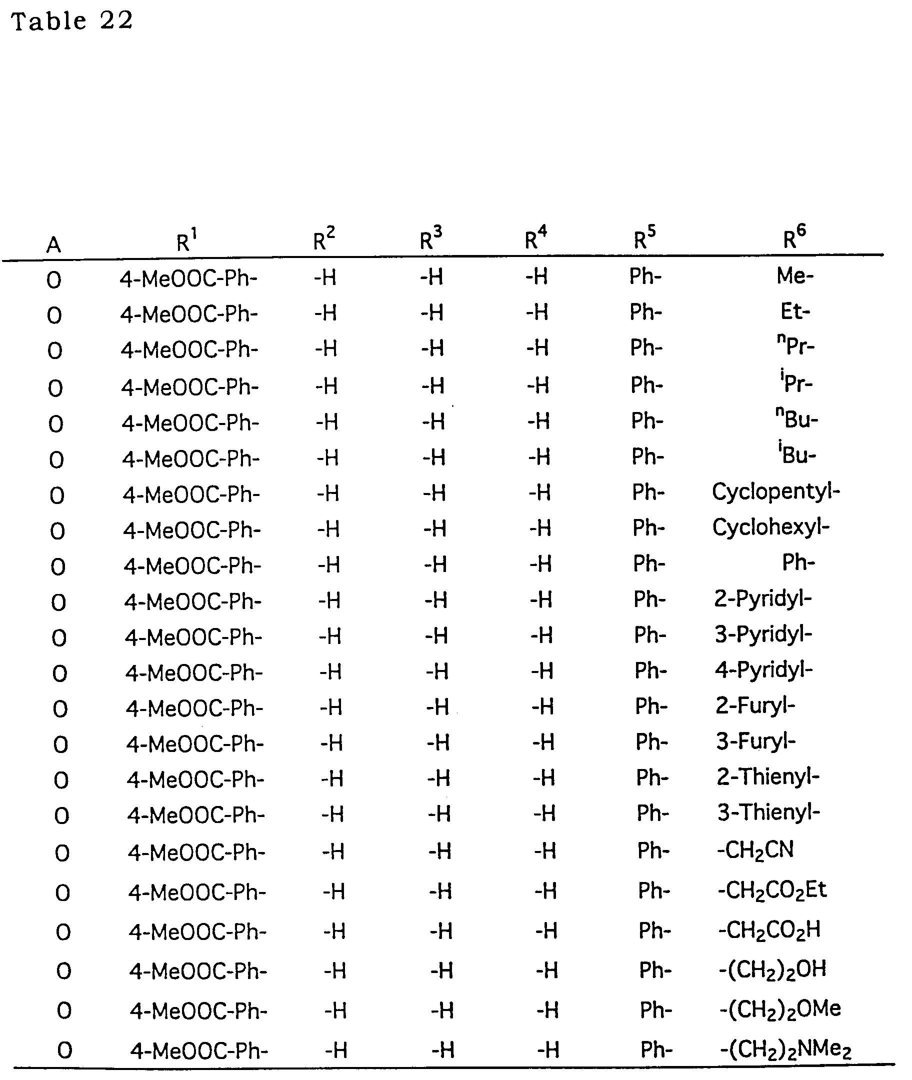

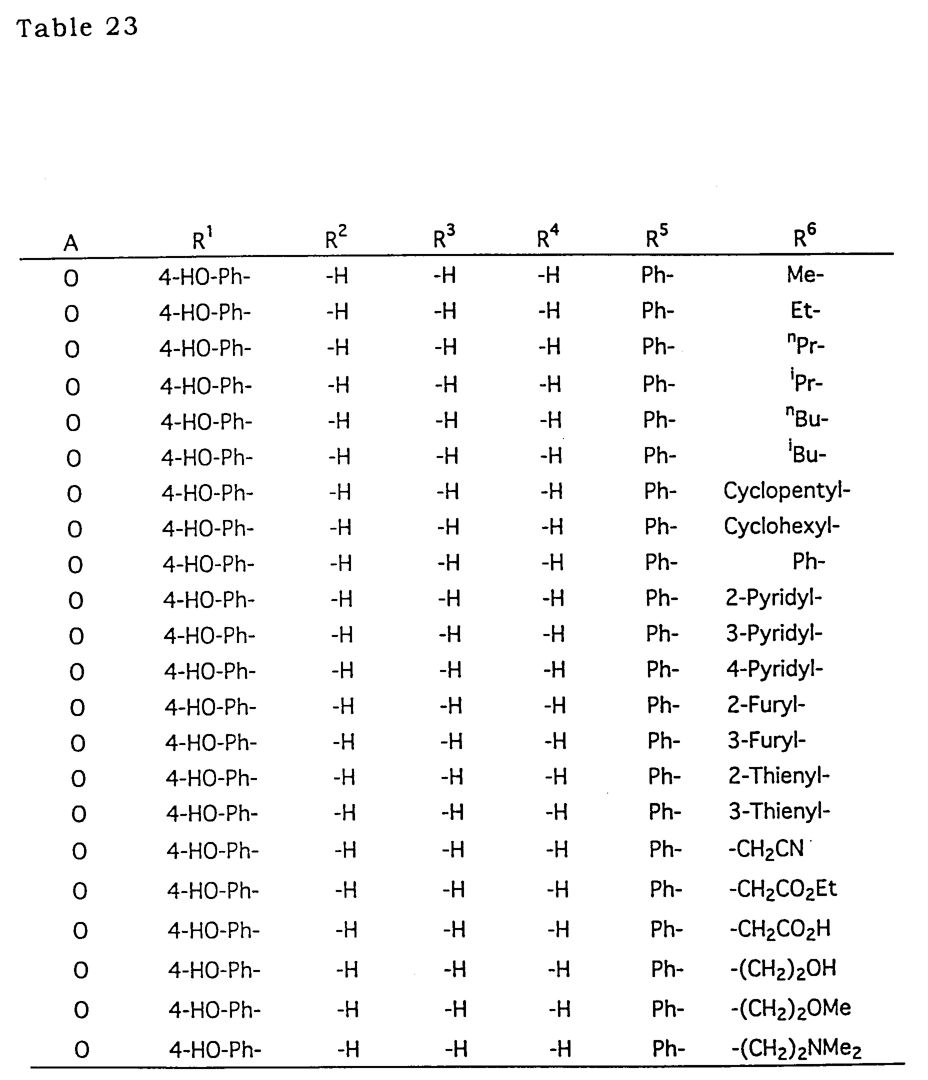

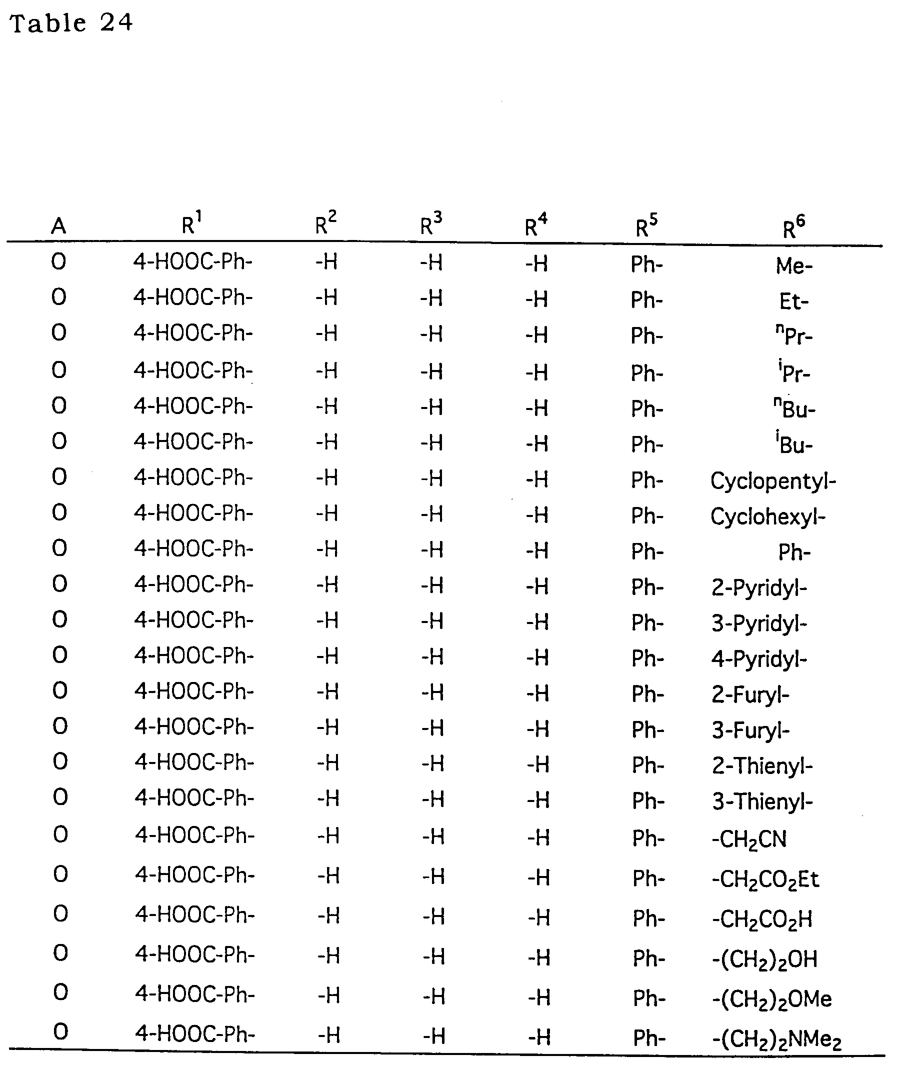

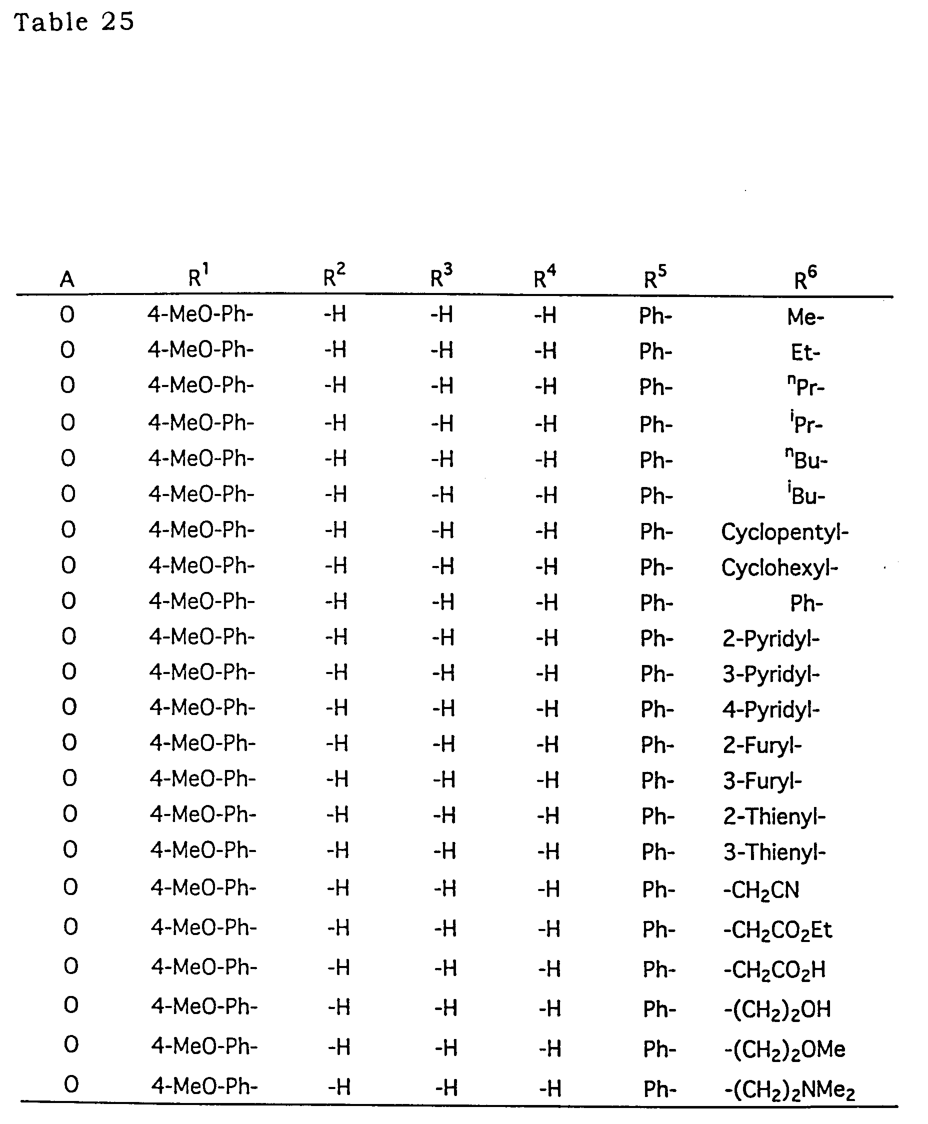

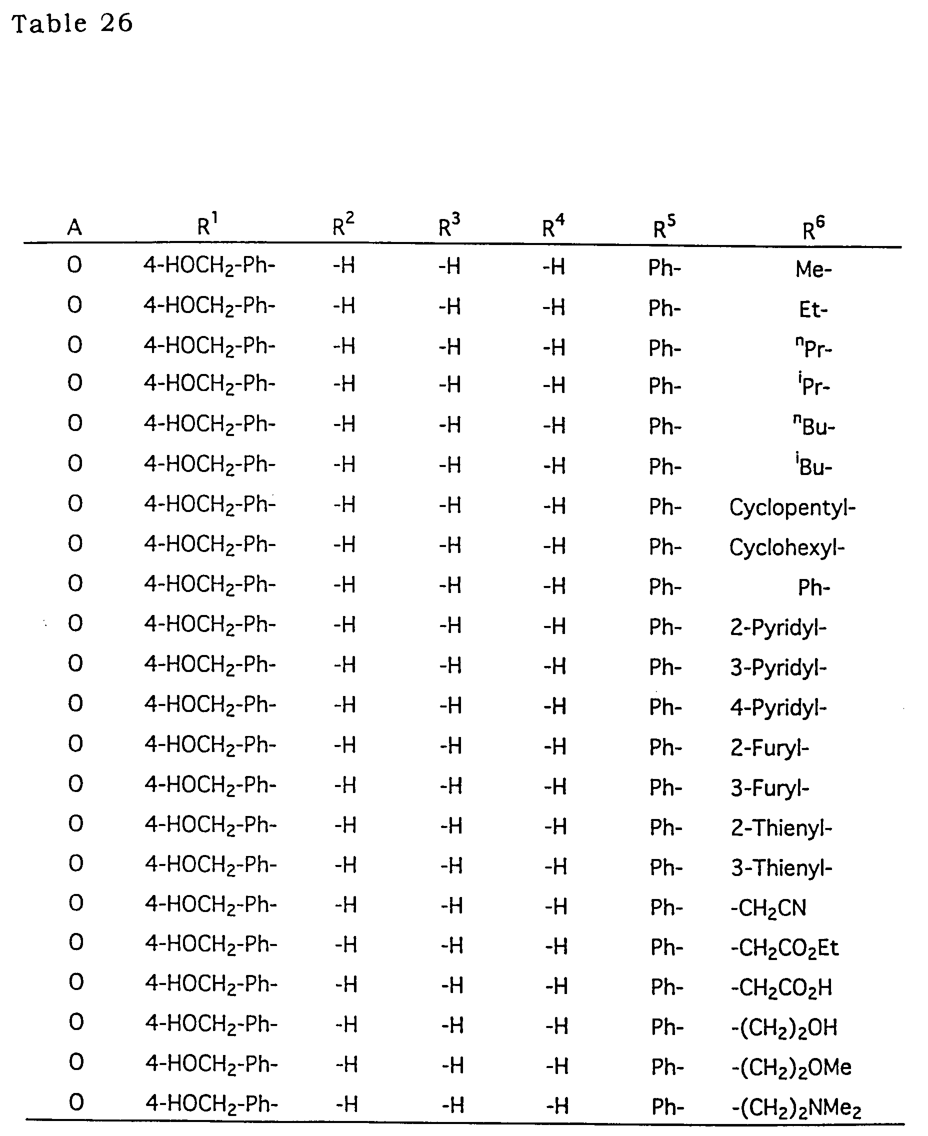

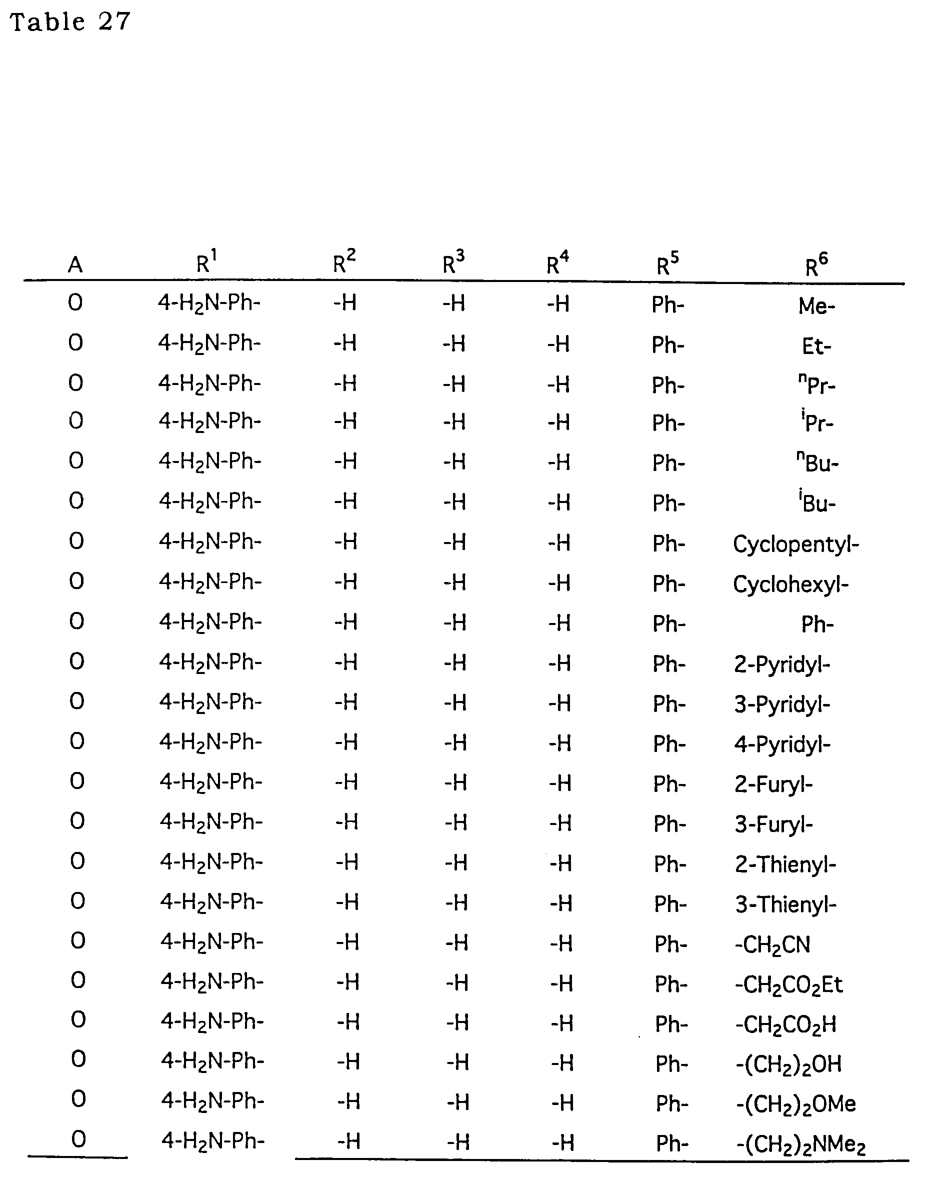

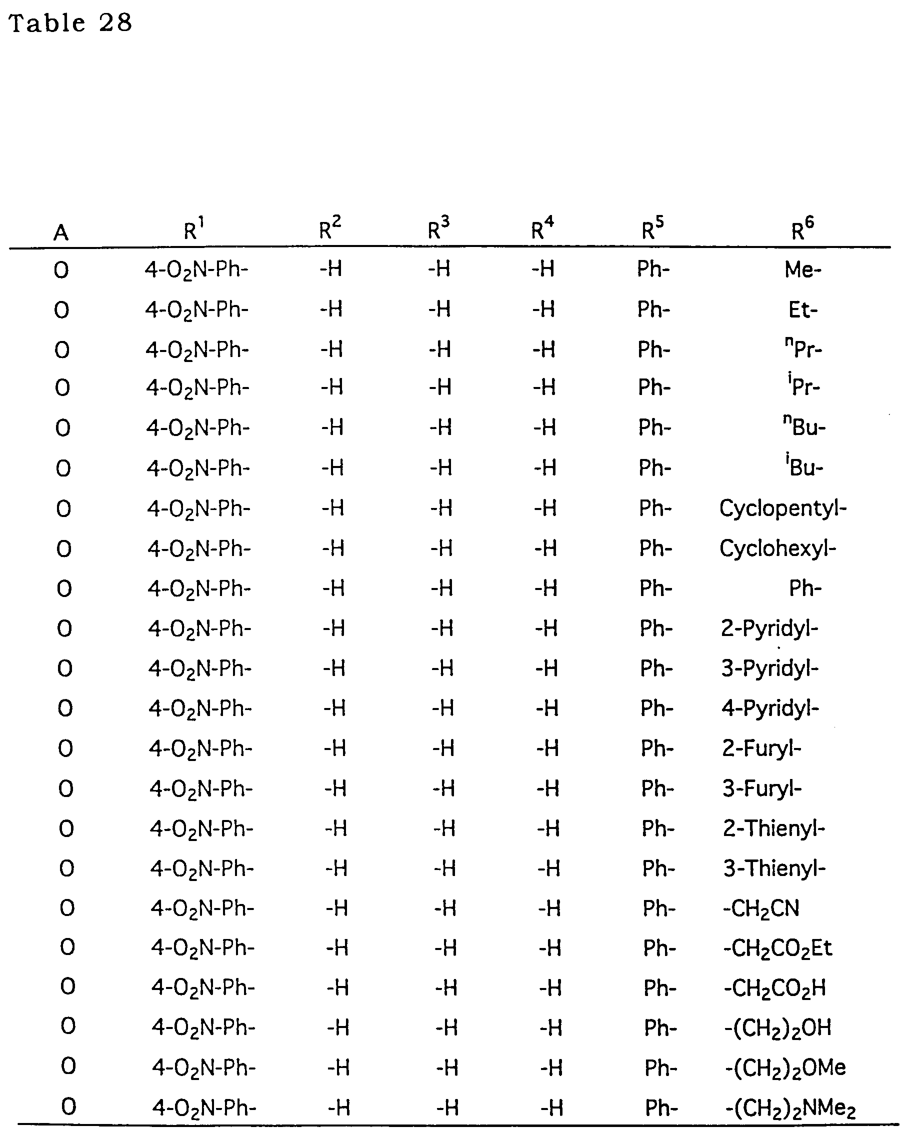

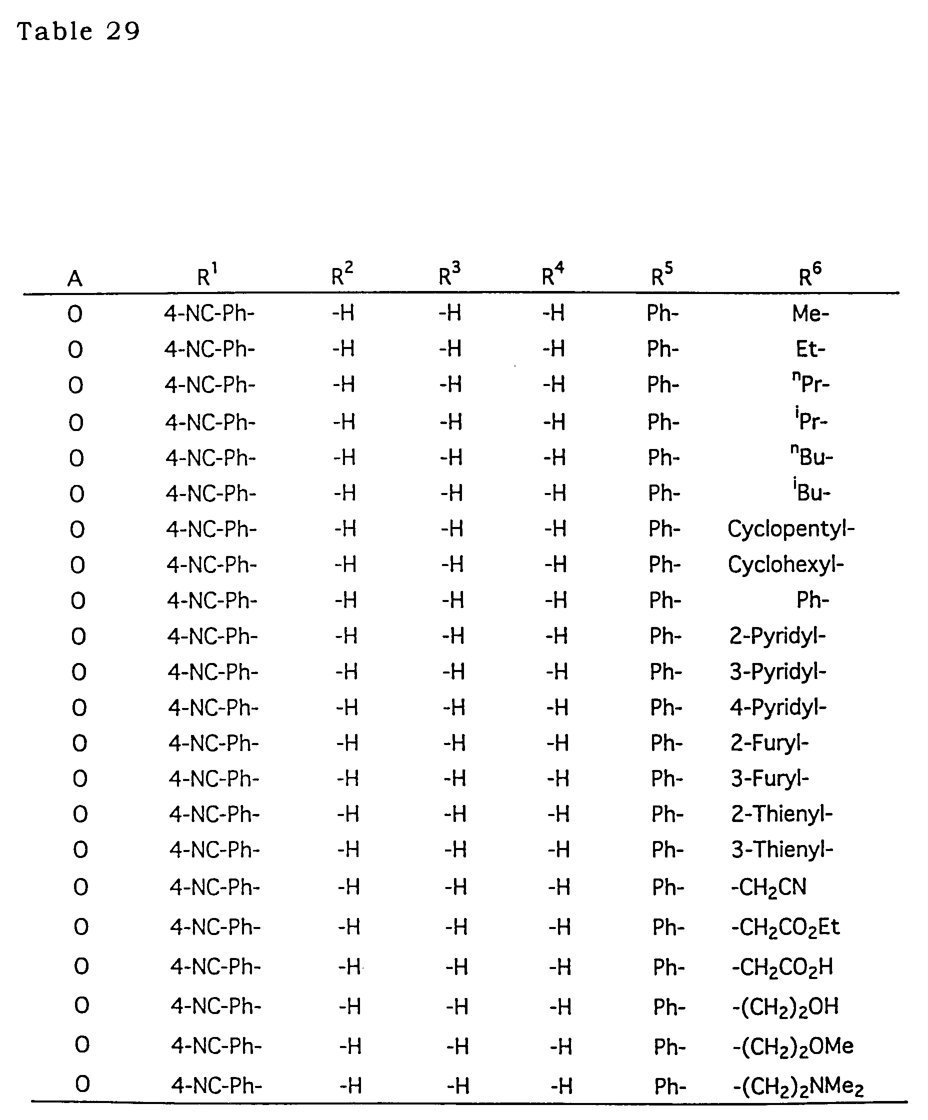

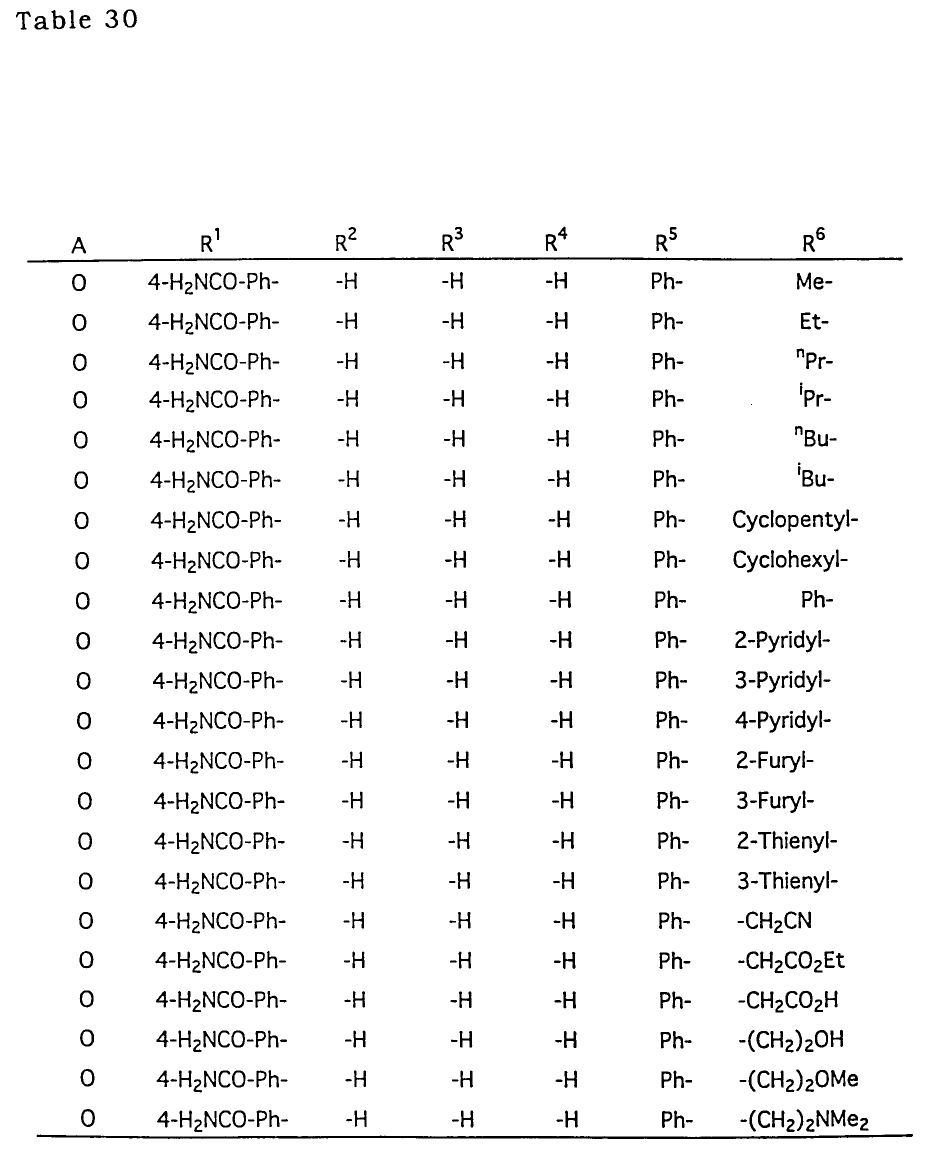

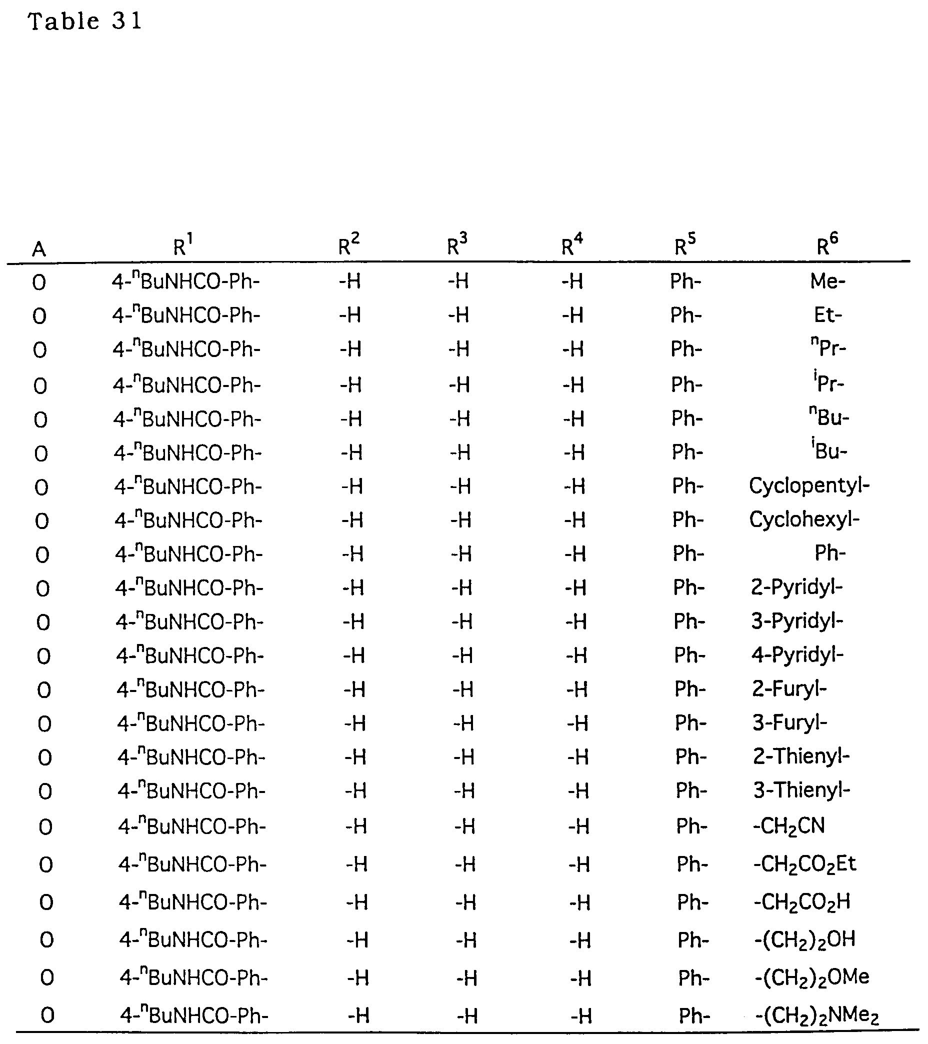

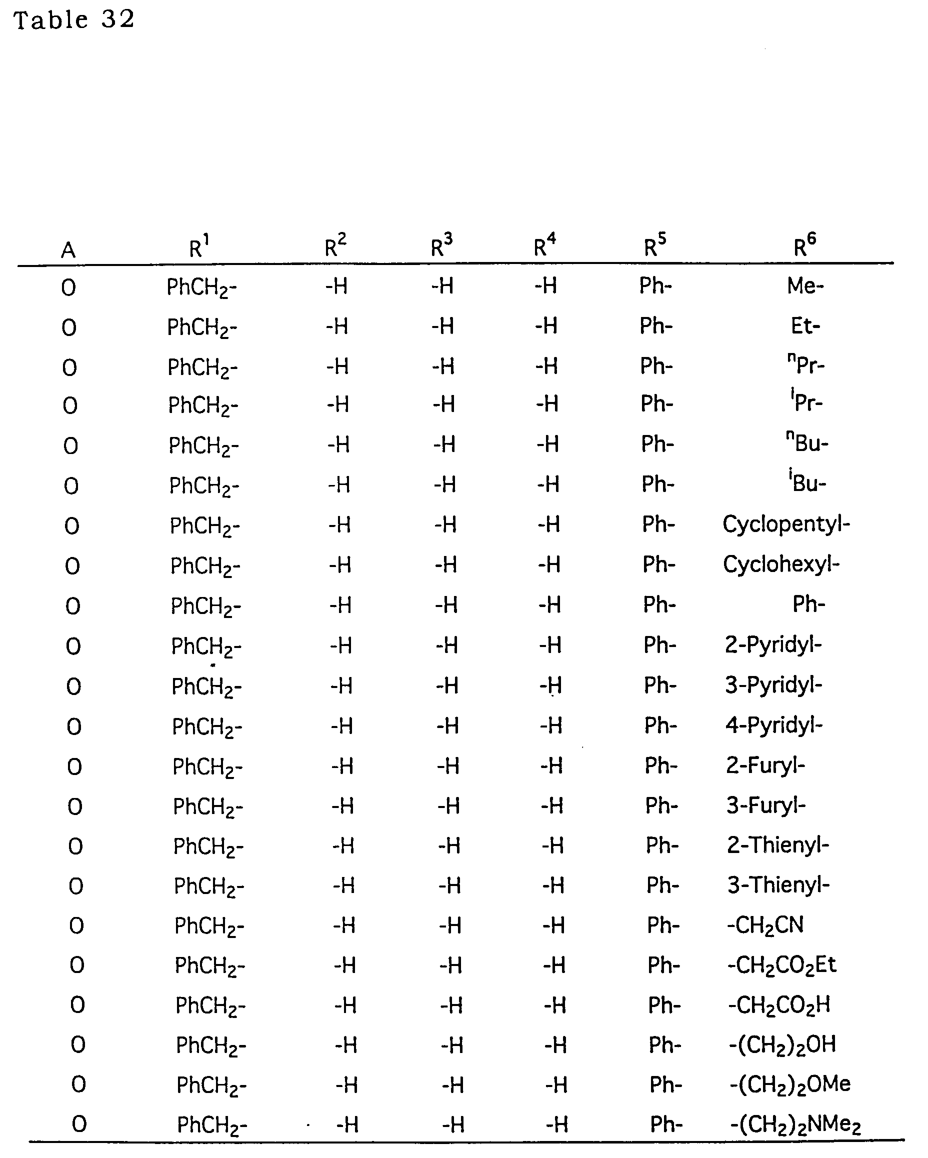

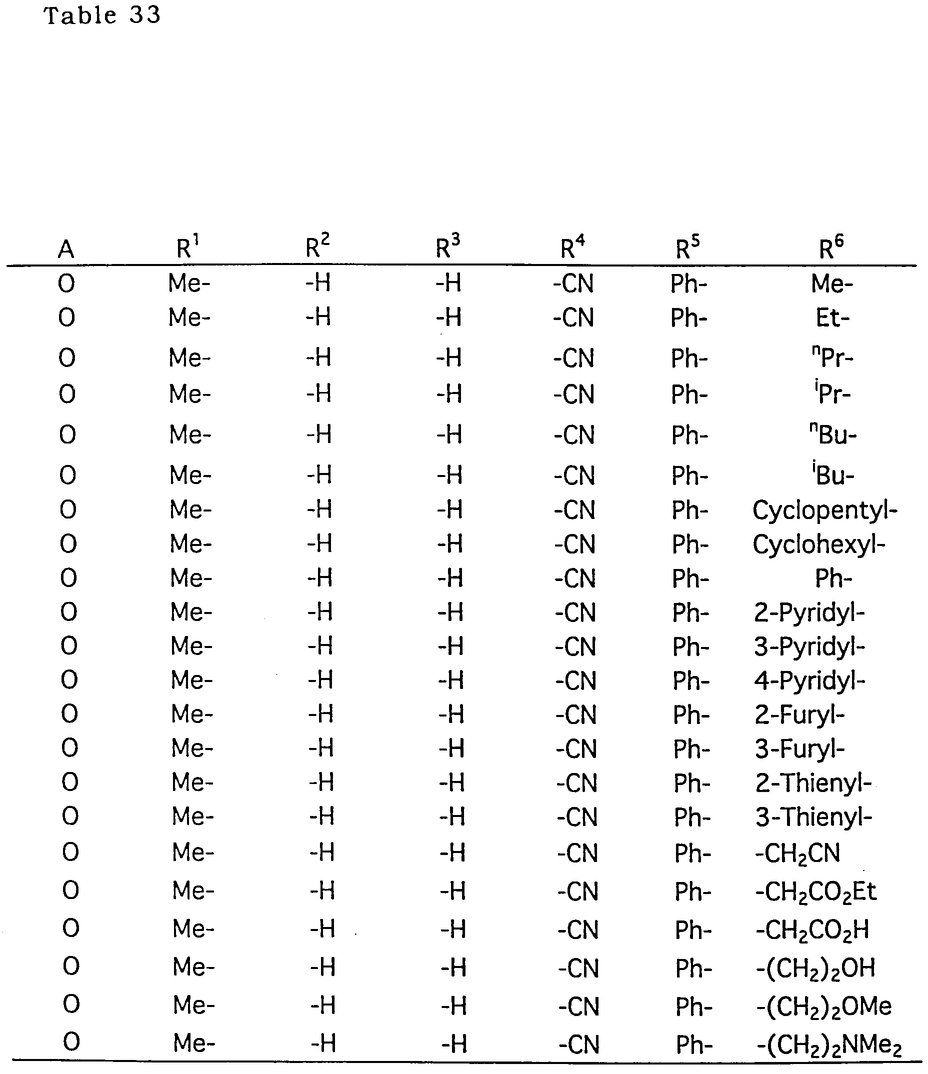

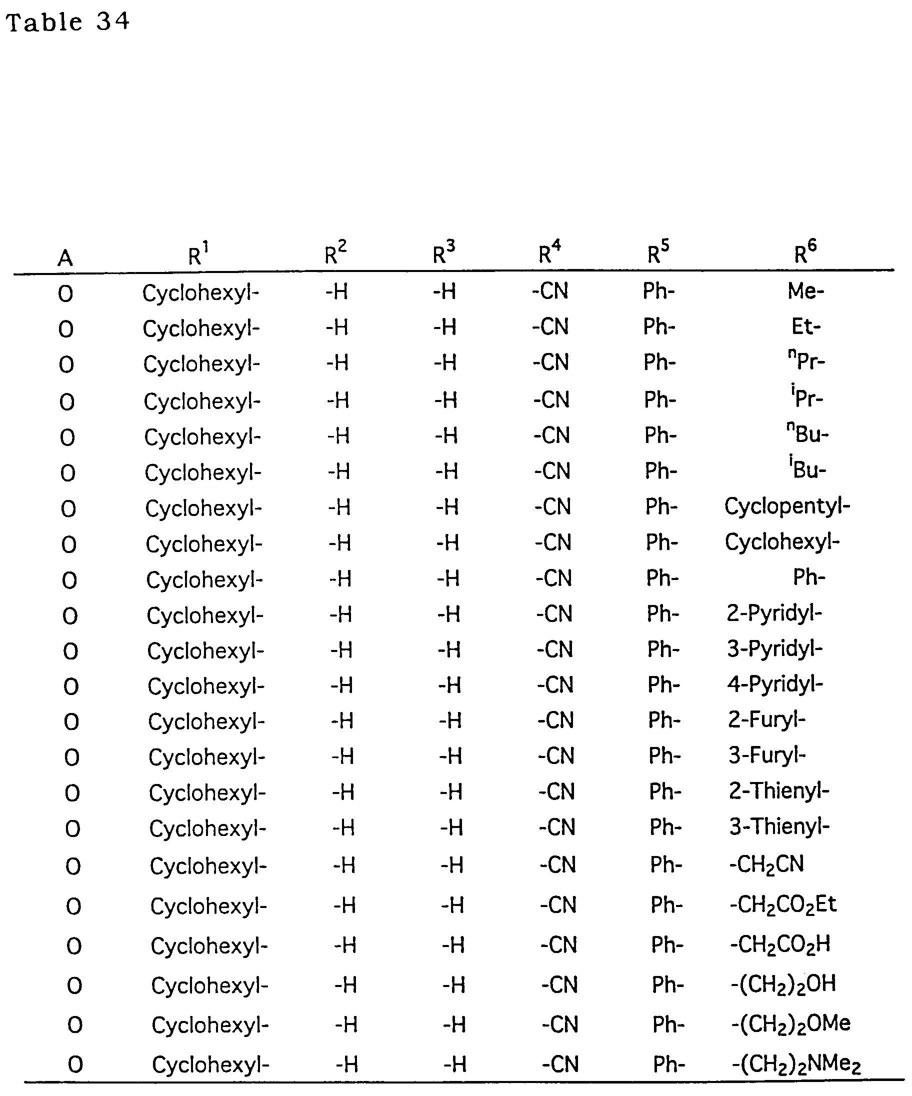

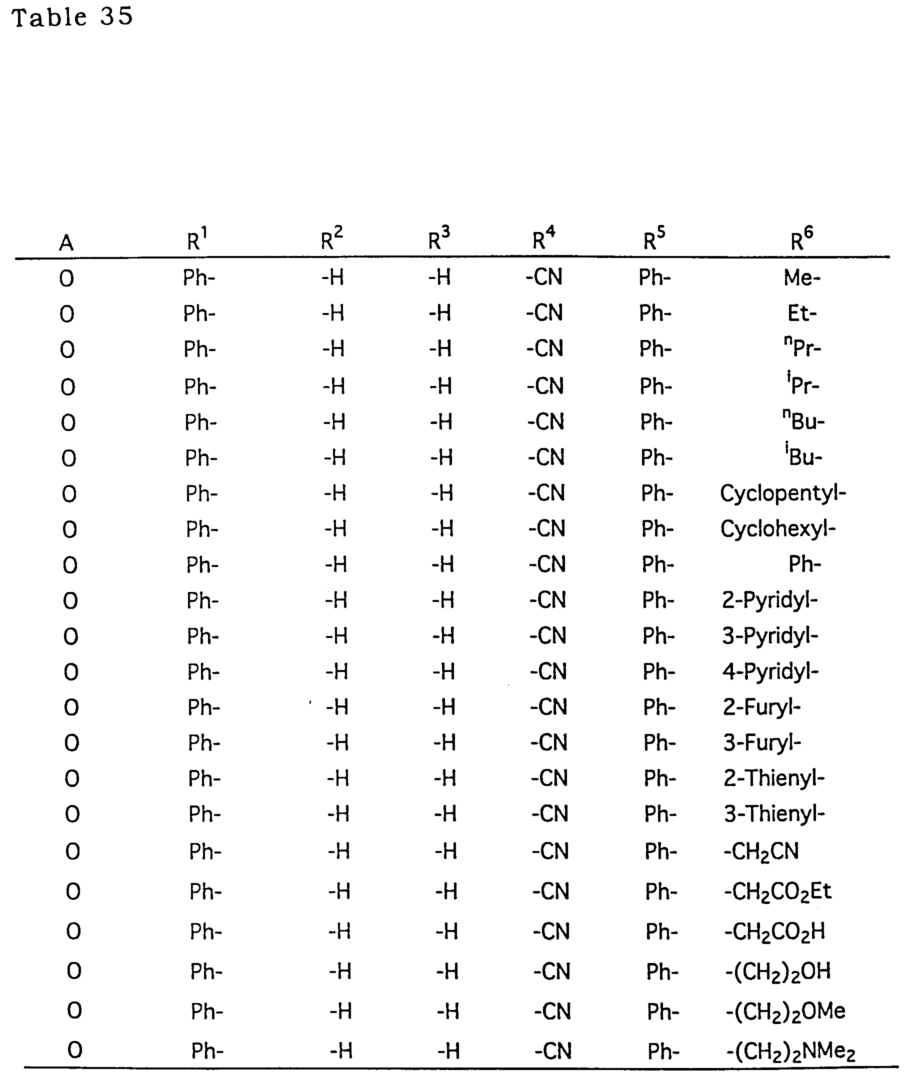

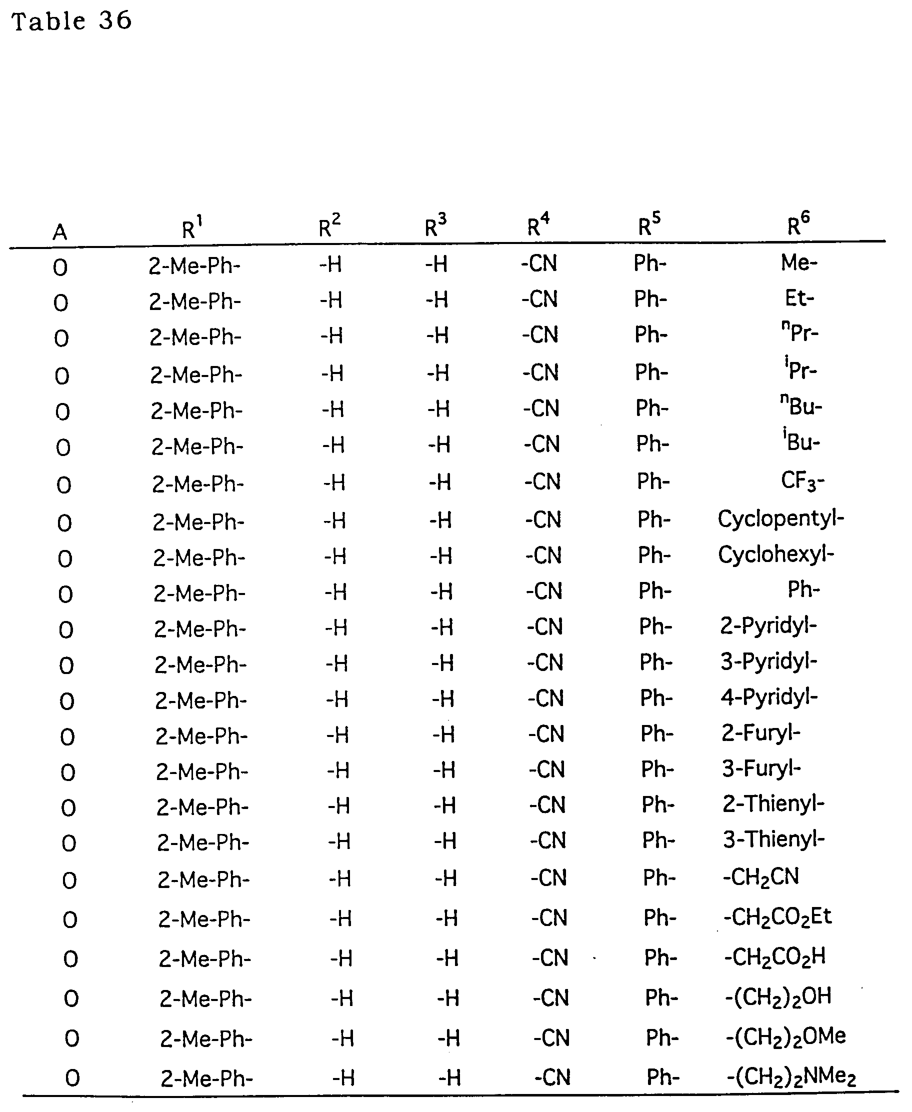

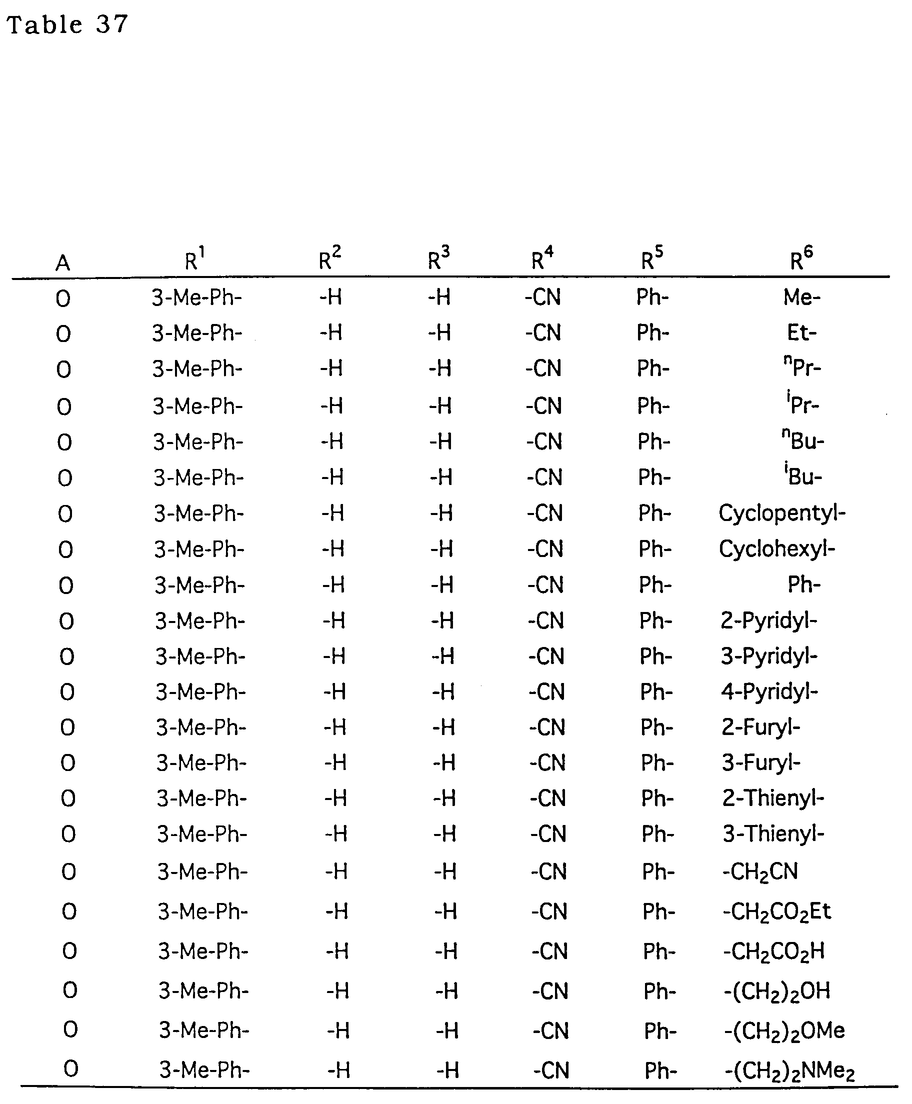

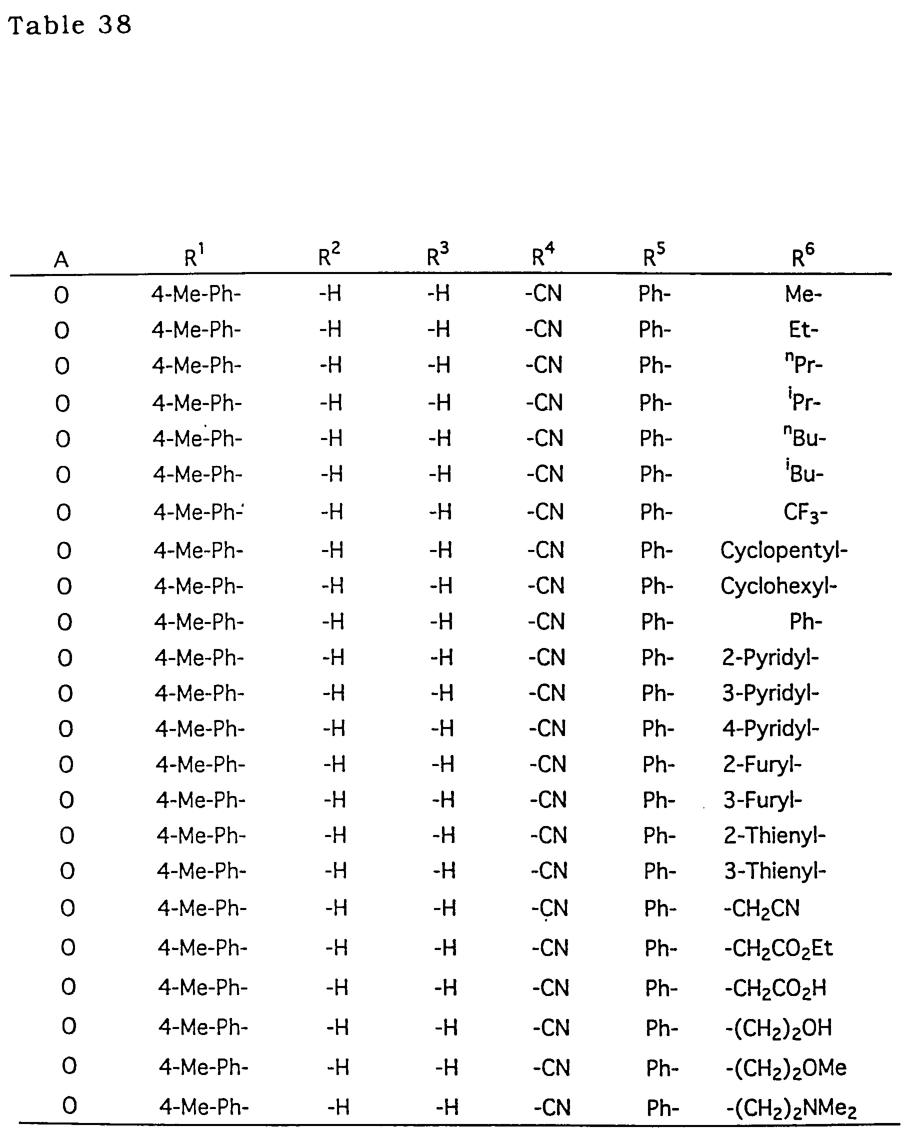

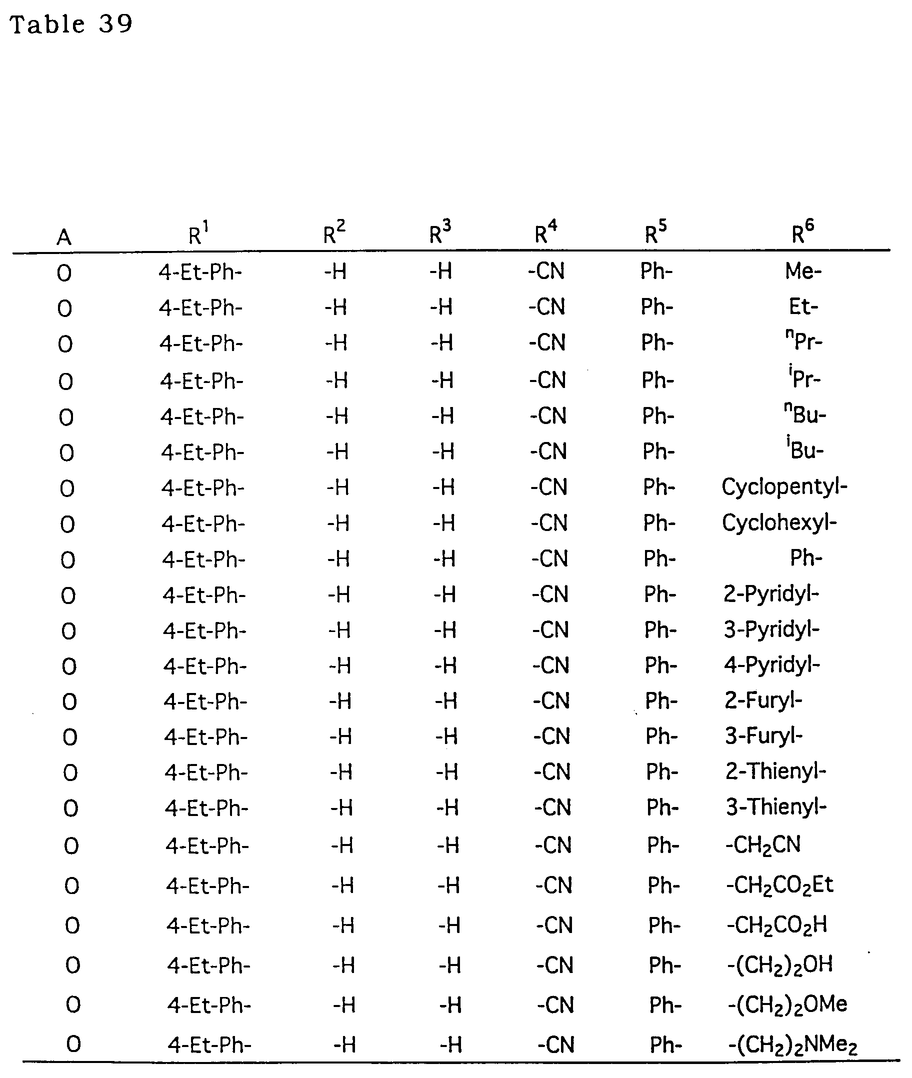

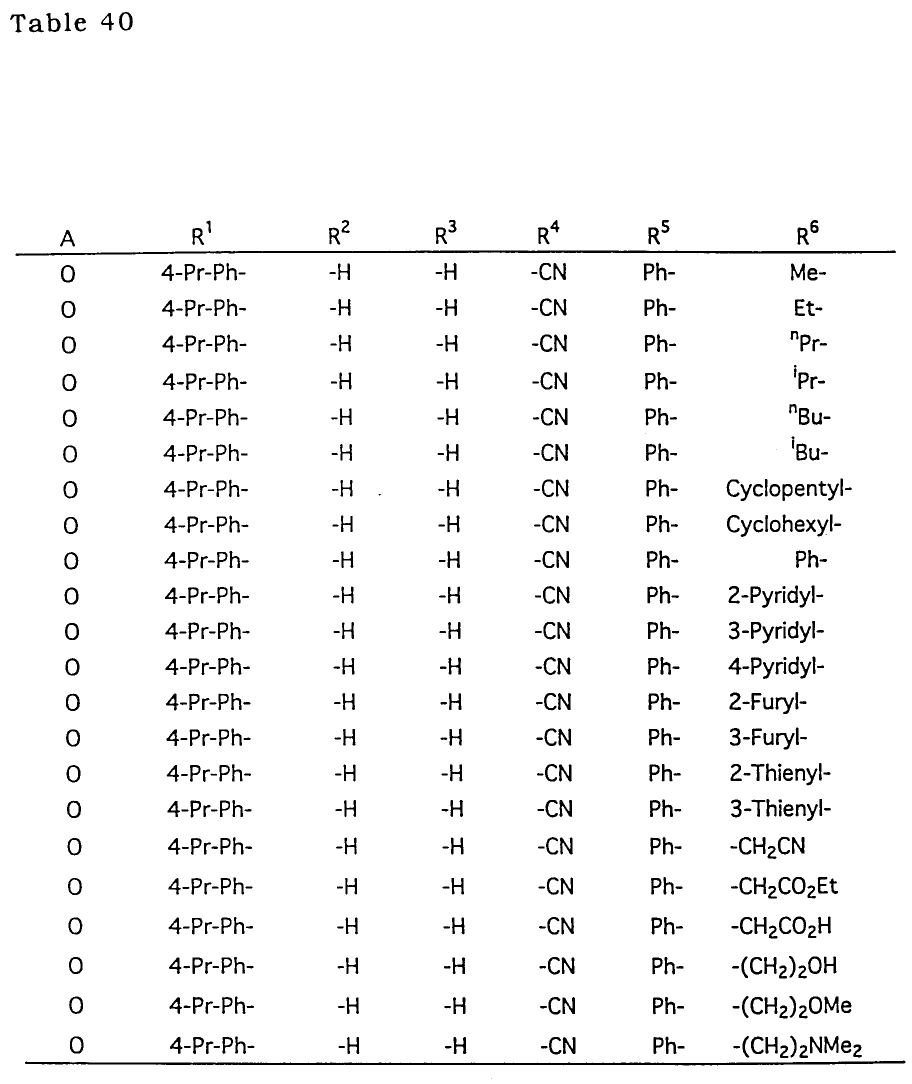

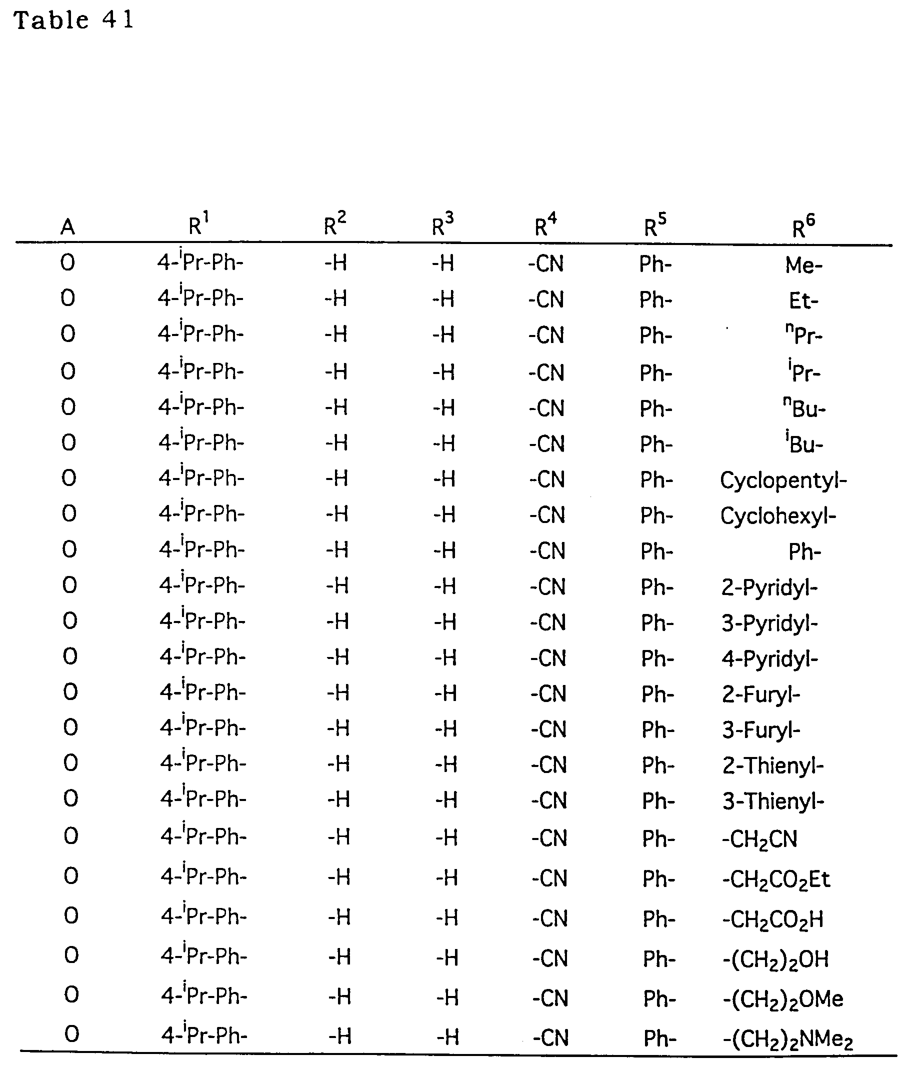

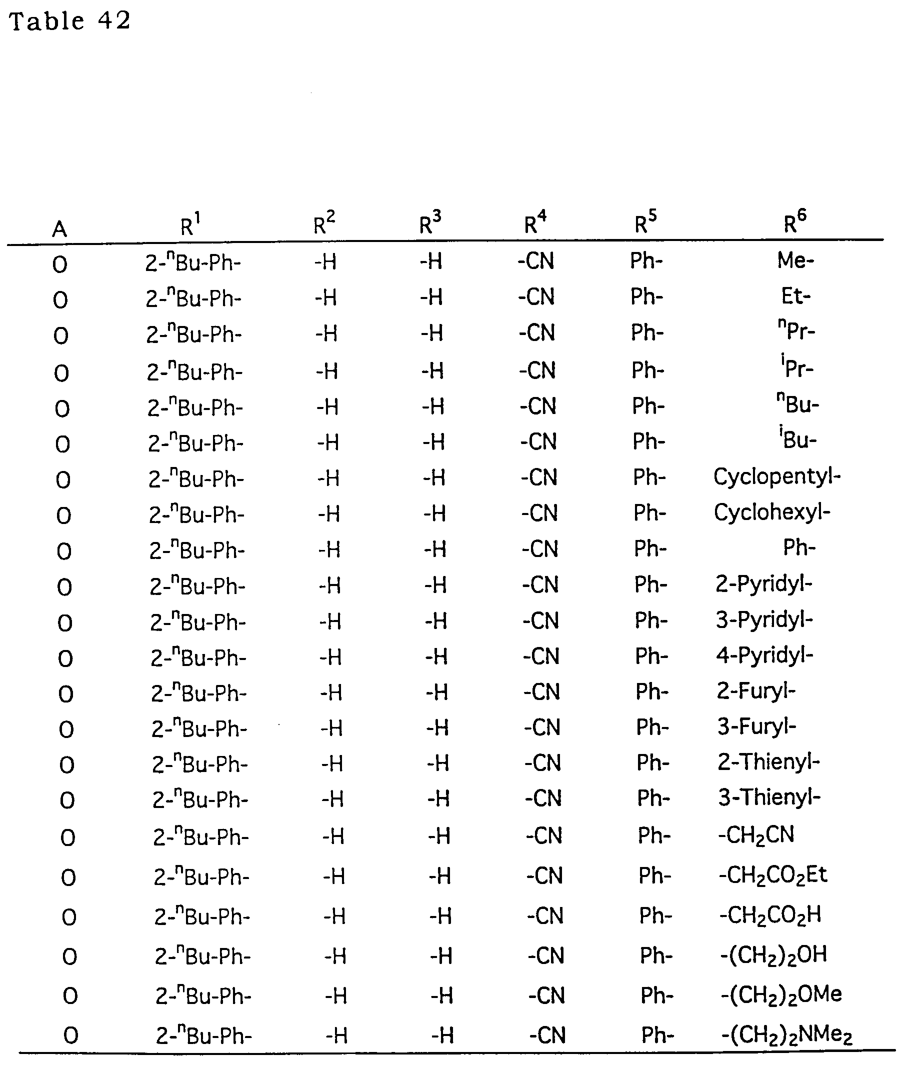

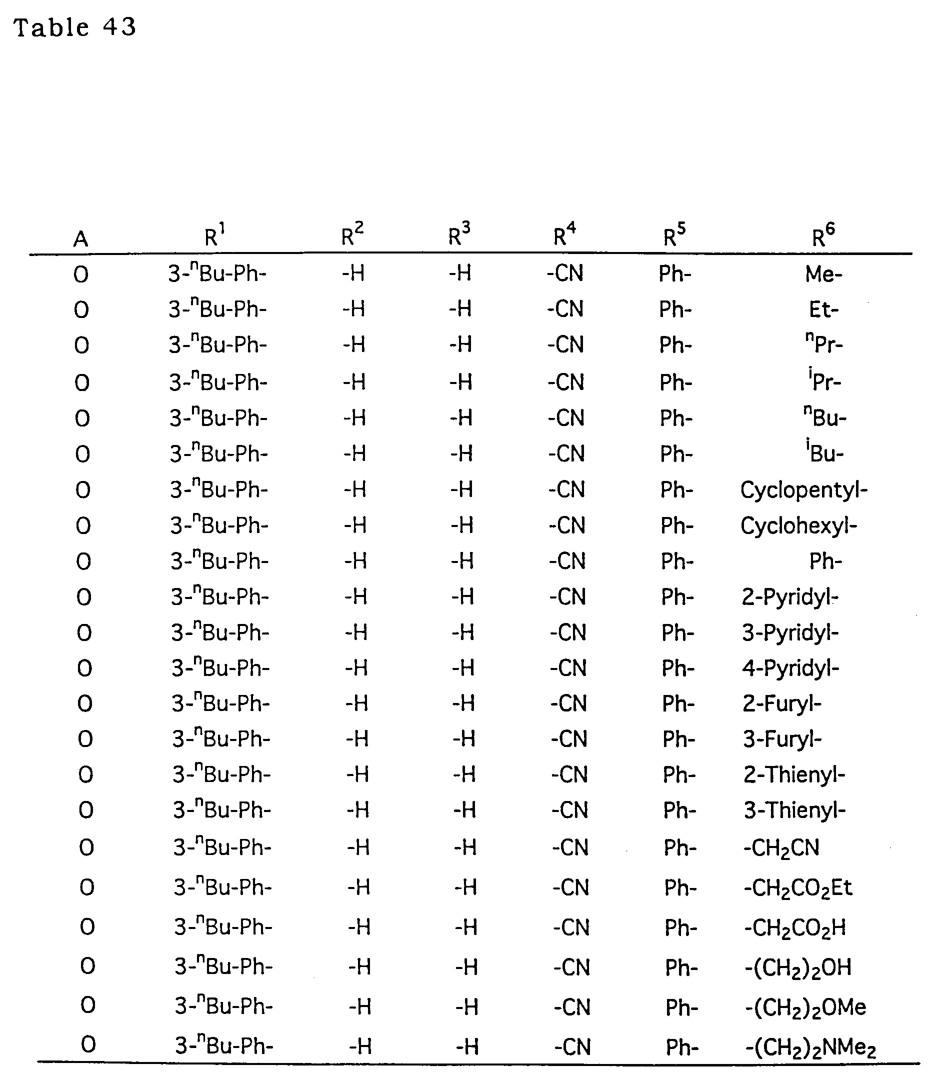

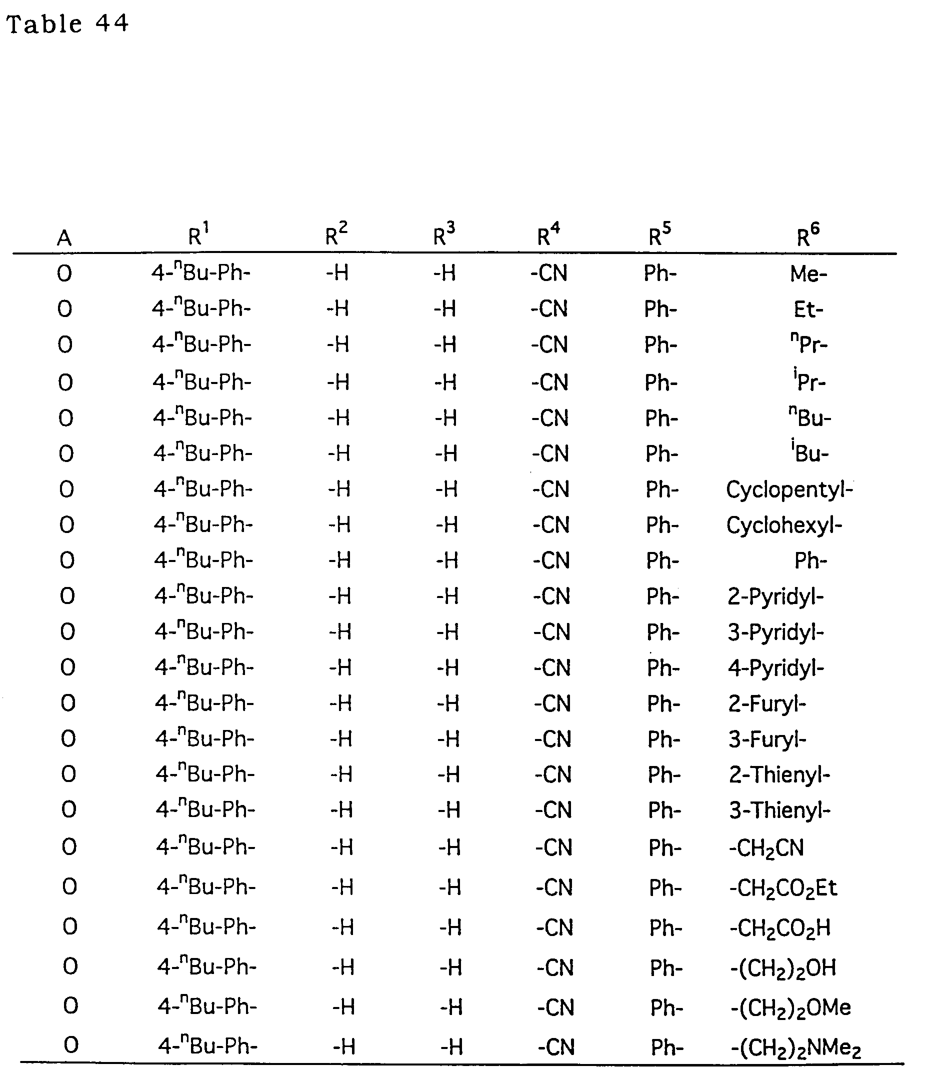

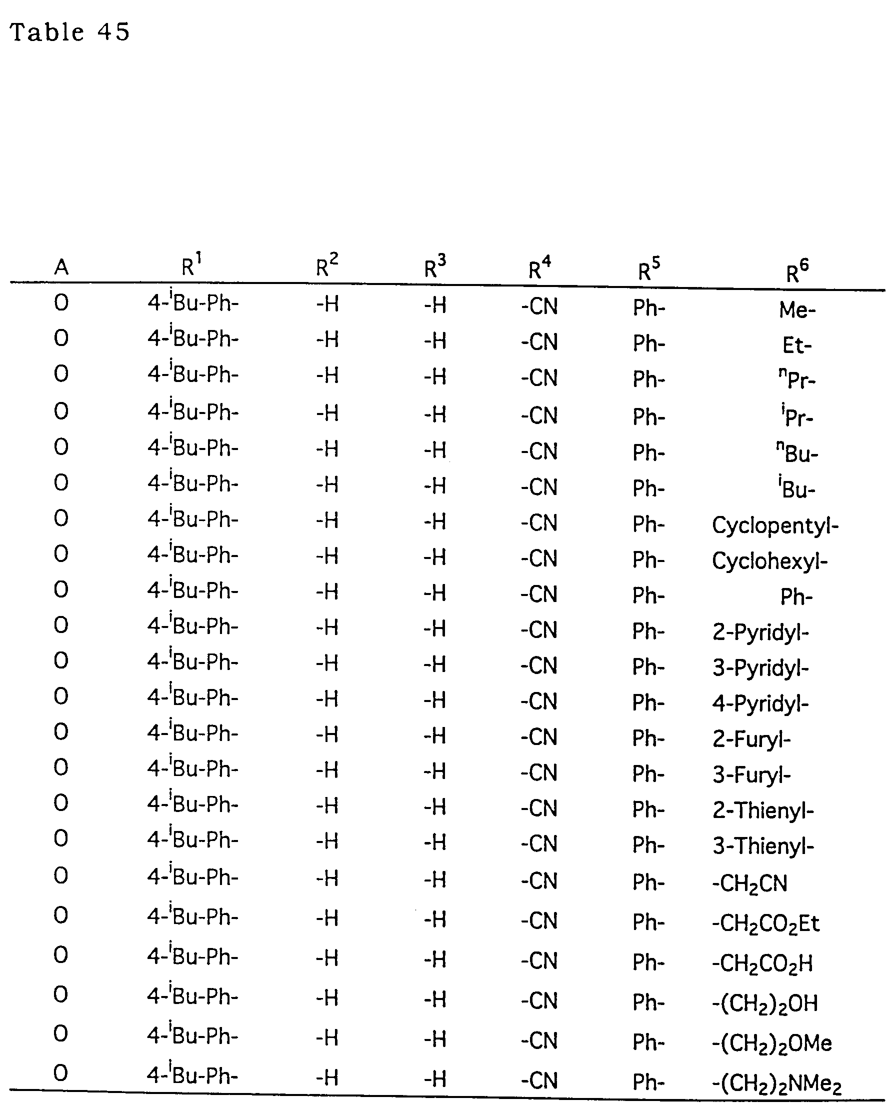

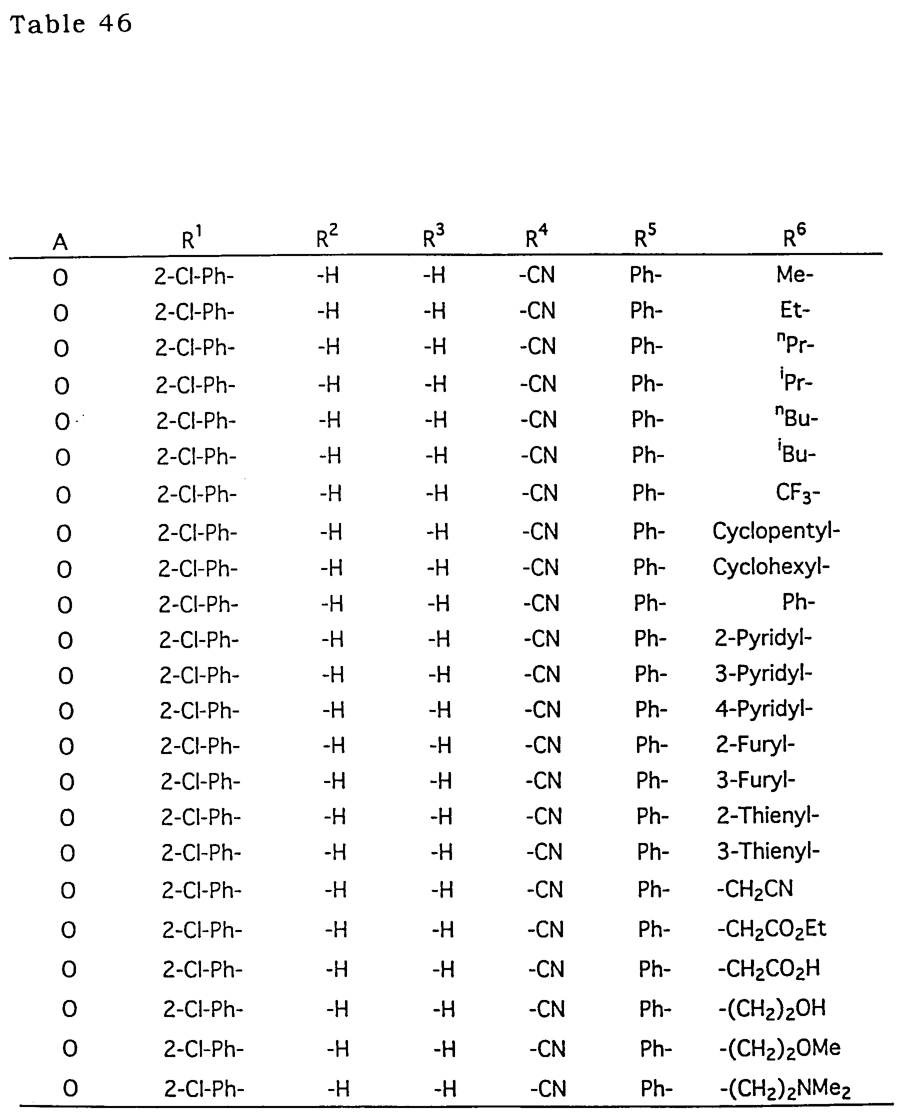

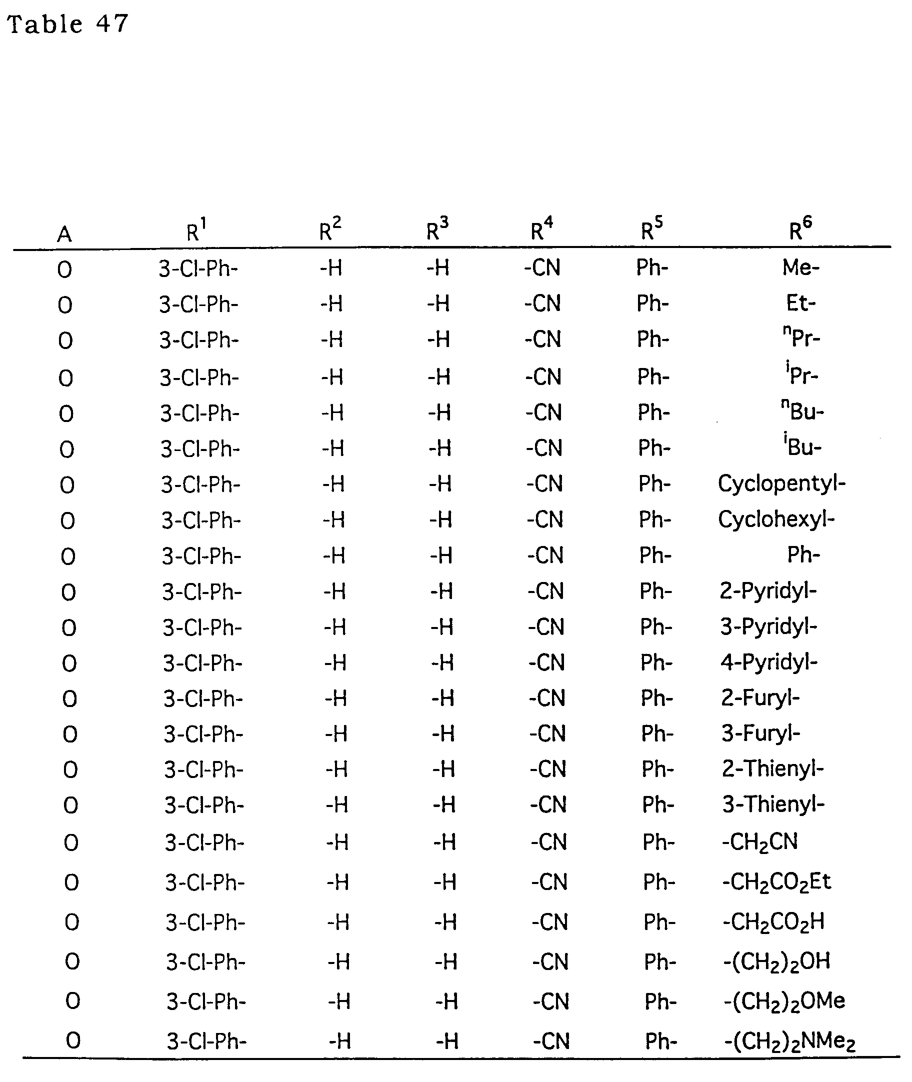

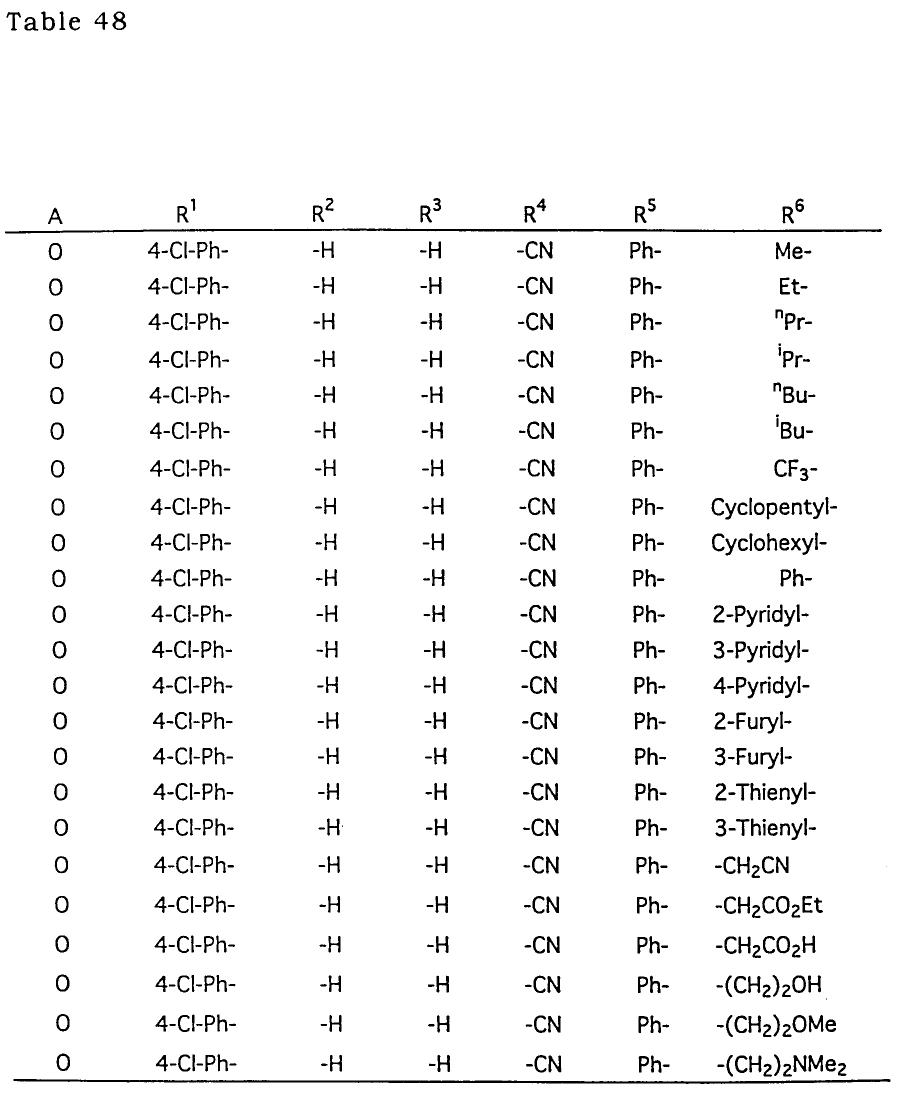

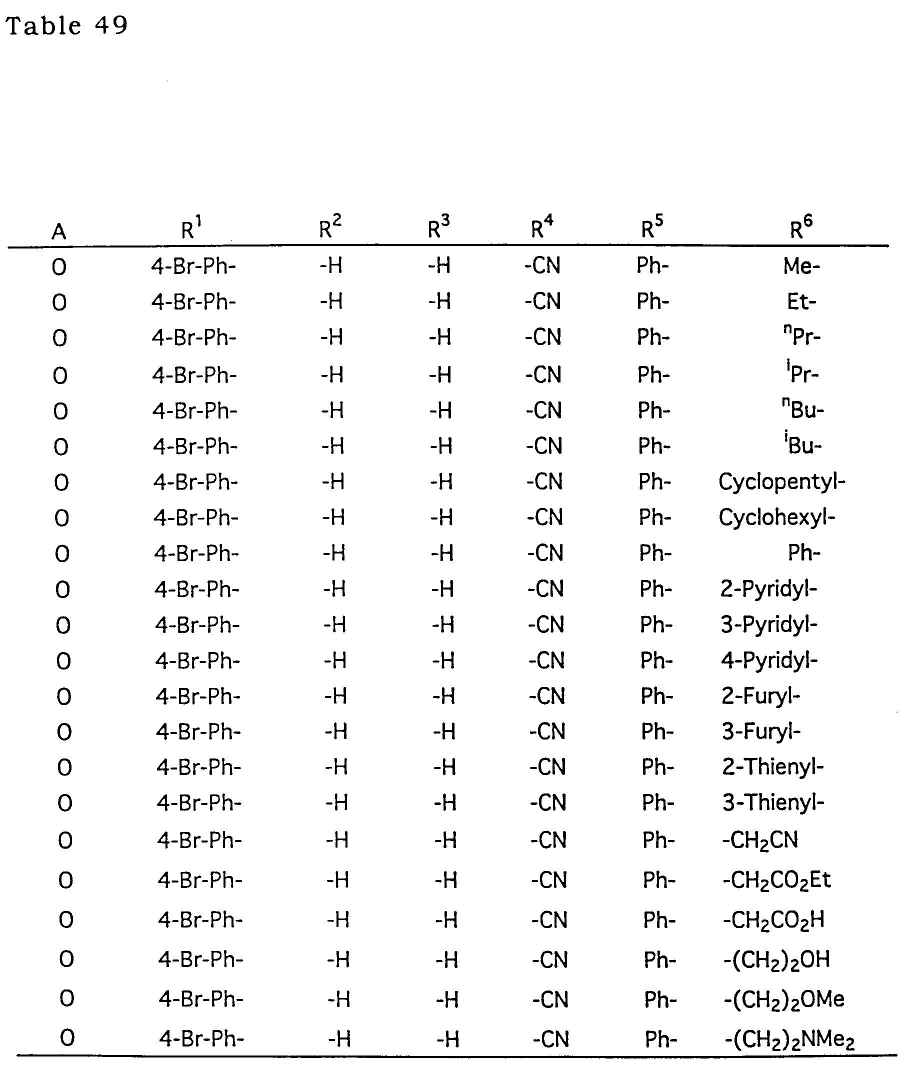

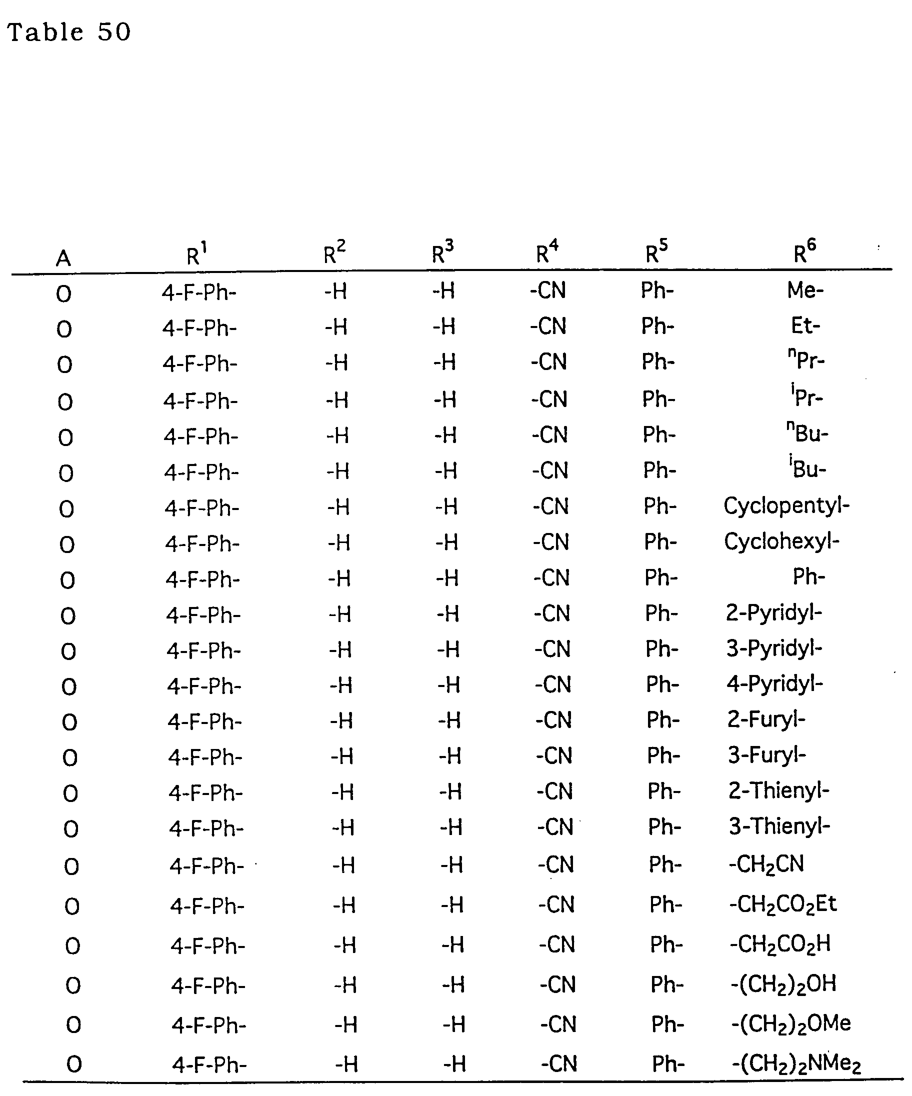

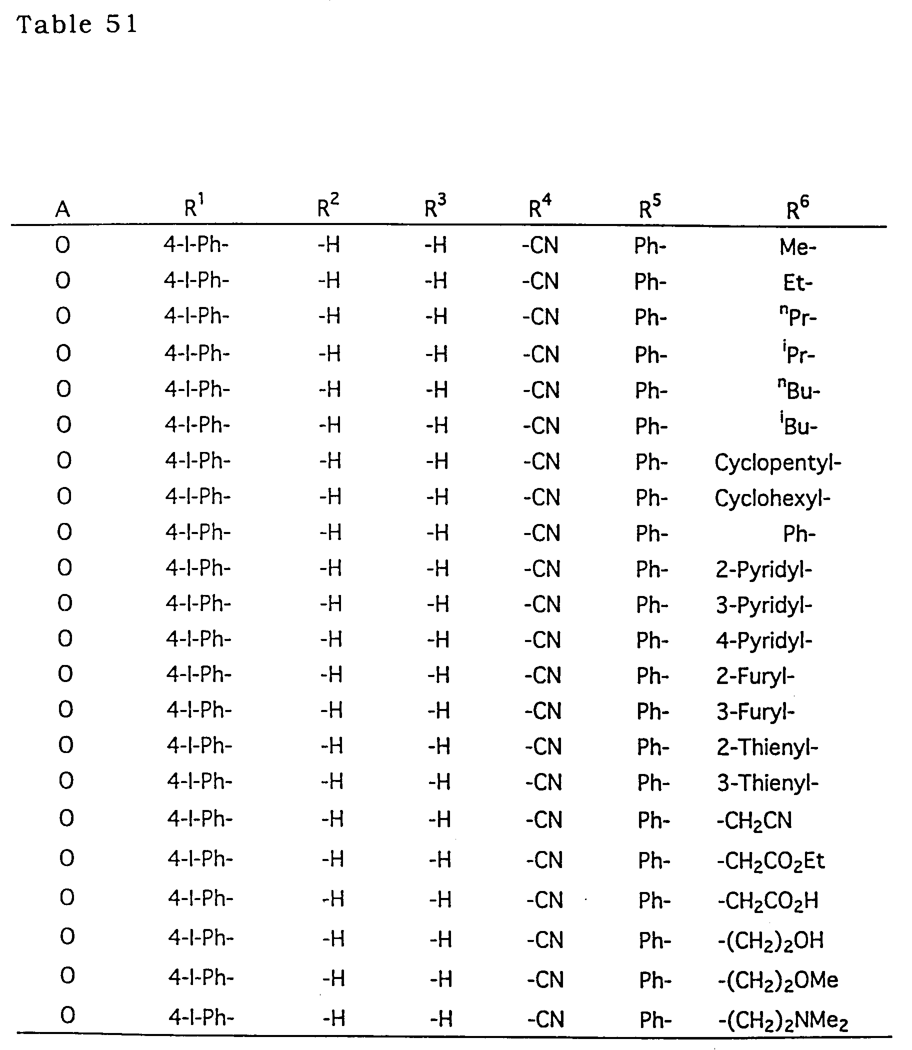

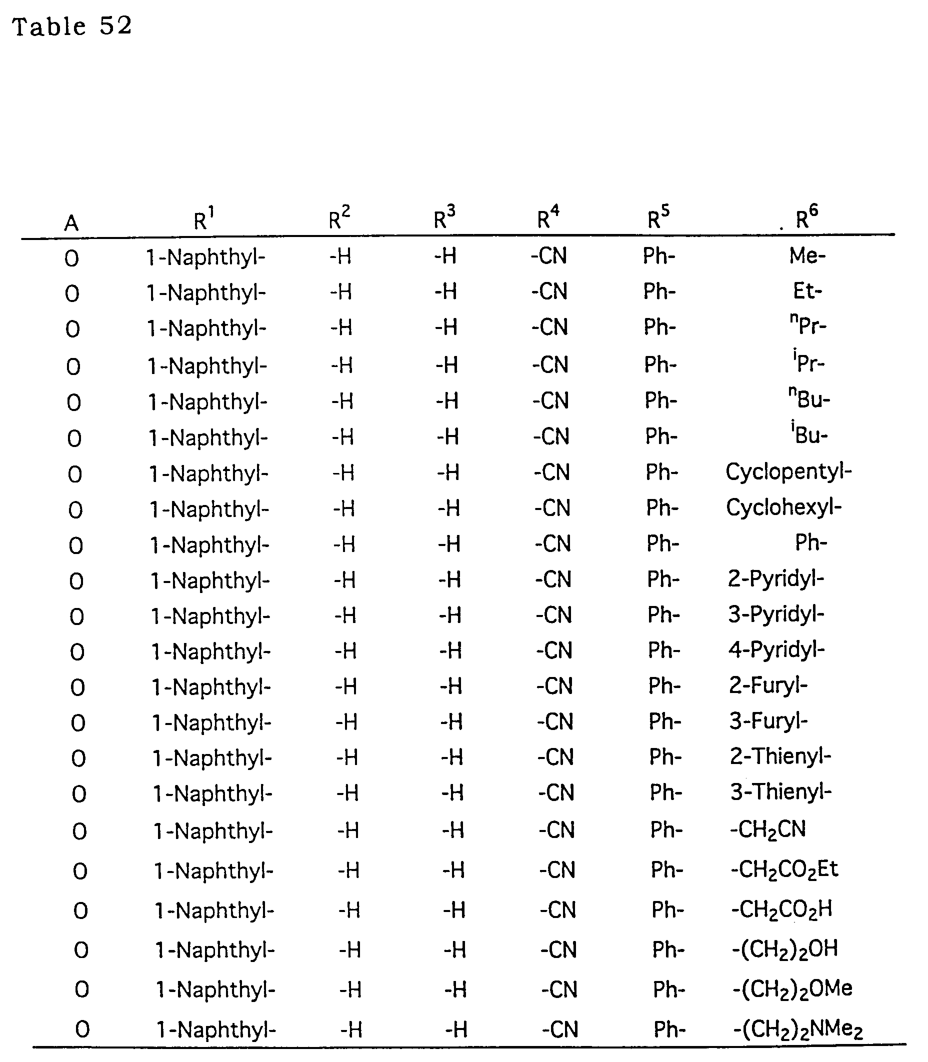

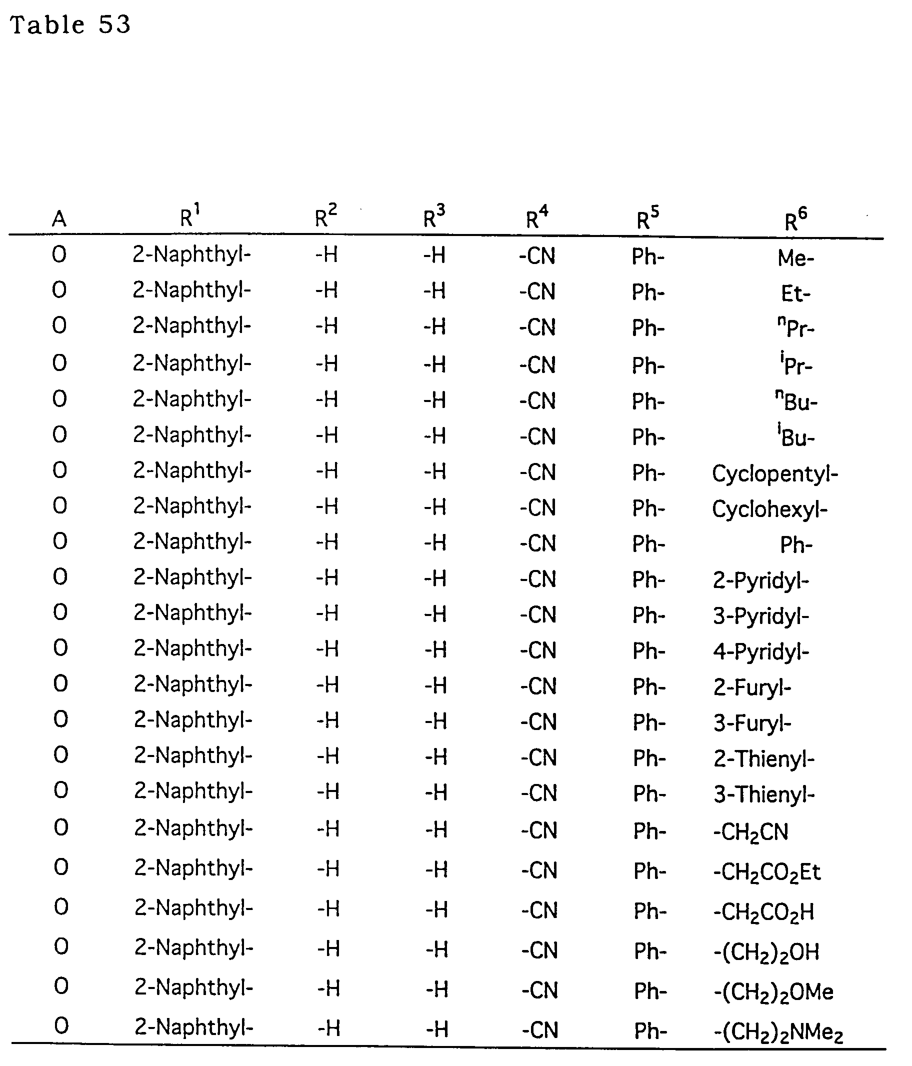

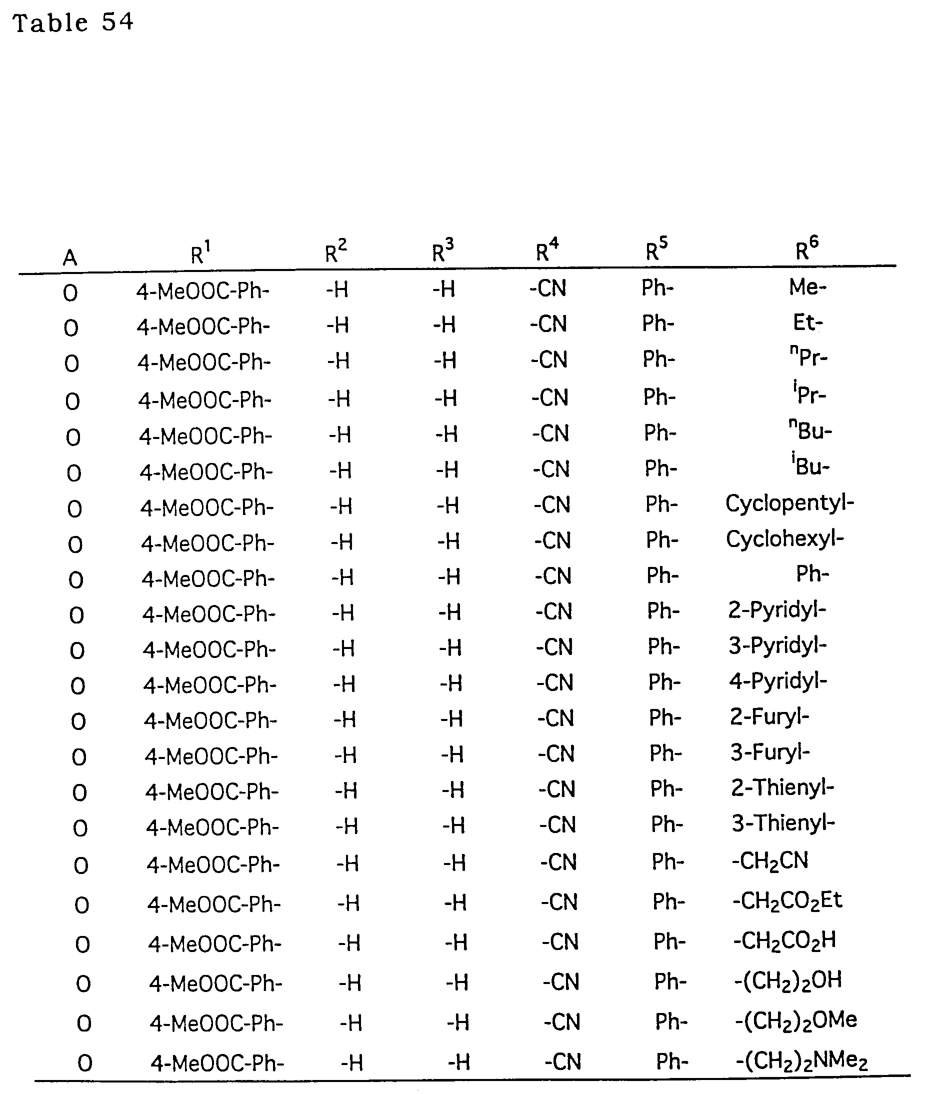

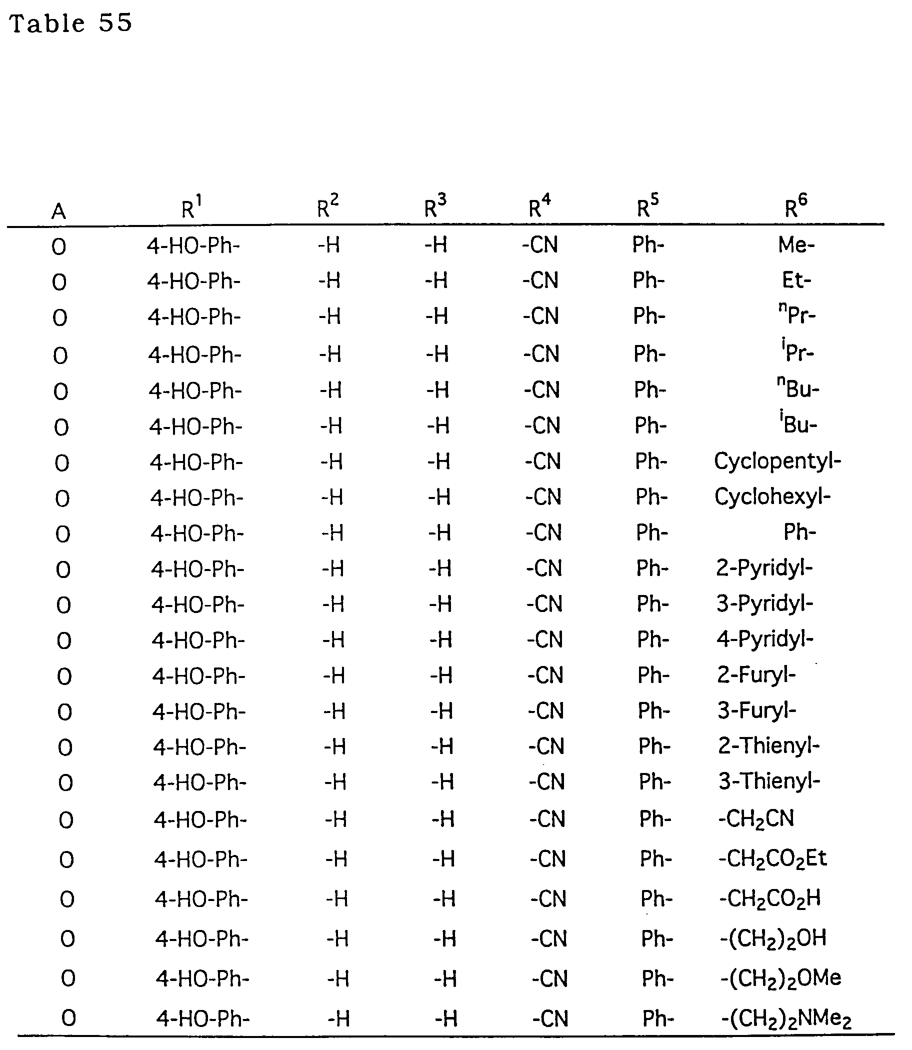

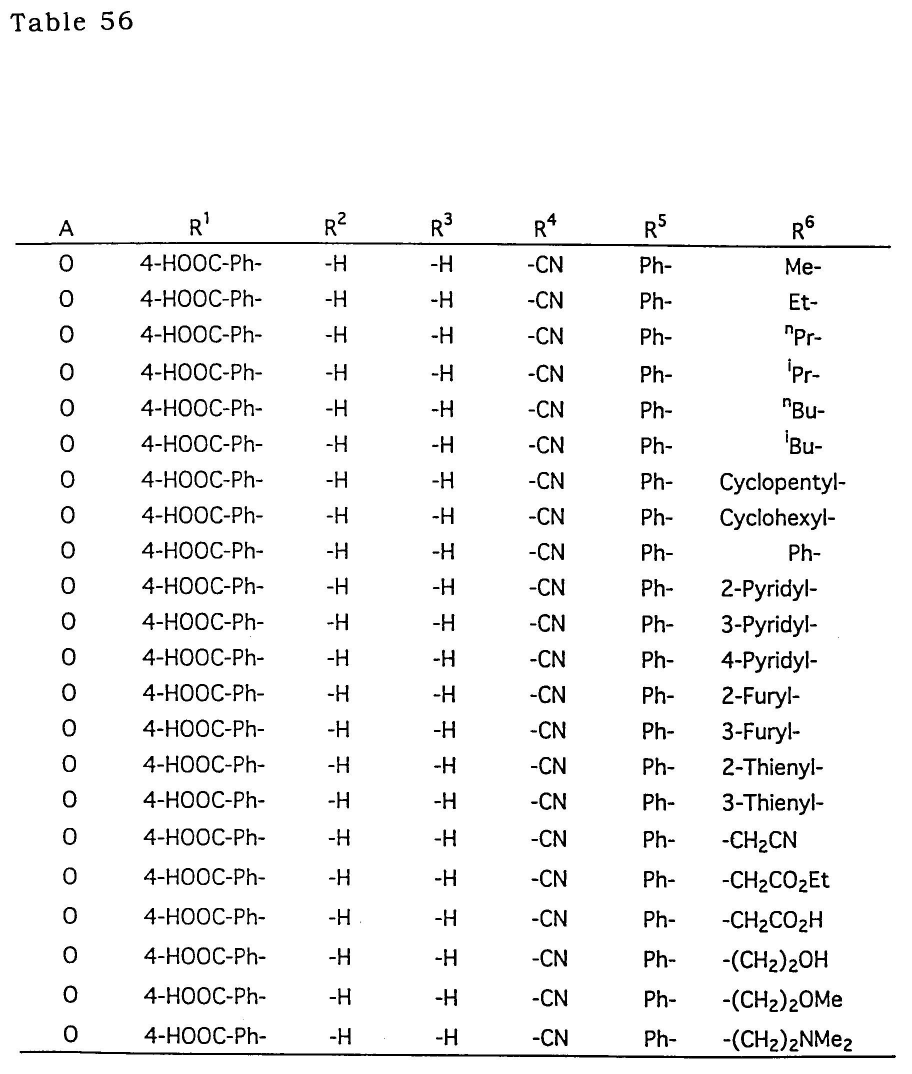

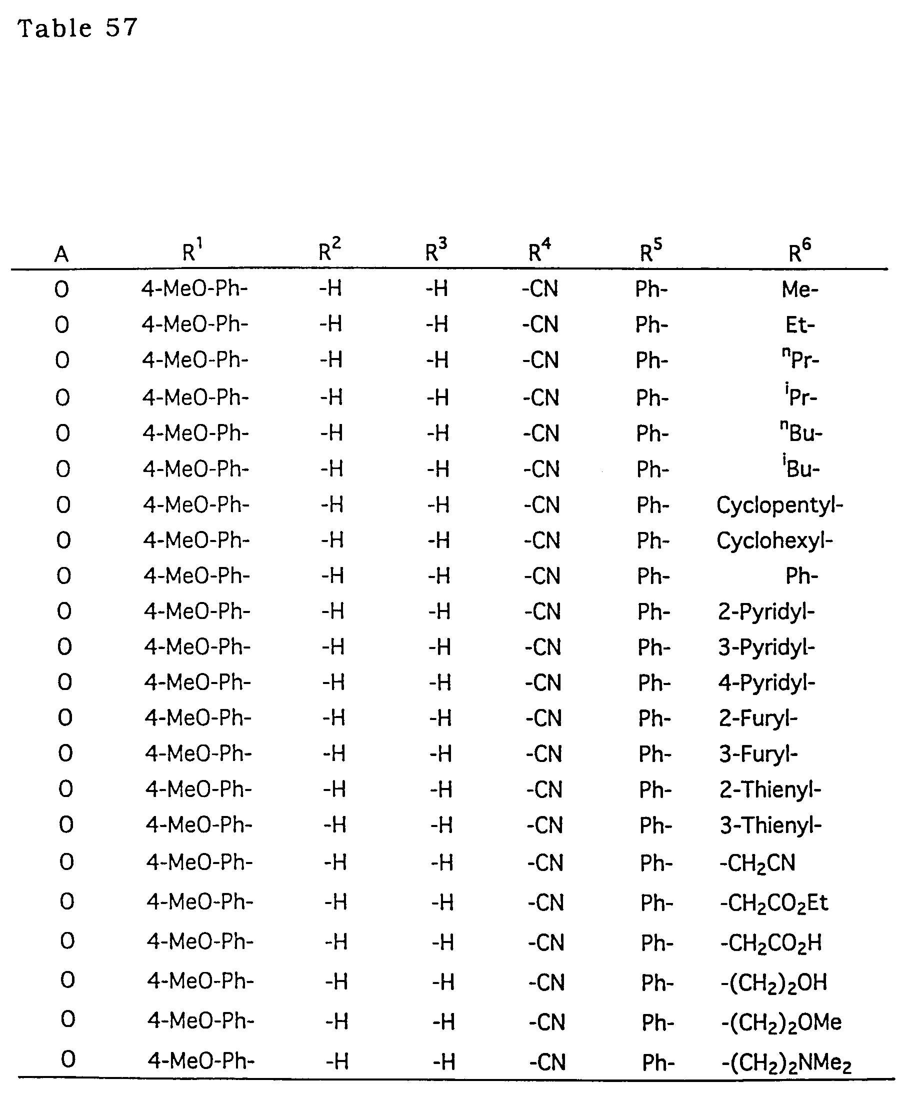

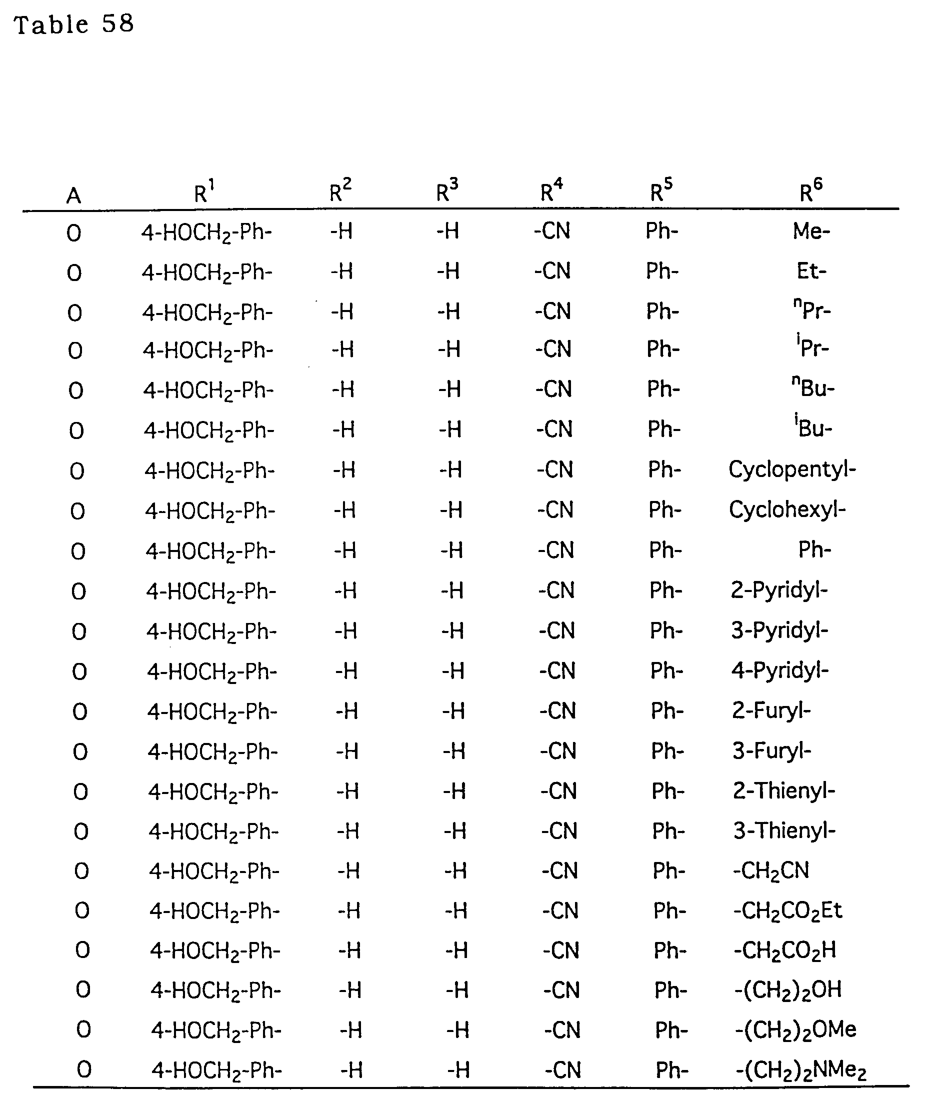

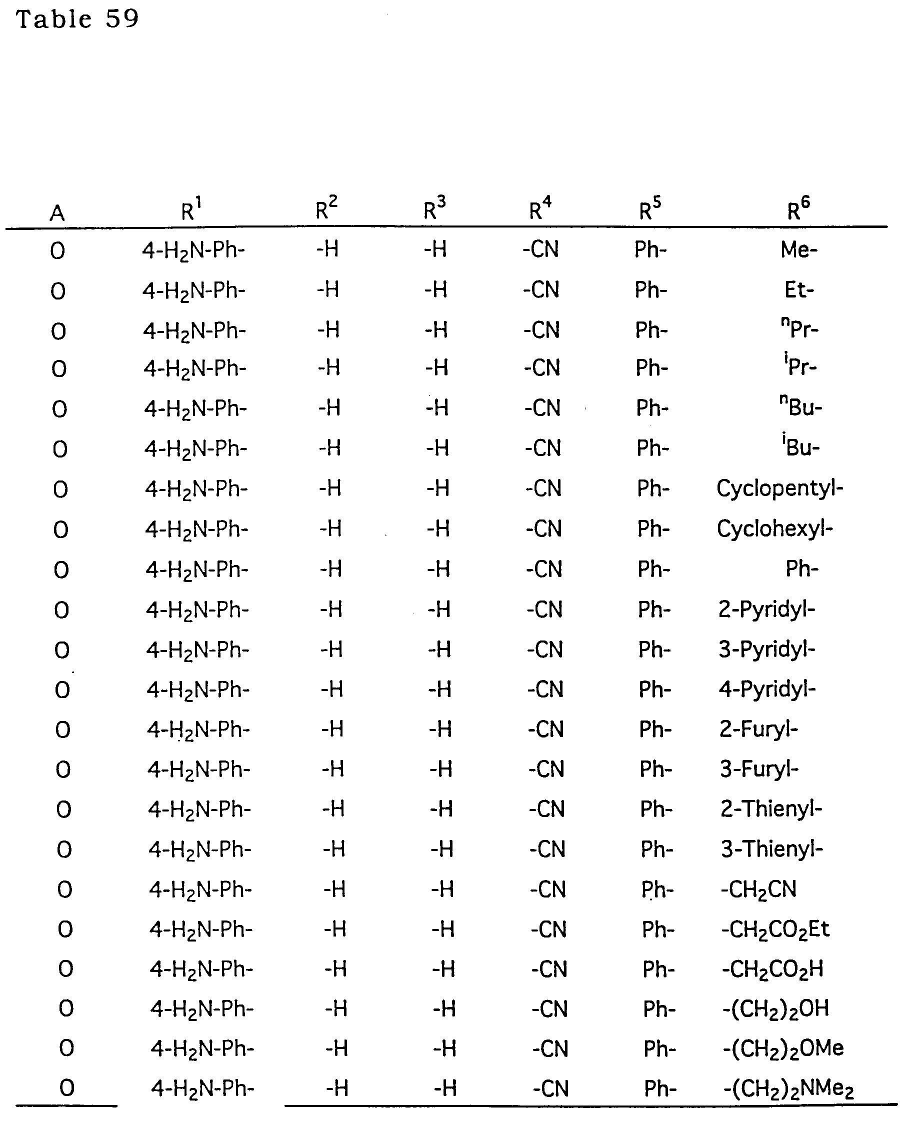

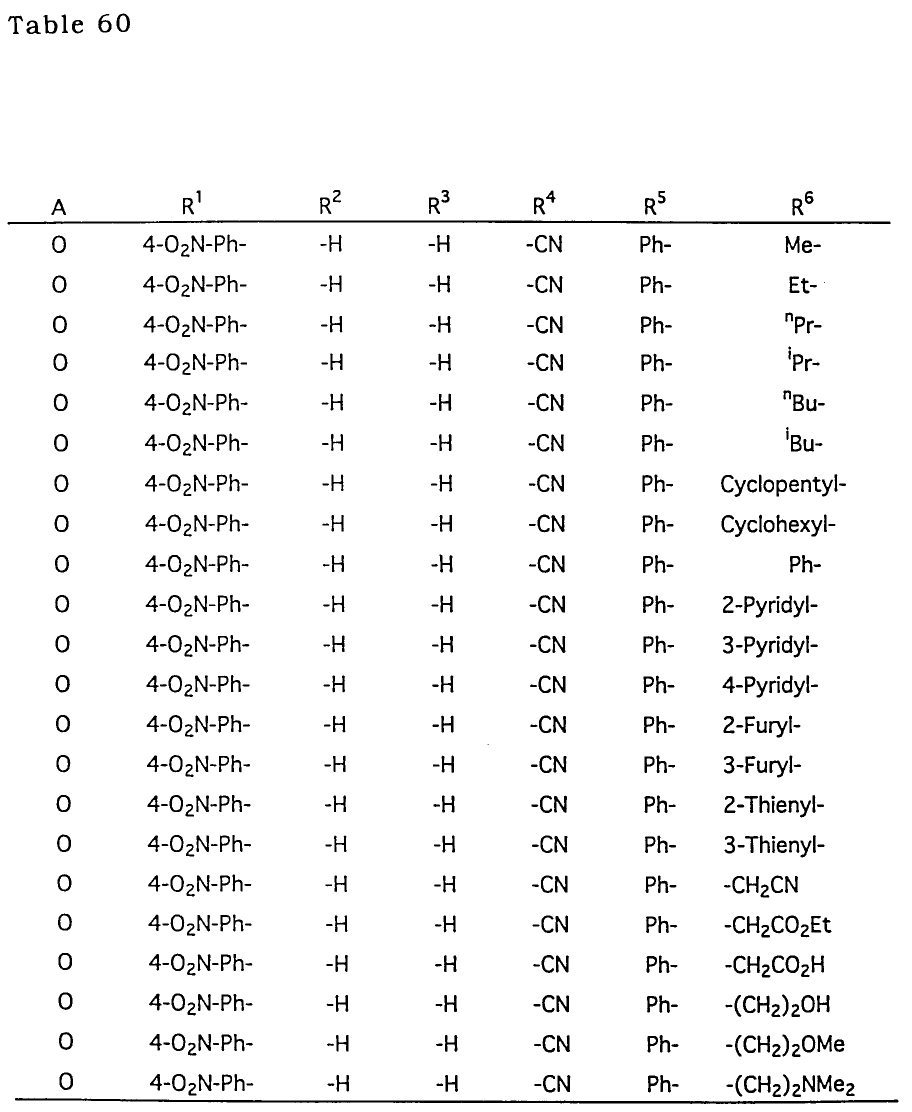

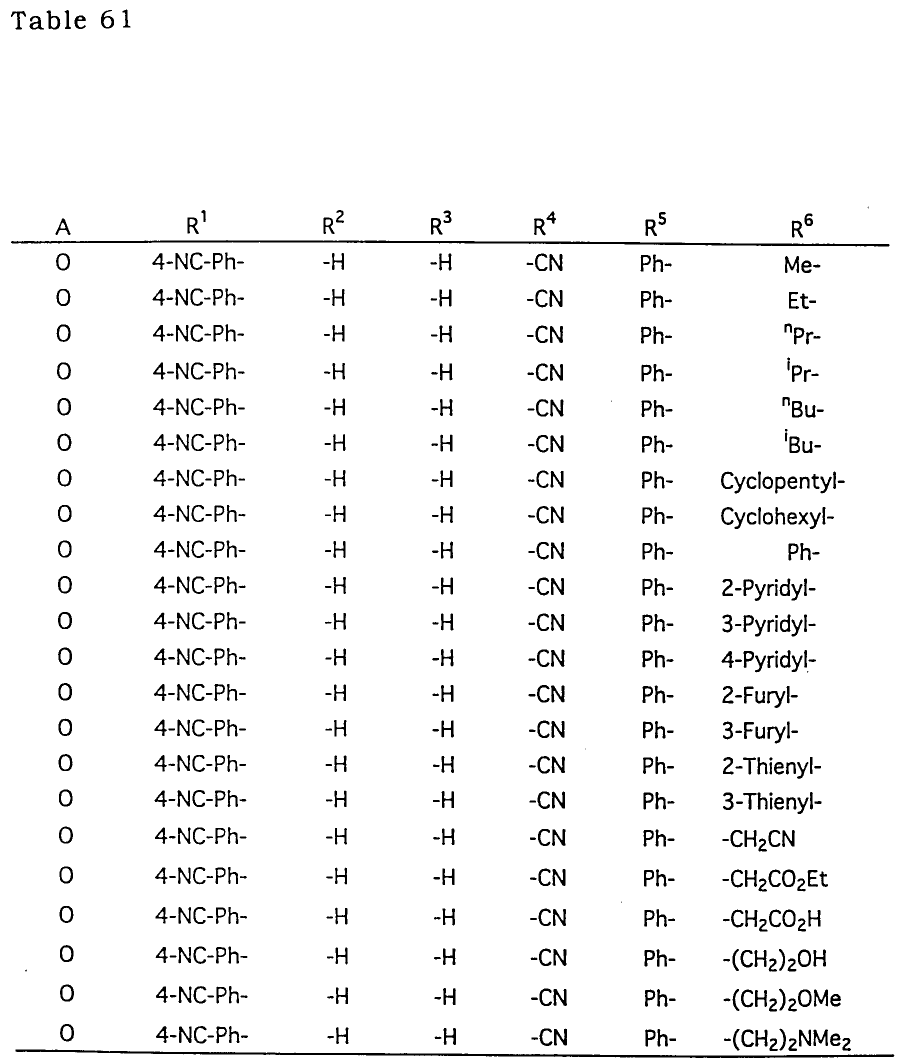

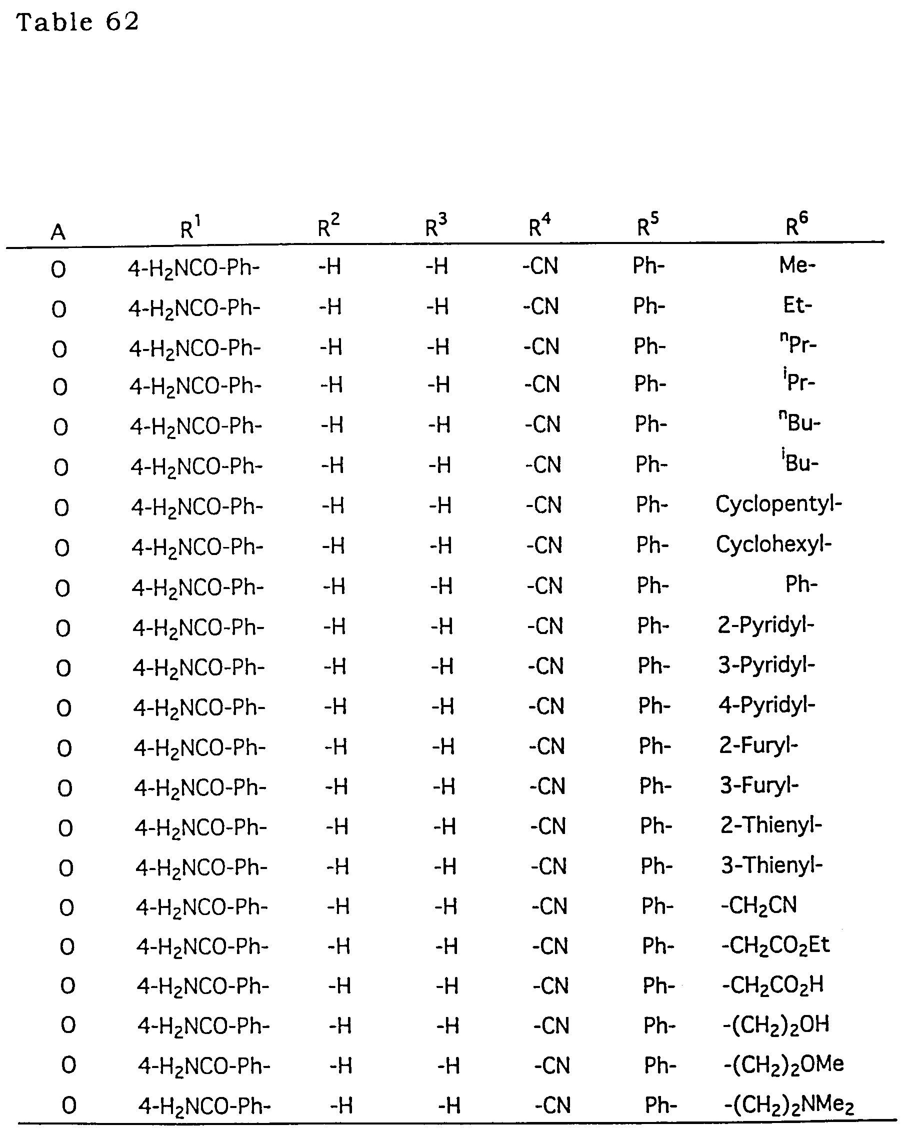

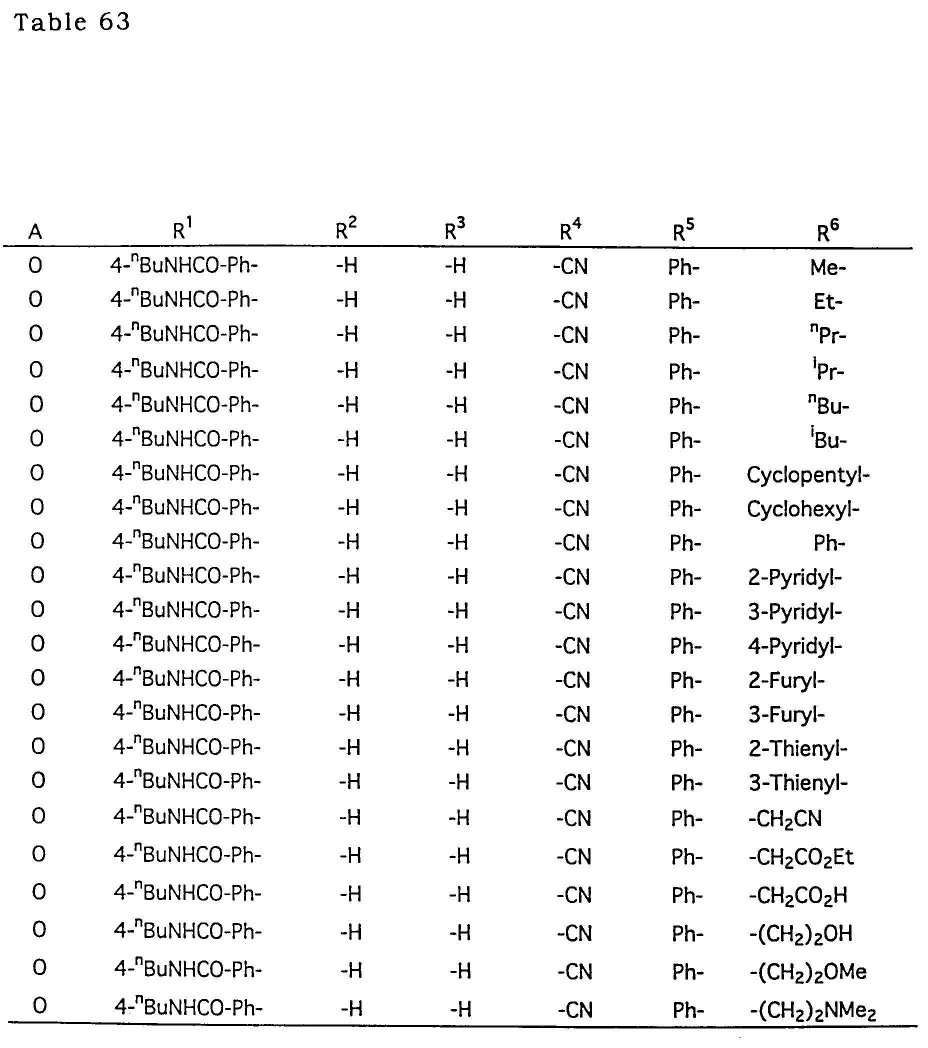

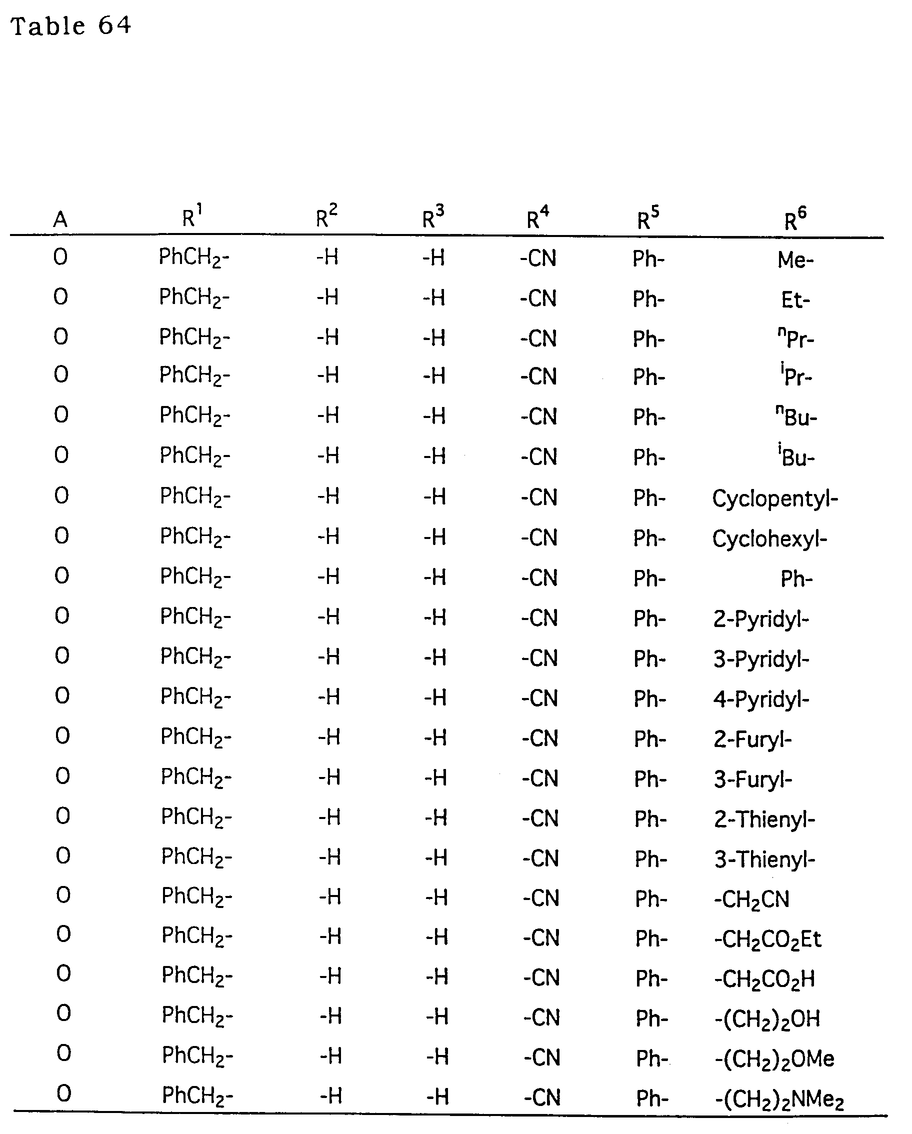

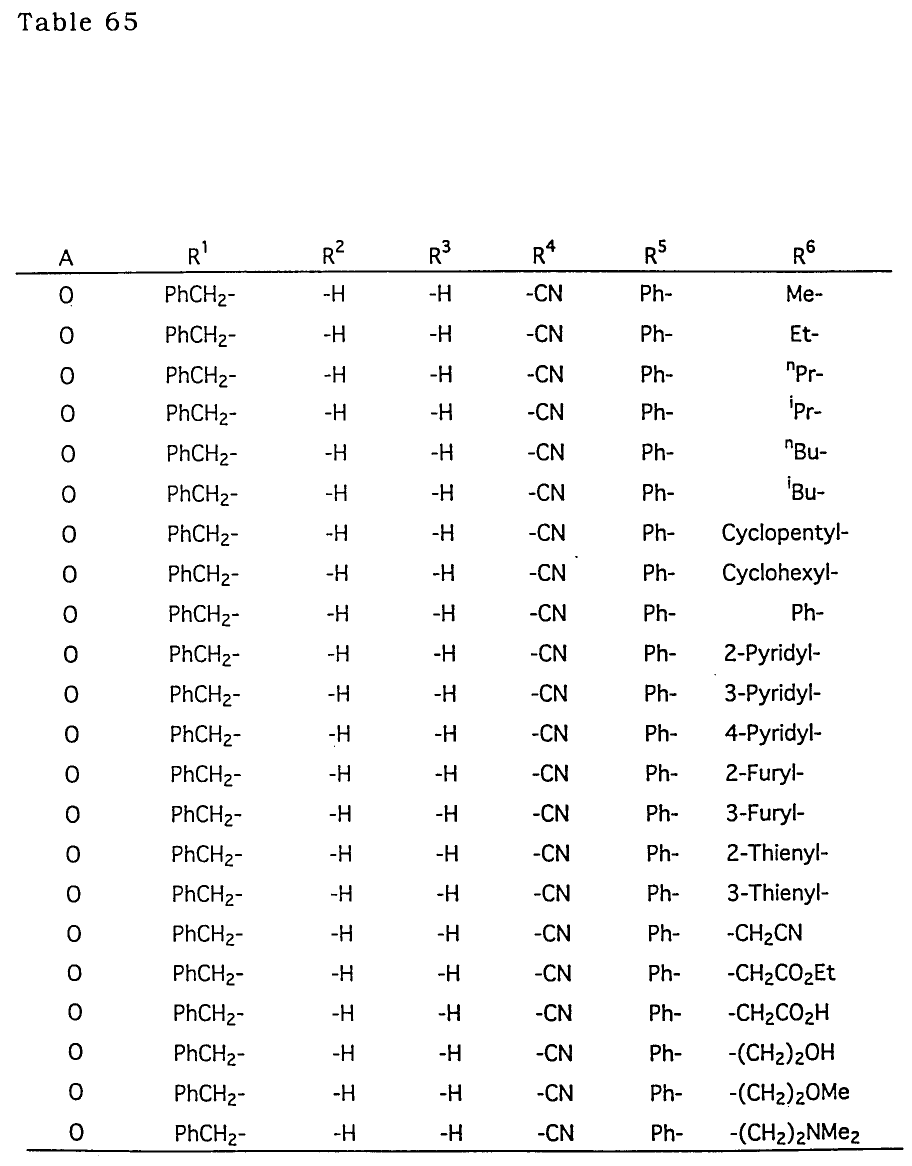

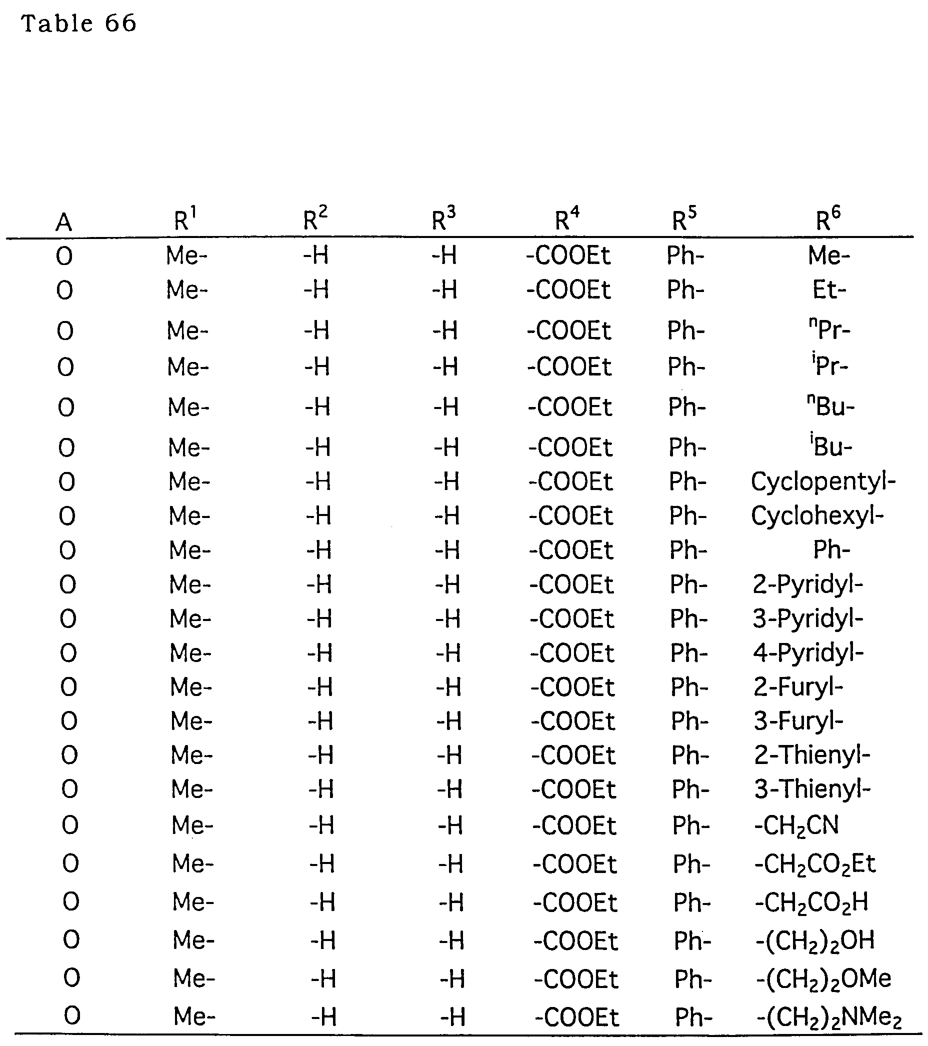

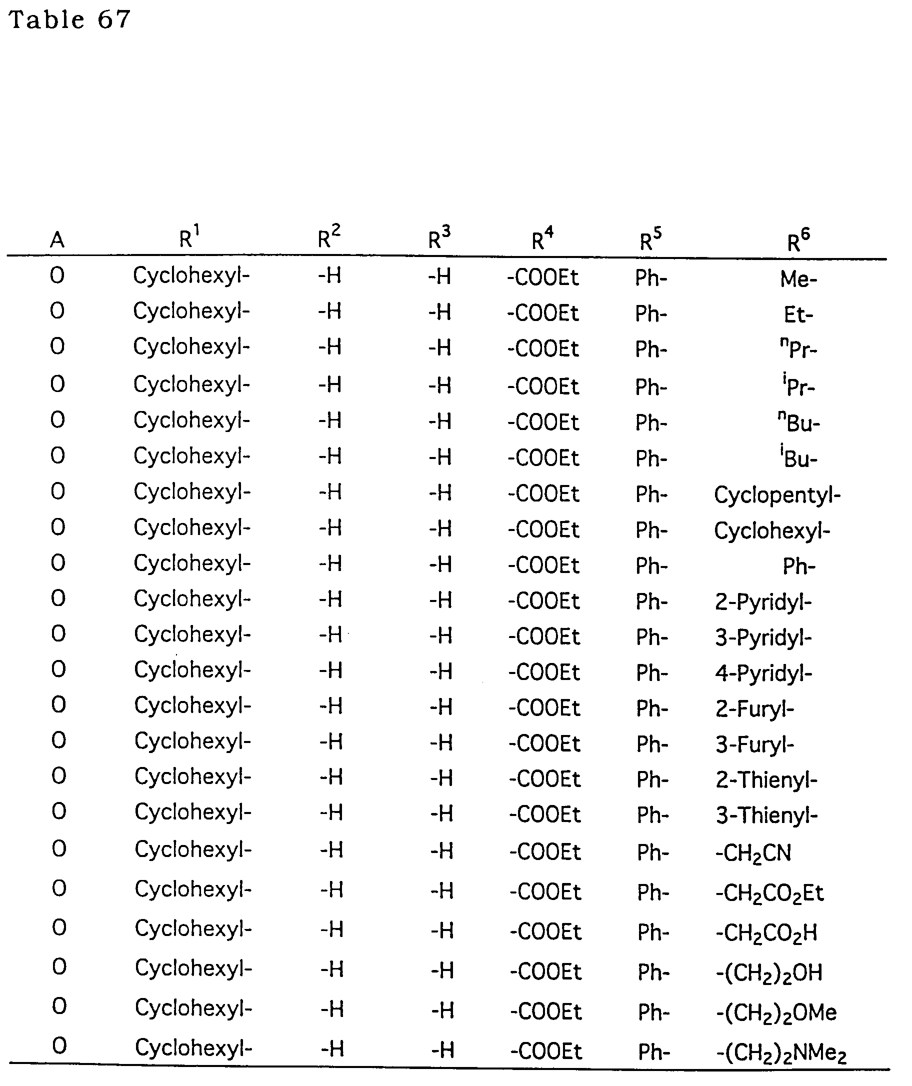

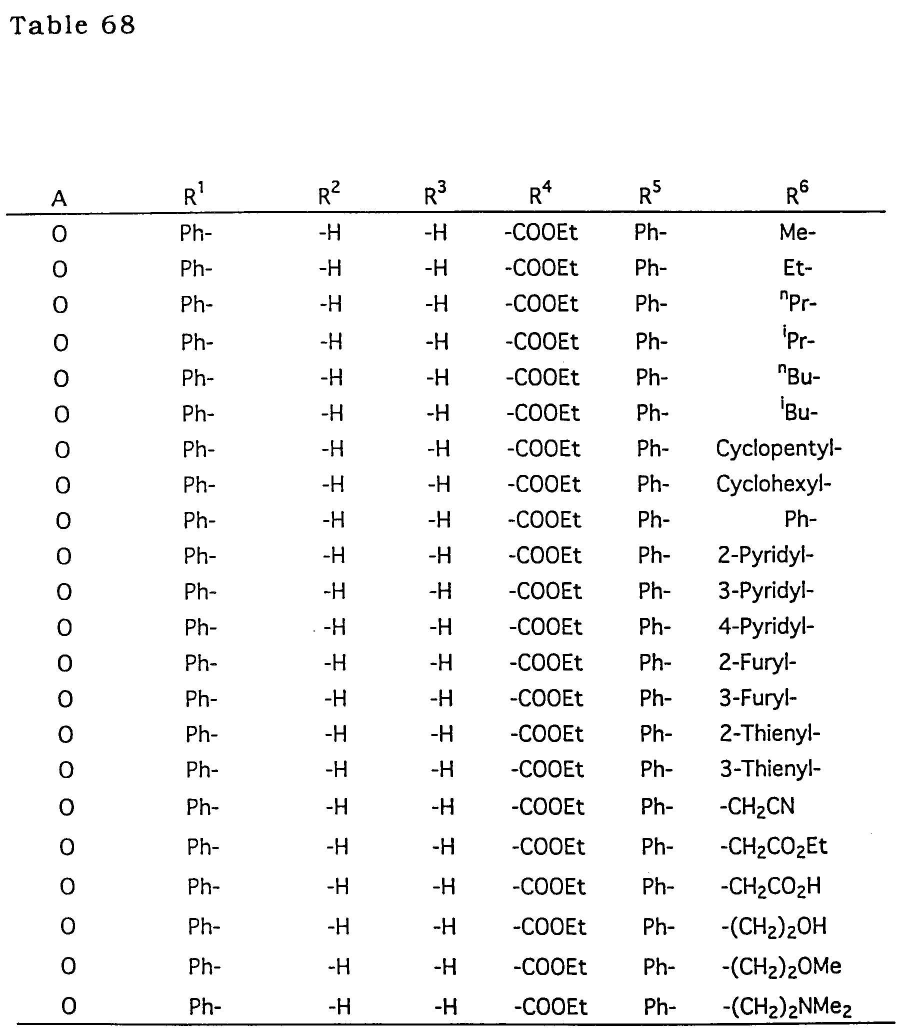

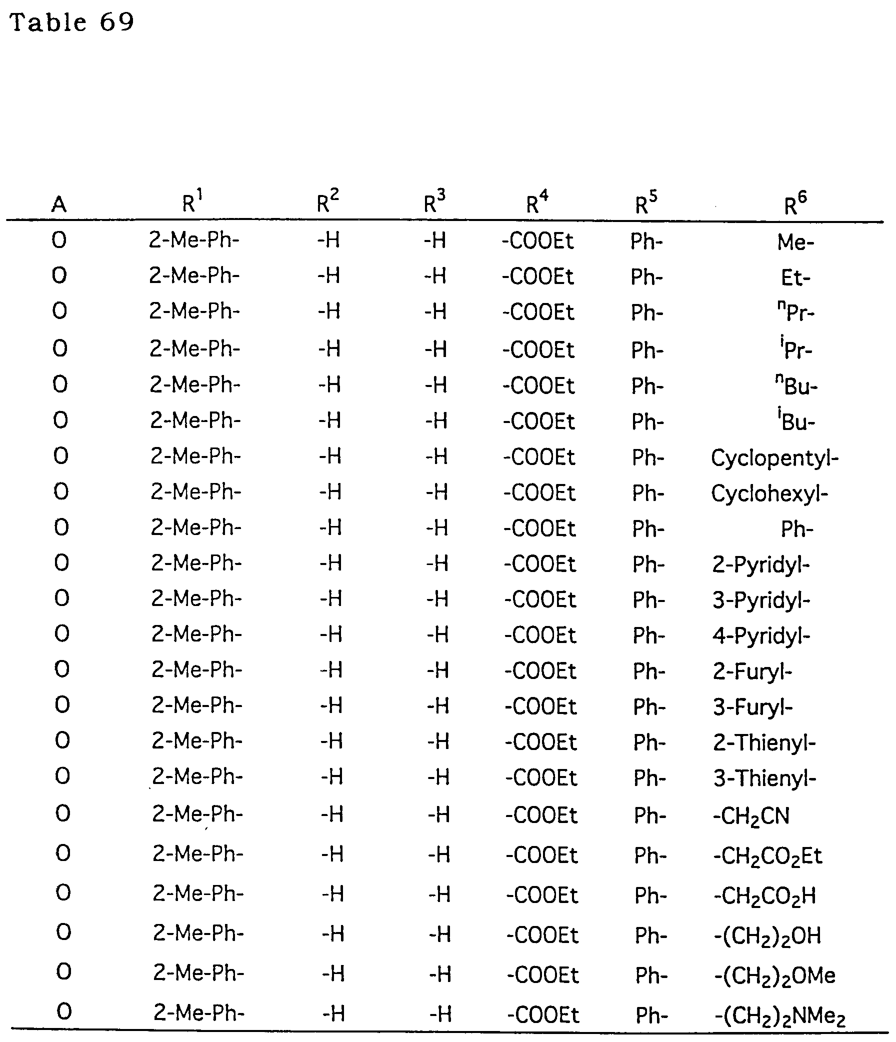

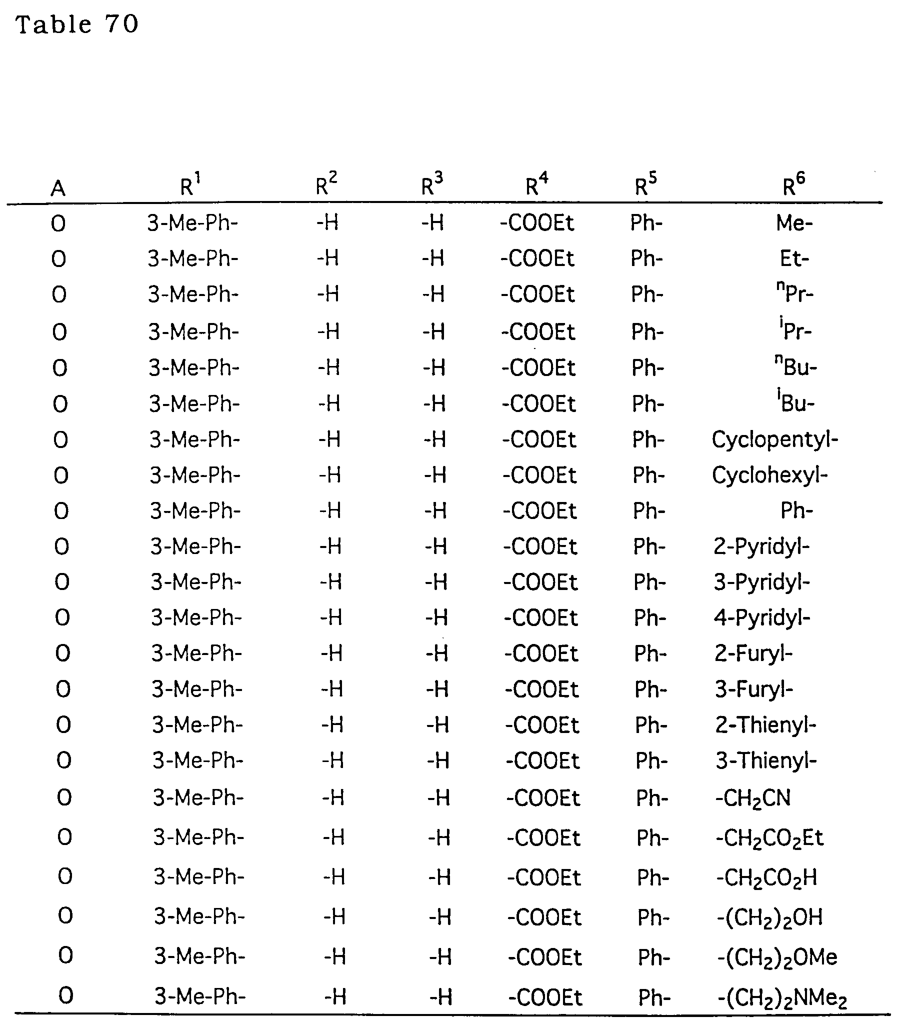

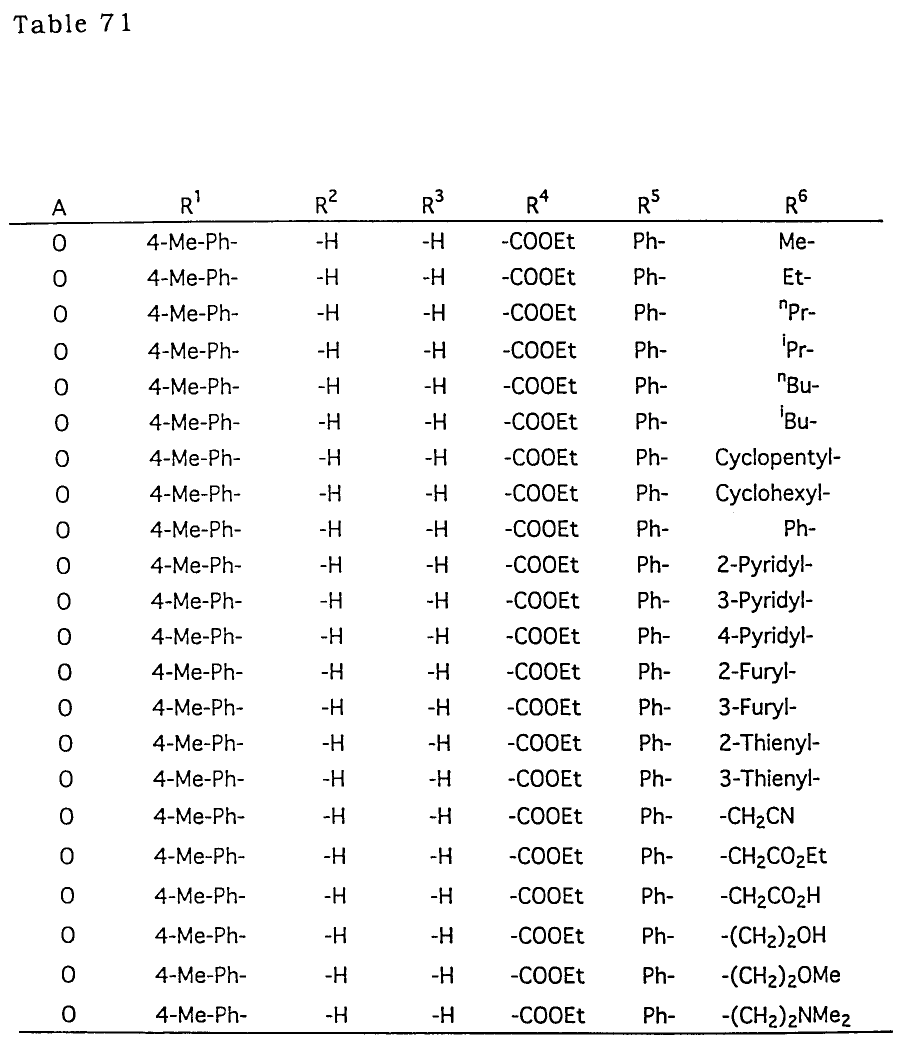

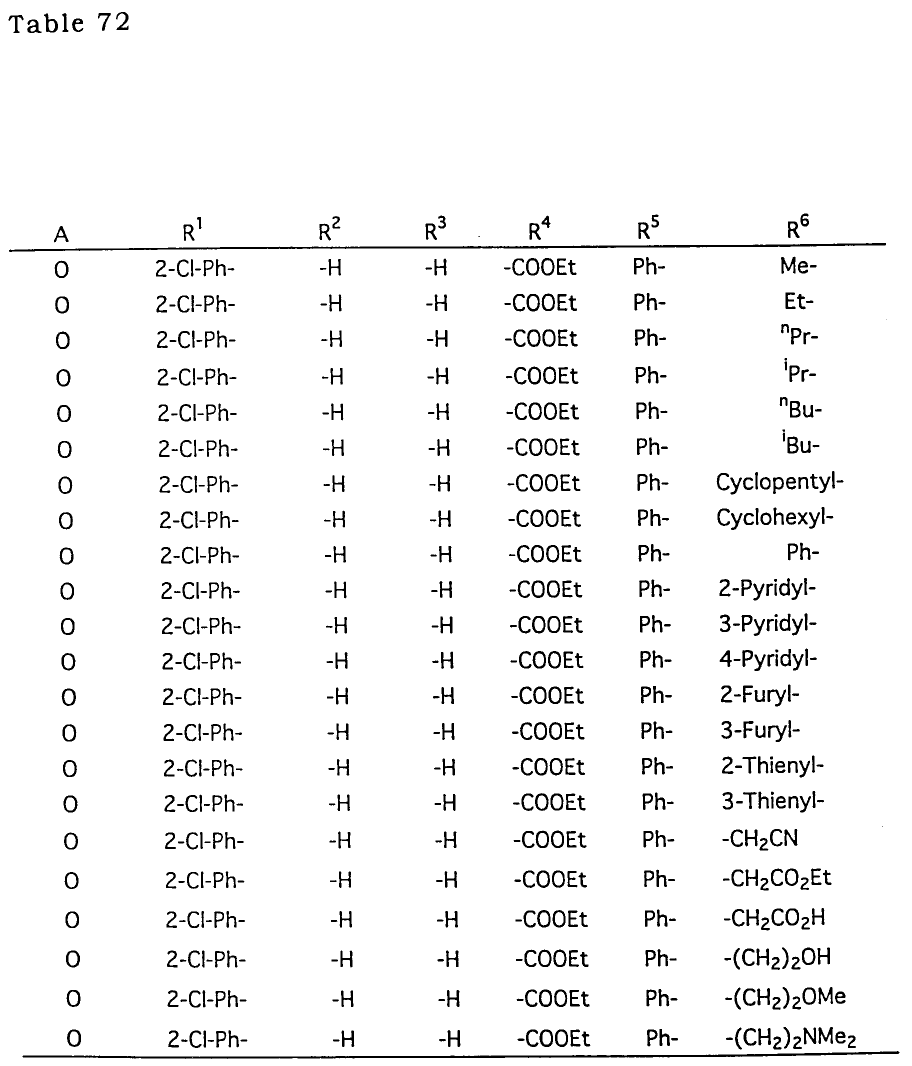

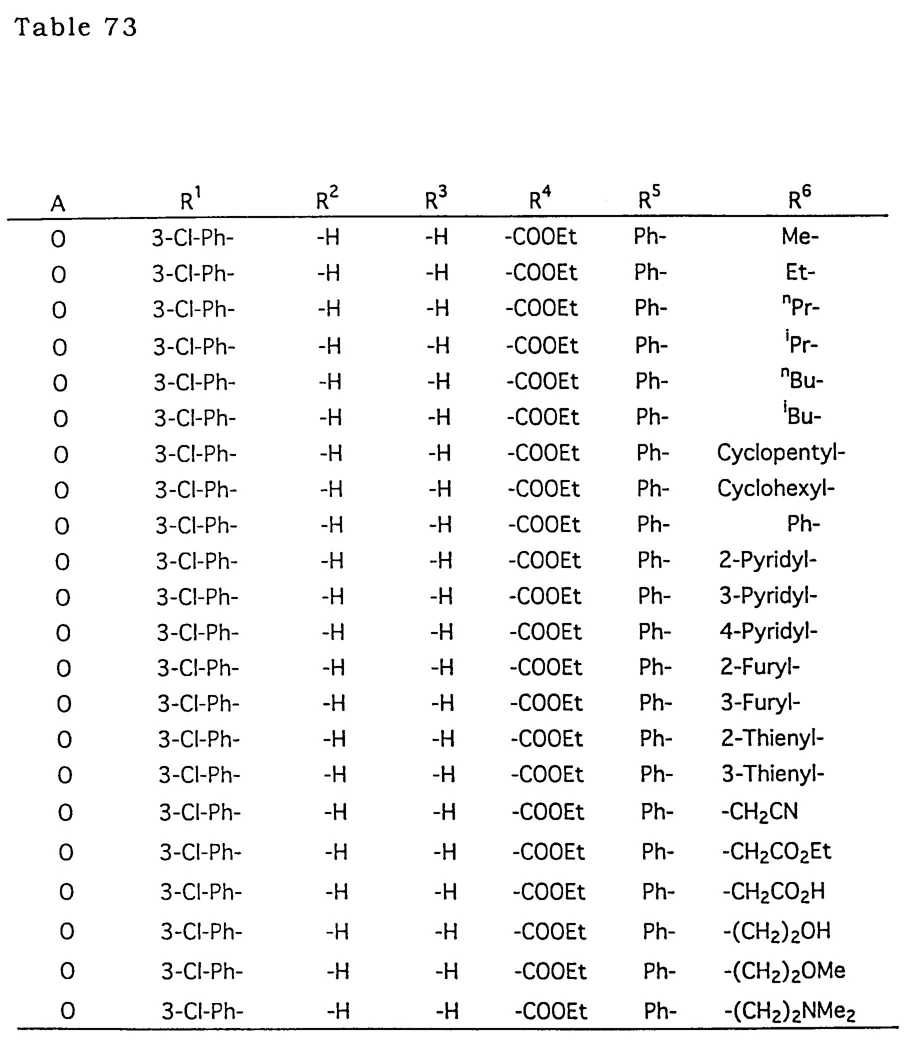

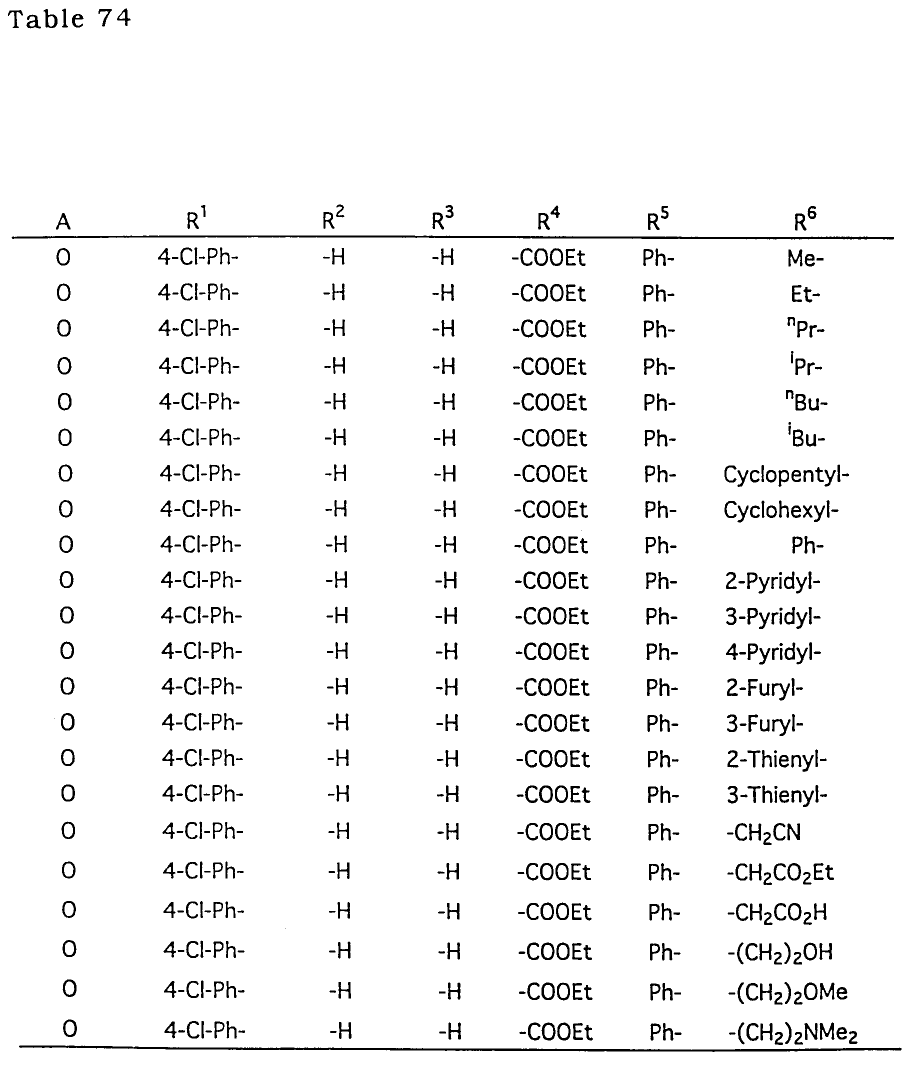

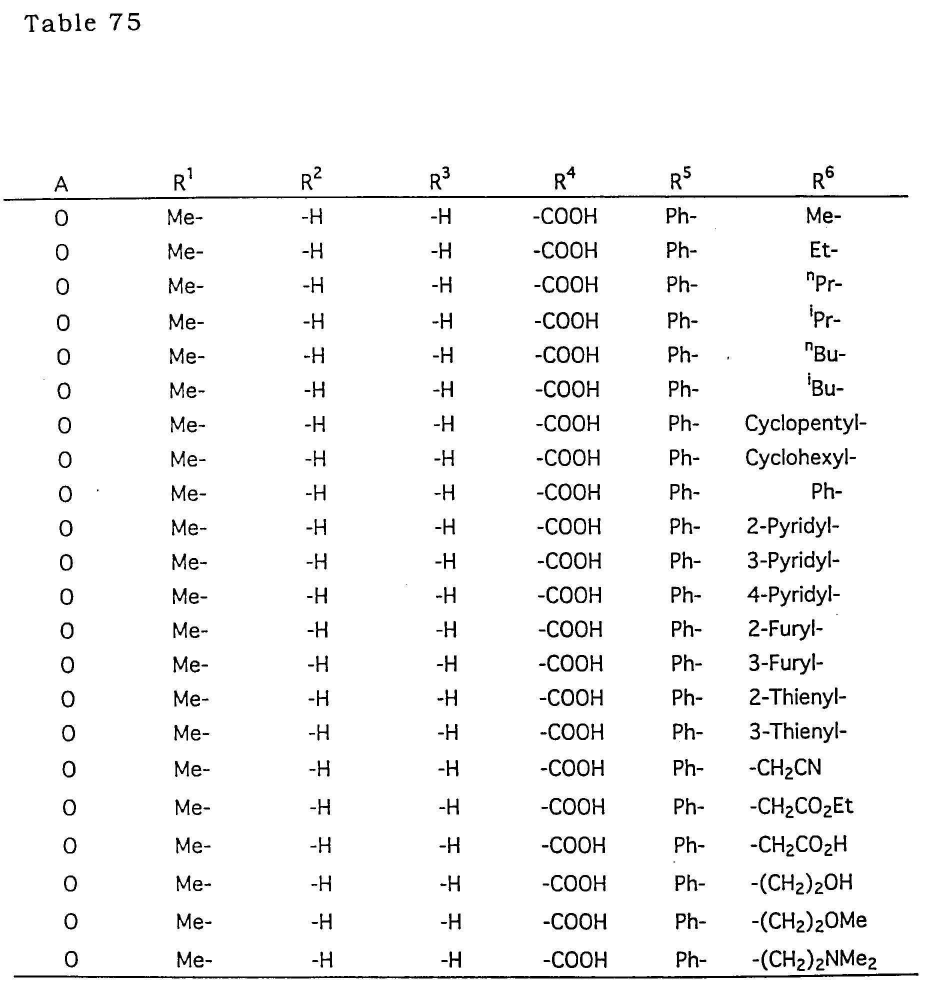

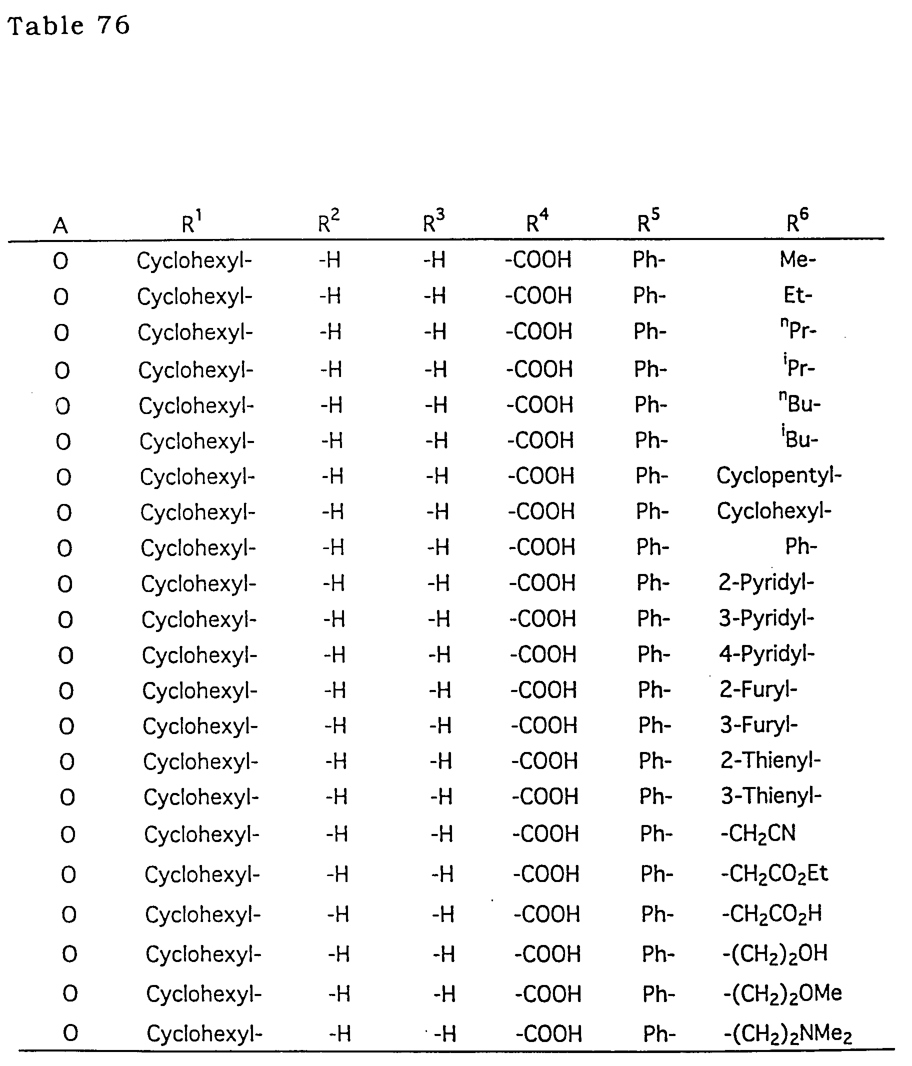

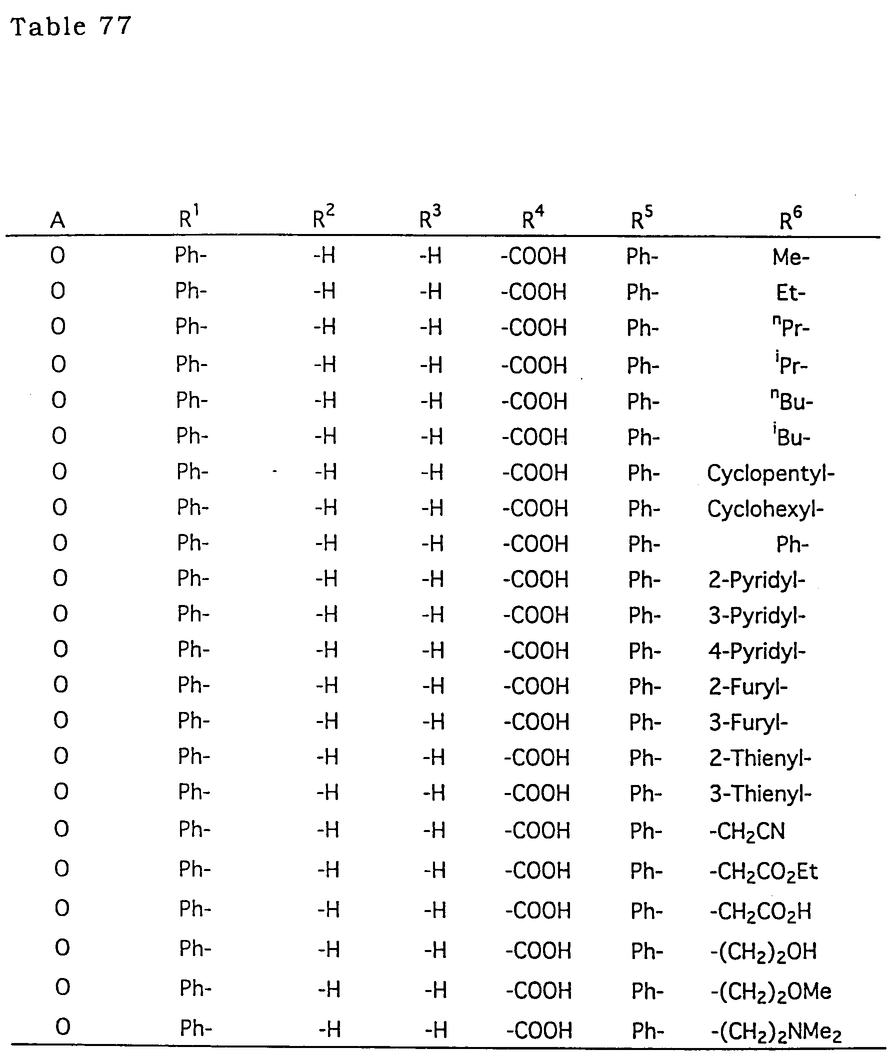

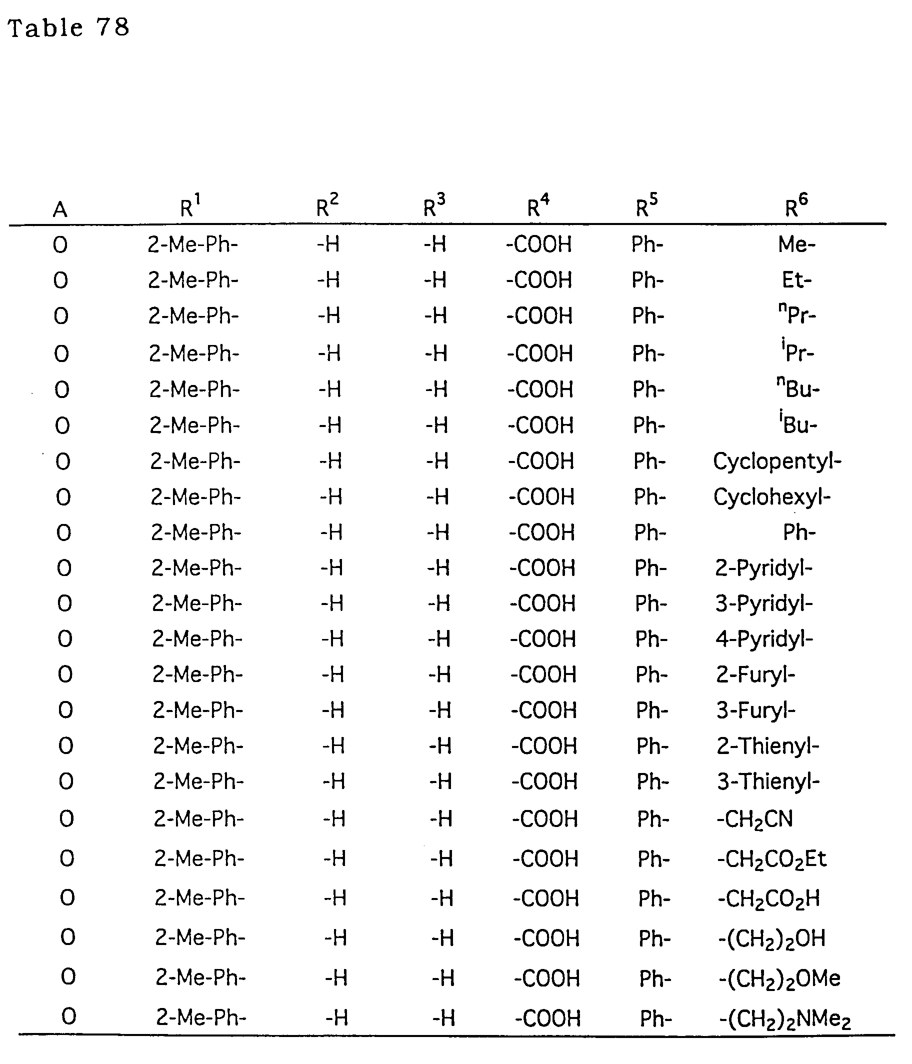

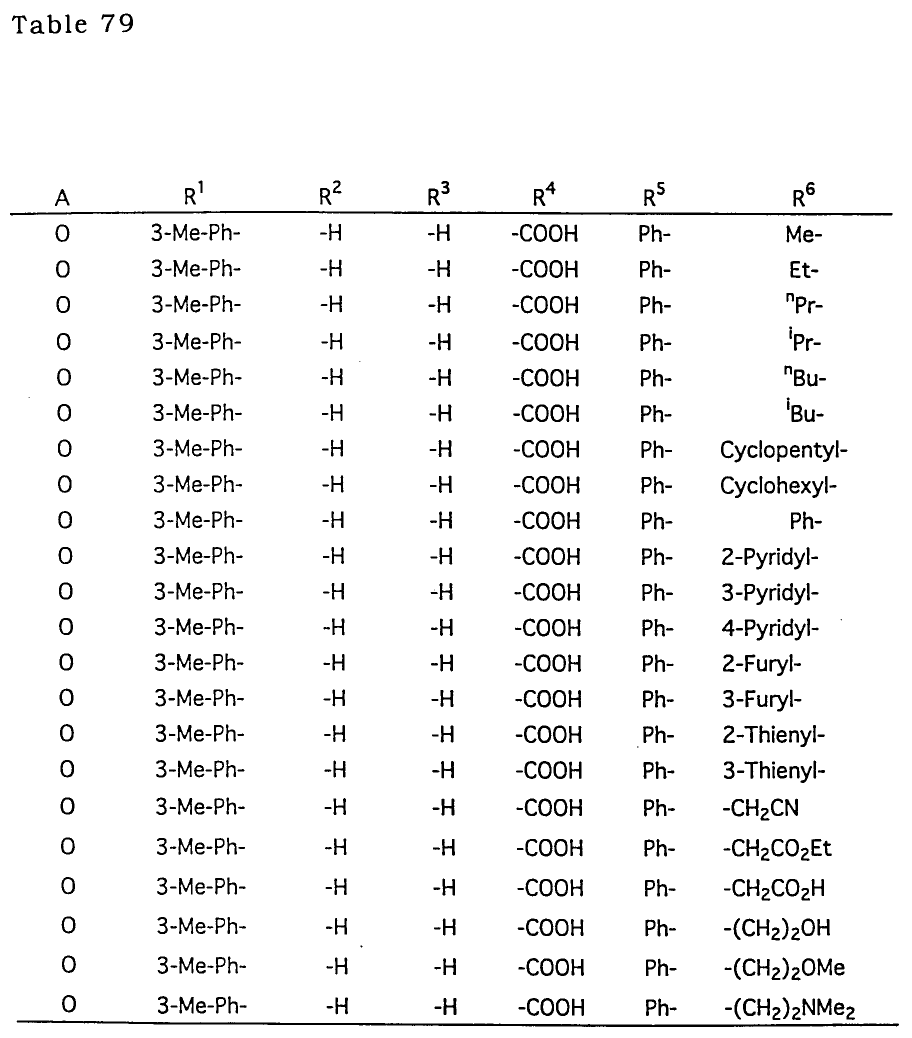

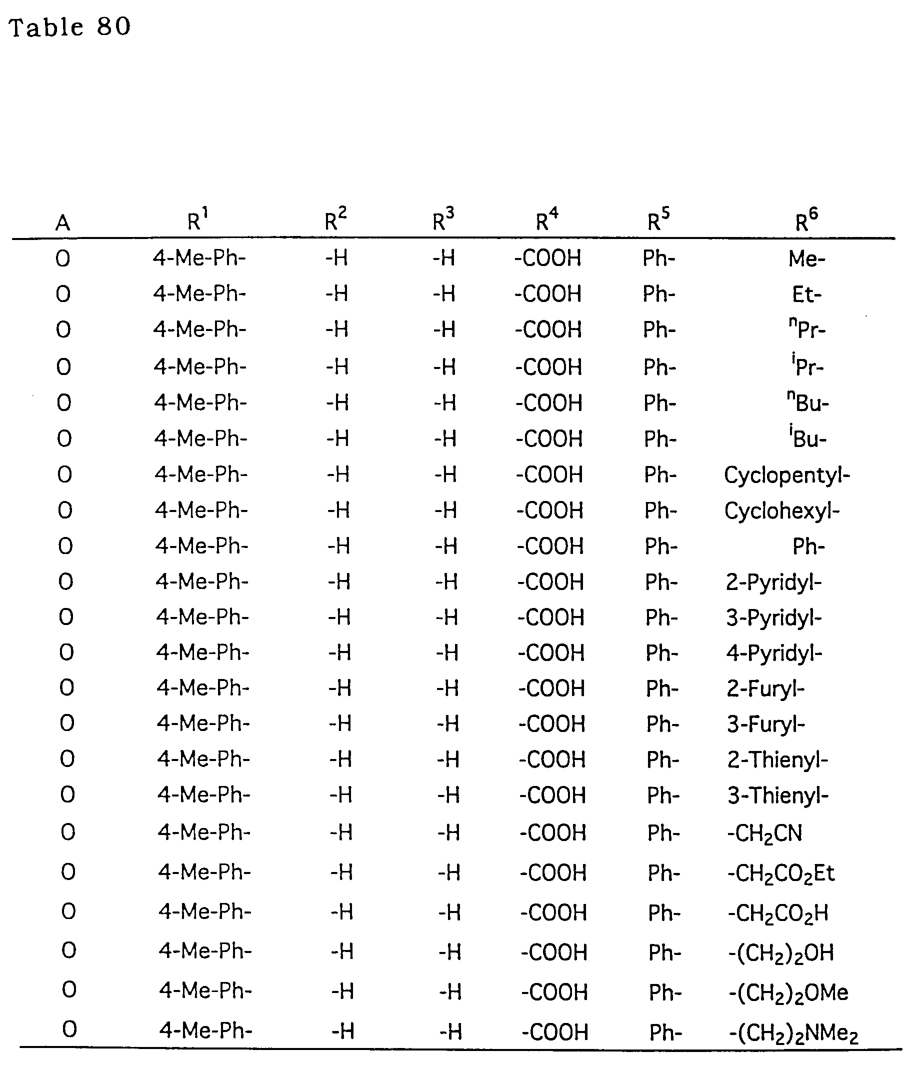

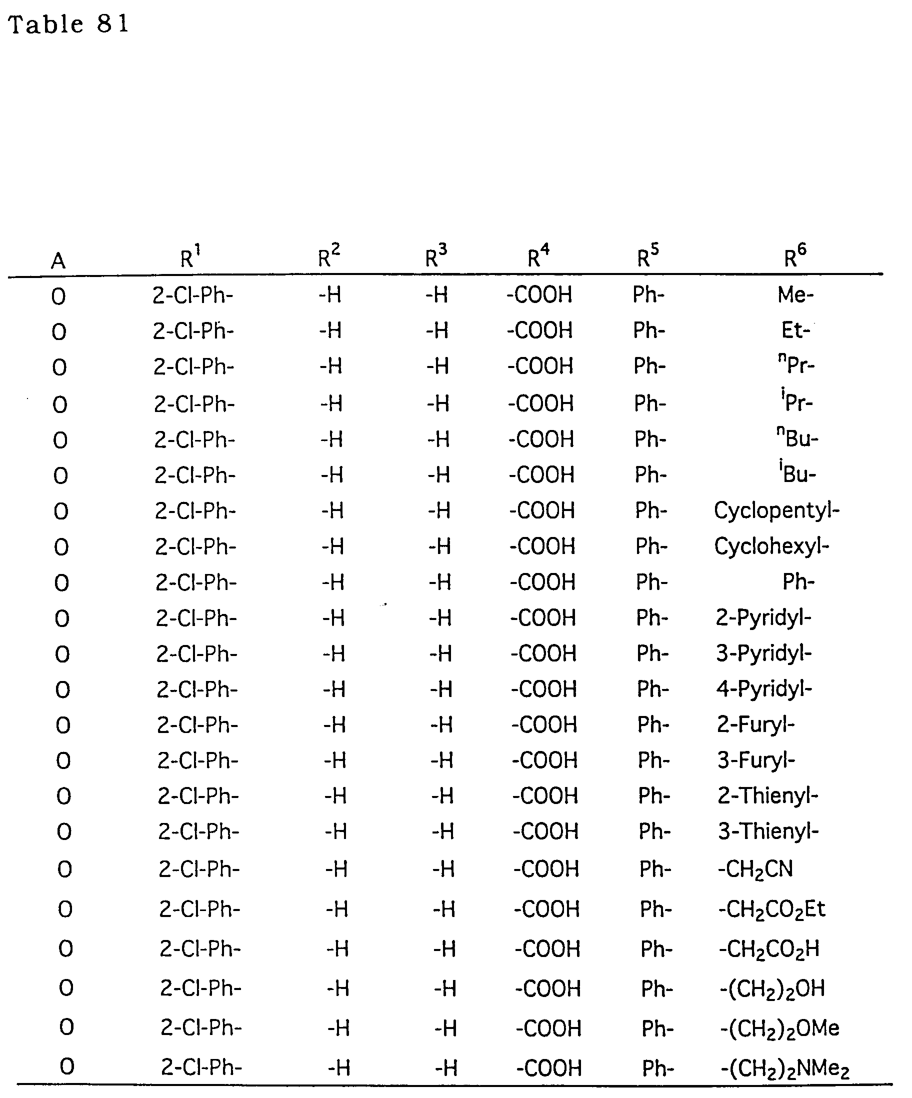

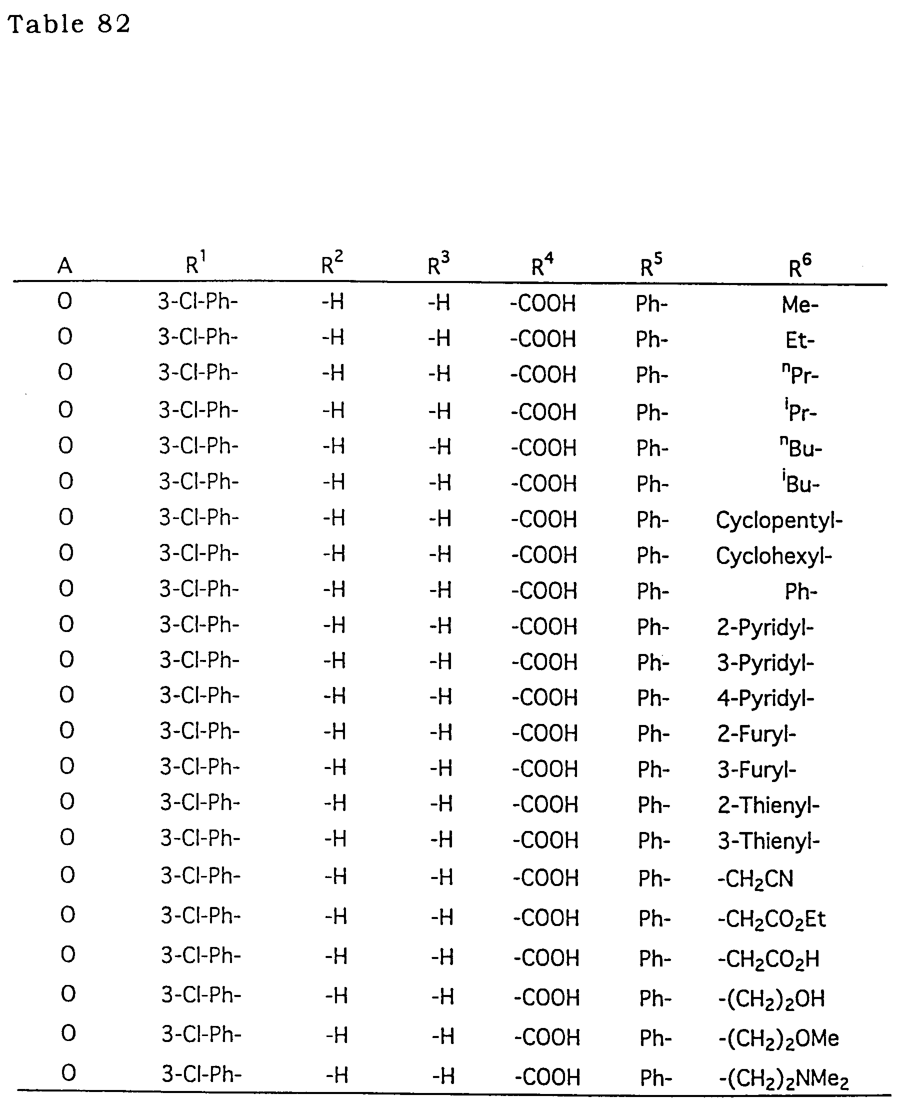

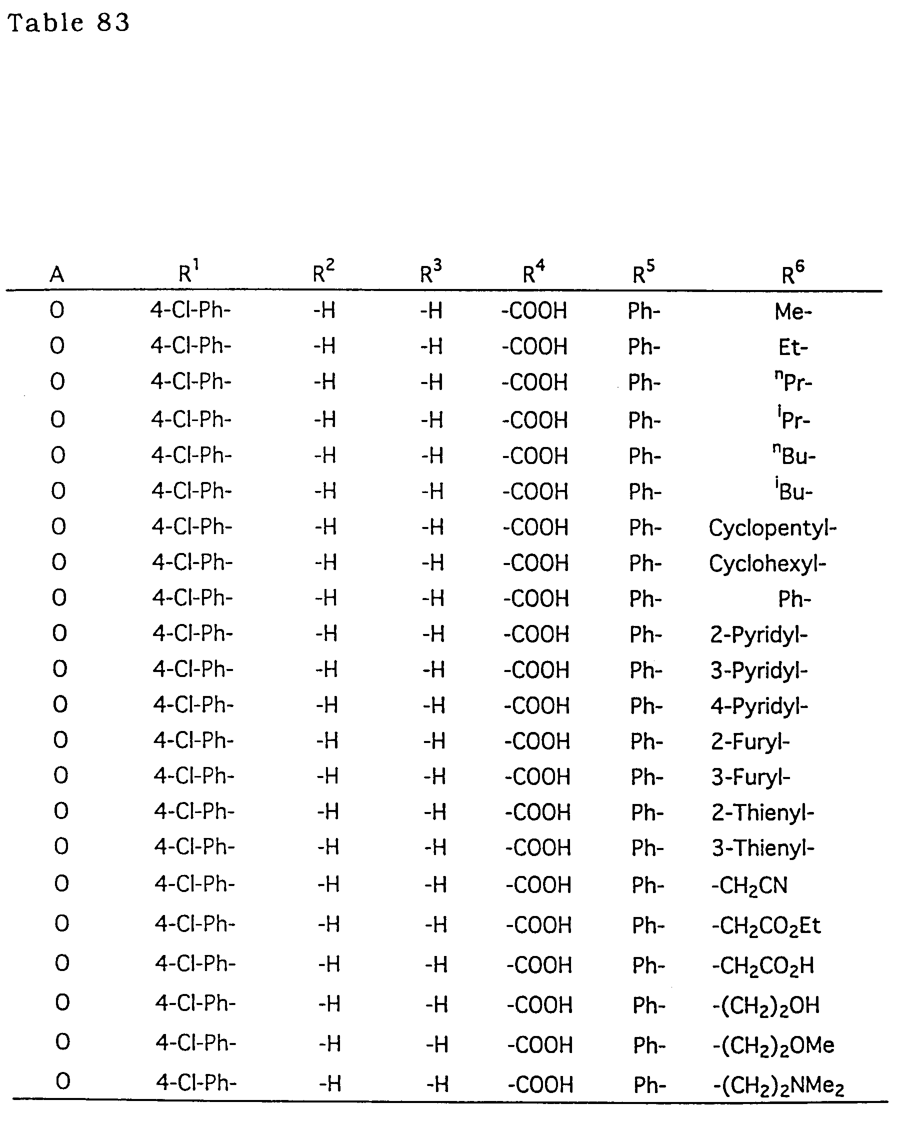

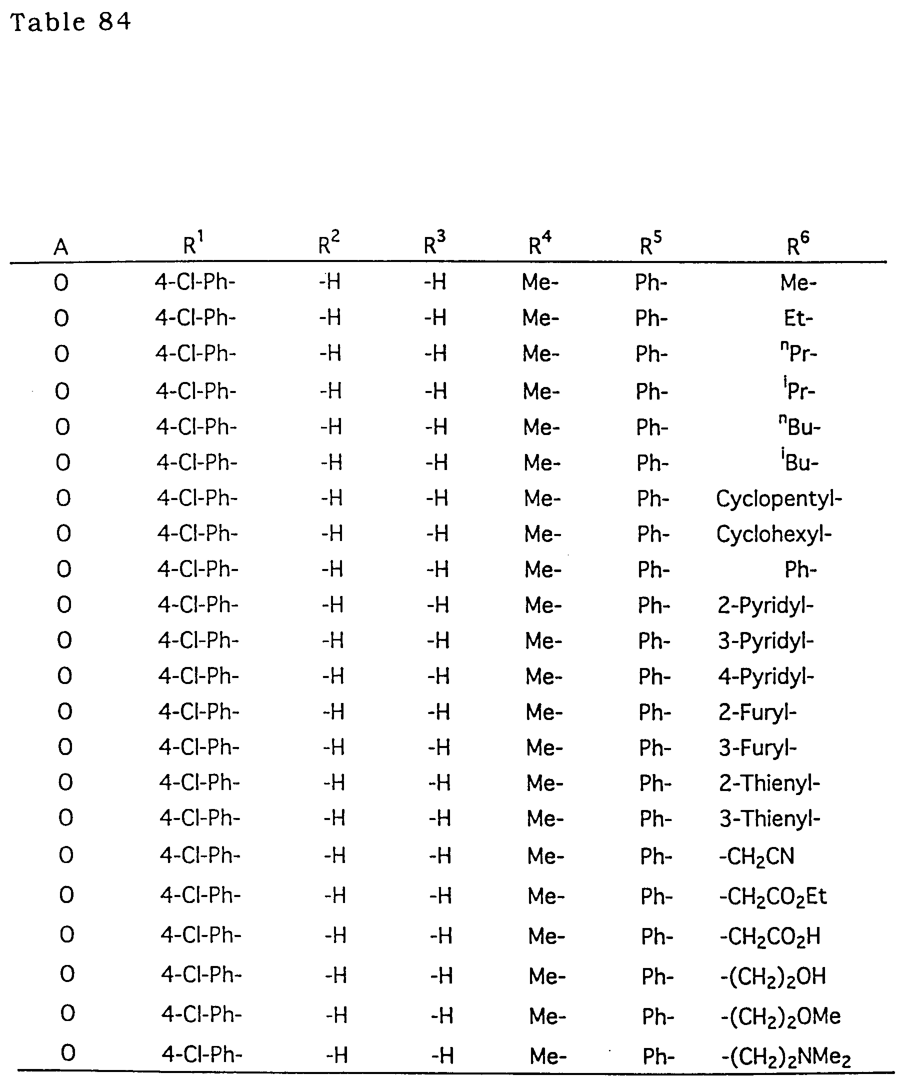

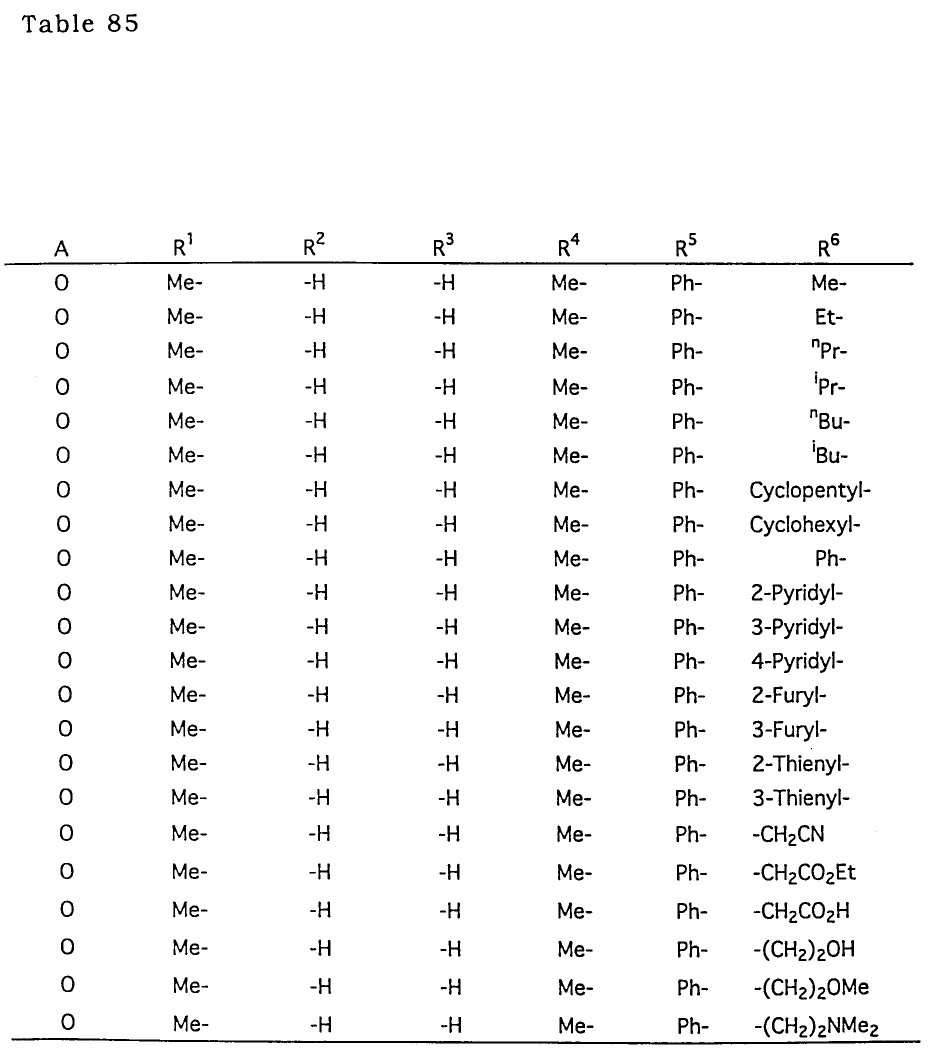

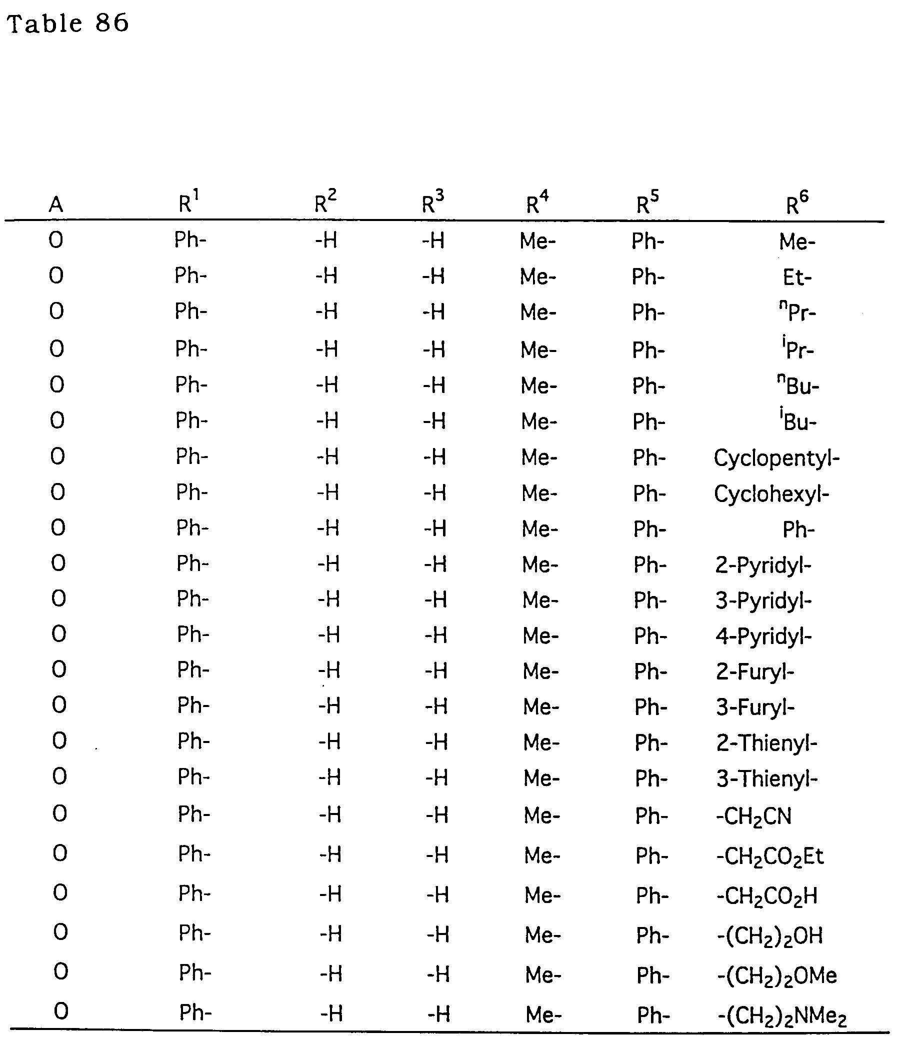

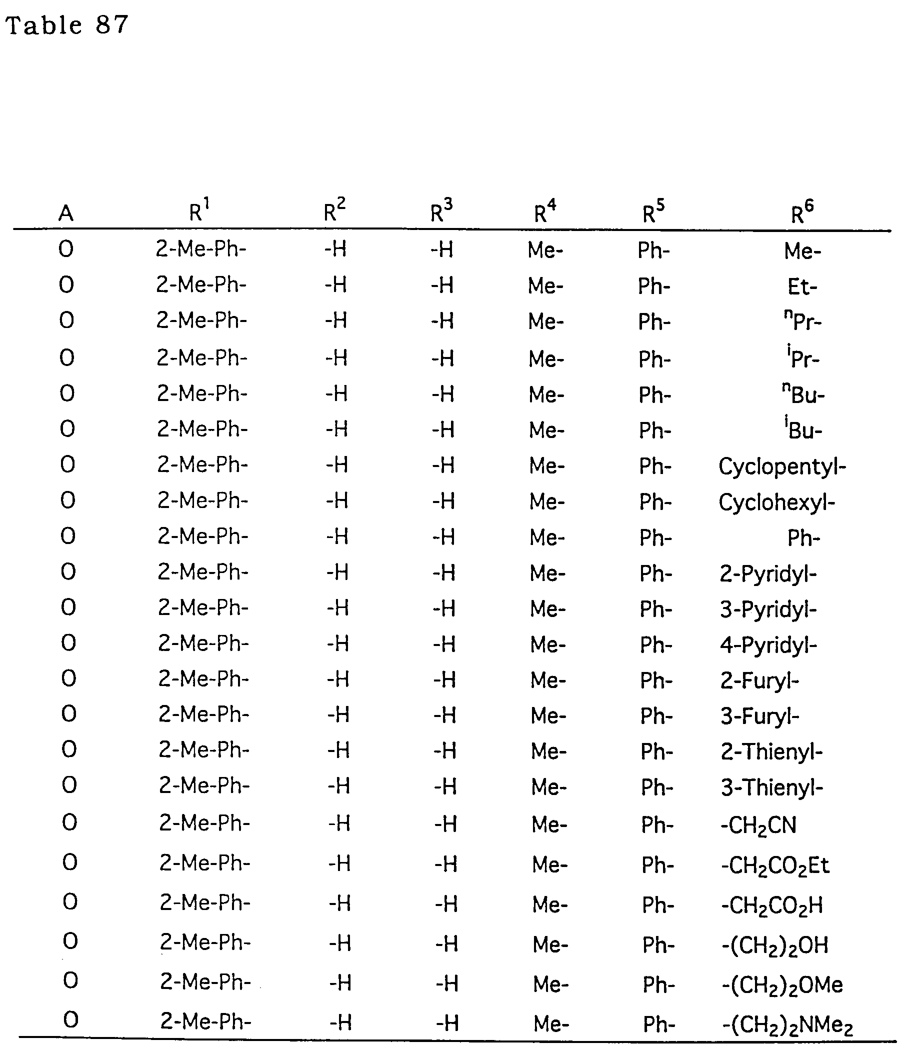

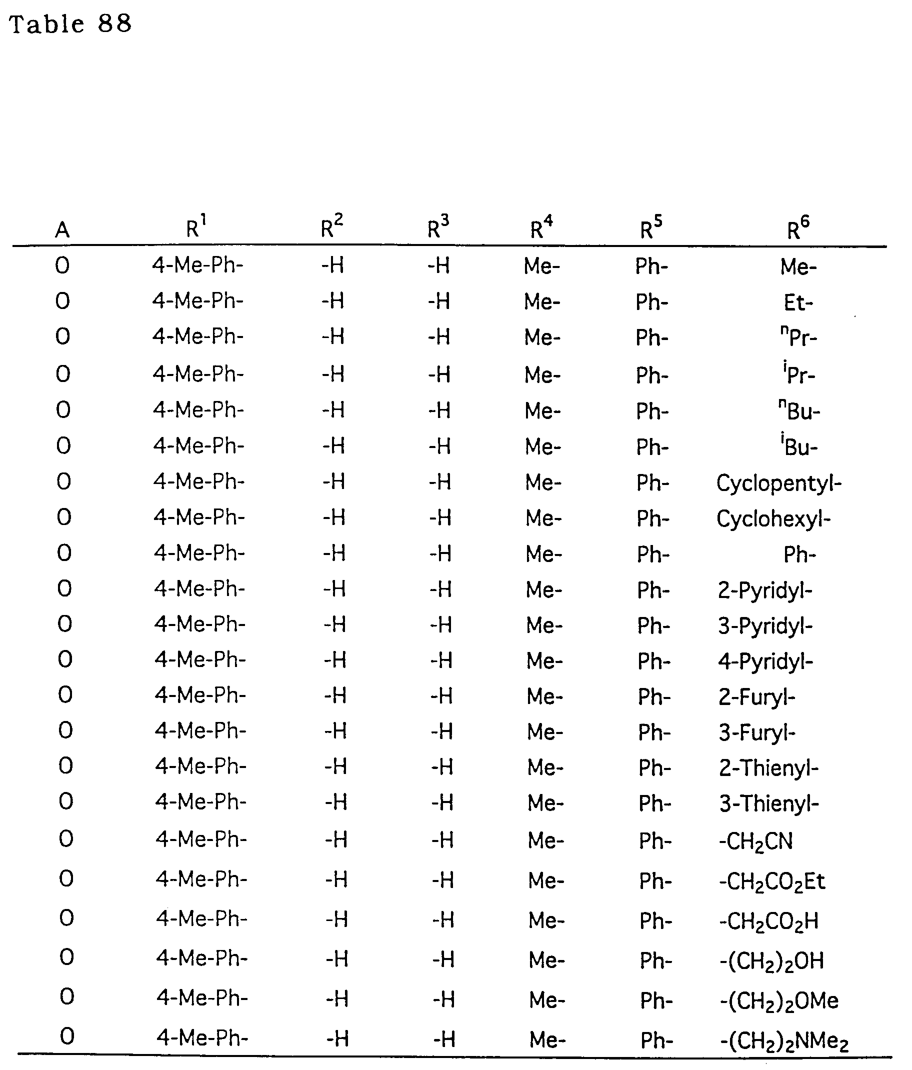

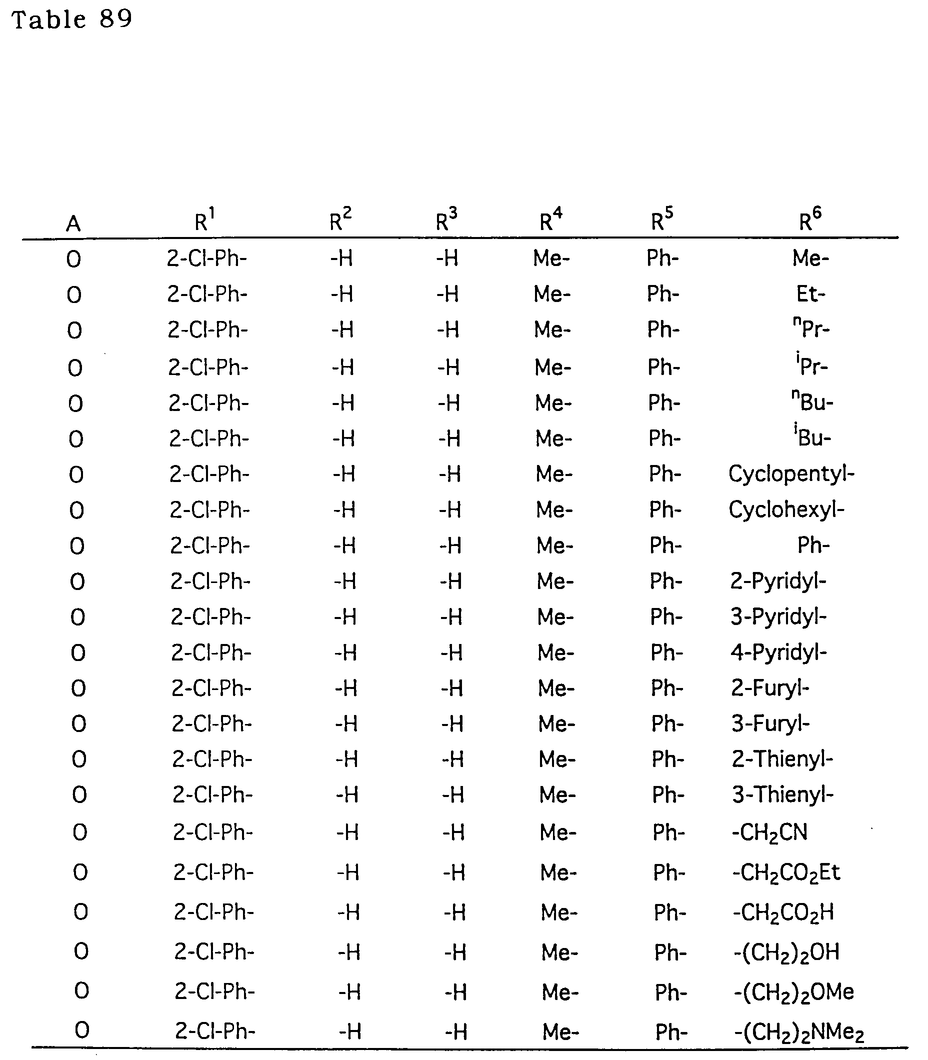

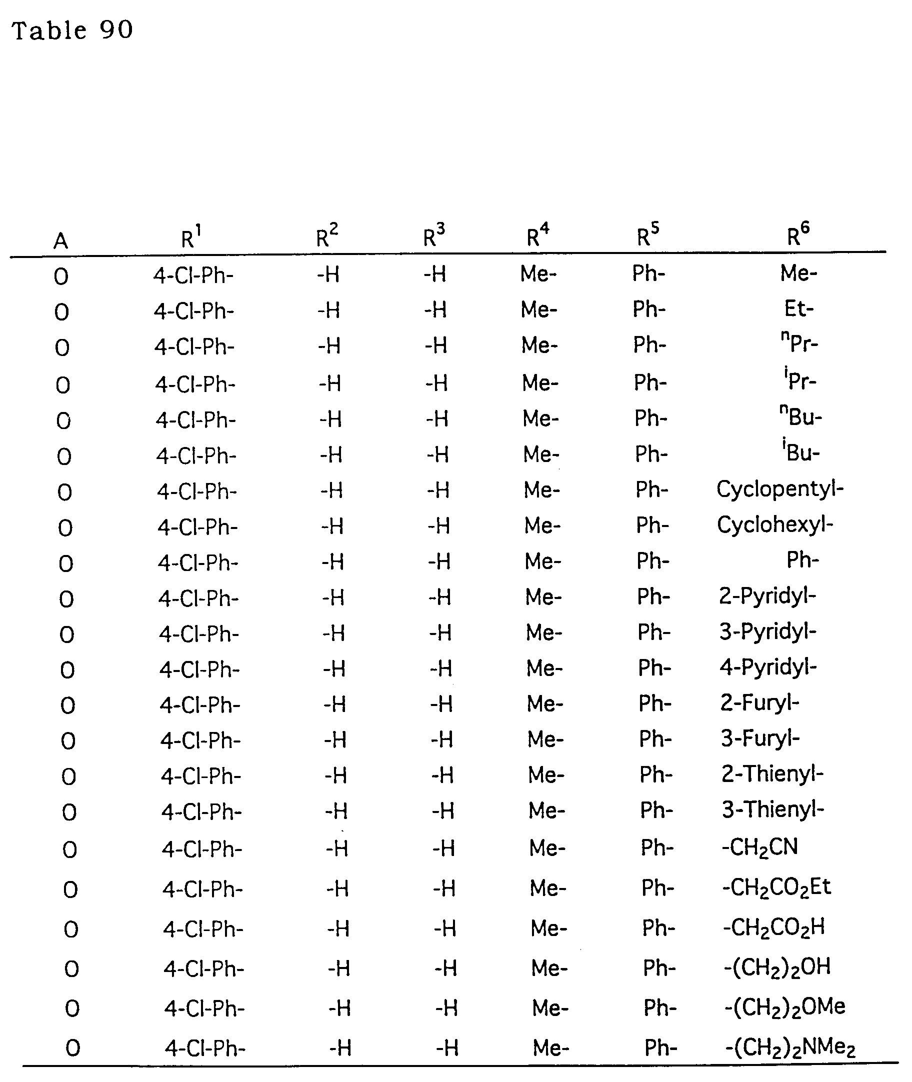

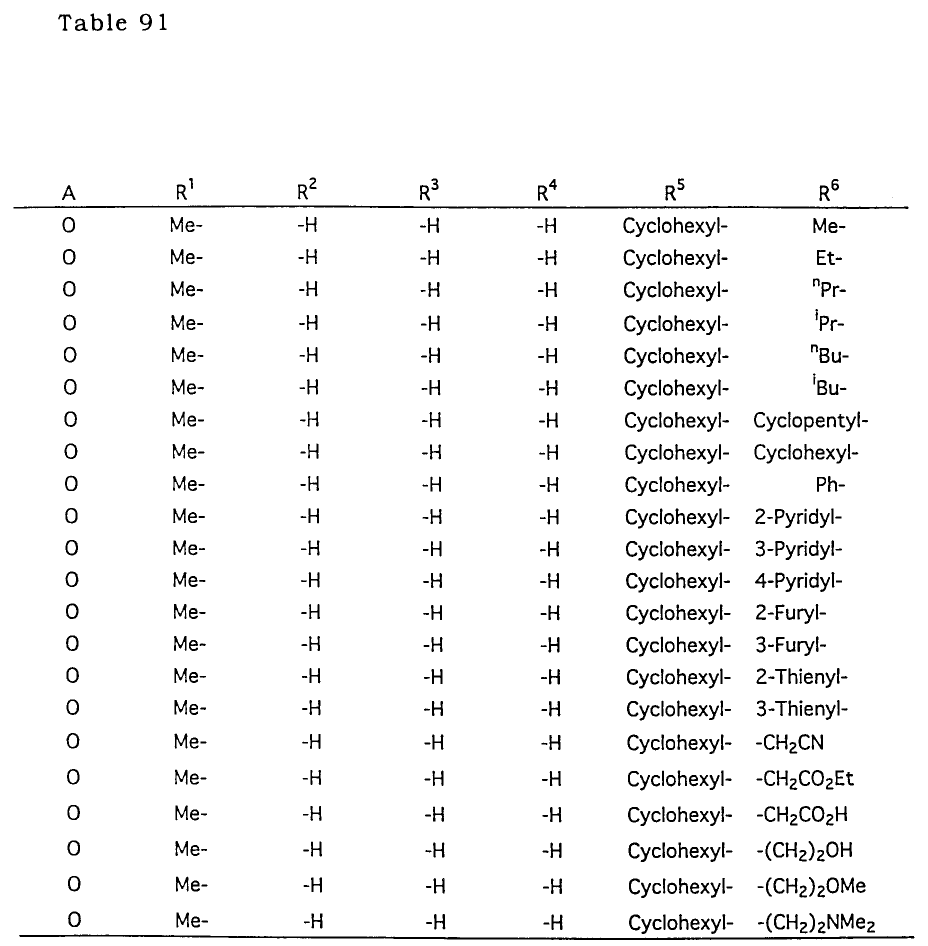

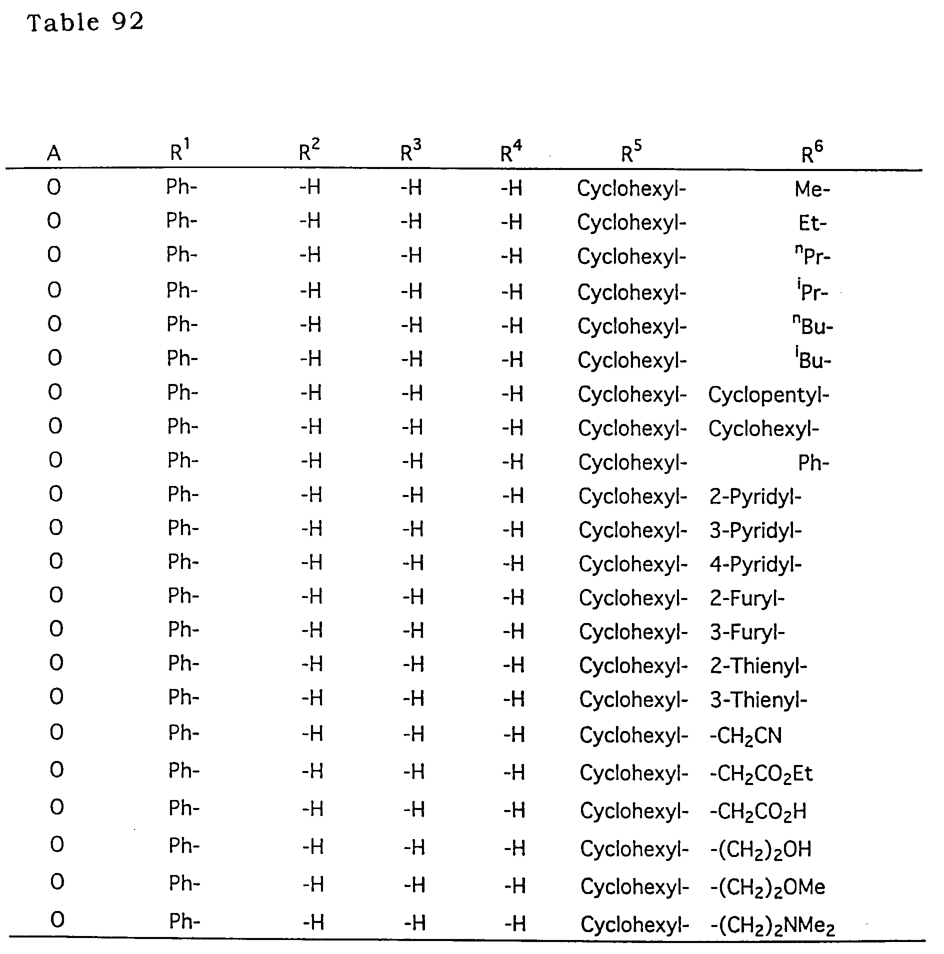

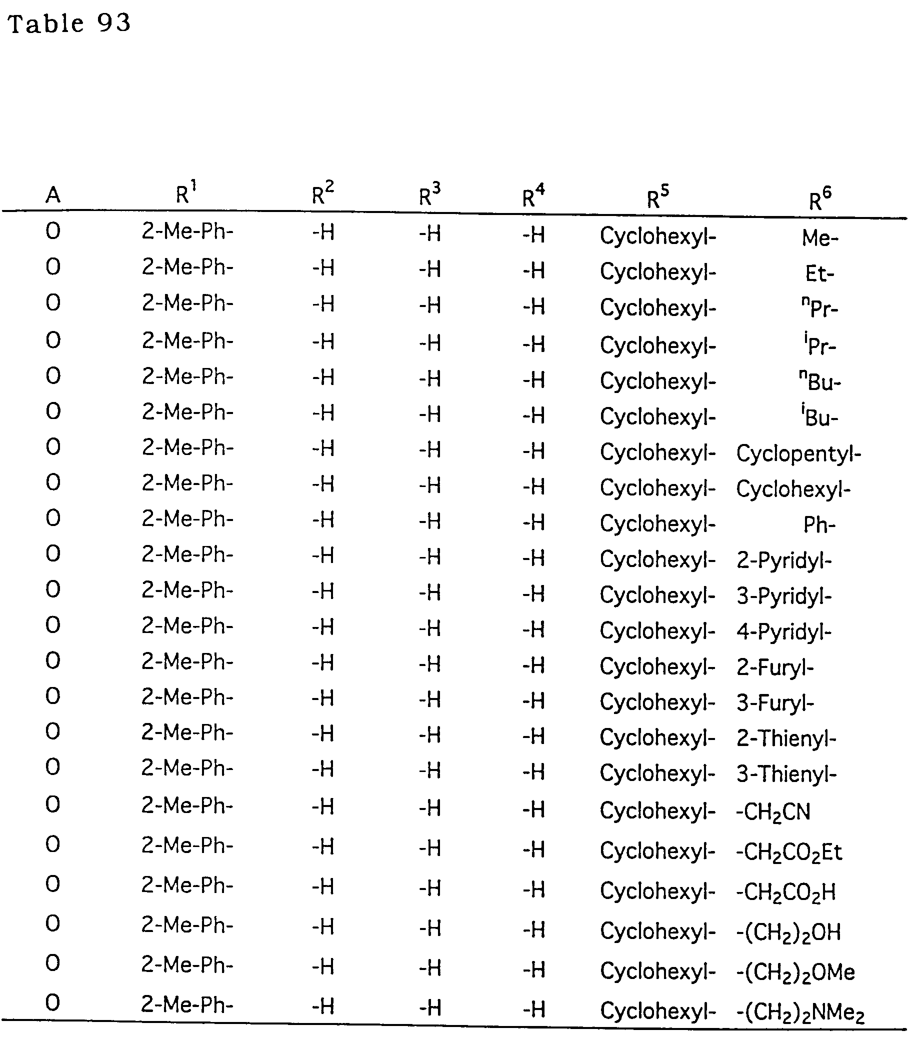

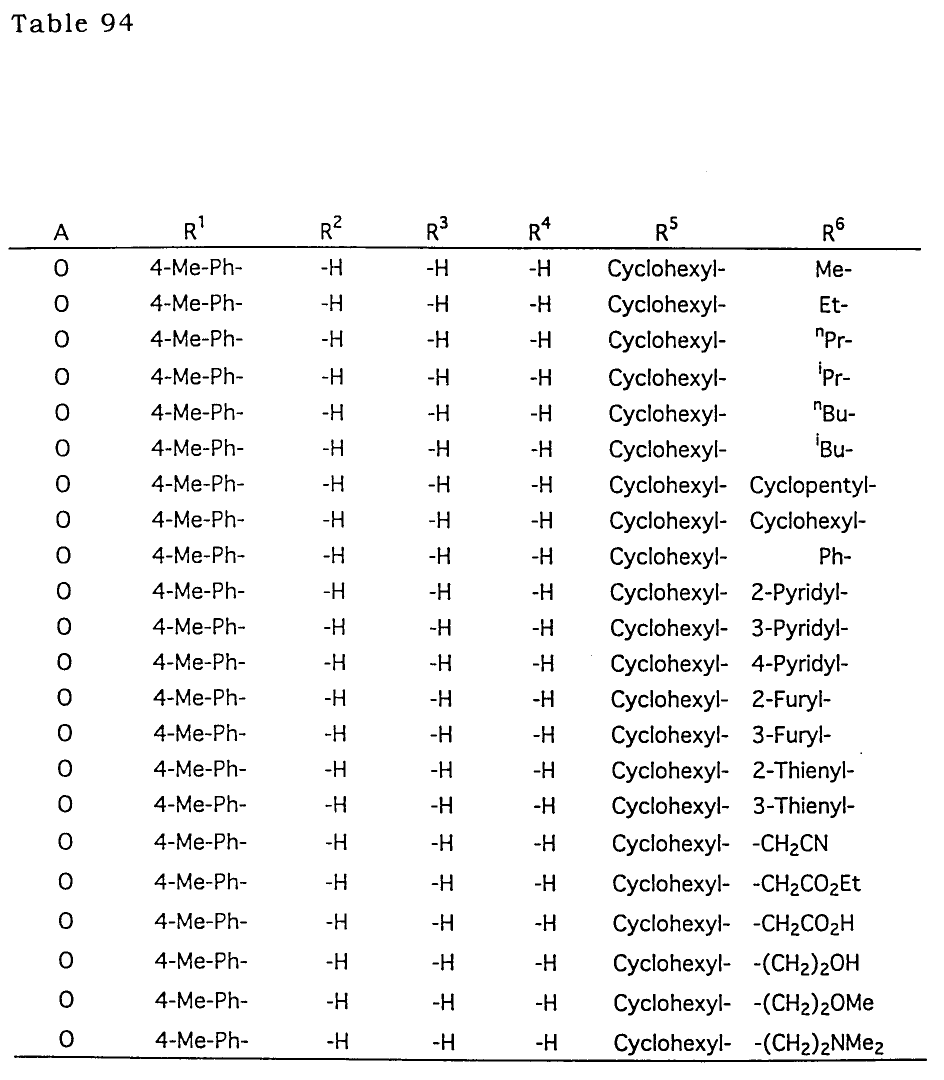

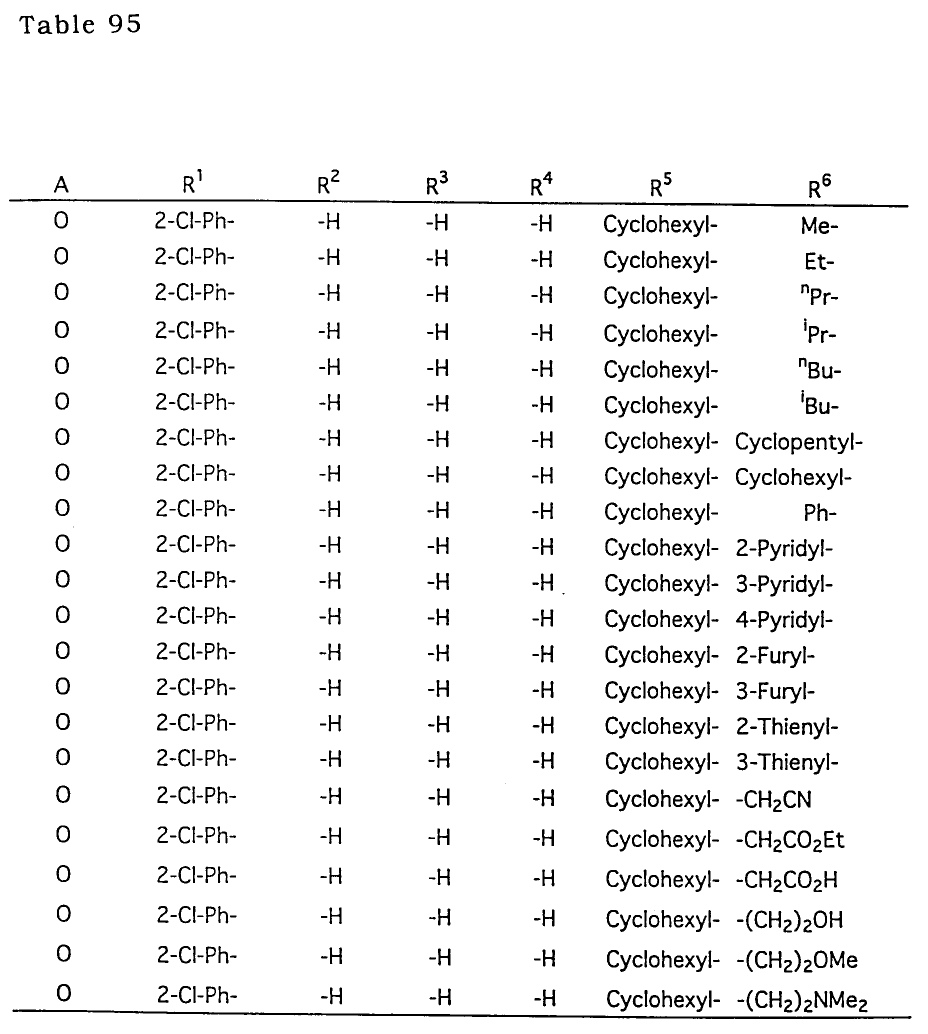

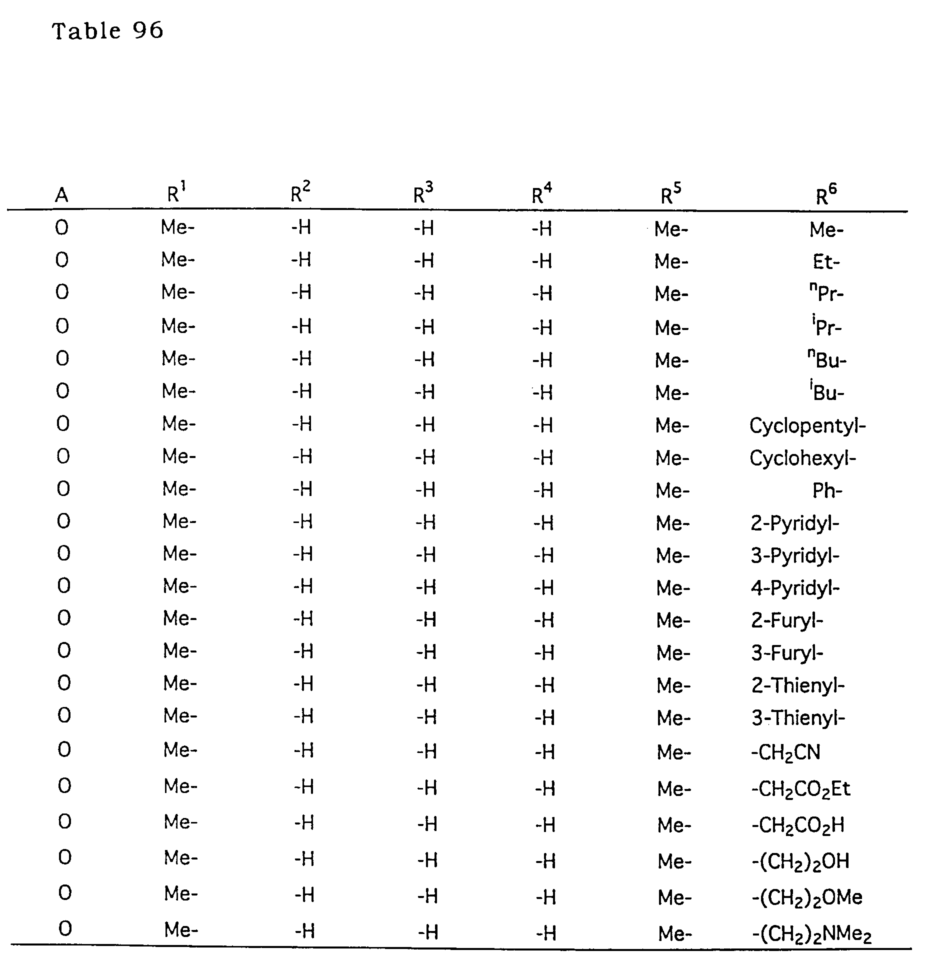

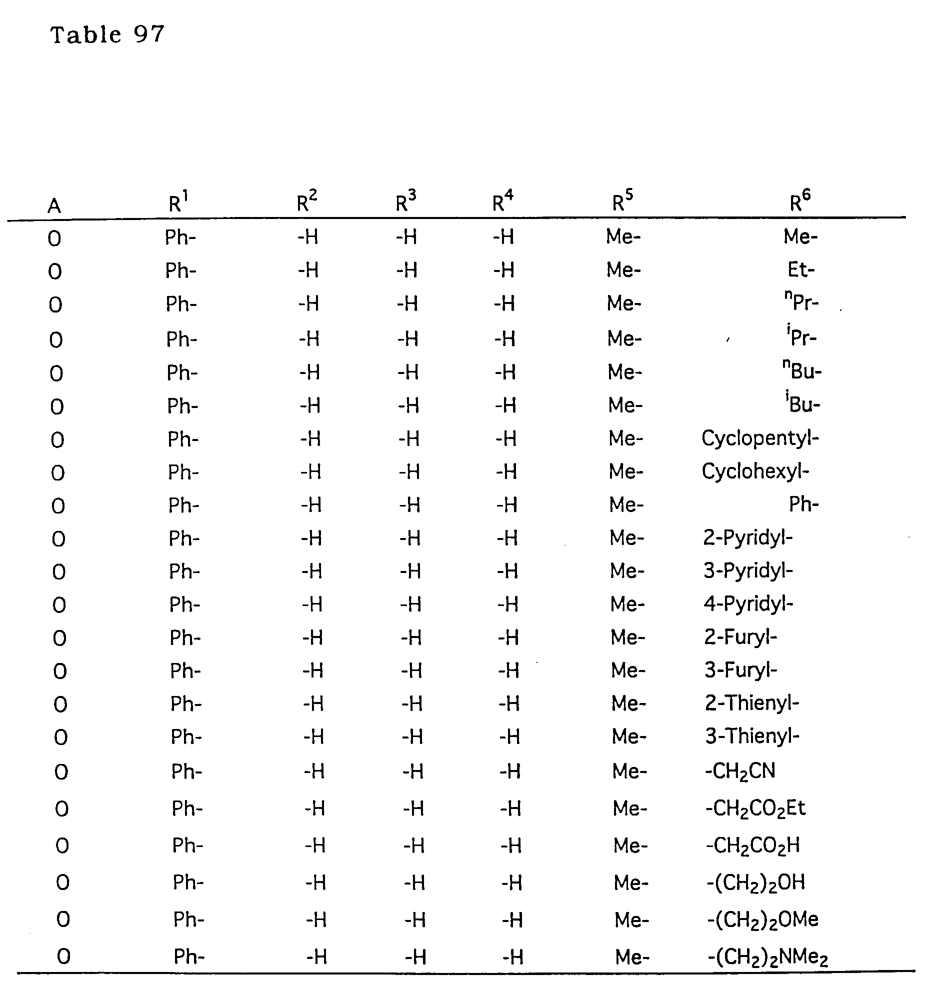

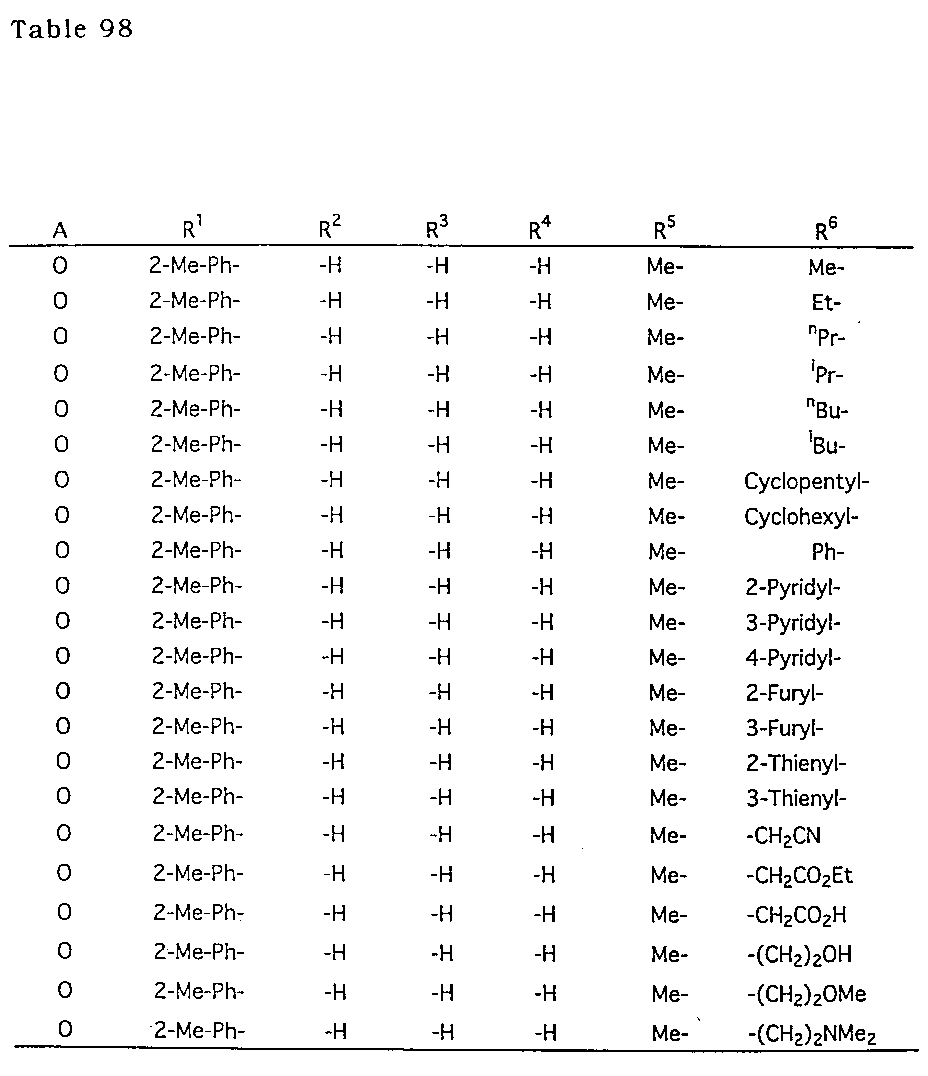

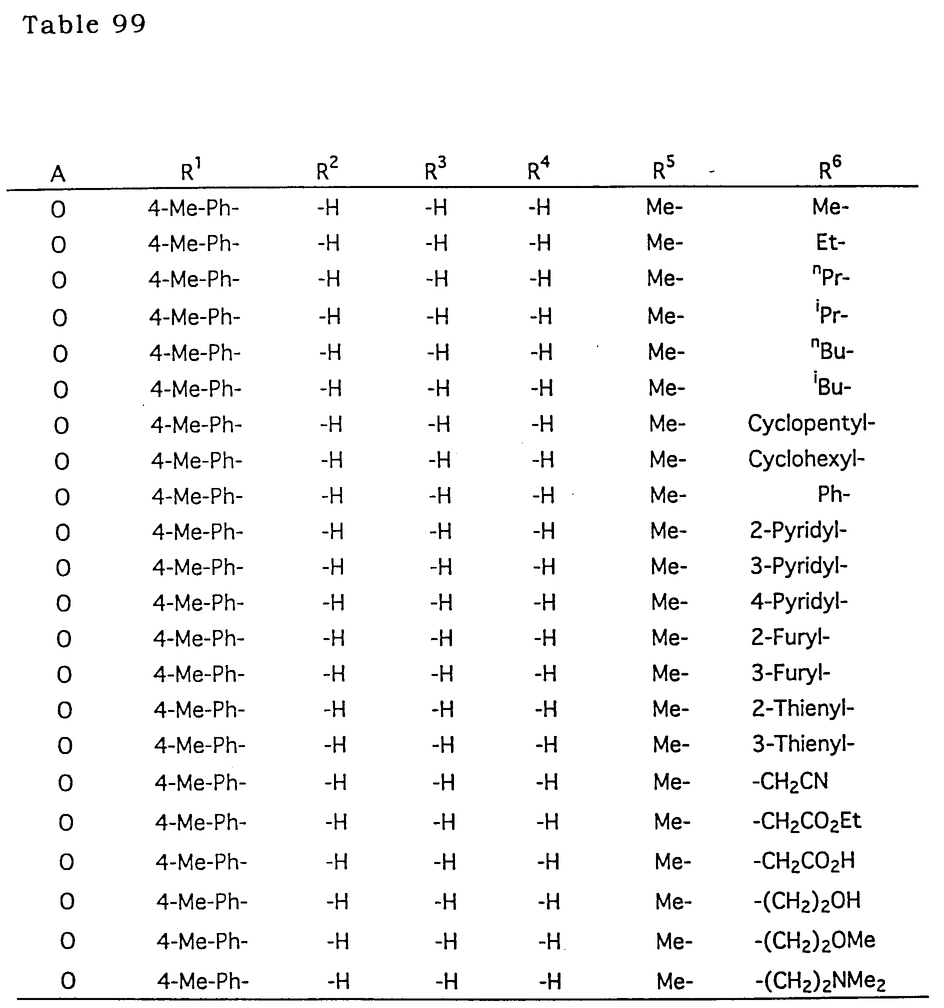

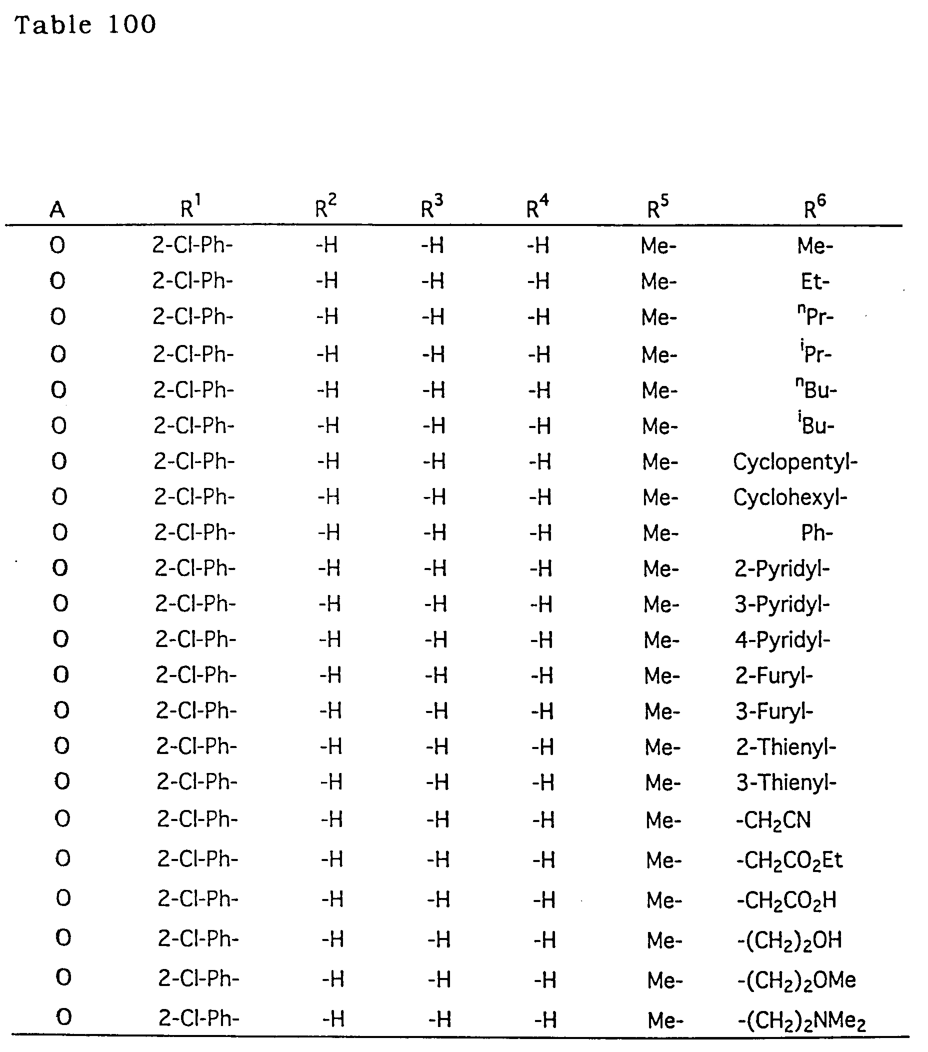

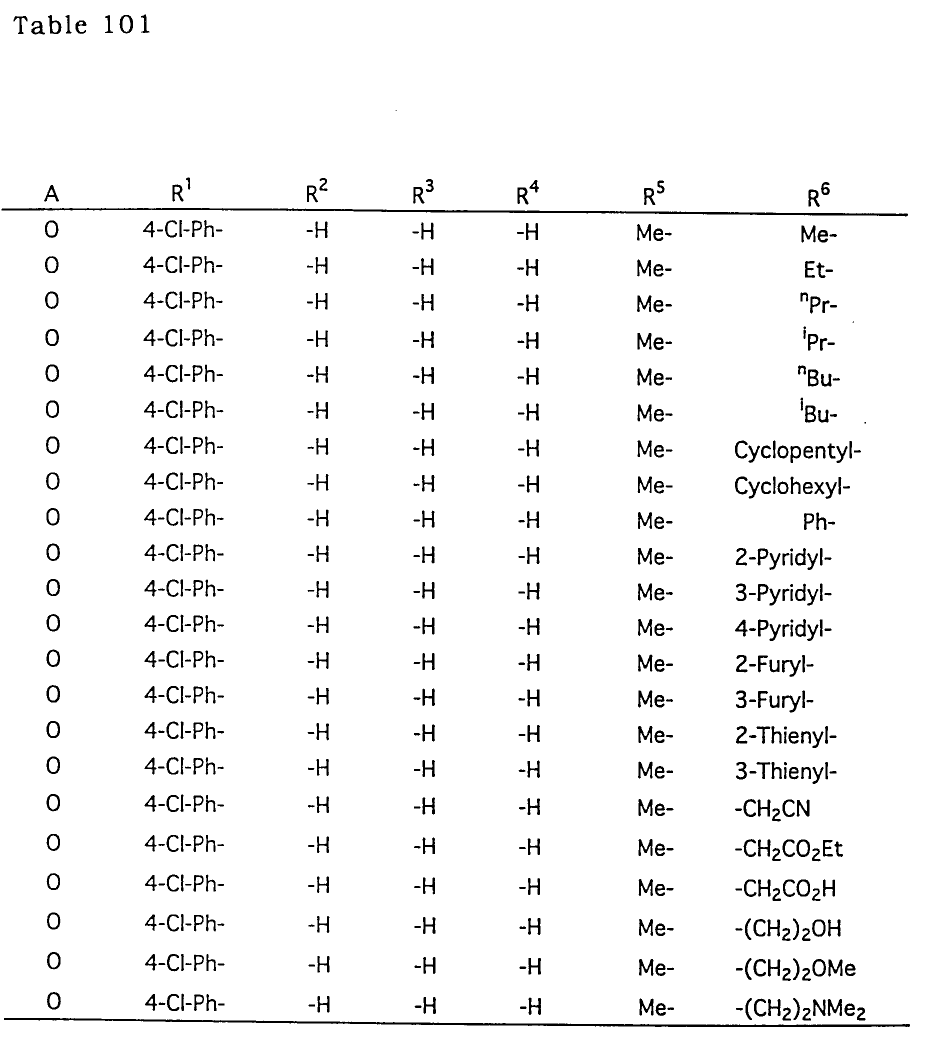

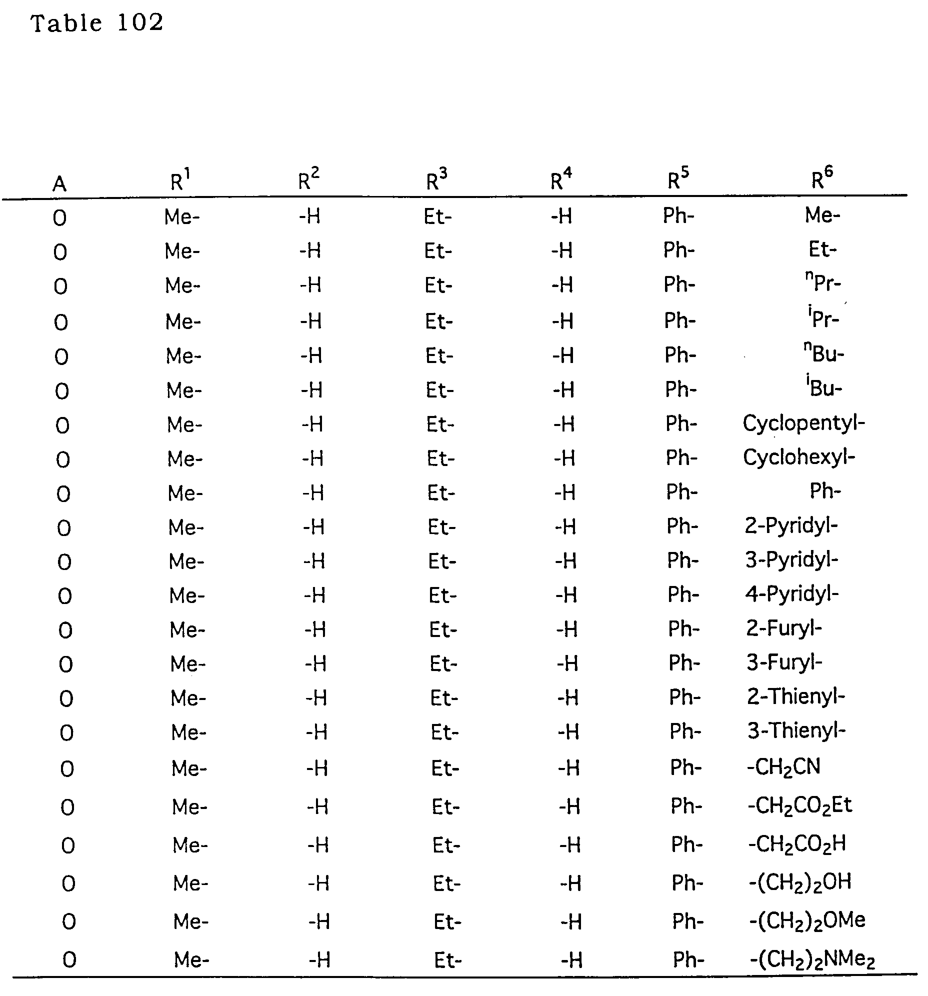

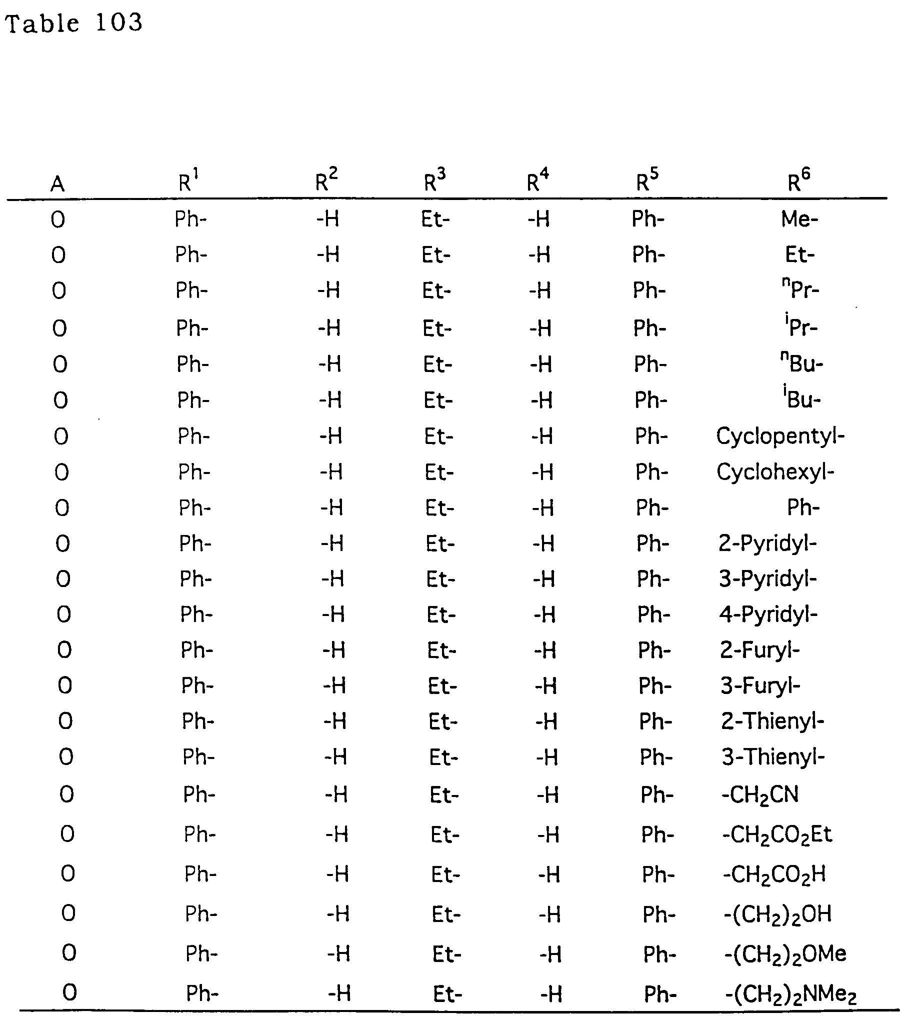

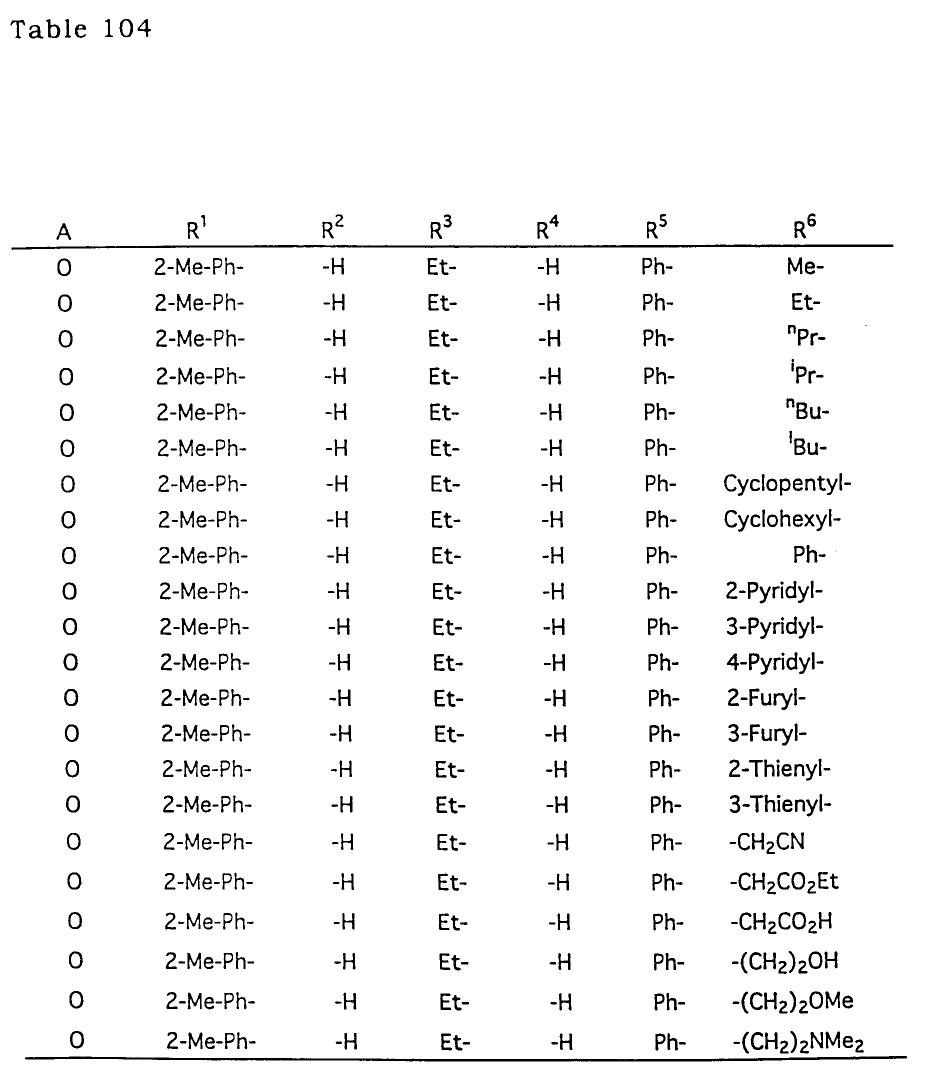

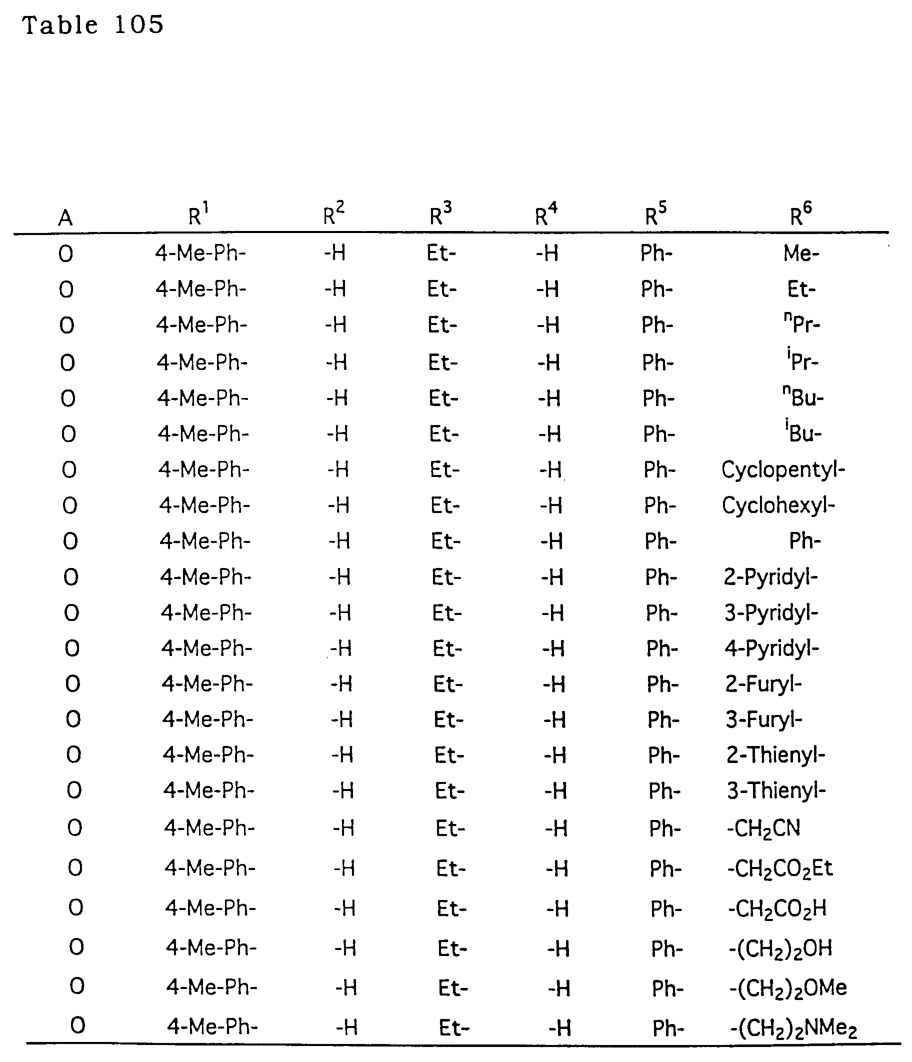

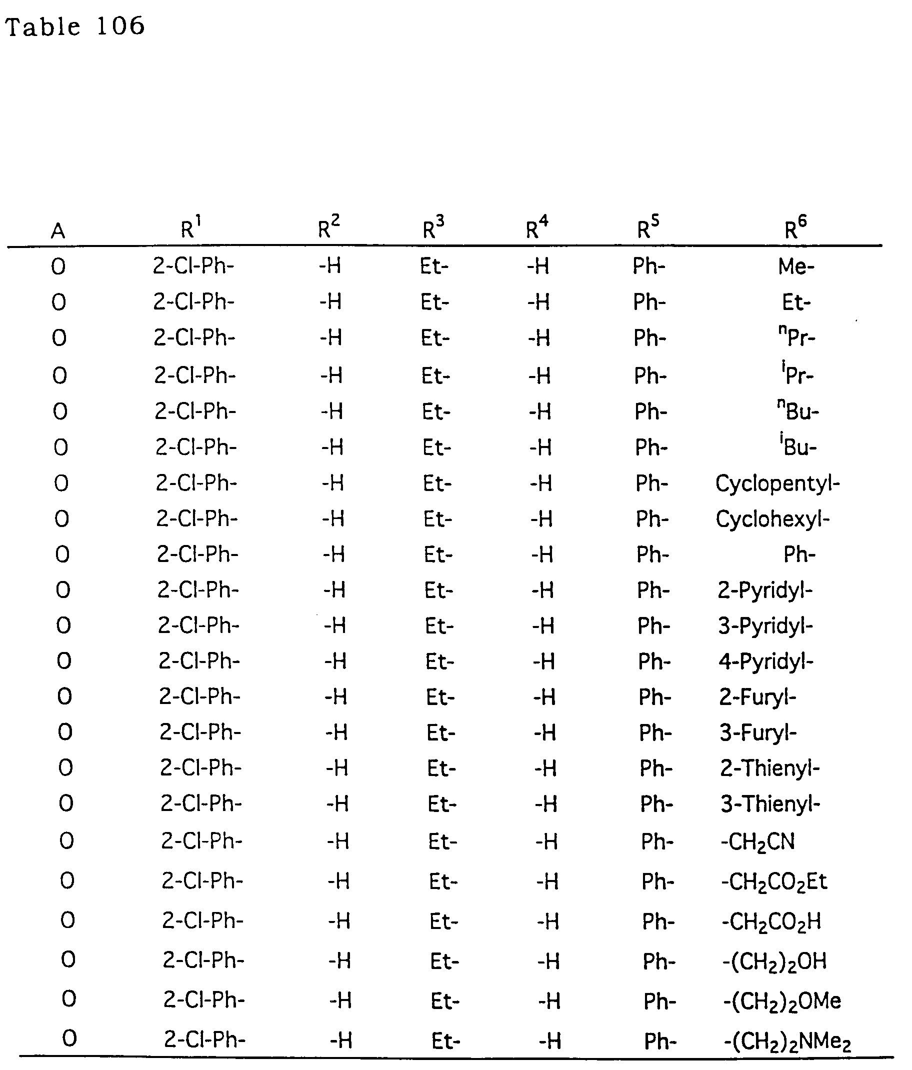

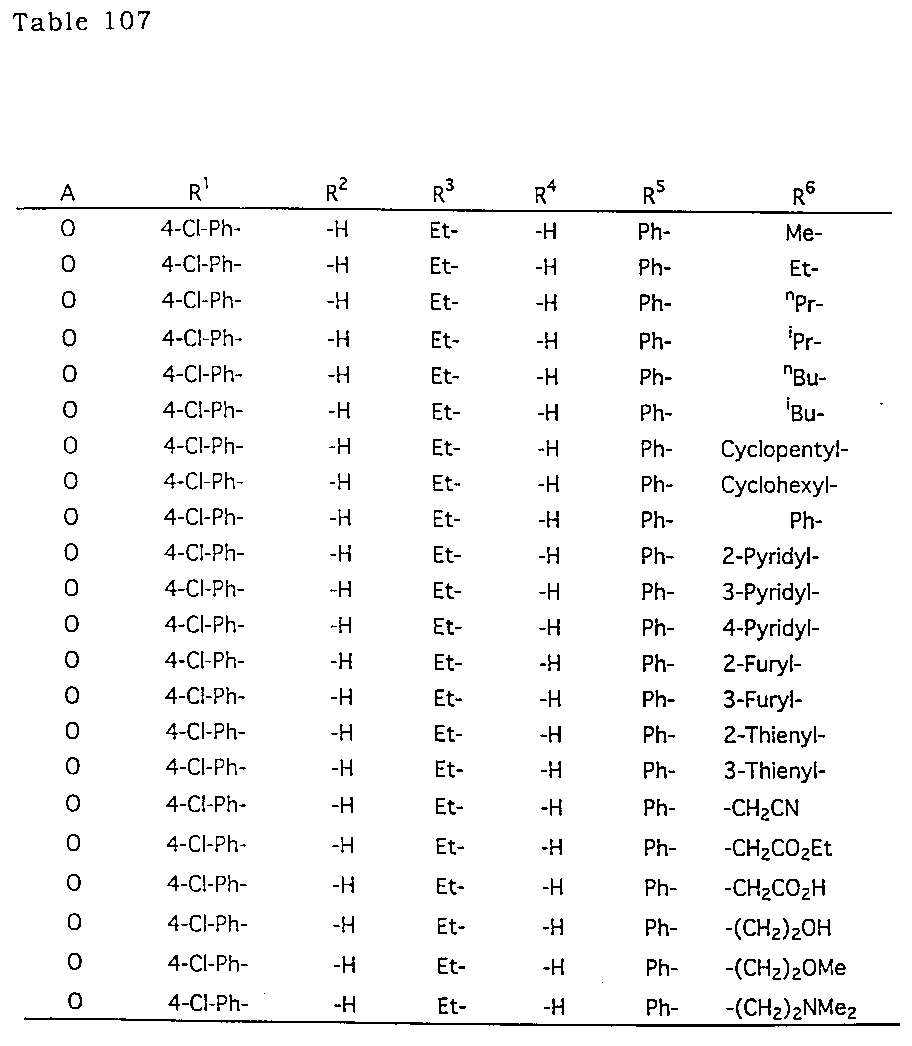

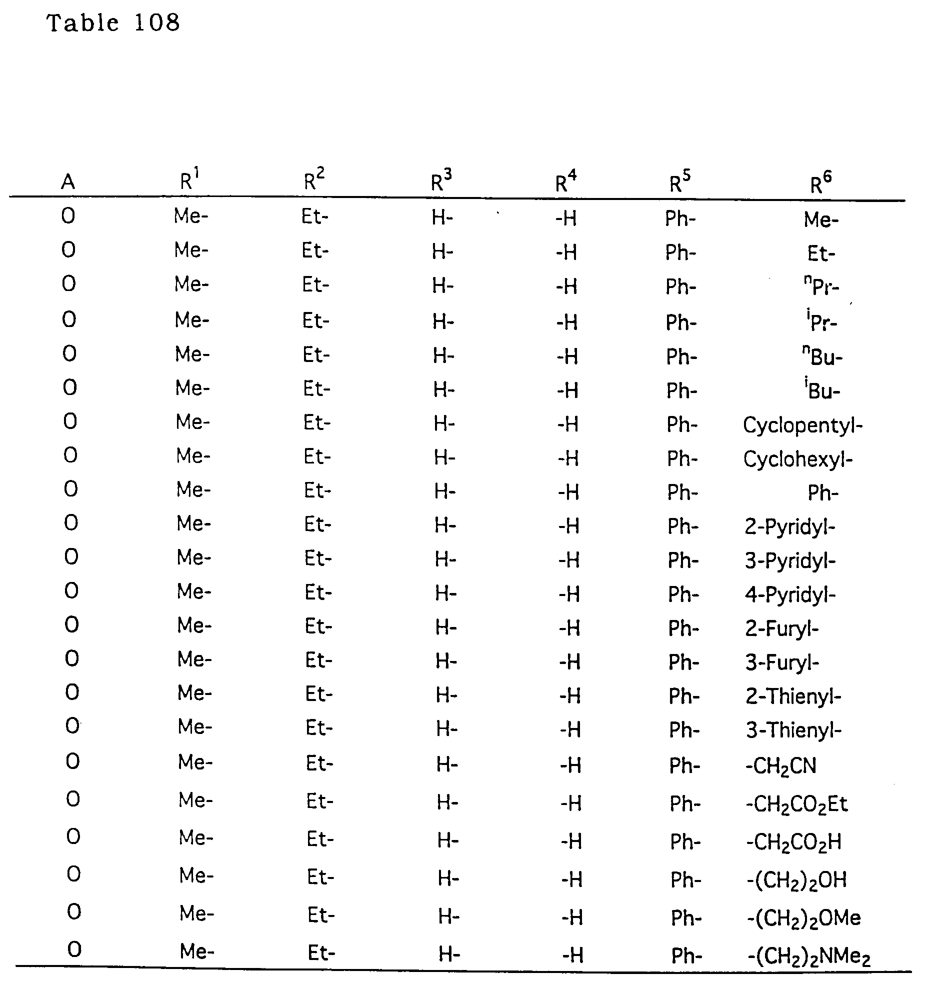

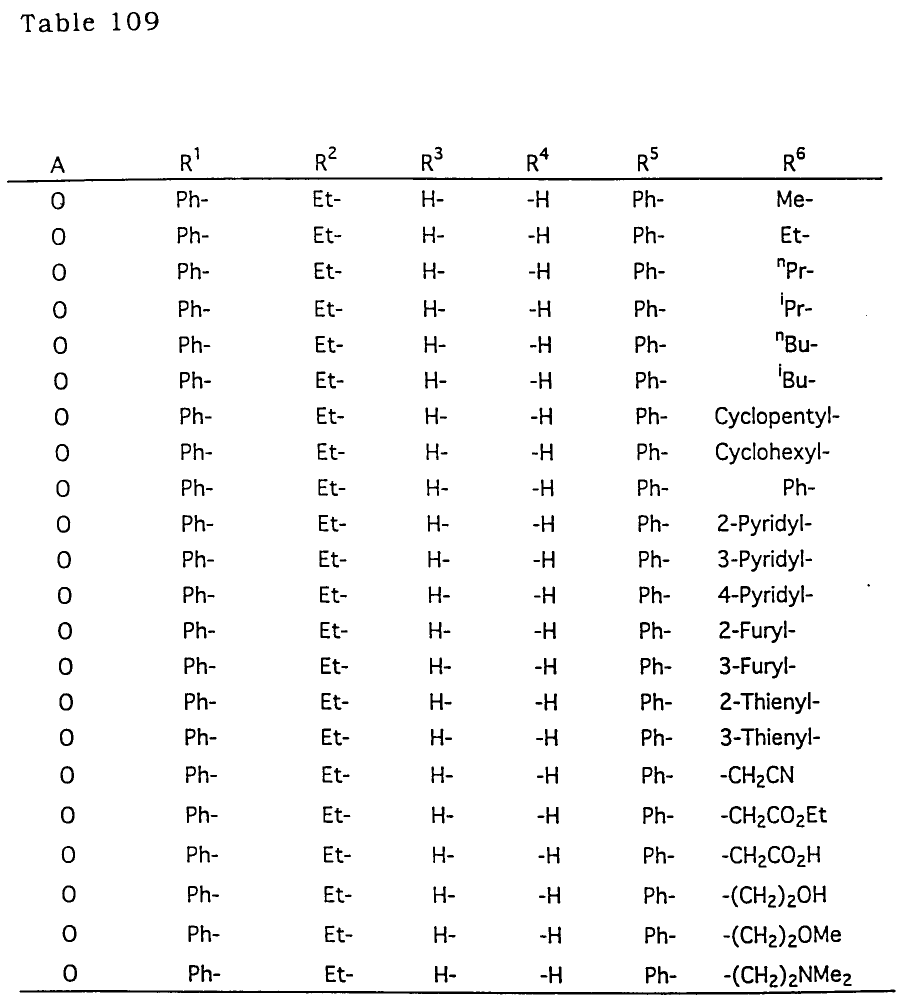

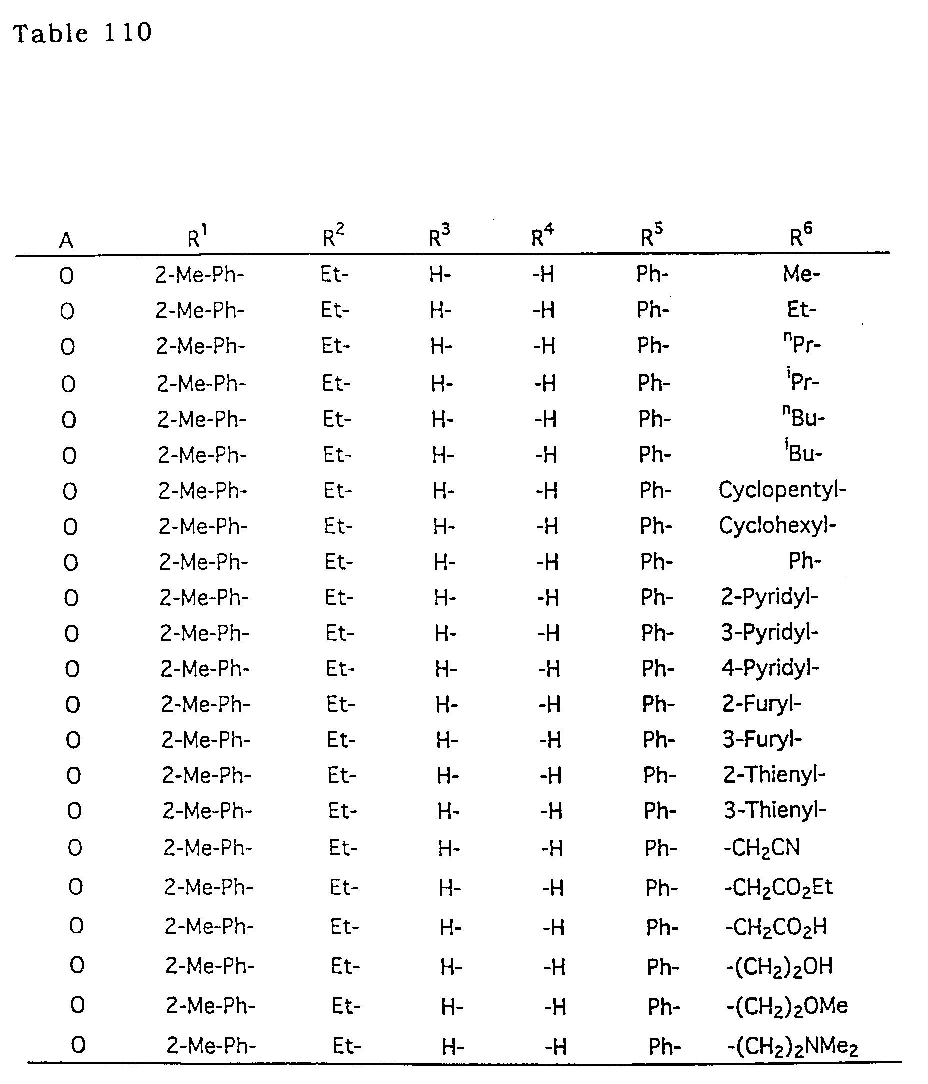

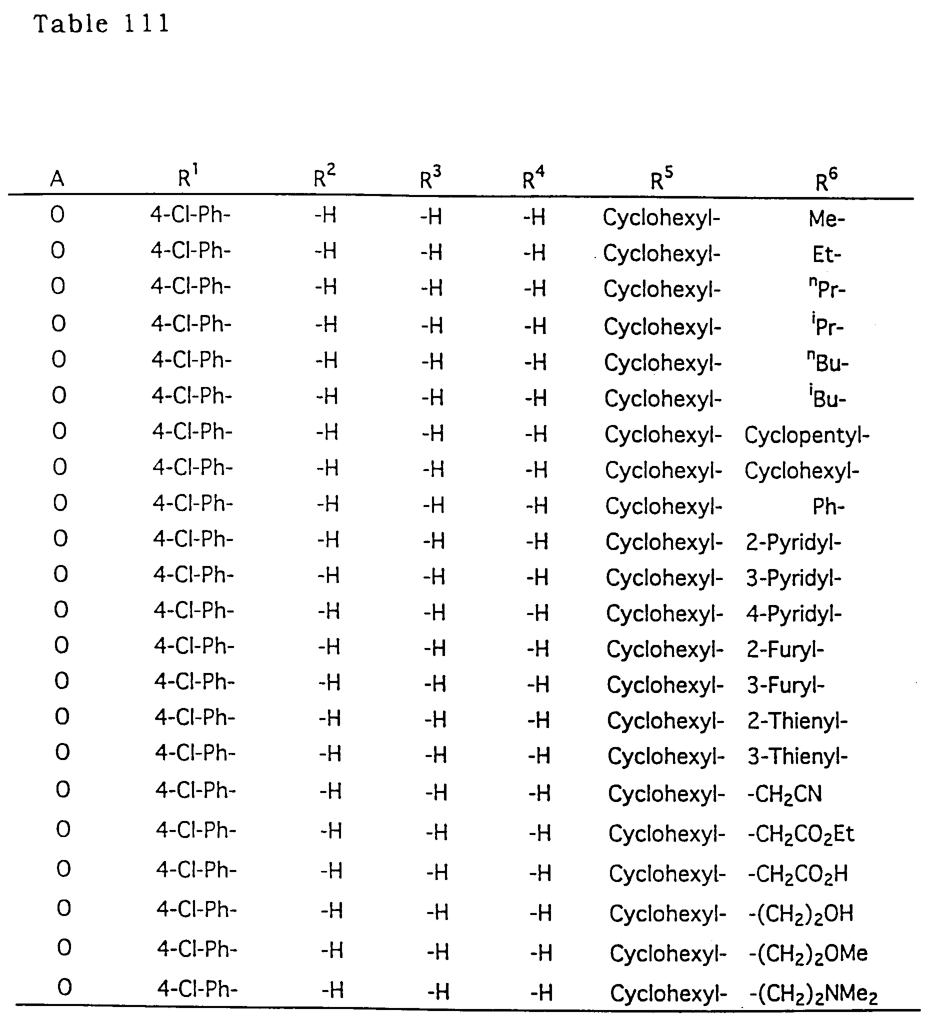

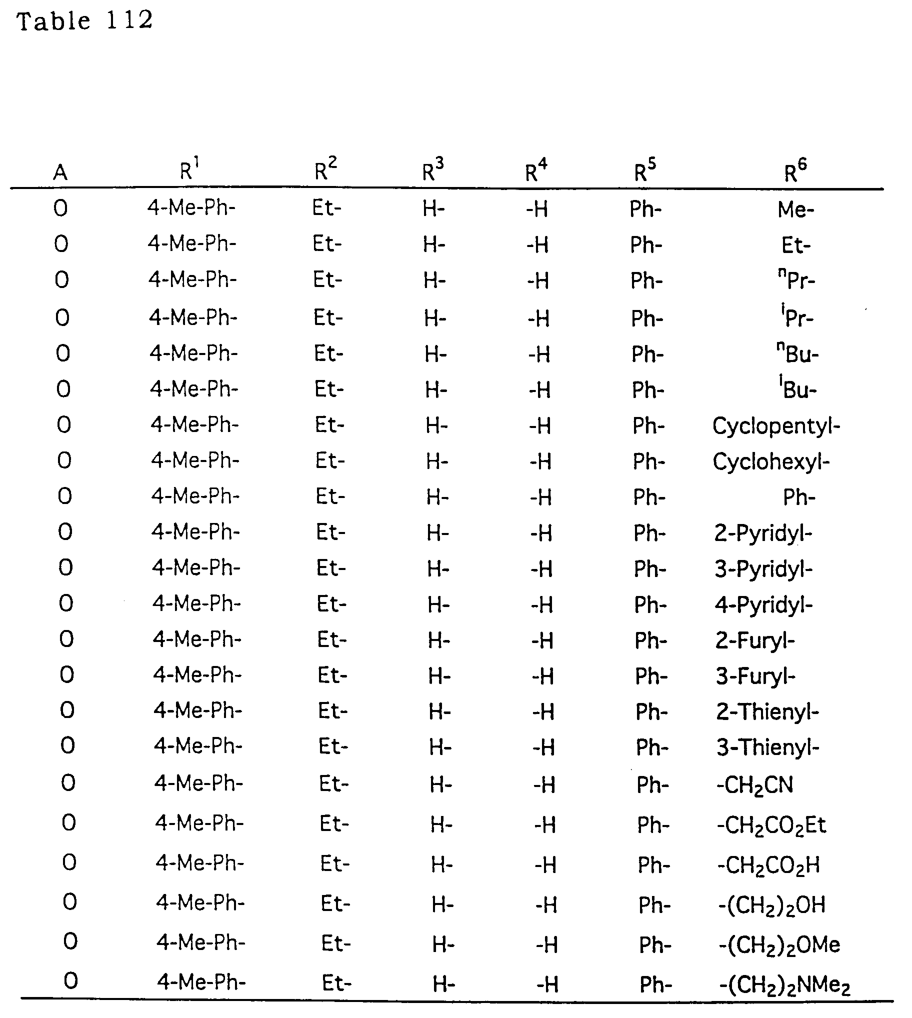

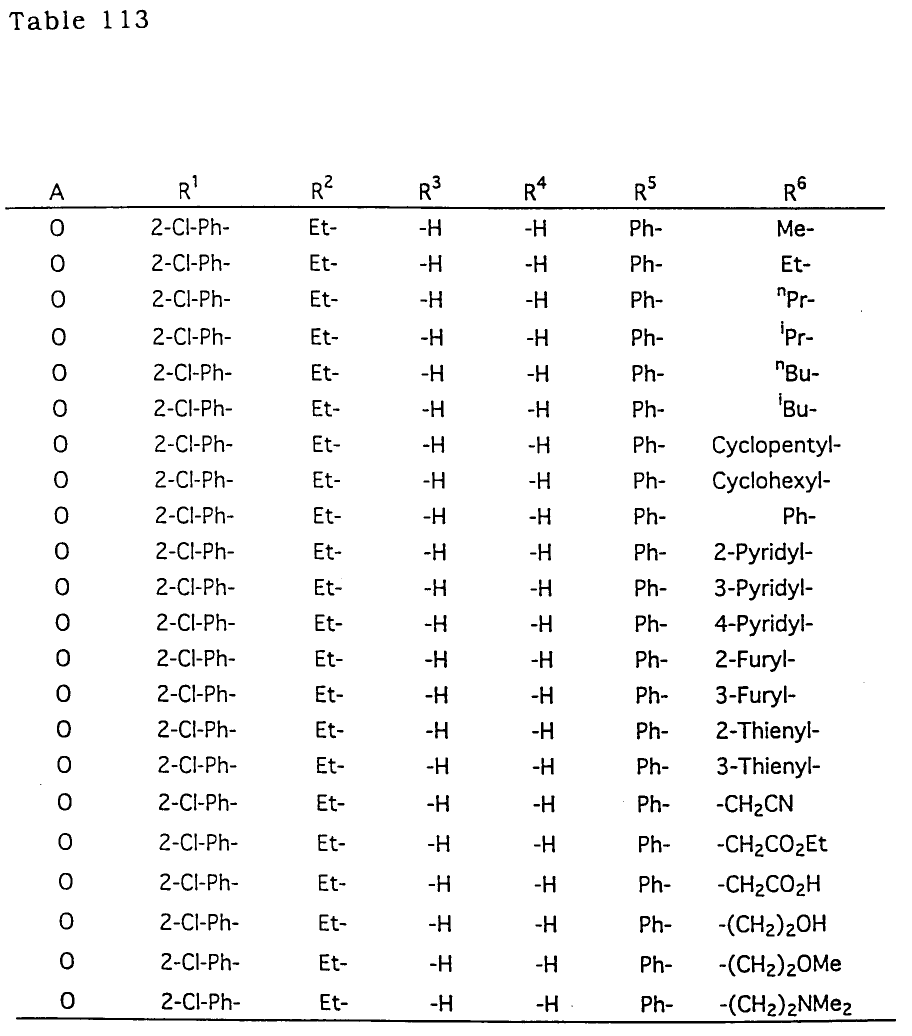

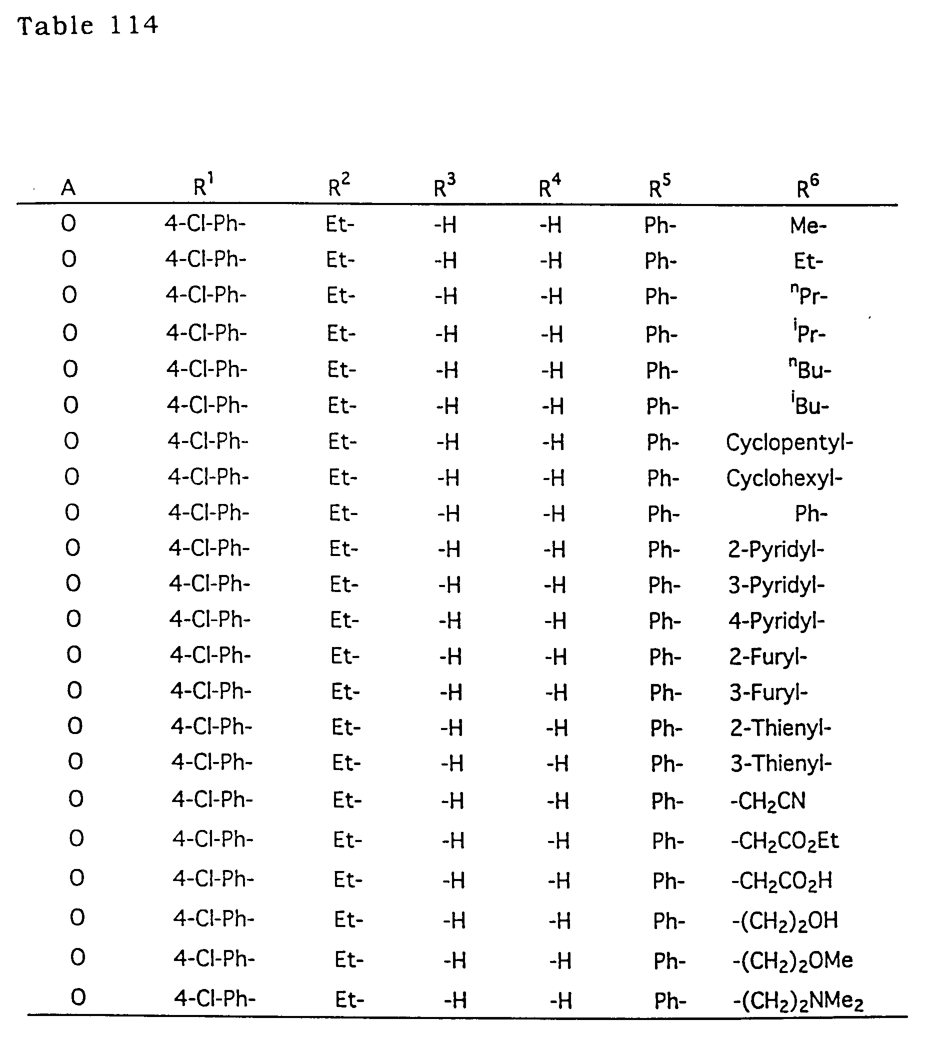

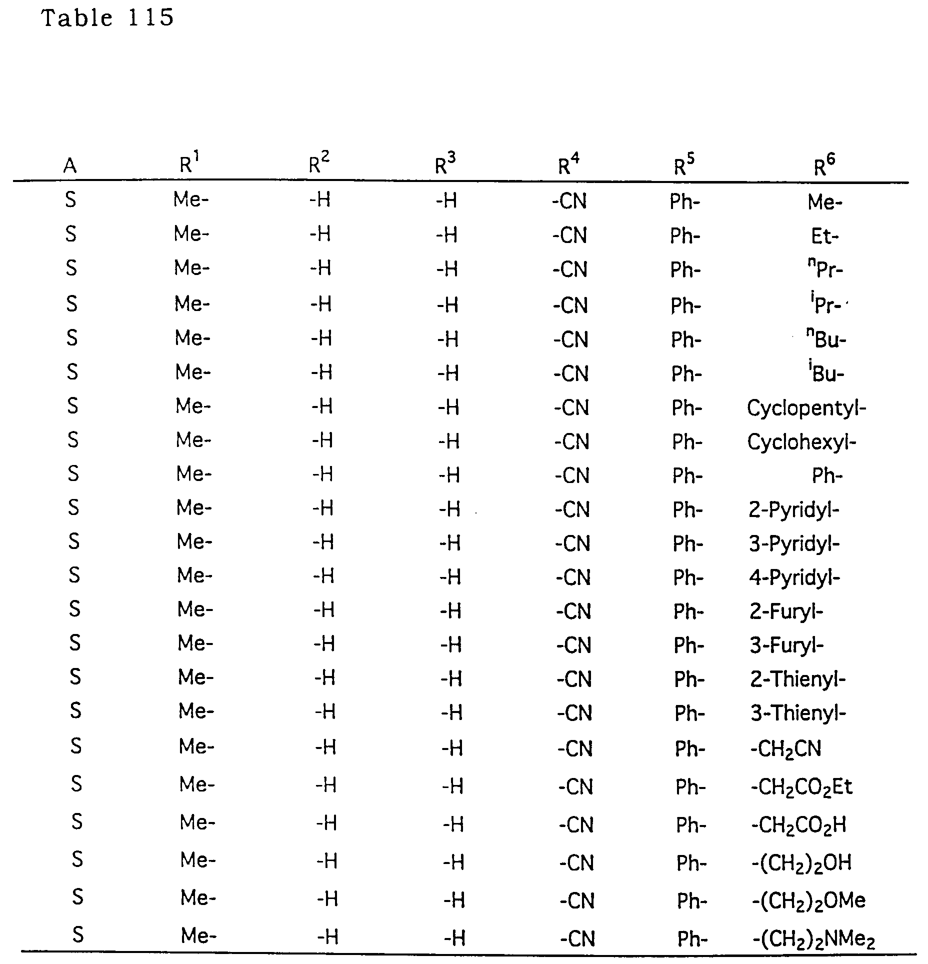

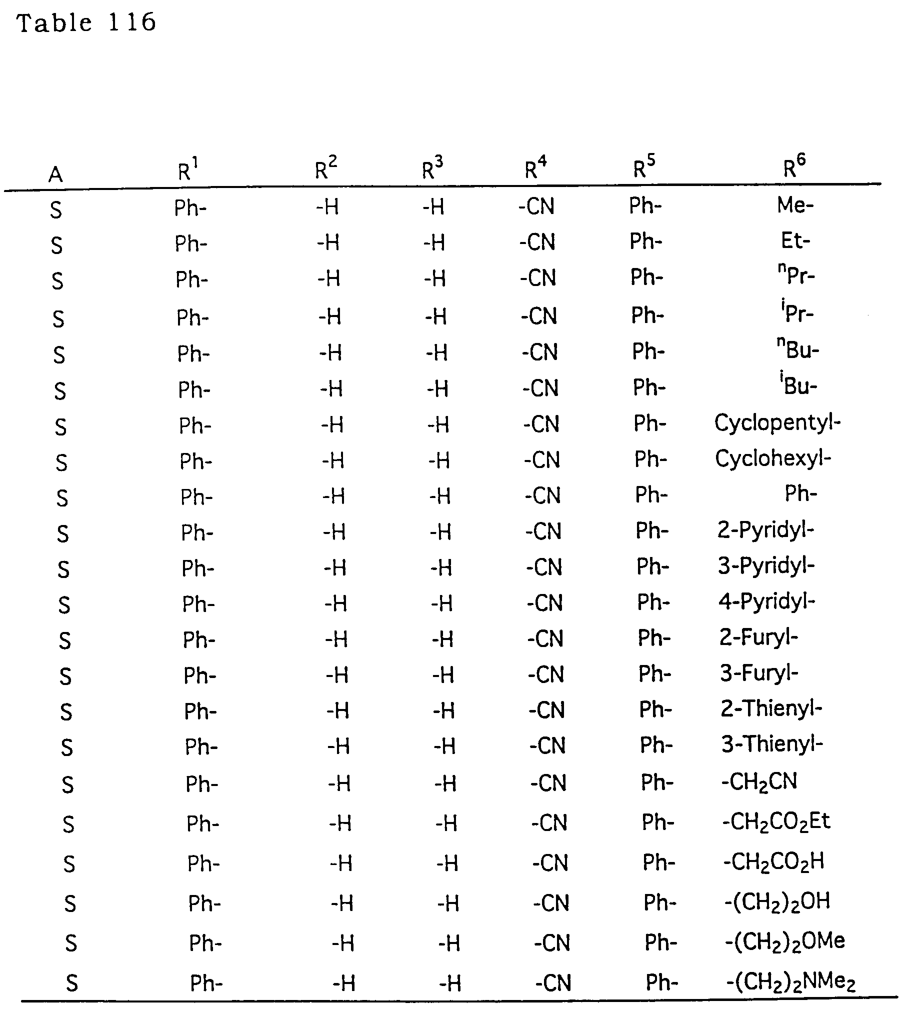

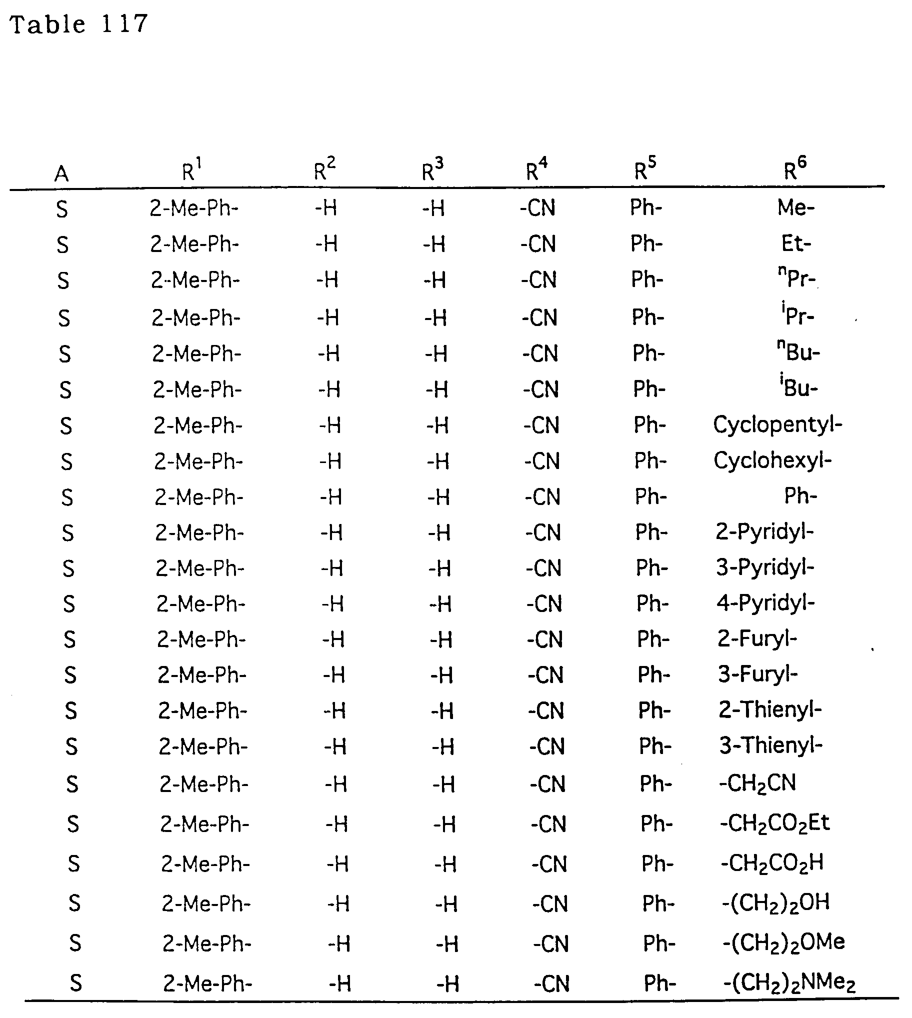

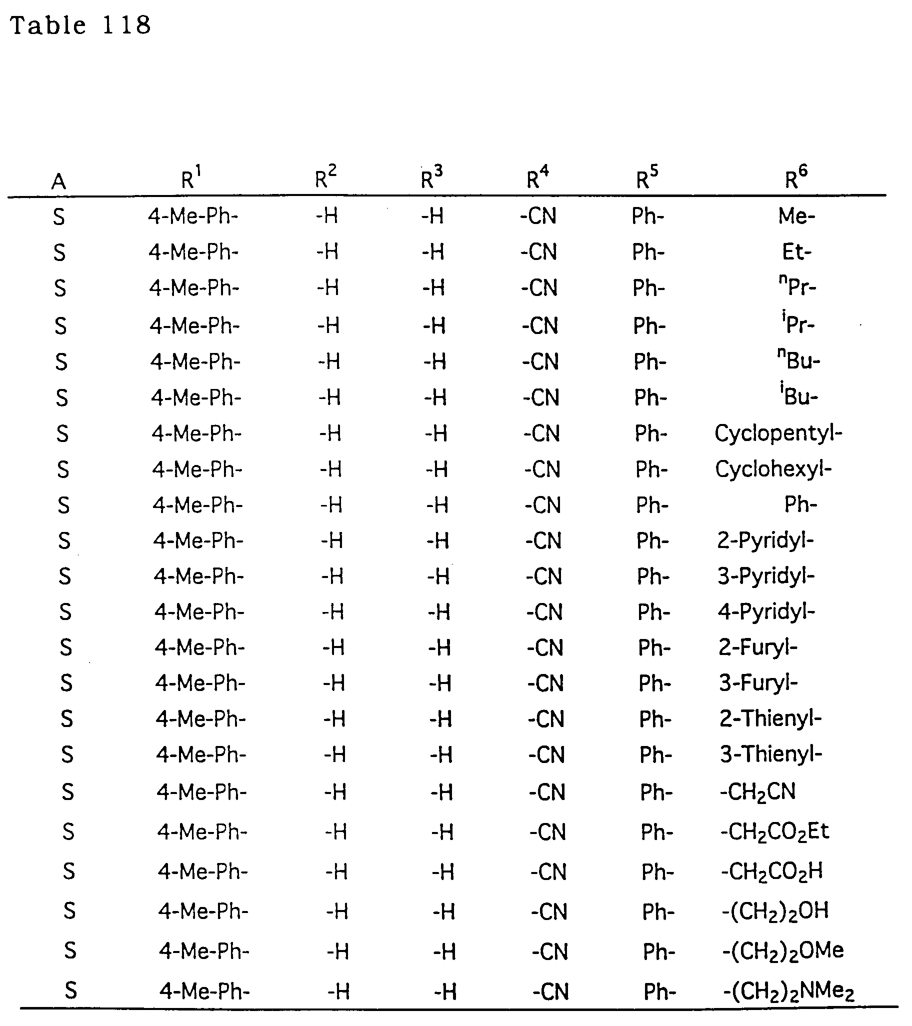

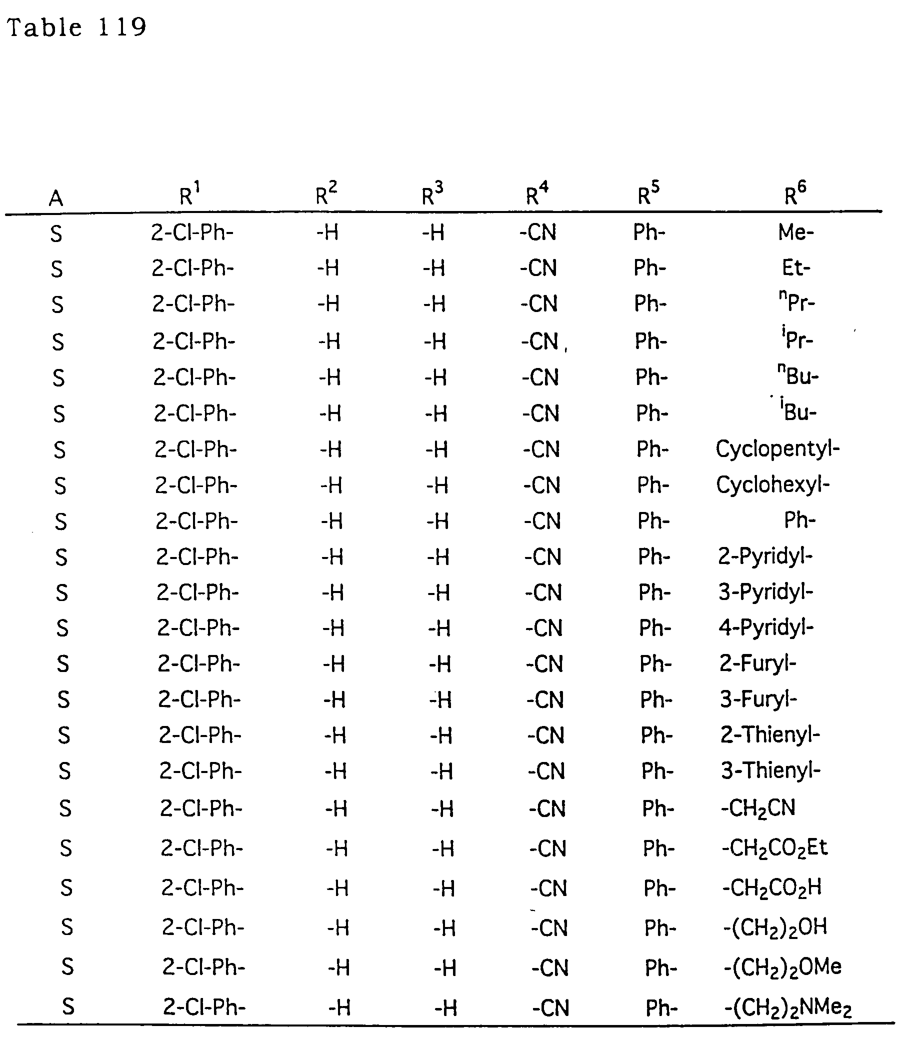

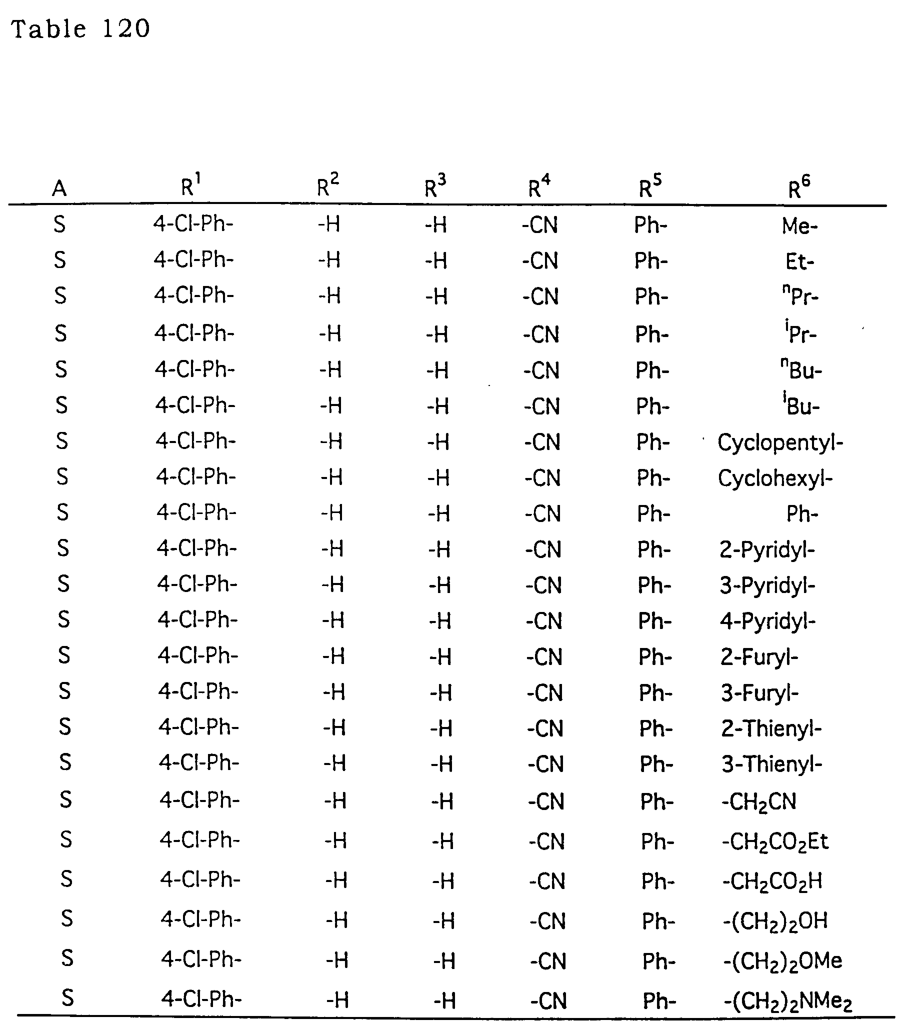

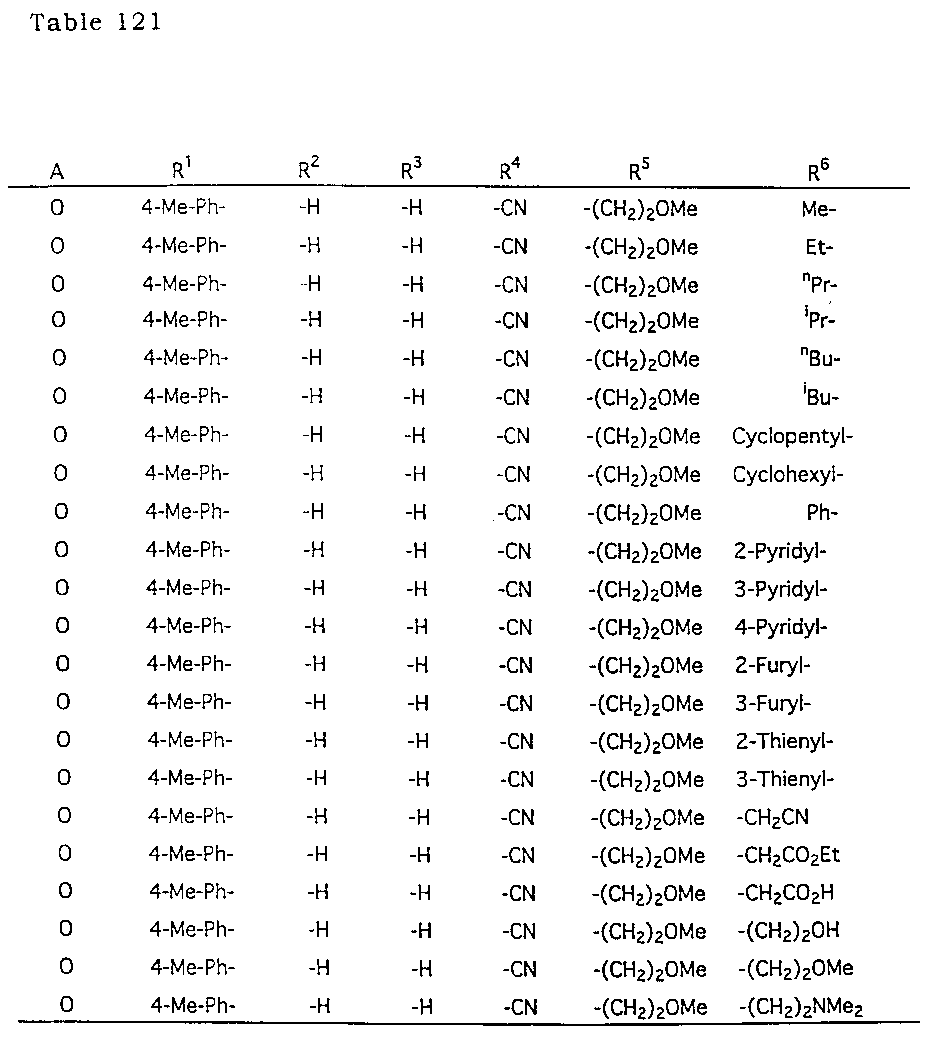

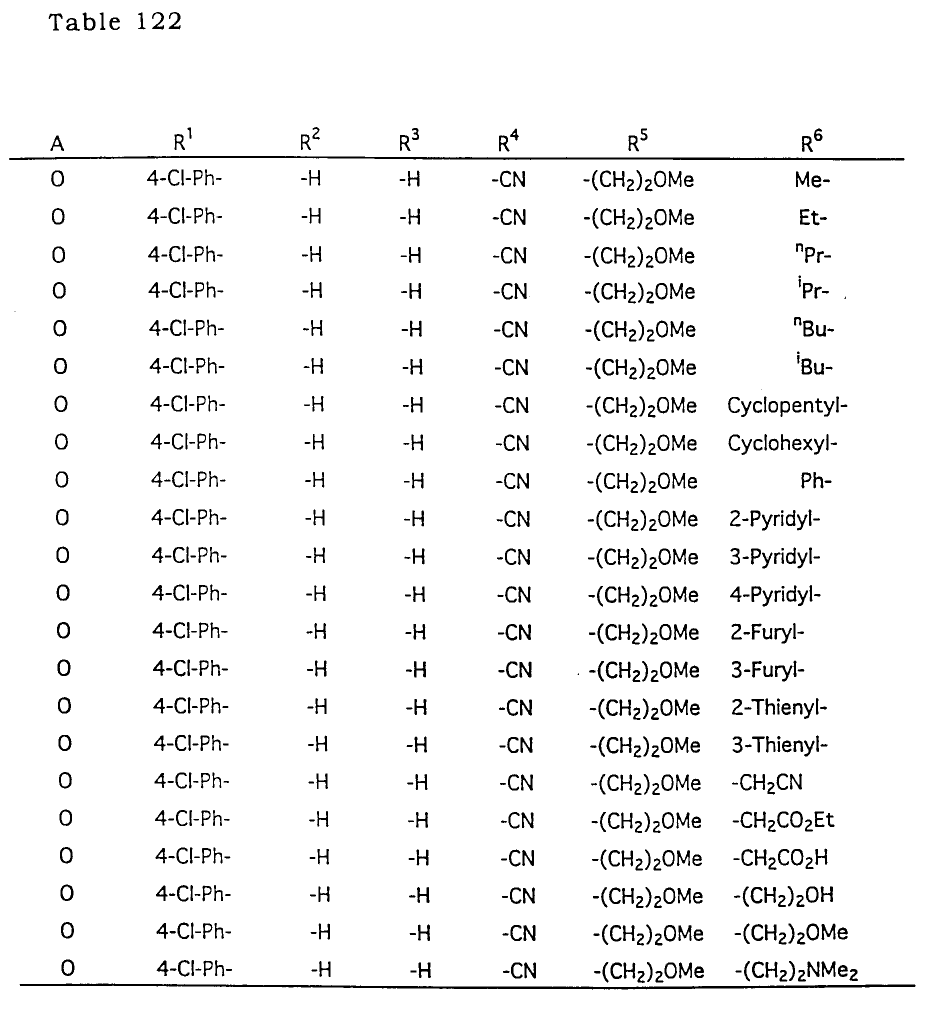

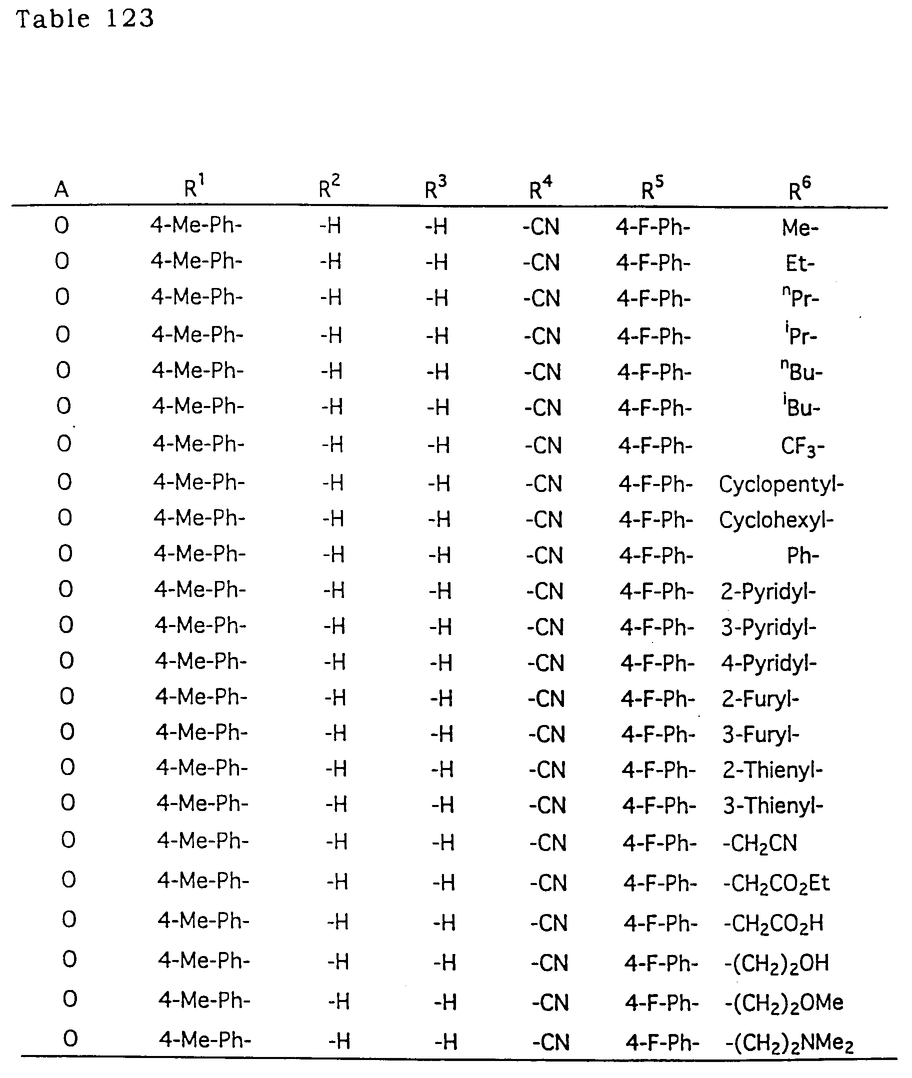

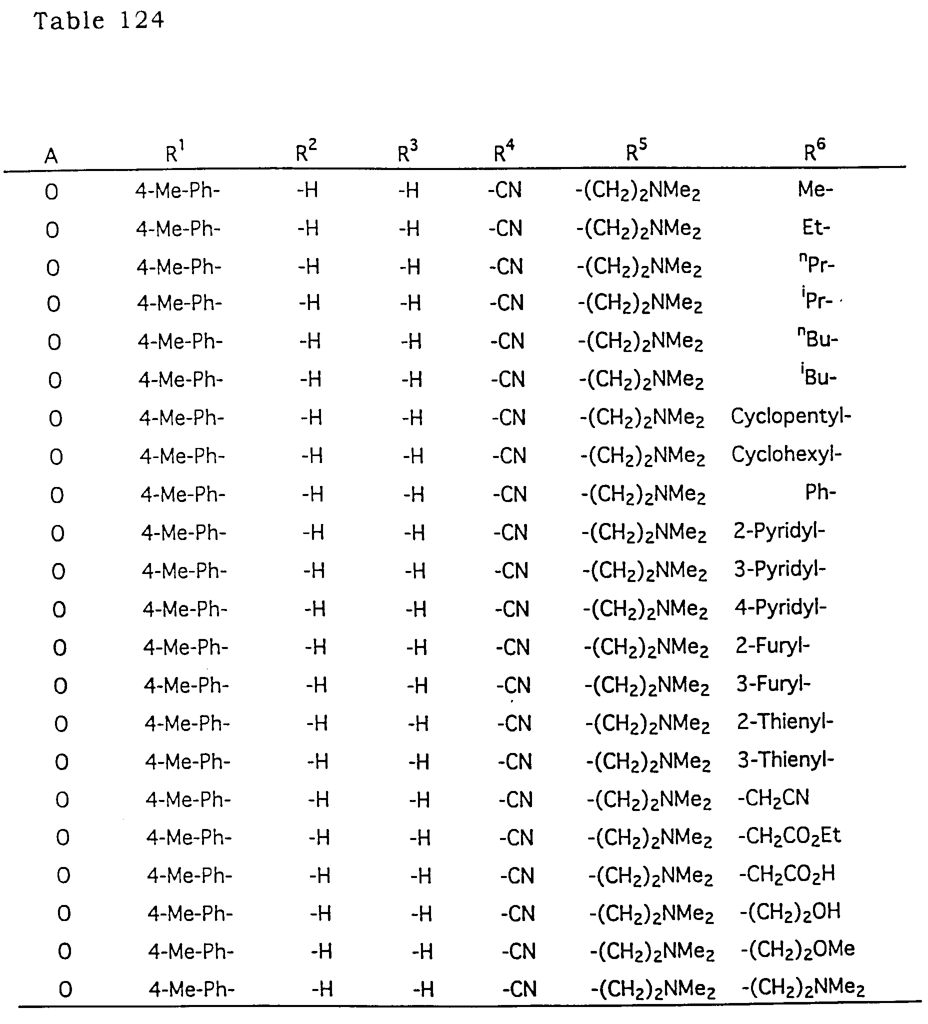

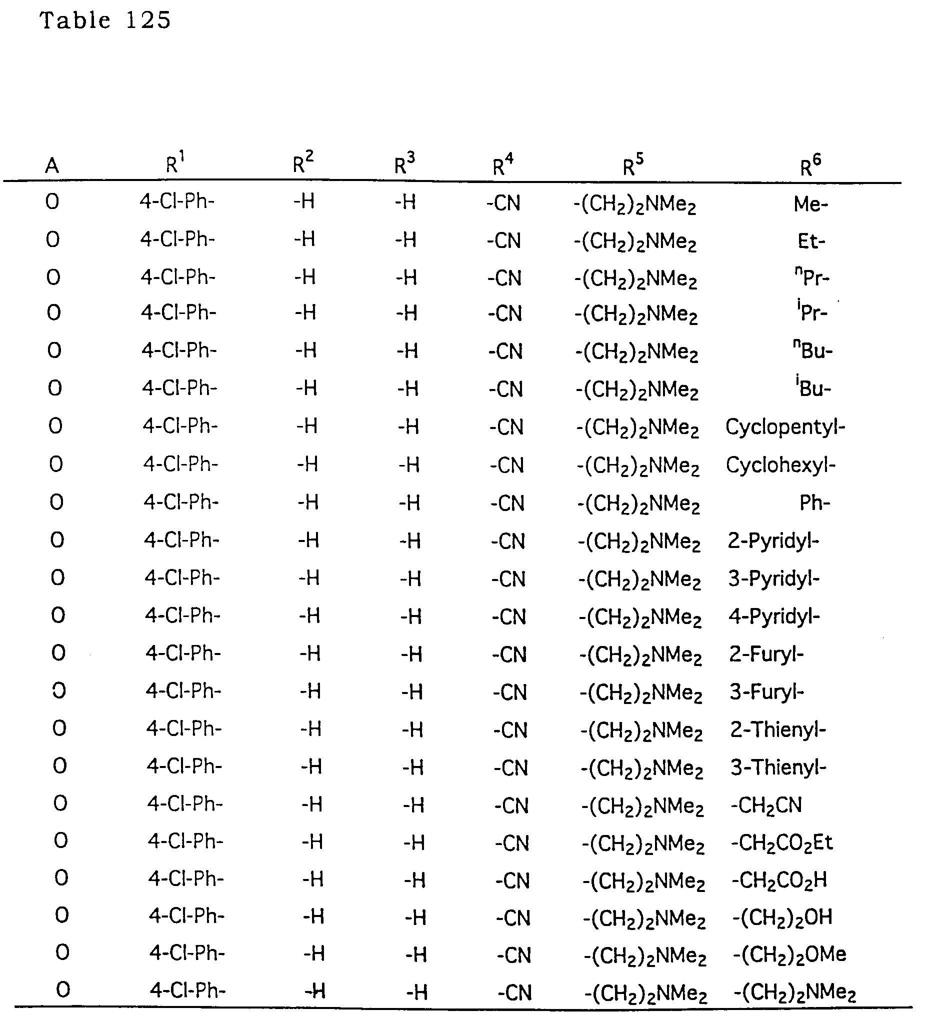

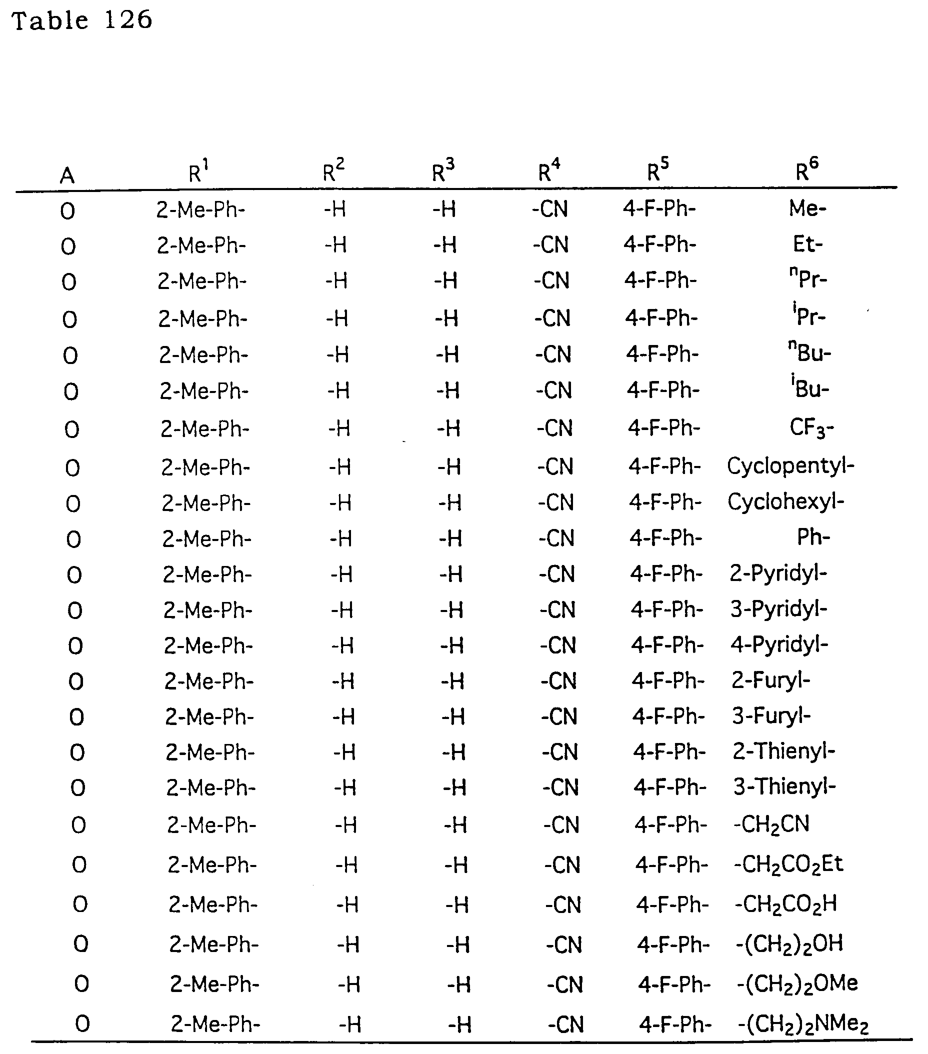

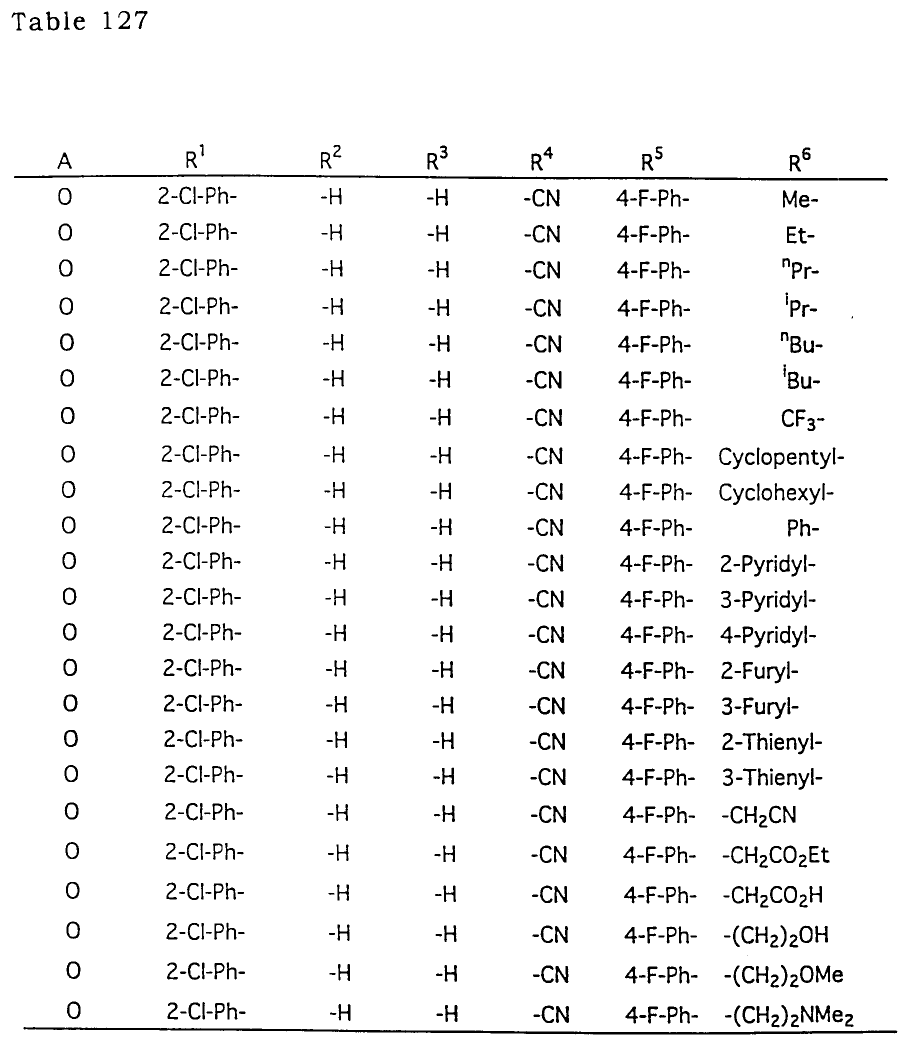

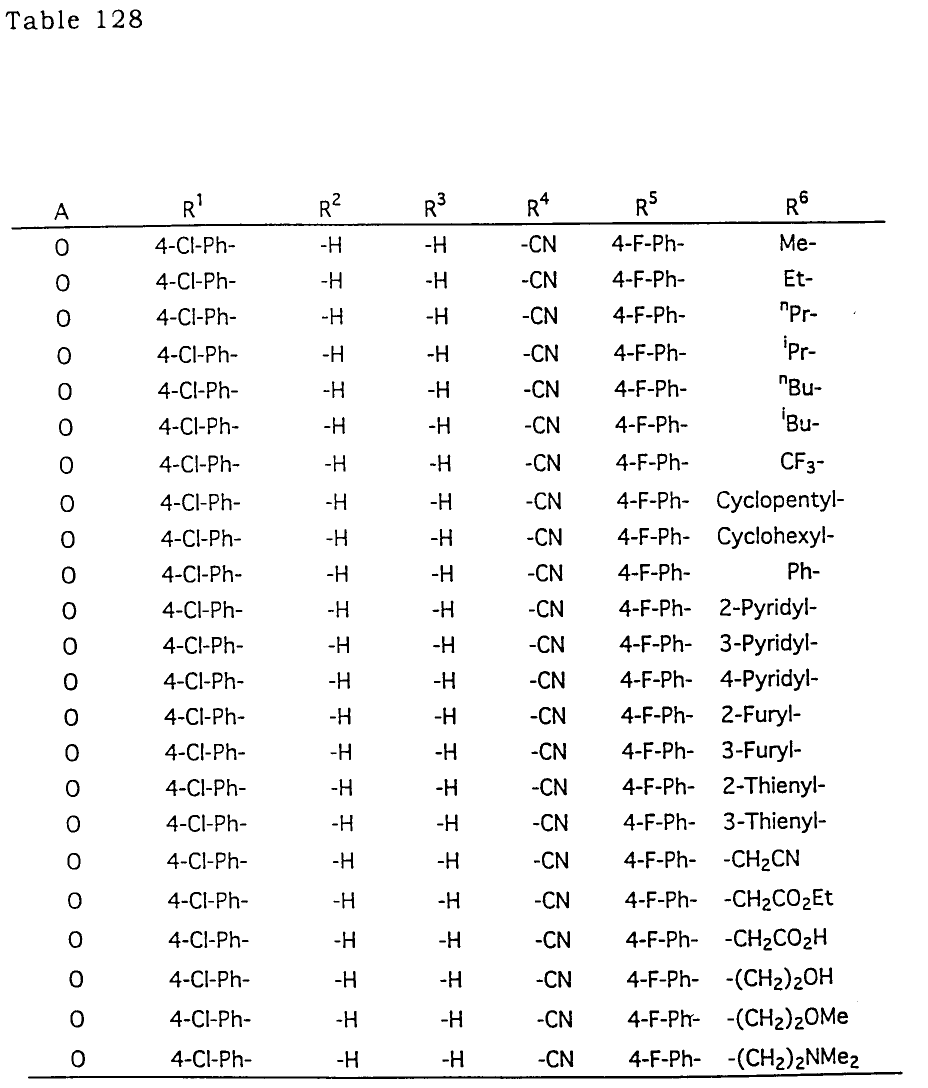

If necessary, the compounds of formula (1) or (2) can be reacted with inorganic or organic acids to form pharmaceutically acceptable acid addition salts or to form alkali addition salts. Such acid addition salts include those formed with inorganic acids such as hydrochloride, hydrobromide, sulfate, phosphate, and the like; those formed with organic carboxylic acid such as formate, acetate, fumarate, maleate, oxalate, citrate, malate, tartrate, aspartate, glutamate, and the like; those formed with sulfonic acids such as methanesulfonate, benzenesulfonate, p-toluenesulfonate, hydroxybenzenesulfonate, dihydroxybenzenesulfonate, and the like. The pharmaceutically acceptable alkali addition salts include ammonium salts, lithium salts, sodium salts, potassium salts, calcium salts, magnesium salts, and the like.

The compounds of the present invention are effective in oral administration as well as intravenous administration. The compounds of formula (1) or (2), or acid addition salts or alkali addition salts thereof can be administered parenterally or orally in employing them as agents for treatment or prevention. In particular, the compounds can be administered orally in the form employed commonly in the art, such as, for example, powders, granules, tablets, capsules, syrups, suspensions, and the like. Alternatively, the compounds can also be administered parenterally in the form of, for example, injectable solutions, emulsions or suspensions. The compounds can also be administered by rectal route in the form of suppositories. Such suitable formulations can be prepared by combining the active compounds with conventional carriers, excipients, binders, stabilizing agents, or diluents, which are acceptable. The injectable formulations can additionally contain buffers, solubilizing agents, or isotonizating agents, which are acceptable. Such formulations can be prepared by conventional procedures. Doses and frequencies vary depending on, for example, the disease and condition to be treated, the age and weight, a method for administration and the like, and a typical daily dose for adults may range 0.1 mg to 2000 mg, preferably, 1 to 200 mg, which is administered at a time or in portions.

### BEST MODE FOR CARRYING OUT THE INVENTION

The following preparations, formulations, and experiments are presented for purpose of further illustration of the invention, and such examples are not intended to be limiting the invention in any respect.

### Preparation 1

### Synthesis of 4-cyano-1-phenyl-5-{3-(4-toluenesulfonyl)ureido}-(1H)-pyrazole

To a solution of 5-amino-4-cyano-1-phenyl-(1H)-pyrazole (471 mg, 2.257 mmol) [compound described in J. Org. Chem., 1240, 21, (1956)] in dichloromethane (15 ml) was added dropwise 4-toluenesulfonyl isocyanate (523 mg, 2.652 mmol) at 0°C while stirring. The solution was stirred for 7.7 hours while allowing to warm gradually up to room temperature. The precipitated crystals were filtered off, washed with dichloromethane, and dried in vacuo, to yield desired 4-cyano-1-phenyl-5-{3-(4-toluenesulfonyl)ureido}-(1H)-pyrazole (820 mg, 84.1 %).
Melting point: 162-164°C.

### Preparation 2

### Synthesis of 1-phenyl-5-{3-(4-toluenesulfonyl)ureido}-(1H)-pyrazole [compound described in Pol. J. Pharmacol. Pharm. (1974), 26 (4), 479-482]

According to a procedure substantially same as that in Preparation 1, 5-amino-1-phenyl-(1H)-pyrazole and 4-toluenesulfonyl isocyanate were reacted to yield 1-phenyl-5-{3-(4-toluenesulfonyl)ureido}-(1H)-pyrazole. Melting point: 160-163°C.

### Preparation 3

### Synthesis of 3-methyl-1-phenyl-5-{3-(4-toluenesulfonyl)ureido}-(1H)-pyrazole [compound described in Pol. J. Pharmacol. Pharm. (1974), 26 (4), 479-482]

According to a procedure substantially same as that in Preparation 1, 5-amino-3-methyl-1-phenyl-(1H)-pyrazole [compound described in J. Org. Chem. 6155, 58, (1993)] and 4-toluenesulfonyl isocyanate were reacted to yield 3-methyl-1-phenyl-5-{3-(4-toluenesulfonyl)ureido)-(1H)-pyrazole. Melting point: 145-147°C.

### Preparation 4

### Synthesis of a sodium salt of 4-cyano-1-phenyl-5-{3-(4-toluenesulfonyl)ureido}-(1H)-pyrazole

According to a procedure substantially same as that in Example 44 hereinafter, a sodium salt of 4-cyano-1-phenyl-5-{3-(4-toluenesulfonyl)ureido}-(1H)-pyrazole was obtained from 4-cyano-1-phenyl-5-{3-(4-toluenesulfonyl)ureido}-(1H)-pyrazole.
IR (KBr) 3420, 2235, 1638, 1531, 1498, 1282, 1138 cm⁻¹.

### Preparation 5

### Synthesis of 3-{3-(4-toluenesulfonyl)ureido}-(1H)-pyrazole [compound described in the treatise of the department of agriculture in Meijo university 28, 49-59, (1992)]

To a solution of 3-amino-(1H)-pyrazole (3.830 g, 46.095 mmol) in a mixture of dichloromethane (20 ml) and tetrahydrofuran (20 ml) was added dropwise 4-toluenesulfonylisocyanate (7.10 ml, 46.443 mmol) at 0°C, and the mixture was stirred at 0°C for 40 minutes. After removing an ice-water bath, the mixture was stirred for 2 hours while allowing to warm gradually to room temperature. The solvent was evaporated in vacuo, the residue was recrystallized from dichloromethane, and the crystals were again recrystallized from a mixture of toluene and tetrahydrofuran, to yield 3-{3-(4-toluenesulfonyl)ureido}-(1H)-pyrazole.
IR (KBr) 3332, 1699, 1507, 1145, 1088 cm⁻¹.

### Preparation 6

### 4-ethoxycarbonyl-3-{3-(4-toluenesulfonyl)ureido}-(1H)-pyrazole [compound described in the treatise of the department of agriculture in Meijo university 28, 49-59, (1992)]

To a solution of 3-amino-4-ethoxycarbonyl-(1H)-pyrazole (5.283 g, 34.049 mmol) in a mixture of dichloromethane (20 ml) and tetrahydrofuran (20 ml) was added dropwise 4-toluenesulfonylisocyanate (5.50 ml, 35.977 mmol) at 0°C, and the mixture was stirred at 0°C for 30 minutes. After removing an ice-water bath, the mixture was stirred for 2.3 hours while allowing to warm gradually to room temperature. The precipitated crystals were filtered off, washed with dichloromethane, and then recrystallized from dichloromethane, to yield 4-ethoxycarbonyl-3-{3-(4-toluenesulfonyl)ureido}-(1H)-pyrazole.
IR (KBr) 3310, 1737, 1668, 1596, 1500, 1352, 1279, 1148, 1121, 1089, 944 cm⁻¹.

### Preparation 7

### Synthesis of 4-carboxy-3-{3-(4-toluenesulfonyl)ureido}-(1H)-pyrazole

To a solution of 4-ethoxycarbonyl-3-{3-(4-toluenesulfonyl)ureido}-(1H)-pyrazole (1.615 g, 4.583 mmol) in ethanol (60 ml) was added 40 ml of a 10N aqueous solution of potassium hydroxide at room temperature. The mixture was stirred for 60 minutes at room temperature, and then further stirred at 60 °C for an hour. The mixture was cooled to room temperature, neutralized by adding a 4 N solution of hydrochloric acid thereto, and the precipitated crystals were filtered off, washed with water, and dried in vacuo, to yield 4-carboxy-3-{3-(4-toluenesulfonyl)ureido}-(1H)-pyrazole (787 mg, 53.0 %).
IR (KBr) 3271, 1720, 1599, 1510, 1352, 1280, 1155, 1090 cm⁻¹.

### Example 1

### Synthesis of 4-cyano-1-phenyl-5-{(3-benzenesulfonyl)ureido}-(1H)-pyrazole

According to a procedure substantially same as that in Preparation 1, 5-amino-4-cyano-1-phenyl-(1H)-pyrazole [compound described in J. Org. Chem., 1240, 21, (1956)] and benzenesulfonylisocyanate were reacted to yield 4-cyano-1-phenyl-5-{(3-benzenesulfonyl)ureido}-(1H)-pyrazole.
Melting point: 159-161°C.

### Example 2

### Synthesis of 4-cyano-1-(2-pyridyl)-5-{3-(4-toluenesulfonyl)ureido}-(1H)-pyrazole

According to a procedure substantially same as that in Preparation 1, 5-amino-4-cyano-1-(2-pyridyl)-(1H)-pyrazole [compound described in Japanese Patent Publication (kokai) No. 195376/1987] and 4-toluenesulfonylisocyanate were reacted to yield 4-cyano-1-(2-pyridyl)-5-{3-(4-toluenesulfonyl)ureido}-(1H)-pyrazole.
Melting point: 253-256 °C.

### Example 3

### Synthesis of 4-cyano-1-methyl-5-{3-(4-toluenesulfonyl)ureido}-(1H)-pyrazole

According to a procedure substantially same as that in Preparation 1, 5-amino-4-cyano-1-methyl-(1H)-pyrazole [compound described in J. Org. Chem., 1240, 21, (1956)] and 4-toluenesulfonylisocyanate were reacted to yield 4-cyano-1-methyl-5-{3-(4-toluenesulfonyl)ureido}-(1H)-pyrazole.
Melting point: 300 °C or above.

### Example 4

### Synthesis of 4-cyano-1-cyclohexyl-5-{3-(4-toluenesulfonyl)ureido}-(1H)-pyrazole

According to a procedure substantially same as that in Preparation 1, 5-amino-4-cyano-1-cyclohexyl-(1H)-pyrazole [compound described in Chem. Abstract. 1459, 56, (1962)] and 4-toluenesulfonylisocyanate were reacted to yield 4-cyano-1-cyclohexyl-5-{3-(4-toluenesulfonyl)ureido}-(1H)-pyrazole.
Melting point: 273-277 °C.

### Example 5

### Synthesis of 4-cyano-1-(7-chloroquinolin-4-yl)-5-{3-(4-toluenesulfonyl)ureido}-(1H)-pyrazole

According to a procedure substantially same as that in Preparation 1, 5-amino-4-cyano-1-(7-chloroquinolin-4-yl)-(1H)-pyrazole [compound described in U.S. Patent No. 4622330] and 4-toluenesulfonylisocyanate were reacted to yield 4-cyano-1-(7-chloroquinolin-4-yl)-5-{3-(4-toluenesulfonyl)ureido}-(1H)-pyrazole.
Melting point: 300°C or above.

### Example 6

### Synthesis of 4-cyano-1-(4-nitrophenyl)-5-{3-(4-toluenesulfonyl)ureido}-(1H)-pyrazole

According to a procedure substantially same as that in Preparation 1, 5-amino-4-cyano-1-(4-nitrophenyl)-(1H)-pyrazole [compound described in J. Org. Chem., 1240, 21, (1956)] and 4-toluenesulfonylisocyanate were reacted to yield 4-cyano-1-(4-nitrophenyl)-5-{3-(4-toluenesulfonyl)ureido}-(1H)-pyrazole.
Melting point: 300°C or above.

### Example 7

### Synthesis of 4-cyano-1-(4-aminophenyl)-5-{3-(4-toluenesulfonyl)ureido}-(1H)-pyrazole

A solution of 4-cyano-1-(4-nitrophenyl)-5-{3-(4-toluenesulfonyl)ureido}-(1H)-pyrazole (480 mg, 1.126 mmol) and a 20% aqueous solution of titanium trichloride (8.70 ml, 11.281 mmol) in acetone (150 ml) was stirred for 4 hours at room temperature. The reaction solution was poured into an ice-water, and the mixture was made to pH 9 by adding saturated aqueous solution of sodium bicarbonate. The mixture was extracted with ethyl acetate, and the organic layer was washed with saturated brine, dried over anhydrous sodium sulfate, filtered, and evaporated in vacuo. The residue was purified by chromatography on silica gel (ethyl acetate-methanol 20:1) to yield 4-cyano-1-(4-aminophenyl)-5-{3-(4-toluenesulfonyl)ureido}-(1H)-pyrazole (88 mg, 19.7 %).
Melting point: 275°C (dec.) (recrystallized from dichloromethane).

### Example 8

### Synthesis of 4-cyano-1-(4-acetylaminophenyl)-5-{3-(4-toluenesulfonyl)ureido}-(1H)-pyrazole

A solution of 4-cyano-1-(4-aminophenyl)-5-{3-(4-toluenesulfonyl)ureido}-(1H)-pyrazole (8.8 mg, 0.0222 mmol), acetyl chloride (3 µl, 0.0422 mmol) and triethylamine (20 µl, 0.143 mmol) in a mixture of dichloromethane (2 ml) and tetrahydrofuran (2 ml) was stirred for an hour while allowing to warm from 0°C to room temperature. The mixture was poured into an ice-water, and extracted with ethyl acetate. The organic layer was washed with saturated brine, dried over anhydrous sodium sulfate, filtered, and evaporated in vacuo. The residue was purified by preparative thin layer chromatography (ethyl acetate-methanol 10:1) to yield 4-cyano-1-(4-acetylaminophenyl)-5-{3-(4-toluenesulfonyl)ureido}-(1H)-pyrazole (2.8 mg, 28.8 %).
IR (KBr) 3228, 2239, 1675, 1572, 1519, 1414, 1371, 1309, 1265 cm⁻¹.

### Example 9

### Synthesis of 4-cyano-1-(4-methylphenyl)-5-{3-(4-toluenesulfonyl)ureido}-(1H)-pyrazole

According to a procedure substantially same as that in Preparation 1, 5-amino-4-cyano-1-(4-methylphenyl)-(1H)-pyrazole [compound described in J. Org. Chem., 1240, 21, (1956)] and 4-toluenesulfonyl-isocyanate were reacted to yield 4-cyano-1-(4-methylphenyl)-5-{3-(4-toluenesulfonyl)ureido}-(1H)-pyrazole.
Melting point: 172-174°C.

### Example 10

### Synthesis of 4-cyano-1-(4-chlorophenyl)-5-{3-(4-toluenesulfonyl)ureido}-(1H)-pyrazole

According to a procedure substantially same as that in Preparation 1, 5-amino-cyano-1-(4-chlorophenyl)-(1H)-pyrazole [compound described in J. Org. Chem., 1240, 21, (1956)] and 4-toluenesulfonyl-isocyanate were reacted to yield 4-cyano-1-(4-chlorophenyl)-5-{3-(4-toluenesulfonyl)ureido}-(1H)-pyrazole.
Melting point: 168-170°C.

### Example 11

### Synthesis of 4-cyano-1-(4-bromophenyl)-5-{3-(4-toluenesulfonyl)ureido}-(1H)-pyrazole

According to a procedure substantially same as that in Preparation 1, 5-amino-4-cyano-1-(4-bromophenyl)-(1H)-pyrazole [compound described in J. Org. Chem., 1240, 21, (1956)] and 4-toluenesulfonyl-isocyanate were reacted to yield 4-cyano-1-(4-bromophenyl)-5-{3-(4-toluenesulfonyl)ureido}-(1H)-pyrazole.
Melting point: 179-181°C.

### Example 12

### Synthesis of 4-cyano-1-(1-naphthyl)-5-{3-(4-toluenesulfonyl)ureido}-(1H)-pyrazole

### a) Synthesis of 4-cyano-5-amino-1-(1-naphthyl)-(1H)-pyrazole

A solution of ethoxymethylene malononitrile (4.04 g, 33.080 mmol), 1-naphthyl hydrazine hydrochloride (6.44 g, 33.083 mmol), and sodium ethylate (2.26 g, 33.211 mmol) in ethanol (150 ml) was heated under reflux for 4 hours, and after cooling, the solution was evaporated in vacuo. To the residue was added water, and the mixture was extracted with ethyl acetate. The organic layer was washed with saturated brine, dried over sodium sulfate, filtered, and evaporated in vacuo.

The residue was purified by chromatography on silica gel (chloroform-ethyl acetate 10:1) to yield 5-amino-4-cyano-1-(1-naphthyl)-(1H)-pyrazole (2.299 g, 29.7 %). IR (KBr) 3646, 3381, 3164, 2216, 1662, 1536 cm⁻¹.

### b) Synthesis of 4-cyano-1-(1-naphthyl)-5-{3-(4-toluenesulfonyl)ureido}-(1H)-pyrazole

According to a procedure substantially same as that in Preparation 1, 5-amino-4-cyano-1-(1-naphthyl)-(1H)-pyrazole and 4-toluenesulfonylisocyanate were reacted to yield 4-cyano-1-(1-naphthyl)-5-{3-(4-toluenesulfonyl)ureido}-(1H)-pyrazole.
Melting point: 176-178°C.

### Example 13

### Synthesis of 4-cyano-1-(2-benzothiazolyl)-5-{3-(4-toluenesulfonyl)ureido}-(1H)-pyrazole

### a) Synthesis of 5-amino-4-cyano-1-(2-benzothiazolyl)-(1H)-pyrazole

A solution of ethoxymethylene malononitrile (1.944 g, 15.917 mmol), 2-hydrazinobenzothiazole (2.631 g, 15.924 mmol) in ethanol(150 ml) was heated under reflux for 4.8 hours. Then, while heating under reflux, about 100 ml of the ethanol was evaporated, and the mixture was cooled. The precipitaed crystals were collected by filtration, washed with ethanol, and evaporated in vacuo, to yield 5-amino-4-cyano-1-(2-benzothiazolyl)-(1H)-pyrazole (3.293 g, 85.7 %). Melting point: 248-250°C.

### b) Synthesis of 4-cyano-1-(2-benzothiazolyl)-5-{3-(4-toluenesulfonyl)ureido}-(1H)-pyrazole

According to a procedure substantially same as that in Preparation 1, 5-amino-4-cyano-1-(2-benzothiazolyl)-(1H)-pyrazole and 4-toluenesulfonylisocyanate were reacted to yield 4-cyano-1-(2-benzothiazolyl)-5-{3-(4-toluenesulfonyl)ureido}-(1H)-pyrazole.
Melting point: 300°C or above.

### Example 14

### Synthesis of 4-cyano-1-benzyl-5-{3-(4-toluenesulfonyl)ureido}-(1H)-pyrazole

According to a procedure substantially same as that in Preparation 1, the well-known 5-amino-4-cyano-1-benzyl-(1H)-pyrazole [compound described in Japanese Patent Publication (kokai) No. 115581/1985] and 4-toluenesulfonyl isocyanate were reacted to yield 4-cyano-1-benzyl-5-{3-(4-toluenesulfonyl)ureido}-(1H)-pyrazole.
Melting point: 153-156°C.

### Example 15

### Synthesis of 4-cyano-1-(2-imidazolinyl)-5-{3-(4-toluenesulfonyl)ureido}-(1H)-pyrazole

### a) Synthesis of 5-amino-4-cyano-1-(2-imidazolinyl)-(1H)-pyrazole

A solution of ethoxymethylenemalononitrile(4.070 g, 33.325 mmol), 2-hydrazino-2-imidazoline hydrobromide (6.030 g, 33.308 mmol), and sodium ethylate (2.404 g, 33.327 mmol) in ethanol (150 ml) was heated under reflux for 2.5 hours. After cooling, the solution was evaporated in vacuo. To the residue was added water, and the mixture was extracted with ethyl acetate. The organic layer was washed with saturated brine, dried over sodium sulfate, filtered, and evaporated in vacuo. The residue was recrystallized from ethanol. The material was further purified by chromatography on silica gel (chloroform-ethyl acetate 4:1), to yield 5-amino-4-cyano-1-(2-imidazolinyl)-(1H)-pyrazole (2.050 g, 35.1 %).
IR (KBr) 3363, 3208, 2233, 1647, 1556 cm⁻¹.

### b) Synthesis of 4-cyano-1-(2-imidazolinyl)-5-{3-(4-toluenesulfonyl)ureido}-(1H)-pyrazole

According to a procedure substantially same as that in Preparation 1, 5-amino-4-cyano-1-(2-imidazolinyl)-(1H)-pyrazole and 4-toluenesulfonylisocyanate were reacted to yield 4-cyano-1-(2-imidazolinyl)-5-{3-(4-toluenesulfonyl)ureido}-(1H)-pyrazole.
Melting point: 280°C (dec.).

### Example 16

### Synthesis of 4-cyano-1-(2-nitrophenyl)-5-{3-(4-toluenesulfonyl)ureido}-(1H)-pyrazole

According to a procedure substantially same as that in Preparation 1, 5-amino-4-cyano-1-(2-nitrophenyl)-(1H)-pyrazole [compound described in J. Heterocyclic. Chem., 511, 20, (1983)] and 4-toluenesulfonylisocyanate were reacted to yield 4-cyano-(2-nitrophenyl)-5-{3-(4-toluenesulfonyl)ureido}-(1H)-pyrazole.
Melting point: 167-170°C.

### Example 17

### Synthesis of 4-cyano-1-(2-aminophenyl)-5-{3-(4-toluenesulfonyl)ureido}-(1H)-pyrazole

A solution of 1-(2-nitrophenyl)-4-cyano-5-{3-(4-toluenesulfonyl)ureido}-(1H)-pyrazole (123 mg, 0.288 mmol), 10% palladium on carbon (34 mg) in a mixture of ethyl acetate (10 ml) and methanol(10 ml) was subjected to hydrogenation for two hours at room temperature. The solution was filtered through CELITE, and the filtrate was evaporated in vacuo. The residue was purified by chromatography on silica gel (methanol-chloroform-aqueous ammonia 10:90:1) to yield 4-cyano-1-(2-aminophenyl)-5-{3-(4-toluenesulfonyl)ureido}-(1H)-pyrazole (113 mg, 99.0 %).
Melting point: 193-196°C.

### Example 18

### Synthesis of 4-cyano-1-(2-methylphenyl)-5-{3-(4-toluenesulfonyl)ureido}-(1H)-pyrazole

According to a procedure substantially same as that in Preparation 1, 5-amino-4-cyano-1-(2-methylphenyl)-(1H)-pyrazole [compound described in Japanese Patent Publication (kohyo) No. 503069/1994] and 4-toluenesulfonyl isocyanate were reacted to yield 4-cyano-1-(2-nitrophenyl)-5-{3-(4-toluenesulfonyl)ureido}-(1H)-pyrazole.
Melting point: 156-159°C.

### Example 19

### Synthesis of 4-cyano-1-(2-chlorophenyl)-5-{3-(4-toluenesulfonyl)ureido}-(1H)-pyrazole

According to a procedure substantially same as that in Preparation 1, 5-amino-4-cyano-1-(2-chlorophenyl)-(1H)-pyrazole [compound described in J. Med. Chem. 2892, 34, (1991)] and 4-toluenesulfonyl isocyanate were reacted to yield 4-cyano-1-(2-chlorophenyl)-5-{3-(4-toluenesulfonyl)ureido}-(1H)-pyrazole.
Melting point: 174-177°C.

### Example 20

### Synthesis of 4-cyano-1-phenyl-5-{3-(2-toluenesulfonyl)ureido}-(1H)-pyrazole

According to a procedure substantially same as that in Preparation 1, 5-amino-4-cyano-1-phenyl-(1H)-pyrazole [compound described in J. Org. Chem., 1240, 21, (1956)] and 2-toluenesulfonyl isocyanate were reacted to yield 4-cyano-1-phenyl-5-{3-(2-toluenesulfonyl)ureido}-(1H)-pyrazole.
Melting point: 165-168°C.

### Example 21

### Synthesis of 4-cyano-1-cyclohexyl-5-{3-(2-toluenesulfonyl)ureido}-(1H)-pyrazole

To a solution of 5-amino-cyano-1-cyclohexyl-(1H)-pyrazole [compound described in Chem. Abstract. 1459, 56, (1962)] (210 mg, 1.105 mmol) in dichloromethane (10 ml) was added 2-toluenesulfonyl isocyanate (200 µl, 1.370 mmol) at 0°C, and the mixture was stirred at 0°C for 20 minutes. After removal of an ice-water bath, the mixture was further stirred for 100 minutes while allowing to gradually warm to room temperature. Ether was added to the mixture, and the precipitated crystals were collected by filtration. The crystals were purified by chromatography on silica gel (methanol-chloroform 1:9), and further purified by preparative thin layer chromatography (silica gel, methanol-chloroform 1:9) to yield 4-cyano-1-cyclohexyl-5-{3-(2-tolenesulfonyl)ureido}-(1H)-pyrazole (34 mg, 8.0 %).
IR (KBr) 2935, 2233, 1720, 1615, 1278 cm⁻¹.

### Example 22

### Synthesis of 1-phenyl-5-{3-(2-toluenesulfonyl)ureido}-(1H)-pyrazole

According to a procedure substantially same as that in Preparation 1, 5-amino-1-phenyl-(1H)-pyrazole and 2-toluenesulfonyl isocyanate were reacted to yield 1-phenyl-5-{3-(2-toluenesulfonyl)ureido}-(1H)-pyrazole.
Melting point: 160-163°C.

### Example 23

### Synthesis of 4-ethoxycarbonyl-1-phenyl-5-{3-(4-toluenesulfonyl)ureido}-(1H)-pyrazole

According to a procedure substantially same as that in Preparation 1, 5-amino-4-ethoxycarbonyl-1-phenyl-(1H)-pyrazole [compound described in J. Med. Chem., 3263, 35, (1992)] and 4-toluenesulfonyl isocyanate were reacted to yield 4-ethoxycarbonyl-1-phenyl-5-{3-(4-toluenesulfonyl)ureido}-(1H)-pyrazole.
IR (KBr) 3374, 2982, 1701, 1601, 1503, 1304, 1240, 1129, 1086, 759, 667, 556 cm⁻¹.

### Example 24

### Synthesis of 4-carboxy-1-phenyl-5-{3-(4-toluenesulfonyl)ureido}-(1H)-pyrazole

A solution of 4-ethoxycarbonyl-1-phenyl-5-{3-(4-toluenesulfonyl)ureido}-(1H)-pyrazole (471 mg, 1.136 mmol) and a 10N aqueous solution of potassium hydroxide in ethanol (10 ml) was stirred for 9.7 hours at room temperature. The mixture was cooled to 0°C, and added dropwise with a 4N solution of hydrochloric acid until the pH was 5. The precipitated crystals were collected by filtration, washed with water, and evaporated in vacuo, to yield 4-carboxy-1-phenyl-5-{3-(4-toluenesulfonyl)ureido}-(1H)-pyrazole (202 mg , 47.2 %).
Melting point: 204-207°C.

### Example 25

### Synthesis of 4-cyano-1-phenyl-3-methyl-5-{3-(4-toluenesulfonyl)ureido}-(1H)-pyrazole

According to a procedure substantially same as that in Preparation 1, 5-amino-4-cyano-1-phenyl-3-methyl-(1H)-pyrazole [compound described in J. Org. Chem., 1240, 21, (1956)] and 4-toluenesulfonyl isocyanate were reacted to yield 4-cyano-1-phenyl-3-methyl-5-{3-(4-toluenesulfonyl)ureido}-(1H)-pyrazole.
Melting point: 169-172°C.

### Example 26

### Synthesis of 4-cyano-1-phenyl-3-methyl-5-{3-(2-toluenesulfonyl)ureido}-(1H)-pyrazole

To a solution of 5-amino-4-cyano-1-phenyl-3-methyl-(1H)-pyrazole [compound described in J. Org. Chem., 1240, 21, (1956)] (228 mg, 1.149 mmol) in dichloromethane (10 ml) was added dropwise 2-toluenesulfonyl isocyanate (200 µl, 1.370 mmol) at 0°C, and the mixture was stirred at 0°C for 20 minutes. After removing an ice-water bath, the mixture was further stirred for 100 minutes while allowing to gradually warm to room temperature. Ether was added to the reaction, and the precipitated crystals were filtered off. The crystals were purified by chromatography on silica gel (methanol-chloroform 1:9) to yield 4-cyano-1-phenyl-3-methyl-5-{3-(2-toluenesulfonyl)ureido}-(1H)-pyrazole (32 mg, 7.0 %).
IR (KBr) 3418, 2231, 1617, 1310, 1272 cm⁻¹.

### Example 27

### Synthesis of 4-cyano-3-ethyl-1-phenyl-5-{3-(4-toluenesulfonyl)ureido}-(1H)-pyrazole

According to a procedure substantially same as that in Preparation 1, 5-amino-4-cyano-1-phenyl-3-ethyl-(1H)-pyrazole [compound described in Chem. Abstract. 79, 146518] and 4-toluenesulfonyl isocyanate were reacted to yield 4-cyano-3-ethyl-1-phenyl-5-{3-(4-toluenesulfonyl)ureido}-(1H)-pyrazole.
Melting point: 161-164°C.

### Example 28

### Synthesis of 4-cyano-3-ethyl-1-phenyl-5-{3-(2-toluenesulfonyl)ureido}-(1H)-pyrazole

According to a procedure substantially same as that in Preparation 1, 5-amino-4-cyano-3-ethyl-1-phenyl-(1H)-pyrazole [compound described in Chem. Abstract. 79, 146518] and 2-toluenesulfonyl isocyanate were reacted to yield 4-cyano-3-ethyl-1-phenyl-5-{3-(2-toluenesulfonyl)ureido}-(1H)-pyrazole.
Melting point: 159-161°C.

### Example 29

### Synthesis of 3-n-butyl-4-cyano-1-phenyl-5-{3-(4-toluenesulfonyl)ureido}-(1H)-pyrazole

### a) Synthesis of 1-ethoxypentylidene malononitrile

A solution of triethyl orthovalerate (15.773 g, 77.201 mmol) and malononitrile (5.100g, 77.203 mmol) in anhydrous acetic acid (100 ml) was heated under reflux for three hours. The anhydrous acetic acid was evaporated in vacuo. To the residue was added water, and the mixture was extracted with ethyl acetate. The organic layer was washed with saturated brine for many times, dried over anhydrous sodium sulfate, and evaporated in vacuo. The residue was purified by chromatography on silica gel (hexane-ethyl acetate 3:1 ∼ 1:1) to yield 1-ethoxypentylidene malononitrile (10.566 g, 76.8 %).
IR (KBr) 2970, 2940, 2880, 2225, 1570, 1470, 1380, 1345, 1225, 1050 cm⁻¹.

### b) Synthesis of 5-amino-3-n-butyl-4-cyano-1-phenyl-(1H)-pyrazole

A solution of 1-ethoxypentylidene malononitrile (2.701 g, 15.154 mmol) and phenyl hydrazine (1.664 g, 15.397 mmol) in ethanol (60 ml) was heated under reflux for five hours. After cooling, the solvent was evaporated in vacuo to give the residue. The residue was purified by chromatography on silica gel (hexane-ethyl acetate 10:1 ∼ 1:1) to give 1-ethoxypentylidene malononitrile (3.640 g, 100 %).
IR (neat) 3340, 3225, 2950, 2930, 2880, 2225, 1630, 1600, 1560, 1540, 1500, 1455, 1070, 760, 690 cm⁻¹.

### c) Synthesis of 3-n-butyl-4-cyano-1-phenyl-5-{3-(4-toluenesulfonyl)ureido}-(1H)-pyrazole

According to a procedure substantially same as that in Preparation 1, 5-amino-4-cyano-1-phenyl-2-n-butyl-(1H)-pyrazole and 4-toluenesulfonyl isocyanate were reacted to yield 3-n-butyl-4-cyano-1-phenyl-5-{3-(4-toluenesulfonyl)ureido}-(1H)-pyrazole.
Melting point: 229-231°C.

### Example 30

### Synthesis of 3-n-butyl-4-cyano-1-phenyl-5-{3-(2-toluenesulfonyl)ureido}-(1H)-pyrazole

According to a procedure substantially same as that in Example 21, reaction was performed starting from 5-amino-4-cyano-1-phenyl-2-n-butyl-(1H)-pyrazole and 2-toluenesulfonyl isocyanate, and then purification was performed by preparative thin layer chromatography (silica gel, methanol-chloroform 1:9), to yield 3-n-butyl-4-cyano-1-phenyl-5-{3-(2-toluenesulfonyl)ureido}-(1H)-pyrazole.
IR (KBr) 2958, 2230, 1623, 1260 cm⁻¹.

### Example 31

### Synthesis of 4-cyano-1, 3-diphenyl-5-{3-(4-toluenesulfonyl)ureido}-(1H)-pyrazole

According to a procedure substantially same as that in Preparation, 1, 5-amino-4-cyano-1, 3-diphenyl-(1H)-pyrazole [compound described in J. Heterocyclic. Chem. 647, 27, (1990)] and 4-toluenesulfonyl isocyanate were reacted to yield 4-cyano-1, 3-diphenyl-5-{3-(4-toluenesulfonyl)ureido}-(1H)-pyrazole.
Melting point: 224-226°C.

### Example 32

### Synthesis of 4-cyano-1, 3-diphenyl-5-{3-(2-toluenesulfonyl)ureido}-(1H)-pyrazole

According to a procedure substantially same as that in Preparation 1, 5-amino-4-cyano-1, 3-diphenyl-(1H)-pyrazole [compound described in J. Heterocyclic. Chem. 647, 27, (1990)] and 2-toluenesulfonyl isocyanate were reacted to yield 4-cyano-1, 3-diphenyl-5-{3-(2-toluenesulfonyl)ureido}-(1H)-pyrazole.
Melting point: 219-221°C.

### Example 33

### Synthesis of 4-cyano-3-cyanomethyl-1-phenyl-5-{3-(4-toluenesulfonyl)ureido}-(1H)-pyrazole

According to a procedure substantially same as that in Preparation 1, 5-amino-4-cyano-3-cyanomethyl-1-phenyl-(1H)-pyrazole [compound described in J. Am. Chem. Soc., 2456, 81, (1959)] and 4-toluenesulfonyl isocyanate were reacted to yield 4-cyano-3-cyanomethyl-1-phenyl-5-{3-(4-toluenesulfonyl)ureido}-(1H)-pyrazole.
Melting point: 159-161°C.

### Example 34

### Synthesis of 4-cyano-3-ethoxycarbonylmethyl-1-phenyl-5-{3-(4-toluenesulfonyl)ureido}-(1H)-pyrazole

According to a procedure substantially same as that in Preparation 1, 5-amino-4-cyano-3-ethoxycarbonylmethyl-1-phenyl-(1H)-pyrazole [compound described in J. Am. Chem. Soc., 2456, 81, (1959)] and 4-toluenesulfonylisocyanate were reacted to yield 4-cyano-3-ethoxycarbonylmethyl-1-phenyl-5-{3-(4-toluenesulfonyl)ureido}-(1H)-pyrazole.
Melting point: 108-110°C.

### Example 35

### Synthesis of 4-cyano-3-carboxymethyl-1-phenyl-5-{3-(4-toluenesulfonyl)ureido}-(1H)-pyrazole

To a solution of 4-cyano-3-ethoxycarbonylmethyl-1-phenyl-5-{3-(4-toluenesulfonyl)ureido}-(1H)-pyrazole (502 mg, 1.073 mmol) in ethanol (20 ml) was added dropwise a 4N aqueous solution of potassium hydroxide (5 ml) at 0°C. The solution was allowed to warm to 10°C over 1.5 hours while stirring. The mixture was cooled to 0°C, and was added with a 2N solution of hydrochloric acid until the pH was 3. The mixture was extracted with ethyl acetate, washed with saturated brine, dried over sodium sulfate, filtered, and evaporated in vacuo, to yield 4-cyano-3-carboxymethyl-1-phenyl-5-{3-(4-toluenesulfonyl)ureido}-(1H)-pyrazole (364 mg, 77.1 %).
IR (KBr) 3216, 2232, 1702, 1598, 1534, 1478, 1351, 1235, 1162 cm⁻¹.

### Example 36

### Synthesis of 4-cyano-1-phenyl-5-{3-(4-chlorobenzenesulfonyl)ureido}-(1H)-pyrazole

According to a procedure substantially same as that in Preparation 1, 5-amino-4-cyano-1-phenyl-(1H)-pyrazole [compound described in J. Org. Chem., 1240, 21, (1956)] and 4-chlorobenzenesulfonyl isocyanate were reacted to yield 4-cyano-1-phenyl-5-{3-(4-chlorobenzenesulfonyl)ureido}-(1H)-pyrazole.
Melting point: 159-161°C.

### Example 37

### Synthesis of 4-cyano-3-methyl-1-phenyl-5-{3-(4-chlorobenzenesulfonyl)ureido}-(1H)-pyrazole

According to a procedure substantially same as that in Preparation 1, 5-amino-4-cyano-3-methyl-1-phenyl-(1H)-pyrazole [compound described in J. Org. Chem., 1240, 21, (1956)] and 4-chlorobenzenesulfonyl isocyanate were reacted to yield 4-cyano-3-methyl-1-phenyl-5-{3-(4-chlorobenzenesulfonyl)ureido}-(1H)-pyrazole.
Melting point: 163-165°C.

### Example 38

### Synthesis of 4-cyano-3-ethyl-1-phenyl-5-{3-(4-chlorobenzenesulfonyl)ureido}-(1H)-pyrazole

According to a procedure substantially same as that in Preparation 1, 5-amino-4-cyano-3-ethyl-1-phenyl-(1H)-pyrazole [compound described in Chem. Abstract. 79, 146518] and 4-chlorobenzenesulfonyl isocyanate were reacted to yield 4-cyano-3-ethyl-1-phenyl-5-{3-(4-chlorobenzenesulfonyl)ureido}-(1H)-pyrazole.
Melting point: 152-154°C.

### Example 39

### Synthesis of 4-cyano-3-(2-dimethylaminoethyl)-1-phenyl-5-{3-(4-chlorobenzenesulfonyl)ureido}-(1H)-pyrazole

### a) Synthesis of 4-cyano-5-{di-(tert-butoxycarbonyl)-amino}-3-ethoxycarbonylmethyl-1-phenyl-(1H)-pyrazole

A solution of 5-amino-4-cyano-3-ethoxycarbonylmethyl-1-phenyl-(1H)-pyrazole (6.025 g, 22.291 mmol), di-*tert*-butyl carbonate (10.640 g, 48.751 mmol), and dimethylamino-pyridine (300 mg, 2.456 mmol) in dichloromethane (50 ml) was stirred for four hours at room temperature. To the mixture was added water, then the organic layer was separated and the aqueous layer was extracted with chloroform. The combined organic layers were washed with saturated brine, dried over anhydrous sodium sulfate, filtered, and evaporated in vacuo. The residue was purified by chromatography on silica gel (chloroform-ethyl acetate 4:1) to yield 4-cyano-5-{di-(*tert*-butoxycarbonyl)-amino}-3-ethoxycarbonylmethyl-1-phenyl-(1H)-pyrazole (10.488 g, 100 %).
IR (neat) 3000, 2950, 2245, 1815, 1780, 1740, 1575, 1505, 1455, 1400, 1375, 1255, 1150, 1120, 1100 cm⁻¹.

### b) Synthesis of 4-cyano-5-tert-butoxycarbonylamino-3-(2-hydroxyethyl)-1-phenyl-(1H)-pyrazole

In a nitrogen atmosphere, a solution of 4-cyano-5-{di-(*tert*-butoxycarbonyl)-amino)-3-ethoxycarbonylmethyl-1-phenyl-(1H)-pyrazole (12.115 g, 25.748 mmol) in tetrahydrofuran (100 ml) was added dropwise to a suspension of lithium aluminum hydride (977 mg, 25.744 mmol) in tetrahydrofuran (50 ml) over 20 minutes at 0°C. The solution was stirred at 0°C for 70 minutes. To the reaction solution was added dropwise a mixture of 1:1 tetrahydrofuran and water at 0°C. The resultant solution was filtered through CELITE, and the filtrate was evaporated in vacuo. To the residue was added saturated brine, and the mixture was extracted with ethyl acetate. The organic layer was washed with saturated brine, dried over anhydrous sodium sulfate, filtered and evaporated in vacuo. The residue was purified by chromatography on silica gel (chloroform-ethyl acetate 4:1→ 1:1) to yield 4-cyano-5-*tert*-butoxycarbonylamino-3-(2-hydroxyethyl)-1-phenyl-(1H)-pyrazole (3.252 g, 38.5 %).
IR (KBr) 3339, 2981, 2230, 1700, 1568, 1535, 1372, 1356, 1159, 774 cm⁻¹.

### c) Synthesis of 4-cyano-5-tert-butoxycarbonylamino-3-(2-dimethylaminoethyl)-1-phenyl-(1H)-pyrazole

A solution of 4-cyano-5-*tert*-butoxycarbonylamino-3-(2-hydroxyethyl)-1-phenyl-(1H)-pyrazole (789 mg, 2.403 mmol), methanesulfonyl chloride (195 µl, 2.519 mmol) and triethylamine(670 µl, 4.807 mmol) in dichloromethane (40 ml) was stirred at 0°C for 2.5 hours. To the solution was added water, then the organic layer was separated and the aqueous layer was extracted with chloroform. The combined organic layers were washed with saturated brine, dried over anhydrous sodium sulfate, and evaporated in vacuo. The residue was purified by chromatography on silica gel (chloroform-ethyl acetate 4:1) to yield a residue containing 4-cyano-5-*tert*-butoxycarbonylamino-3-(2-methanesulfonyloxyethyl)-1-phenyl-(1H)-pyrazole (982 mg). A mixture of a portion of this residue (857 mg), and a solution of 50% aqueous dimethylamine (570 µl) in dimethylformamide (40 ml) was stirred for two hours at room temperature. Additional 50% aqueous dimethylamine (600 µl) was added, and the mixture was stirred for two hours at room temperature. To the reaction was added water, and the mixture was extracted with ethyl acetate. The organic layer was washed with saturated brine, dried over anhydrous sodium sulfate, and evaporated in vacuo. The residue was purified by chromatography on silica gel (chloroform-methanol 97:3) to yield 4-cyano-5-*tert*-butoxycarbonylamino-3-(2-dimethylaminoethyl)-1-phenyl-(1H)-pyrazole (650 mg, 76.1 %).
IR (KBr) 2983, 2786, 2232, 1731, 1597, 1575, 1504, 1456, 1395, 1369, 1280, 1256, 1160, 1012, 767 cm⁻¹.

### d) Synthesis of 5-amino-4-cyano-3-(2-dimethylaminoethyl)-1-phenyl-(1H)-pyrazole

To a solution of 4-cyano-5-*tert*-butoxycarbonylamino-3-(2-dimethylaminoethyl)-1-phenyl-(1H)-pyrazole (9.8 mg, 0.0276 mmol) in dichloromethane (1 ml) was added trifluoroacetic acid (145 µl, 1.882 mmol) at 0°C, and the mixture was stirred for nine hours while allowing to warm gradually to room temperature. The mixture was evaporated in vacuo. To the residue was added aqueous ammonia, and the mixture was extracted with chloroform. The organic layer was washed with saturated brine, dried over anhydrous sodium sulfate, and evaporated in vacuo. The residue was purified by preparative thin layer chromatography (silica gel, methanol-chloroform-aqueous ammonia 10:90:1) to yield 5-amino-4-cyano-3-(2-dimethyl-aminoethyl)-1-phenyl-(1H)-pyrazole (1.8 mg, 25.5 %).
IR (KBr) 3364, 2826, 2209, 1654, 1573, 1535, 1495, 1465, 779, 697 cm⁻¹.

### e) Synthesis of 4-cyano-3-(2-dimethylaminoethyl)-1-phenyl-5-{3-(4-chlorobenzenesulfonyl)ureido}-(1H)-pyrazole

According to a procedure substantially same as that in Preparation 1, 5-amino-4-cyano-3-(2-dimethylaminoethyl)-1-phenyl-(1H)-pyrazole and 4-chlorobenzenesulfonylisocyanate were reacted to yield 4-cyano-3-(2-dimethylaminoethyl)-1-phenyl-5-{3-(4-chlorobenzenesulfonyl)ureido}-(1H)-pyrazole.
Melting point: 168-170°C.

### Example 40

### Synthesis of 4-cyano-1-phenyl-5-{3-(4-nitrobenzenesulfonyl)ureido}-(1H)-pyrazole

To a solution of 5-amino-4-cyano-1-phenyl-(1H)-pyrazole [compound described in J. Org. Chem., 1240, 21, (1956)] (960 mg, 5.212 mmol) in dichloromethane (30 ml) was added dropwise a solution of 4-nitrobenzenesulfonyl isocyanate [compound described in Tetrahedron Letters, 2839, 34, (1993)] (1.124 g, 4.926 mmol) in dichloromethane(5 ml) at 0°C, and the mixture was stirred at 0°C for 20 minutes. After removal of an ice-water bath, the mixture was further stirred for 100 minutes while allowing to gradually warm to room temperature. The mixture was poured into an ice-water, and the mixture was extracted with chloroform. The organic layer was washed with saturated brine, dried over anhydrous sodium sulfate, and evaporated in vacuo. The residue was purified twice by chromatography on silica gel (methanol-chloroform 1:9) to yield 4-cyano-1-phenyl-5-{3-(4-nitrobenzenesulfonyl)ureido}-(1H)-pyrazole (62 mg, 3.1 %).
¹H-NMR (DMSO-d₆) δ; 8.37 (2H, m), 8.20 (1H, m), 8.17 (2H, m), 7.78 (2H, m), 7.53 (2H, m), 7.42 (1H, m).

### Example 41

### Synthesis of 4-cyano-1-phenyl-5-{3-(4-aminobenzenesulfonyl)ureido}-(1H)-pyrazole

A solution of 4-cyano-1-phenyl-5-{3-(4-nitrobenzenesulfonyl)ureido}-(1H)-pyrazole (60.0 mg, 0.145 mmol) and 20% aqueous titanium trichloride (1.20 ml, 1.556 mmol) in acetone (5 ml) was stirred for seven fours at room temperature. The mixture was poured into an ice-water, then a saturated solution of sodium bicarbonate was added, and the mixture was extracted with ethyl acetate. The organic layer was washed with saturated brine, dried over anhydrous sodium sulfate, and evaporated in vacuo. The residue was purified by preparative thin layer chromatography (silica gel, methanol-ethyl acetate 1:10) to yield 4-cyano-1-phenyl-5-{3-(4-aminobenzenesulfonyl)ureido}-(1H)-pyrazole (5.6 mg, 10.1 %).
IR (KBr) 3358, 2925, 1592, 1502, 1144, 1082 cm⁻¹.

### Example 42

### Synthesis of 4-cyano-3-isopropyl-1-phenyl-5-{3-(4-toluenesulfonyl)ureido}-(1H)-pyrazole

### a) Synthesis of isopropylhydroxymethylene malononitrile

To a solution of malononitrile (2.591 g, 39.222 mmol) and triethylamine (7.986 g, 78.921 mmol) in benzene (50 ml) was added dropwise isobutyryl chloride (4.084 g, 38.329 mmol) at 0°C. After removal of an ice-water bath, the mixture was stirred for two hours while allowing to gradually warm to room temperature. To the reaction was added water, and the mixture was extracted with ethyl acetate. The aqueous layer was made to pH 1 by adding a 4N solution of sulfuric acid, and the mixture was extracted with ethyl acetate. The organic layer was dried over anhydrous sodium sulfate, and evaporated in vacuo to yield isopropylhydroxymethylene malononitrile (4.894 g, 94.5 %).
IR (KBr) 3202, 2983, 2242, 2228, 1560, 1464, 1253, 1095, 980 cm⁻¹.

### b) Synthesis of isopropylmethoxymethylene malononitrile

A solution of isopropylhydroxymethylene malononitrile (4.307 g, 31.869 mmol), dimethyl sulfate (10 ml, 105.685 mmol), sodium carbonate (10.375 g, 97.887 mmol) and water (8 ml) in 1, 4-dioxane (75 ml) was heated under reflux at 70-80°C for 6 hours. To the reaction was added an ice-water, and the mixture was extracted with ethyl acetate. The organic layer was washed with saturated brine, and evaporated in vacuo. The residue was purified by chromatography on silica gel (hexane-ethyl acetate 10:1) to yield isopropylmethoxymethylene malononitrile (1.470 g, 30.7 %).
IR (neat) 2990, 2225, 1570, 1470, 1335, 1215, 1105, 1000, 955 cm⁻¹.

### c) Synthesis of 5-amino-4-cyano-3-isopropyl-1-phenyl-(1H)-pyrazole

A solution of isopropylmethoxymethylene malononitrile (937 mg, 6.239 mmol) and phenyl hydrazine (681 mg, 6.301 mmol) in ethanol (20 ml) was heated under reflux for three hours. After cooling, the ethanol was evaporated in vacuo. The residue was purified by chromatography on silica gel (hexane-ethyl acetate 10:1→3:1) to yield 5-amino-4-cyano-3-isopropyl-1-phenyl-(1H)-pyrazole (1.370 g, 97.0 %).
IR (KBr) 3364, 2826, 2209, 1654, 1573, 1535, 1495, 1465, 779, 697 cm⁻¹.

### d) Synthesis of 4-cyano-3-isopropyl-1-phenyl-5-{3-(4-toluenesulfonyl)ureido}-(1H)-pyrazole

According to a procedure substantially same as that in Preparation 1, 5-amino-4-cyano-3-isopropyl-1-phenyl-(1H)-pyrazole and 4-toluenesulfonyl isocyanate were reacted to yield 4-cyano-3-isopropyl-1-phenyl-5-{3-(4-toluenesulfonyl)ureido}-(1H)-pyrazole.
Melting point: 161-163°C.

### Example 43

### Synthesis of 4-cyano-3-isopropyl-1-phenyl-5-{3-(4-chlorobenzenesulfonyl)ureido}-(1H)-pyrazole

According to a procedure substantially same as that in Preparation 1, 5-amino-4-cyano-3-isopropyl-1-phenyl-(1H)-pyrazole and 4-chlorobenzenesulfonyl isocyanate were reacted to yield 4-cyano-3-isopropyl-1-phenyl-5-{3-(4-chlorobenzenesulfonyl)ureido}-(1H)-pyrazole.
IR (KBr) 3324, 2972, 2233, 1720, 1582, 1508, 1468, 1159, 1091 cm⁻¹.

### Example 44

### Synthesis of a sodium salt of 4-cyano-3-methyl-1-phenyl-5-{3-(4-chlorobenzenesulfonyl)ureido}-(1H)-pyrazole

4-Cyano-3-methyl-1-phenyl-5-{3-(4-chlorobenzenesulfonyl)ureido}-(1H)-pyrazole (388 mg, 0.750 mmol) was suspended in water (40 ml), and to the suspension was added 750 µl of a 1N aqueous solution of sodium hydroxide while stirring at room temperature, and then the mixture was stirred for one hour. The insoluble material was filtered out, and the filtrate was evaporated in vacuo to yield a sodium salt of 4-cyano-3-methyl-1-phenyl-5-{3-(4-chlorobenzenesulfonyl)-ureido}-(1H)-pyrazole (327 mg, 80.0 %)
IR (KBr) 3411, 2230, 1638, 1572, 1535, 1497, 1395, 1307, 1257, 1148, 1074 cm⁻¹.

### Example 45

### Synthesis of a sodium salt of 4-cyano-3-methyl-1-phenyl-5-{3-(4-toluenesulfonyl)ureido}-(1H)-pyrazole

According to a procedure substantially same as that in Example 44, a sodium salt of 4-cyano-3-methyl-1-phenyl-5-{3-(4-toluenesulfonyl)ureido}-(1H)-pyrazole was obtained starting from 4-cyano-3-methyl-1-phenyl-5-{3-(4-toluenesulfonyl)ureido)-(1H)-pyrazole.
IR (KBr) 3422, 2229, 1640, 1536, 1497, 1307, 1260, 1145, 1074 cm⁻¹.

### Example 46

### Synthesis of a sodium salt of 4-cyano-1-cyclohexyl-5-{3-(4-toluenesulfonyl)ureido}-(1H)-pyrazole

According to a procedure substantially same as that in Example 44, a sodium salt of 4-cyano-1-cyclohexyl-5-{3-(4-toluenesulfonyl)ureido}-(1H)-pyrazole was obtained starting from 4-cyano-1-cyclohexyl-5-{3-(4-toluenesulfonyl)ureido}-(1H)-pyrazole.
IR (KBr) 3399, 2931, 2856, 2236, 1629, 1575, 1455, 1275, 1144, 1099 cm⁻¹.

### Example 47

### Synthesis of 4-cyano-1-phenyl-5-{3-(4-isopropylbenzenesulfonyl)ureido}-(1H)-pyrazole

To a solution of 5-amino-4-cyano-1-phenyl-(1H)-pyrazole [compound described in J. Org. Chem. 1240, 21, (1956)] (354 mg, 1.922 mmol) in dichloromethane (10 ml) was added dropwise a solution of 4-isopropylbenzenesulfonyl isocyanate [compound described in German Patent No. 1289526] in cumene (2 ml) at 0°C. The mixture was stirred at 0°C for 20 minutes, and after removing an ice-water bath, the mixture was further stirred for 100 minutes while allowing to gradually warm to room temperature. The reaction was evaporated in vacuo. The residue was purified by chromatography on silica gel (methanol-chloroform 1:19), and further purified by preparative thin layer chromatography (silica gel, methanol-chloroform 1:9) to yield 4-cyano-1-phenyl-5-{3-(4-isopropyl-benzenesulfonyl)ureido}-(1H)- pyrazole (2.6 mg, 3.3 %).
¹H-NMR (CD₃OD) δ; 7.93 (1H, s), 7.74 (2H, m), 7.41-7.49 (5H, m), 7.27 (2H, m), 2.95 (1H, Hep, J=6.9Hz), 1.26 (6H, d, J=6.9Hz).

### Example 48

### Synthesis of 4-cyano-3-methyl-1-phenyl-5-{3-(4-isopropyl-benzenesulfonyl)ureido}-(1H)-pyrazole

According to a procedure substantially same as that in Example 47, 5-amino-4-cyano-3-methyl-1-phenyl-(1H)-pyrazole [compound described in J. Org. Chem. 1240, 21, (1956)] and 4-isopropylbenzenesulfonyl isocyanate[compound described in German Patent No. 1289526] were reacted to yield 4-cyano-3-methyl-1-phenyl-5-{3-(4-isopropylbenzenesulfonyl)ureido}-(1H)-pyrazole.
¹H-NMR (CD₃OD) δ; 7.76 (2H, m), 7.41 (2H, m), 7.31 (2H, m), 2.97 (1H, Hep, J=6.9Hz), 2.35 (3H, s), 1.27 (6H, d, J =6.9Hz).

### Example 49

### Synthesis of 1-phenyl-5-{3-(4-chlorobenzenesulfonyl)ureido}-(1H)-pyrazole

According to a procedure substantially same as that in Preparation 1, 5-amino-1-phenyl-(1H)-pyrazole and 4-chlorobenzenesulfonyl isocyanate were reacted to yield 1-phenyl-5-{3-(4-chlorobenzenesulfonyl)ureido}-(1H)-pyrazole.
Melting point: 176-178°C.

### Example 50

### Synthesis of 3-methyl-1-phenyl-5-{3-(4-chlorobenzenesulfonyl)ureido}-(1H)-pyrazole

According to a procedure substantially same as that in Preparation 1, 5-amino-3-methyl-1-phenyl-(1H)-pyrazole [compound described in J. Org. Chem. 6155, 58, (1993)] and 4-chlorobenzenesulfonyl isocyanate were reacted to yield 3-methyl-1-phenyl-5-{3-(4-chlorobenzenesulfonyl)ureido}-(1H)-pyrazole.
Melting point: 157-159°C.

### Example 51

### Synthesis of 5-{3-(4-chlorobenzenesulfonyl)-3-methylureido}-4-cyano-3-methyl-1-phenyl-(1H)-pyrazole

A mixture of a sodium salt of 5-{3-(4-chlorobenzenesulfonyl)ureido}-4-cyano-3-methyl-1-phenyl-(1H)-pyrazole (73 mg, 0.167 mmol), and a solution of iodomethane (15 µl, 0.241 mmol) in dimethylformamide (2.0 ml) was stirred for 4.3 hours at room temperature. The mixture was poured into an ice-water, and the mixture was extracted with ethyl acetate. The organic layer was washed with saturated brine, dried over anhydrous sodium sulfate, and evaporated in vacuo. The residue was purified by chromatography on silica gel (chloroform) to yield 5-{3-(4-chlorobenzenesulfonyl)-3-methylureido}-4-cyano-3-methyl-1-phenyl-(1H)-pyrazole (34 mg, 47.5 %).
IR (KBr) 3328, 2231, 1710, 1575, 1506, 1358, 1157, 1086, 969, 761, 625 cm⁻¹.

### Example 52

### Synthesis of 5-{3-(4-chlorobenzenesulfonyl)-1-benzylureido}-4-cyano-3-methyl-1-phenyl-(1H)-pyrazole

### a) Synthesis of 5-benzylamino-4-cyano-3-methyl-1-phenyl-(1H)-pyrazole

A solution of 5-amino-4-cyano-3-methyl-1-phenyl-(1H)-pyrazole (1.024 g, 5.166 mmol), benzyl bromide (0.6 ml, 5.044 mmol), potassium carbonate (2.303 g, 16.663 mol) in dimethylformamide (25 ml) was stirred for 70 minutes at room temperature. The mixture was poured into an ice-water, and the mixture was extracted with ethyl acetate. The organic layer was washed with saturated brine, and evaporated in vacuo. The residue was purified by chromatography on silica gel (hexane-ethyl acetate 10:1) to yield 5-benzylamino-4-cyano-3-methyl-1-phenyl-(1H)-pyrazole (125 mg, 8.4 %). ¹H-NMR (CDCl₃); 7.26-7.48 (10H, m), 4.65 (2H, s), 2.31 (3H, s). b) According to a procedure substantially same as that in Preparation 1, 5-benzylamino-4-cyano-3-methyl-1-phenyl-(1H)-pyrazole and 4-chlorobenzenesulfonyl isocyanate were reacted to yield 5-{3-(4-chlorobenzenesulfonyl)-1-benzylureido}-4-cyano-3-methyl-1-phenyl-(1H)-pyrazole.
IR (KBr) 3436, 2232, 1582, 1504, 1380, 1321, 1251, 1133, 1086, 868, 758, 696, 640 cm⁻¹.

### Example 53

### Synthesis of 5-{3-(4-chlorobenzenesulfonyl)ureido}-4-cyano-3-methyl-1-(1-benzylpiperidin-4-yl)-(1H)-pyrazole

### a) Synthesis of 4-(2-benzoylhydrazino)-1-benzylpiperidine

To a solution of benzoylhydrazine (7.70 g, 56.555 mmol) in methanol (50 ml) was added dropwise 1-benzyl-4-piperidone (10.5 ml, 56.644 mmol) over 10 minutes at 0°C. Then, after removal of an ice-water bath, the mixture was heated under reflux at 60°C for six hours. The mixture was again cooled to 0°C. To the mixture was added portionwise sodium borohydride (1.97 mg, 52.075 mmol), and the mixture was stirred for two hours. The methanol was evaporated in vacuo to yield a residue, to which was added water, and the mixture was extracted with dichloromethane. The organic layer was dried over magnesium sulfate, and evaporated in vacuo. The residue was recrystallized from ethanol to yield 4-(2-benzoylhydrazino)-1-benzylpiperidine (11.897 g, 70.7 %).
¹H-NMR(CDCl₃) 7.74 (2H, m), 7.40-7.59 (4H, m), 7.22-7.32 (6H, m), 4.88 (1H, m), 3.50 (2H, s), 2.83-2.99 (3H, m), 2.04 (2H, m), 1.86 (2H, m), 1.47-1.61 (2H, m).

### b) Synthesis of 4-hydrazino-1-benzylpiperidine dihydrochloride

A solution of 4-(2-Benzoylhydrazino)-1-benzylpiperidine (10.107 g, 33.985 mmol) in a mixture of concentrated hydrochloric acid (23 ml) and water (13 ml) was heated under reflux for four hours. The mixture was cooled to room temperature, and then the precipitated crystals were filtered off. The mother liquid was evaporated in vacuo. To the residue was added methanol, and the mixture was evaporated in vacuo. The residue was recrystallized from methanol to yield 4-hydrazino-1-benzylpiperidine dihydrochloride (4.336 g, 62.2 %).

| Elemental Analysis | | | | |
|---|---|---|---|---|
| Calculated: | C, 51.80; | H, 7.61; | N, 15.10; | Cl, 25.49 |
| Found: | C, 51.73; | H, 7.66; | N, 15.03; | Cl, 25.78 |

### c) Synthesis of 5-amino-4-cyano-3-methyl-1-(1-benzylpiperidin-4-yl)-(1H)-pyrazole

A solution of 4-hydrazino-1-benzylpiperidine dihydrochloride (2.507 g, 9.011 mmol), sodium ethoxide (1.226 g, 18.016 mmol), and methylmethoxymethylene malononitrile (1.226 g, 9.010 mmol) in ethanol (100 ml) was heated under reflux for four hours. After cooling, the ethanol was evaporated in vacuo. To the residue was added water, and the mixture was extracted with ethyl acetate. The organic layer was washed with saturated brine, dried over sodium sulfate, and evaporated in vacuo. The residue was purified by chromatography on silica gel (methanol-chloroform 3:97) to yield 5-amino-4-cyano-3-methyl-1-(1-benzylpiperidin-4-yl)-(1H)-pyrazole (2.651g, 99.6%).
IR (KBr) 3326, 3190, 2946, 2805, 2209, 1649, 1568, 1536cm⁻¹.

### d) Synthesis of 5-{3-(4-chlorobenzenesulfonyl)ureido}-4-cyano-3-methyl-1-(1-benzylpiperidin-4-yl)-(1H)-pyrazole

According to a procedure substantially same as that in Preparation 1, 5-amino-4-cyano-3-methyl-1-(1-benzylpiperidin-4-yl)-(1H)-pyrazole and 4-chlorobenzenesulfonyl isocyanate were reacted to yield 5-{3-(4-chlorobenzenesulfonyl)ureido}-4-cyano-3-methyl-1-(1-benzylpiperidin-4-yl)-(1H)-pyrazole.
Melting point: 175-177°C.

### Example 54

### Synthesis of 5-{3-(4-chlorobenzenesulfonyl)-1-methylureido}-4-cyano-3-methyl-1-phenyl-(1H)-pyrazole

### a) Synthesis of 5-methylamino-4-cyano-3-methyl-1-phenyl-(1H)-pyrazole

A solution of 5-amino-4-cyano-3-methyl-1-phenyl-(1H)-pyrazole (1.30 g, 5.196 mmol) [compound described in J. Org. Chem., 1240, 21, (1956)], iodomethane (330 µl, 5.301 mmol), potassium carbonate (1.573 g, 11.381 mmol) in dimethylformamide (20 ml) was stirred for 5.0 hours at room temperature. This reaction was poured into an ice-water, and the mixture was extracted with ethyl acetate. The organic layer was washed with saturated brine, dried over magnesium sulfate, and evaporated in vacuo. The residue was purified by chromatography on silica gel (chloroform-ethyl acetate 20:1) to yield 5-methylamino-4-cyano-3-methyl-1-phenyl-(1H)-pyrazole (35 mg, 3.0 %).
¹H-NMR (CDCl₃) 7.38-7.53 (5H, m), 4.39 (1H, m), 3.15 (3H, d, J=5.3Hz), 2.31 (3H, s).

### b) Synthesis of 5-{3-(4-chlorobenzenesulfonyl)-1-methylureido}-4-cyano-3-methyl-1-phenyl-(1H)-pyrazole

According to a procedure substantially same as that in Preparation 1, 5-methylamino-4-cyano-3-methyl-1-phenyl-(1H)-pyrazole and 4-chlorobenzenesulfonylisocyanate were reacted to yield 5-{3-(4-chlorobenzenesulfonyl)-1-methylureido}-4-cyano-3-methyl-1-phenyl-(1H)-pyrazole.
IR (KBr) 3491, 2243, 1618, 1506, 1478, 1456, 1396, 1319, 1242, 1134, 1085, 1049, 1015, 876, 841, 751, 630 cm⁻¹.

### Example 55

### Synthesis of 5-{3-(4-chlorobenzenesulfonyl)ureido}-4-cyano-3-methyl-1-{tetrahydro-(4H)-pyran-4-yl}-(1H)-pyrazole

### a) Synthesis of 4-(2-benzoylhydrazino)-tetrahydro-(4H)-pyran

According to a procedure substantially same as that in Example 53(a), reaction was performed using 4-tetrahydro-(4H)-pyran-4-one, benzoylhydrazine, and sodium borohydride, to yield 4-(2-benzoylhydrazino)-tetrahydro-(4H)-pyran.
IR (KBr) 3298, 2939, 2853, 1637, 1548, 1479, 1319, 1095, 904 cm⁻¹.

### b) Synthesis of 5-amino-4-cyano-3-methyl-1-{4-tetrahydro-(4H)-pyran-4-yl}-(1H)-pyrazole

A solution of 4-(2-Benzoylhydrazino)tetrahydro-(4H)-pyran (3.900 g, 17.706 mmol) in a mixture of concentrated hydrochloric acid (30 ml) and water (30 ml) was heated under reflux for four hours. The mixture was cooled to room temperature, and the precipitated crystals were filtered out. The mother liquor was evaporated in vacuo. To the residue was added methanol, and the mixture was evaporated in vacuo. The residue was recrystallized from methanol to yield crystals containing 4-hydrazinotetrahydro-(4H)-pyran hydrochloride (1.279 g). Then, a solution of a portion (1.260 g, 10.847 mmol) of the crystals containing 4-hydrazinotetrahydro-(4H)-pyran hydrochloride (1.279 g), methoxymethylene malononitrile (1.200 g), sodium ethylate (1.000 g, 14.695 mmol) in ethanol (50 ml) was heated under reflux for six hours. After cooling, the ethanol was evaporated in vacuo. To the residue was added water, and the mixture was extracted with ethyl acetate. The organic layer was washed with saturated brine, dried over sodium sulfate, and evaporated in vacuo. The residue was purified by chromatography on silica gel (methanol-chloroform 3:97) to yield 5-amino-4-cyano-3-methyl-1-{4-tetrahydro-(4H)-pyran-4-yl}-(1H)-pyrazole (316 mg, 17.4 %).
IR (KBr) 3381, 3340, 3241, 2213, 1656, 1567, 1540, 1489, 1383, 1141, 1088, 1014, 820 cm⁻¹.

### c) Synthesis of 5-{3-(4-chlorobenzenesulfonyl)ureido}-4-cyano-3-methyl-1-{tetrahydro-(4H)-pyran-4-yl}-(1H)-pyrazole

According to a procedure substantially same as that in Preparation 1, 5-amino-4-cyano-3-methyl-1-{tetrahydro-(4H)-pyran-4-yl}-(1H)-pyrazole and 4-chlorobenzenesulfonyl isocyanate were reacted to yield 5-{3-(4-chlorobenzenesulfonyl)ureido}-4-cyano-3-methyl-1-{tetrahydro-(4H)-pyran-4-yl}-(1H)-pyrazole.
Melting point: 176-178°C (dec.).

### Example 56

### Synthesis of 5-{3-benzyl-3-(4-chlorobenzenesulfonyl)ureido}-4-cyano-3-methyl-1-phenyl-(1H)-pyrazole

A mixture of a sodium salt of 4-cyano-3-methyl-1-phenyl-5-{3-(4-chlorobenzenesulfonyl)ureido}-(1H)-pyrazole (200 mg, 0.457 mmol), benzyl bromide (65 µl, 0.546 mmol), and tetra-n-butyl ammonium sulfate (30 mg, 0.0884 mmol) in a mixture of a 1N aqueous solution of sodium hydroxide (20 ml) and toluene (20 ml) was heated under reflux at 100°C for two hours. After cooling, the reaction was added with a 1N solution of hydrochloric acid until the pH was 7, and the mixture was extracted with ethyl acetate. The organic layer was washed with saturated brine, dried over sodium sulfate, and evaporated in vacuo. The residue was purified by chromatography on silica gel(methanol-chloroform 10:90) to yield 5-{3-benzyl-3-(4-chlorobenzenesulfonyl)ureido}-4-cyano-3-methyl-1-phenyl-(1H)-pyrazole (149 mg, 66.3 %).
IR (KBr) 3436, 2231, 1598, 1506, 1395, 1308, 1256, 1132, 1076, 756, 632 cm⁻¹.

### Example 57

### Synthesis of 5-{3-(4-bromobenzenesulfonyl)ureido}-4-cyano-3-methyl-1-phenyl-(1H)-pyrazole

### a) Synthesis of 5-(N-methoxycarbonylamino)-4-cyano-3-methyl-1-phenyl-(1H)-pyrazole

To a solution of 5-amino-4-cyano-3-methyl-1-phenyl-(1H)-pyrazole [compound described in J. Org. Chem., 1240, 21, (1956)] (5.302 g, 0.0267 mol), triethylamine (18.6 ml, 0.133 mol), and 4-dimethylaminopyridine (327 mg, 0.0027 mol) in dichloromethane (400 ml) was added dropwise methyl chloroformate (5.20 ml, 0.0673 mol) at 0°C. After removing an ice-water bath, the mixture was stirred for four hours while allowing to gradually warm to room temperature. The mixture was poured into an ice-water, the organic layer was separated, and the aqueous layer was extracted with dichloromethane. The combined organic layers were washed with saturated brine, dried over magnesium sulfate, and evaporated in vacuo. The residue was dissolved in ethanol (300 ml), a 1N aqueous solution of sodium hydroxide (30 ml) was added thereto, and the mixture was stirred for four hours. After cooling to 0°C, the mixture was added with a 1N solution of hydrochloric acid until the pH was 7, and evaporated in vacuo. To the residue was added water, and the mixture was extracted with ethyl acetate. The organic layer was washed with saturated brine, dried over sodium sulfate, and evaporated in vacuo. The residue was purified by chromatography on silica gel (chloroform-ethyl acetate 4:1) to yield a material, which then was recrystallized from methanol, and 5-(N-methoxycarbonylamino)-4-cyano-3-methyl-1-phenyl-(1H)-pyrazole (6.09 g, 88.8 %) was obtained.
¹H-NMR (CDCl₃) 7.40-7.54 (5H, m), 6.77 (1H, brs), 3.78 (3H, s), 2.42 (3H, s).

### b) Synthesis of a sodium salt of 4-bromobenzenesulfonamide

A solution of 4-bromobenzenesulfonamide (5.101 g, 21.606 mmol) and sodium ethylate (1.470 g, 21.602 mmol) in ethanol (700 ml) was heated under reflux for 30 minutes. After cooling, the reaction was evaporated in vacuo. The residue was dried in vacuo to yield a sodium salt of 4-bromobenzenesulfonamide.
IR (KBr) 3213, 1154, 1118, 991, 820 cm⁻¹.

### c) Synthesis of 5-{3-(4-bromobenzenesulfonyl)-ureido}-4-cyano-3-methyl-1-phenyl-(1H)-pyrazole

A solution of 5-(N-methoxycarbonylamino)-4-cyano-3-methyl-1-phenyl-(1H)-pyrazole (68 mg, 0.265 mmol) and a sodium salt of 4-bromobenzenesulfonamide (72 mg, 0.306 mmol) in tetrahydrofuran (10 ml) was heated under reflux for 16 hours. After cooling, the tetrahydrofuran was evaporated in vacuo. To the residue was added water, and the mixture was extracted with ethyl acetate. The organic layer was washed with saturated brine, dried over sodium sulfate, and evaporated in vacuo. The residue was purified by chromatography on silica gel (methanol-chloroform 10:90), and further purified by preparative thin layer chromatography (silica gel, methanol-chloroform 10:90), to yield 5-{3-(4-bromobenzenesulfonyl)-ureido}-4-cyano-3-methyl-1-phenyl-(1H)-pyrazole (4.9 mg, 4.0 %).
IR (KBr) 3468, 2925, 2229, 1632, 1256, 1148, 1073, 744, 616 cm⁻¹.

### Example 58

### Synthesis of 5-{3-(4-chlorobenzenesulfonyl)-1-isopropylureido}-4-cyano-3-methyl-1-phenyl-(1H)-pyrazole

### a) Synthesis of 5-(N-methoxycarbonylisopropylamino)-4-cyano-3-methyl-1-phenyl-(1H)-pyrazole

In a nitrogen atmosphere, sodium hydride (60 % in oil) (70 mg, 1.750 mmol) was added to a solution of 5-(N-methoxycarbonylamino)-4-cyano-3-methyl-1-phenyl-(1H)-pyrazole (300 mg, 1.171 mmol) in tetrahydrofuran(10 ml) at 0°C, and the mixture was stirred for 30 minutes. To the mixture were added isopropyl bromide (170 µl, 1.811 mmol) and sodium iodide (275 mg, 1.835 mmol), successively. Then, dimethylformamide (10 ml) was added thereto, and the mixture was stirred for four hours at room temperature. The mixture was poured into an ice-water, extracted with ethyl acetate, and the organic layer was washed with saturated brine, dried over sodium sulfate, and evaporated in vacuo. The residue was purified by chromatography on silica gel (hexane-ethyl acetate 4:1) to yield 5-(N-methoxycarbonylisopropylamino)-4-cyano-3-methyl-1-phenyl-(1H)-pyrazole (185 mg, 53.0 %).
¹H-NMR (CDCl₃) 7.36-7.51 (5H, m), 4.27 (1H, m), 3.78 (1H, m), 2.47 (3H, m), 1.21 (3H, d, J=6.6Hz), 0.73 (3H, d, J=6.6Hz).

### b) Synthesis of 5-isopropylamino-4-cyano-3-methyl-1-phenyl-(1H)-pyrazole

A solution of 5-(N-methoxycarbonylisopropylamino)-4-cyano-3-methyl-1-phenyl-(1H)-pyrazole (83 mg, 0.278 mmol) and a 5N aqueous solution of potassium hydroxide (2.0 ml) in diethylene glycol (5.0 ml) was stirred at 100°C for 3.5 hours. The mixture was poured into an ice-water, and the mixture was extracted with ethyl acetate. The organic layer was washed with saturated brine, dried over sodium sulfate, and evaporated in vacuo. The residue was purified by chromatography on silica gel(chloroform-ethyl acetate 10:1) to yield 5-isopropylamino-4-cyano-3-methyl-1-phenyl-(1H)-pyrazole (185 mg, 53.0 %).
¹H-NMR (CDCl₃) 7.39-7.56 (5H, m), 4.12 (1H, m), 2.33 (3H, s), 1.25 (6H, d, J=5.9Hz).

### c) Synthesis of 5-{3-(4-chlorobenzenesulfonyl)-1-isopropylureido}-4-cyano-3-methyl-1-phenyl-(1H)-pyrazole

According to a procedure substantially same as that in Preparation 1, 5-isopropylamino-4-cyano-3-methyl-1-phenyl-(1H)-pyrazole and 4-chlorobenzenesulfonyl isocyanate were reacted to yield 5-{3-(4-chlorobenzenesulfonyl)-1-isopropylureido}-4-cyano-3-methyl-1-phenyl-(1H)-pyrazole.
Melting point: 193-195°C.

### Example 59

### Synthesis of 5-{3-(4-chlorobenzenesulfonyl)-1-(2-dimethylaminoethyl)ureido}-4-cyano-3-methyl-1-phenyl-(1H)-pyrazole

### a) Synthesis of 5-{N-methoxycarbonyl-(2-dimethylaminoethyl)amino}-4-cyano-3-methyl-1-phenyl-(1H)-pyrazole

According to a procedure substantially same as that in Example 58(a), 5-(N-methoxycarbonylamino)-4-cyano-3-methyl-1-phenyl-(1H)-pyrazole, and dimethylamino ethyl chloride hydrochloride were reacted to yield 5-{N-methoxycarbonyl-(2-dimethylaminoethyl)amino}-4-cyano-3-methyl-1-phenyl-(1H)-pyrazole.
¹H-NMR (CDCl₃) 7.38-7.52 (5H, m), 3.31-3.78 (5H, m), 2.44 (3H, s), 2.42 (2H, m), 2.14 (6H, s).

### b) Synthesis of 5-(2-dimethylaminoethylamino)-4-cyano-3-methyl-1-phenyl-(1H)-pyrazole

According to a procedure substantially same as that in Example 58(b), 5-(2-dimethylaminoethylamino)-4-cyano-3-methyl-1-phenyl-(1H)-pyrazole was prepared starting from 5-(N-methoxycarbonyl-(2-dimethylaminoethyl)amino)-4-cyano-3-methyl-1-phenyl-(1H)-pyrazole.
¹H-NMR(CDCl₃) 7.36-7.53 (5H, m), 5.26 (1H, m), 3.51-3.57 (2H, m), 2.51 (2H, m), 2.32 (3H, s), 2.18 (6H, s).

### c) Synthesis of 5-{3-(4-chlorobenzenesulfonyl)-1-(2-dimethylaminoethyl)ureido}-4-cyano-3-methyl-1-phenyl-(1H)-pyrazole

According to a procedure substantially same as that in Preparation 1, 5-(2-dimethylaminoethylamino)-4-cyano-3-methyl-1-phenyl-(1H)-pyrazole and 4-chlorobenzenesulfonyl isocyanate were reacted to yield 5-{3-(4-chlorobenzenesulfonyl)-1-(2-dimethylaminoethyl)ureido}-4-cyano-3-methyl-1-phenyl-(1H)-pyrazole.
Melting point: 93-96°C.

### Example 60

### Synthesis of 5-[3-(4-chlorobenzenesulfonyl)-1-{2-(4-morpholino)ethyl}ureido]-4-cyano-3-methyl-1-phenyl-(1H)-pyrazole

### a) 5-[N-methoxycarbonyl-{2-(4-morpholino)ethyl}amino]-4-cyano-3-methyl-1-phenyl-(1H)-pyrazole

According to a procedure substantially same as that in Example 58(a), 5-(N-methoxycarbonylamino)-4-cyano-3-methyl-1-phenyl-(1H)-pyrazole and 4-(2-chloroethyl)morpholine hydrochloride were reacted to yield 5-[N-methoxycarbonyl-{2-(4-morpholino)ethyl}amino]-4-cyano-3-methyl-1-phenyl-(1H)-pyrazole.
¹H-NMR (CDCl₃) 7.39-7.54 (5H, m), 3.81 (1H, m), 3.66 (3H, s), 3.60 (4H, m), 3.25 (1H, m), 2.45 (3H, s), 2.35 (4H, m), 2.35-2.50 (2H, m), 2.14 (6H, s).

### b) Synthesis of 5-{2-(4-morpholino)ethylamino}-4-cyano-3-methyl-1-phenyl-(1H)-pyrazole

According to a procedure substantially same as that in Example 58(b), 5-{2-(4-morpholino)ethylamino}-4-cyano-3-methyl-1-phenyl-(1H)-pyrazole was prepared starting from 5-[N-methoxycarbonyl-{2-(4-morpholino)ethyl}amino]-4-cyano-3-methyl-1-phenyl-(1H)-pyrazole.
¹H-NMR (CDCl₃) 7.40-7.55 (5H, m), 5.41 (1H, m), 3.52-3.59 (6H, m), 2.59 (2H, m), 2.41 (4H, m), 2.33 (3H, s).

### c) Synthesis of 5-[3-(4-chlorobenzenesulfonyl)-1-{2-(4-morpholino)ethyl}-ureido]-4-cyano-3-methyl-1-phenyl-(1H)-pyrazole

According to a procedure substantially same as that in Preparation 1, 5-{2-(4-morpholino)-ethylamino}-4-cyano-3-methyl-1-phenyl-(1H)-pyrazole and 4-chlorobenzenesulfonyl isocyanate were reacted to yield 5-[3-(4-chlorobenzenesulfonyl)-1-{2-(4-morpholino)ethyl}ureido]-4-cyano-3-methyl-1-phenyl-(1H)-pyrazole.
Melting point: 143-155°C.

### Example 61

### Synthesis of 5-{3-(4-chlorobenzenesulfonyl)ureido}-4-cyano-3-methyl-1-(1-methylpiperidin-4-yl)-(1H)-pyrazole

### a) Synthesis of 5-{di-(tert-butoxycarbonyl)-amino}-4-cyano-3-methyl-1-(1-benzylpiperidin-4-yl)-(1H)-pyrazole

To a solution of 5-amino-4-cyano-3-methyl-1-(1-benzylpiperidin-4-yl)-(1H)-pyrazole (300 mg, 1.016 mmol) in dichloromethane (20 ml) was added di-*tert*-butyl carbonate (280 µl, 3.235 mmol) at room temperature, then 4-dimethylaminopyridine (20 mg) was added thereto, and the mixture was stirred for 30 minutes. To the mixture was added additional di-*tert*-butyl carbonate (280 µl, 3.235 mmol), and the mixture was stirred for one hour, and was poured into an ice-water. The resulting mixture was extracted with ethyl acetate, and the organic layer was washed with saturated brine, dried over sodium sulfate, and evaporated in vacuo. The residue was purified by chromatography on silica gel (methanol-chloroform 10:90) to yield 5-{di-(*tert*-butoxycarbonyl)amino}-4-cyano-3-methyl-1-(1-benzylpiperidine-4-yl)-(1H)-pyrazole (500 mg, quantitative).
¹H-NMR (CDCl₃) 7.22-7.35 (5H, m), 3.81 (1H, m), 3.53 (2H, s), 3.00 (2H, m), 2.36 (2H, s), 2.19 -2.33 (2H, m), 2.01-2.12 (2H, m), 1.75 (2H, m), 1.45 (18H, s).

### b) Synthesis of 5-(N-tert-butoxycarbonylamino)-4-cyano-3-methyl-1-(1-benzylpiperidin-4-yl)-(1H)-pyrazole

A solution of 5-{di-(*tert*-butoxycarbonyl)-amino}-4-cyano-3-methyl-1-(1-benzylpiperidin-4-yl)-(1H)-pyrazole (384 mg, 0.775 mmol), and a 1N aqueous solution of potassium hydroxide (3.0 ml) in ethanol (20 ml) was stirred for four hours at room temperature. After cooling, the solution was added with 1N hydrochloric acid until the pH was 7, and then the ethanol was evaporated in vacuo. To the residue was added water, and the mixture was extracted with ethyl acetate. The organic layer was washed with saturated brine, dried over sodium sulfate, and evaporated in vacuo. The residue was purified by chromatography on silica gel (methanol-chloroform 10:90) to yield 5-(N-*tert*-butoxycarbonylamino)-4-cyano-3-methyl-1-(1-benzylpiperidin-4-yl)-(1H)-pyrazole (290 mg, 94.6 %).
¹H-NMR (CDCl₃) 7.21-7.35 (5H, m), 6.36 (1H, brs), 3.97 (1H, m), 3.54 (2H, s), 3.01 (2H, m), 2.33 (3H, s), 2.03-2.30 (4H, m), 1.84 (2H, m), 1.51 (9H, s).

### c) Synthesis of 5-(N-tert-butoxycarbonylamino)-4-cyano-3-methyl-1-(piperidin-4-yl)-(1H)-pyrazole

A solution of 5-(N-*tert*-butoxycarbonylamino)-4-cyano-3-methyl-1-(1-benzylpiperidin-4-yl)-(1H)-pyrazole (95 mg, 0.240 mmol), 10% palladium on carbon (5.0mg), and ammonium formate (60.0 mg, 0.951 mmol) in ethanol (10 ml) was heated uncer reflux for four hours. After cooling, the mixture was filtered through CELITE, and evaporated in vacuo. The residue was purified by chromatography on silica gel (methanol-chloroform 10:90) to yield 5-(N-*tert*-butoxycarbonylamino)-4-cyano-3-methyl-1-(piperidin-4-yl)-(1H)-pyrazole (73mg, quantitative).
¹H-NMR (CDCl₃) 4.32 (81H, m), 3.29-3.35 (2H, m), 2.83-2.93 (2H, m), 2.30 (3H, s), 1.93-2.26 (4H, m).

### d) Synthesis of 5-(N-tert-butoxycarbonylamino)-4-cyano-3-methyl-1-(1-methylpiperidin-4-yl)-(1H)-pyrazole

A solution of 5-(N-*tert*-butoxycarbonylamino)-4-cyano-3-methyl-1-(piperidin-4-yl)-(1H)-pyrazole (90 mg, 0.295 mmol), iodomethane (25 µl, 0.402 mmol), potassium carbonate (220 mg, 1.592 mmol) in dimethylformamide (5.0 ml) was stirred for three hours at room temperature. The solution was poured into an ice-water. The mixture was extracted with ethyl acetate, and the organic layer was washed with saturated brine, dried over sodium sulfate, and evaporated in vacuo. The residue was purified by chromatography on silica gel (methanol-chloroform 5:95) to yield 5-(N-*tert*-butoxycarbonylamino)-4-cyano-3-methyl-1-(1-methylpiperidin-4-yl)-(1H)-pyrazole (19 mg, 20.2 %).
IR (KBr) 3320, 2976, 2229, 1724, 1582, 1457, 1370, 1277, 1255, 1160 cm⁻¹.

### e) Synthesis of 5-amino-4-cyano-3-methyl-1-(1-methylpiperidin-4-yl)-(1H)-pyrazole

To a solution of 5-(N-*tert*-butoxycarbonylamino)-4-cyano-3-methyl-1-(1-methylpiperidin-4-yl)-(1H)-pyrazole (17 mg, 0.0532 mmol) in dichloromethane (2.0 ml) was added trifluoromethanesulfonic acid (200 µl) at 0 °C, and the mixture was stirred for two hours. The reaction mixture was evaporated in vacuo. To the residue was added aqueous ammonia, and the mixture was extracted with ethyl acetate. The organic layer was washed with saturated brine, dried over sodium sulfate, and evaporated in vacuo. The residue was purified by preparative thin layer chromatography (silica gel, methanol-chloroform 10:90) to yield 5-amino-4-cyano-3-methyl-1-(1-methylpiperidein-4-yl)-(1H)-pyrazole (5.6 mg, 48.2 %).
¹H-NMR(CDCl₃) 4.23 (2H, brs), 3.74 (1H, m), 2.99 (2H, m), 2.32 (3H, s), 2.23 (3H, s9, 2.03-2.25 (4H, m), 1.83-1.89 (2H, m).

### f) Synthesis of 5-{3-(4-chlorobenzenesulfonyl)ureido}-4-cyano-3-methyl-1-(1-methylpiperidin-4-yl)-(1H)-pyrazole

According to a procedure substantially same as that in Preparation 1, 5-amino-4-cyano-3-methyl-1-(1-methylpiperidin-4-yl)-(1H)-pyrazole and 4-chlorobenzenesulfonyl isocyanate were reacted to yield 5-{3-(4-chlorobenzenesulfonyl)ureido}-4-cyano-3-methyl-1-(1-methylpiperidin-4-yl)-(1H)-pyrazole.
IR (KBr) 3468, 2236, 1686, 1637, 1258, 1210, 1144, 804, 725, 632 cm⁻¹.

### Example 62

### Synthesis of a sodium salt of 5-{3-(4-chlorobenzenesulfonyl)ureido}-3-methyl-1-phenyl-(1H)-pyrazole

According to a procedure substantially same as that in Example 44, a sodium salt of 5-{3-(4-chlorobenzenesulfonyl)ureido}-3-methyl-1-phenyl-(1H)-pyrazole was prepared starting from 5-{3-(4-chlorobenzenesulfonyl)ureido}-3-methyl-1-phenyl-(1H)-pyrazole.
IR (KBr) 3419, 1622, 1539, 1502, 1478, 1365, 1288, 1137, 1088, 1070, 1013, 826, 755, 643, 622 cm⁻¹.

### Example 63

### Synthesis of 5-{3-(4-chlorobenzenesulfonyl)ureido}-4-cyano-3-methyl-1-(2-tert-butoxycarbonyloxyethyl)-(1H)-pyrazole

### a) Synthesis of 5-amino-4-cyano-3-methyl-1-(2-tert-butoxycarbonyloxyethyl)-(1H)-pyrazole

A solution of 4-cyano-3-methyl-1-(2-hydroxyethyl)-5-amino-(1H)-pyrazole [compound described in J. Heterocycl. Chem., 1199, 12, (1975)] (2.273 g, 13.678 mmol), and di-*tert*-butyl carbonate ( 3.80 ml, 16.540 mmol) in a mixture of dichloromethane (10 ml)-tetrahydrofuran (90 ml) was stirred for seven hours at room temperature. The mixture was poured into an ice-water, and the resultant mixture was extracted with ethyl acetate. The organic layer was washed with saturated brine, dried over sodium sulfate, and evaporated in vacuo. The residue was purified by chromatography on silica gel (methanol-chloroform 3:97) to yield 5-amino-4-cyano-3-methyl-1-(2-*tert*-butoxycarbonyloxyethyl)-(1H)-pyrazole (947 mg, 25.9 %).
¹H-NMR (DMSO-d₆) 6.54 (2H, brs), 4.23 (2H, t, J=5.3Hz), 4.06 (2H, t, J=5.3Hz), 2.05 (3H, s), 1.39 (9H, s).

### b) Synthesis of 5-{3-(4-chlorobenzenesulfonyl)ureido}-4-cyano-3-methyl-1-(2-tert-butoxycarbonyloxyethyl)-(1H)-pyrazole

According to a procedure substantially same as that in Preparation 1, 5-amino-4-cyano-3-methyl-1-(2-*tert*-butoxycarbonyloxyethyl)-(1H)-pyrazole and 4-chlorobenzenesulfonyl isocyanate were reacted to yield 5-{3-(4-chlorobenzenesulfonyl)ureido}-4-cyano-3-methyl-1-(2-*tert*-butoxycarbonyloxyethyl)-(1H)-pyrazole.
Melting point: 144-146 °C.

### Example 64

### Synthesis of 5-{3-(4-chlorobenzenesulfonyl)ureido}-4-cyano-3-methyl-1-(2-hydroxyethyl)-(1H)-pyrazole

A solution of 5-{3-(4-chlorobenzenesulfonyl)ureido}-4-cyano-3-methyl-1-(2-*tert*-butoxycarbonyloxyethyl)-(1H)-pyrazole (69 mg, 0.142 mmol) and a 1N aqueous solution of sodium hydroxide (2.0 ml) in ethanol (5.0 ml) was stirred for 3.0 hours at room temperature. After cooling, the solution was added with 1N hydrochloric acid until the pH was 7, and then the ethanol was evaporated in vacuo. To the residue was added water, and the mixture was extracted with ethyl acetate. The organic layer was washed with saturated brine, dried over sodium sulfate, and evaporated in vacuo. The residue was purified by chromatography on silica gel (methanol-chloroform 10:90) to yield 5-{3-(4-chlorobenzenesulfonyl)ureido}-4-cyano-3-methyl-1-(2-hydroxyethyl)-(1H)-pyrazole (32 mg, 58.7 %).
IR (KBr) 3401, 2925, 2853, 2231, 1736, 1618, 1572, 1478, 1260, 1146, 1088, 1014, 756, 628 cm⁻¹.

### Example 65

### Synthesis of 5-{3-(4-chlorobenzenesulfonyl)-1-(2-methoxyethyl)ureido}-4-cyano-3-methyl-1-phenyl-(1H)-pyrazole

### a) Synthesis of 5-{N-methoxycarbonyl-(2-methoxyethyl)amino}-4-cyano-3-methyl-1-phenyl-(1H)-pyrazole

According to a procedure substantially same as that in Example 58(a), 5-(N-methoxycarbonylamino)-4-cyano-3-methyl-1-phenyl-(1H)-pyrazole, and chloroethyl methyl ether were reacted to yield 5-{N-methoxycarbonyl-(2-methoxyethyl)amino}-4-cyano-3-methyl-1-phenyl-(1H)-pyrazole.
¹H-NMR 7.38-7.51 (5H, m), 3.30-3.90 (7H, m), 3.25 (3H, s), 2.44 (3H, s).

### b) Synthesis of 5-(2-methoxyethylamino)-4-cyano-3-methyl-1-pyrazolephenyl-(1H)-pyrazole

According to a procedure substantially same as that in Example 58(b), 5-(2-methoxyethylamino)-4-cyano-3-methyl-1-phenyl-(1H)-pyrazole was prepared starting from 5-{N-methoxycarbonyl-(2-methoxyethyl)-amino}-4-cyano-3-methyl-1-phenyl-(1H)-pyrazole.
¹H-NMR (CDCl₃) 7.38-7.54 (5H, m), 4.76 (1H, m), 3.68 (2H, m), 3.58 (2H, m), 3.34 (3H, s), 2.33 (3H, s)

### c) Synthesis of 5-{3-(4-chlorobenzenesulfonyl)-1-(2-methoxyethyl)ureido}-4-cyano-3-methyl-1-phenyl-(1H)-pyrazole

According to a procedure substantially same as that in Preparation 1, 5-(2-methoxyethylamino)-4-cyano-3-methyl-1-phenyl-(1H)-pyrazole and 4-chlorobenzenesulfonyl isocyanate were reacted to yield 5-{3-(4-chlorobenzenesulfonyl)-1-(2-methoxyethyl)ureido}-4-cyano-3-methyl-1-phenyl-(1H)-pyrazole.
IR (KBr) 3436, 2930, 2232, 1587, 1380, 1310, 1263, 1137, 1089, 754, 632 cm⁻¹.

### Example 66

### Synthesis of 5-{3-(4-chlorobenzenesulfonyl)ureido}-4-cyano-3-methyl-1-vinyl-(1H)-pyrazole

### a) Synthesis of 5-amino-4-cyano-3-methyl-1-(2-methanesulfonyloxyethyl)-(1H)-pyrazole

A solution of 5-amino-4-cyano-3-methyl-1-(2-hydroxyethyl)-(1H)-pyrazole [compound described in J. Heterocycl. Chem., 1199, 12, (1975)] (2.210 g, 13.299 mmol), methanesulfonyl chloride (1.30 ml, 16.796 mmol), and triethylamine (4.0 ml, 28.698 mmol) in a mixture of dichloromethane (10 ml)-tetrahydrofuran (90 ml) was stirred for 11 hours at room temperature. This solution was poured into an ice-water, and the mixture was extracted with ethyl acetate. The organic layer was washed with saturated brine, dried over sodium sulfate, and evaporated in vacuo. The residue was purified by chromatography on silica gel (methanol-chloroform 3:97) to yield 5-amino-4-cyano-3-methyl-1-(2-methanesulfonyloxyethyl)-(1H)-pyrazole (1.097 g, 33.8 %).
¹H-NMR(DMSO-d₆) 6.61 (2H, brs), 4.43 (2H, t, J=5.3Hz), 4.16 (2H, t, J=5.3Hz), 3.09 (3H, s), 2.08 (3H, s).

### b) Synthesis of 5-amino-4-cyano-3-methyl-1-vinyl-(1H)-pyrazole

A solution of 5-amino-4-cyano-3-methyl-1-(2-methanesulfonyloxyethyl)-(1H)-pyrazole (523 mg, 2.141 mmol), 1, 8-diazabicyclo[5.4.0.]-7-undecene (960 µl, 6.424 mmol) in tetrahydrofuran (20 ml) was heated under reflux for 16 hours. After cooling, to the solution was added an ice-water, and the mixture was extracted with ethyl acetate. The organic layer was washed with saturated brine, dried over sodium sulfate, and evaporated in vacuo. The residue was purified by chromatography on silica gel (chloroform-ethyl acetate 4:1) to yield 5-amino-4-cyano-3-methyl-1-vinyl-(1H)-pyrazole (73 mg, 23.0 %).
¹H-NMR(CDCl₃) 6.71 (1H, dd, J=8.9, 15.5Hz), 5.60 (1H, dd, J=1.0, 15.5Hz), 4.98 (1H, dd, J=1.0, 8.9Hz), 4.64 (2H, brs), 2.28 (3H, s).

### c) Synthesis of 5-{3-(4-chlorobenzenesulfonyl)ureido}-4-cyano-3-methyl-1-vinyl-(1H)-pyrazole

According to a procedure substantially same as that in Preparation 1, 4-cyano-3-methyl-1-vinyl-5-amino-(1H)-pyrazole and 4-chlorobenzenesulfonyl isocyanate were reacted to yield 5-{3-(4-chlorobenzenesulfonyl)ureido}-4-cyano-3-methyl-1-vinyl-(1H)-pyrazole.
Melting point: 184-187 °C.

### Example 67

### Synthesis of 5-{3-(4-chlorobenzenesulfonyl)ureido}-4-cyano-3-methyl-1-ethyl-(1H)-pyrazole

### a) Synthesis of 5-amino-4-cyano-3-methyl-1-ethyl-(1H)-pyrazole

In a hydrogen gas atmosphere, a suspension of 5-amino-4-cyano-3-methyl-1-vinyl-(1H)-pyrazole (36 mg, 0.243 mmol), 10% palladium on carbon (10 mg) in ethyl acetate (3.0 ml) was stirred for one hour at room temperature. After filtration, the filtrate was evaporated in vacuo. The residue was purified by chromatography on silica gel (chloroform-ethyl acetate 4:1) to yield 5-amino-4-cyano-3-methyl-1-ethyl-(1H)-pyrazole (28 mg, 76.7 %).

### b) Synthesis of 5-{3-(4-chlorobenzenesulfonyl)ureido}-4-cyano-3-methyl-1-ethyl-(1H)-pyrazole

According to a procedure substantially same as that in Preparation 1, 5-amino-4-cyano-3-methyl-1-ethyl-(1H)-pyrazole and 4-chlorobenzenesulfonyl isocyanate were reacted to yield 5-{3-(4-chlorobenzenesulfonyl)ureido}-4-cyano-3-methyl-1-ethyl-(1H)-pyrazole.
Melting point: 247-250 °C.

### Example 68

### Synthesis of 5-{3-(4-chlorobenzenesulfonyl)ureido}-4-cyano-3-methyl-1-(2-methoxyethyl)-(1H)-pyrazole

### a) Synthesis of 5-amino-4-cyano-3-methyl-1-(2-methoxyethyl)-(1H)-pyrazole

A solution of 5-amino-4-cyano-3-methyl-1-(2-methanesulfonyloxyethyl)-(1H)-pyrazole (189 mg, 0.774 mmol), sodium methoxide (210 mg, 3.887 mmol) in methanol (5.0 ml) was stirred for six hours at room temperature. The methanol was evaporated in vacuo to give a residue, which was then extracted with ethyl acetate. The organic layer was washed with saturated brine, dried over sodium sulfate, and evaporated in vacuo. The residue was purified by chromatography on silica gel (methanol-chloroform 1:99) to yield 5-amino-4-cyano-3-methyl-1-(2-methoxyethyl)-(1H)-pyrazole (77 mg, 55.2 %).
¹H-NMR(CDCl₃) 4.85 (2H, brs), 4.08 (2H, m), 3.68 (2H, m), 3.37 (3H, s), 2.23 (3H, s).

### b) Synthesis of 5-{3-(4-chlorobenzenesulfonyl)ureido}-4-cyano-3-methyl-1-(2-methoxyethyl)-(1H)-pyrazole

According to a procedure substantially same as that in Preparation 1, 5-amino-4-cyano-3-methyl-1-(2-methoxyethyl)-(1H)-pyrazole and 4-chlorobenzenesulfonyl isocyanate were reacted to yield 5-{3-(4-chlorobenzenesulfonyl)ureido}-4-cyano-3-methyl-1-(2-methoxyethyl)-(1H)-pyrazole.
Melting point: 196-199 °C.

### Example 69

### Synthesis of 5-{3-(2-naphthylsulfonyl)ureido}-3-methyl-1-phenyl-(1H)-pyrazole

According to a procedure substantially same as that in Preparation 1, 5-amino-3-methyl-1-phenyl-(1H-pyrazole [compound described in J. Org. Chem., 6155, 58, (1993)] and 2-naphthylsulfonyl isocyanate[compound described in DE1289526] were reacted to yield 5-{3-(2-naphthylsulfonyl)ureido}-3-methyl-1-phenyl-(1H)-pyrazole.
IR (KBr) 3430, 1602, 1541, 1501, 1385, 1301, 1242, 1122, 771, 694 cm⁻¹.

### Example 70

### Synthesis of 5-{3-(4-ethylbenzenesulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and 4-ethylbenzenesulfonyl isocyanate[described in DE1289526] were reacted to yield 5-{3-(4-ethylbenzenesulfonyl)ureido}-3-methyl-1-phenyl-(1H)-pyrazole.
IR (KBr) 3537, 1732, 1564, 1503, 1468, 1334, 1154, 1090, 1026, 922, 752, 658 cm⁻¹.

### Example 71

### Synthesis of 5-(3-methylsulfonylureido)-4-cyano-3-methyl-1-phenyl-(1H)-pyrazole

### a) Synthesis of a sodium salt of the methanesulfonamide

According to a procedure substantially same as that in Example 57(b), a sodium salt of the methanesulfonamide was prepared starting from the methanesulfonamide.
IR (KBr) 3440, 1652, 1352, 1318, 1213, 1136, 996 cm⁻¹.

### b) Synthesis of 5-{3-methylsulfonylureido}-4-cyano-3-methyl-1-phenyl-(1H)-pyrazole

A solution of 5-(N-methoxycarbonylamino)-4-cyano-3-methyl-1-phenyl-(1H)-pyrazole (323 mg, 1.260 mmol), and a sodium salt of methanesulfonamide (738 mg, 6.302 mmol) in tetrahydrofuran (20 ml) was heated under reflux for nine hours. After cooling, the tetrahydrofuran was evaporated in vacuo. To the residue was added chloroform, and then the mixture was added with 1N hydrochloric acid until the pH was 3. The chloroform layer was separated, washed with saturated brine, dried over sodium sulfate, and evaporated in vacuo. The residue was purified by chromatography on silica gel (chloroform-ethyl acetate 4:1→methanol-chloroform 10:90) to yield 5-{3-methylsulfonylureido}-4-cyano-3-methyl-1-phenyl-(1H)-pyrazole (35 mg, 8.7 %).
IR (KBr) 3436, 2232, 1611, 1501, 1312, 1252, 1146, 1114, 968, 768, 696 cm⁻¹.

### Example 72

### Synthesis of 5-{3-(4-isobutylbenzenesulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and 4-isobutylbenzenesulfonyl isocyanate[compound described in DE1289526] were reacted to yield 5-{3-(4-isobutylbenzenesulfonyl)ureido}-3-methyl-1-phenyl-(1H)-pyrazole.
IR (KBr) 3536, 2958, 1734, 1598, 1564, 1502, 1467, 1334, 1154, 1090, 1027, 921, 754, 691 cm⁻¹.

### Example 73

### Synthesis of 5-{3-(4-chlorobenzenesulfonyl)ureido}-4-cyano-3-methyl-1-(2-dimethylaminoethyl)-(1H)-pyrazole

### a) Synthesis of 5-amino-4-cyano-3-methyl-1-(2-dimethylaminoethyl)-(1H)-pyrazole

A solution of 5-amino-4-cyano-3-methyl-1-(2-methanesulfonyloxyethyl)-(1H)-pyrazole (1.273 g, 5.211 mmol) and 50% aqueous dimethylamine (3.0 ml) in dimethylformamide (30 ml) was stirred for 10.5 hours, while allowing to gradually warm from 0 °C to room temperature. The reaction was poured into an ice-water, and the mixture was extraced with ethyl acetate. The organic layer was washed with saturated brine, dried over sodium sulfate , and evaporated in vacuo. The residue was purified by chromatography on silica gel (methanol-chloroform 3:97→5:95) to yield 5-amino-4-cyano-3-methyl-1-(2-dimethylaminoethyl)-(1H)-pyrazole (528 mg, 52.4 %).
¹H-NMR (CDCl₃) 5.90 (2H, brs), 3.97 (2H, m), 2.61 (2H, m), 2.27 (6H, s), 2.16 (3H, s).

### b) Synthesis of 5-{3-(4-chlorobenzenesulfonyl)ureido}-4-cyano-3-methyl-1-(2-dimethylaminoethyl)-(1H)-pyrazole

According to a procedure substantially same as that in Preparation 1, 5-amino-4-cyano-3-methyl-1-(2-dimethylaminoethyl)-(1H)-pyrazole and 4-chlorobenzenesulfonyl isocyanate were reacted to yield 5-{3-(4-chlorobenzenesulfonyl)ureido}-4-cyano-3-methyl-1-(2-dimethylaminoethyl)-(1H)-pyrazole.
IR (KBr) 3388, 2227, 1634, 1572, 1478, 1258, 1145, 1087, 1014 cm⁻¹.

### Example 74

### Synthesis of 5-{3-(4-chlorobenzenesulfonyl)ureido}-3-methyl-1-cyclohexyl-(1H)-pyrazole

According to a procedure substantially same as that in Preparation 1, 5-amino-3-methyl-1-cyclohexyl-(1H)-pyrazole [compound described in J. Heterocycl. Chem., 523, 12, (1975)] and 4-chlorobenzenesulfonyl isocyanate were reacted to yield 5-{3-(4-chlorobenzenesulfonyl)ureido}-3-methyl-1-cyclohexyl-(1H)-pyrazole.
Melting point: 300 °C or above.

### Example 75

### Synthesis of 5-{3-(4-methoxycarbonylbenzenesulfonyl)ureido}-3-methyl-1-phenyl-(1H)-pyrazole

According to a procedure substantially same as that in Preparation 1, 5-amino-3-methyl-1-cyclohexyl-(1H)-pyrazole [compound described in J. Org. Chem., 6155, 58, (1993)] and 4-methoxycarbonylbenzenesulfonyl isocyanate were reacted to yield 5-{3-(4-methoxycarbonylbenzenesulfonyl)ureido}-3-methyl-1-phenyl-(1H)-pyrazole.
Melting point: 154-156°C.

### Example 76

### Synthesis of 5-{3-(2-chlorobenzenesulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and 2-chlorobenzenesulfonyl isocyanate were reacted to yield 5-{3-(2-chlorobenzenesulfonyl)ureido}-3-methyl-1-phenyl-(1H)-pyrazole.
Melting point: 110-113°C.

### Example 77

### Synthesis of 5-{3-(2-chlorobenzenesulfonyl)ureido}-3-methyl-1-cyclohexyl-(1H)-pyrazole

According to a procedure substantially same as that in Preparation 1, 5-amino-3-methyl-1-cyclohexyl-(1H)-pyrazole [compound described in J. Heterocycl. Chem., 523, 12, (1975)] and 2-chlorobenzenesulfonyl isocyanate were reacted to yield 5-{3-(2-chlorobenzenesulfonyl)ureido}-3-methyl-1-cyclohexyl-(1H)-pyrazole.
Melting point: 187-189°C.

### Example 78

### Synthesis of 5-{3-(4-n-butylbenzenesulfonyl)ureido}-3-methyl-1-phenyl-(1H)-pyrazole

According to a procedure substantially same as that in Preparation 1, 5-amino-3-methyl-1-phenyl- (1H)-pyrazole [compound described in J. Org. Chem., 6155, 58, (1993)., 523, 12, (1975)] and 4-n-butylbenzenesulfonyl isocyanate were reacted to yield 5-{3-(4-n-butylbenzenesulfonyl)ureido}-3-methyl-1-phenyl-(1H)-pyrazole.
Melting point: 100-103°C.

### Example 79

### Synthesis of 5-{3-(4-carboxybenzenesulfonyl)ureido}-3-methyl-1-phenyl-(1H)-pyrazole

A solution of 3-methyl-1-phenyl-5-{3-(4-methoxycarbonylbenzenesulfonyl)ureido}-(1H)-pyrazole (103 mg, 0.249 mmol) and a 5N aqueous solution of potassium hydroxide (5.0 ml) in methanol (10 ml) was stirred for two hours while allowing to gradually warm from 0°C to room temperature. The methanol was evaporated in vacuo to yield an aqueous solution, which was added with 1N hydrochloric acid until the pH was 3. The precipitated crystals were filtered off, washed with water, and dried in vacuo to yield 5-{3-(4-carboxybenzenesulfonyl)ureido}-3-methyl-1-phenyl-(1H)-pyrazole (56 mg, 56.3 %).
IR (KBr) 3068, 1704, 1600, 1560, 1503, 1404, 1343, 1262, 1168, 1090, 764, 695, 614 cm⁻¹.

### Example 80

### Synthesis of 5-{3-(benzylsulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and benzylsulfonyl isocyanate [compound described in J. Org. Chem., 1597, 39, (1974)] were reacted to yield 5-{3-(benzylsulfonyl)ureido}-3-methyl-1-phenyl-(1H)-pyrazole.
Melting point: 163-165°C.

### Example 81

### Synthesis of 5-{3-(4-methoxybenzenesulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and 4-methoxybenzenesulfonyl isocyanate [compound described in DE1289526] were reacted to yield 5-{3-(4-methoxybenzenesulfonyl)ureido}-3-methyl-1-phenyl-(1H)- pyrazole.
Melting point: 110-113°C.

### Example 82

### Synthesis of 5-[3-{4-(piperidin-1-carbonyl)-benzenesulfonyl}ureido]-3-methyl-1-phenyl-(1H)-pyrazole

A solution of 3-methyl-1-phenyl-5-{3-(4-carboxybenzenesulfonyl)ureido}-(1H)-pyrazole (102 mg, 0.255 mmol), piperidine (60 µl, 0.607 mmol), 1-(3-dimethylaminopropyl)-3-ethylcarbodiimide hydrochloride (60 mg, 0.313 mmol) in a mixture of dimethylformamide (10 ml)-tetrahydrofuran (10 ml) was stirred overnight while allowing to gradually warm from 0°C to room temperature. The reaction was evaporated in vacuo. The residue was purified by chromatography on silica gel (methanol-chloroform 3:97) to yield 5-[3-{4-(piperidin-1-carbonyl)-benzenesulfonyl}ureido]-3-methyl-1-phenyl-(1H)-pyrazole (30mg, 25.2%).
IR (KBr) 2941, 1635, 1500, 1446, 1339, 1274, 1164, 758, 676, 603 cm⁻¹.

### Example 83

### Synthesis of 5-{3-(4-hydroxymethyl-benzenesulfonyl)ureido}-3-methyl-1-phenyl-(1H)-pyrazole

A solution of 5-{3-(4-methoxycarbonylbenzenesulfonyl)ureido}-(3-methyl-1-phenyl-1H)-pyrazole (117 mg, 0.282 mmol) in tetrahydrofuran (2.0 ml) was added dropwise to a suspension of lithium aluminum hydride (20.0 mg, 0.527 mmol) in tetrahydrofuran (2.0 ml) at 0°C. After removal of an ice-water bath, the mixture was stirred for two hours while allowing to gradually warm to room temperature. To the reaction was added dropwise tetrahydrofuran-water (1:1), the precipitate was removed by filtration, and the filtrate was evaporated in vacuo. The residue was purified by chromatography on silica gel (methanol-chloroform 3:97) to yield 5-{3-(4-hydroxymethyl-benzenesulfonyl)ureido}-3-methyl-1-phenyl-(1H)-pyrazole (92 mg, 84.4 %).
IR (KBr) 3437, 3079, 1600, 1565, 1501, 1382, 1340, 1163, 1095, 1048, 925, 757, 684 cm⁻¹.

### Example 84

### Synthesis of 5-{3-(4-carbamoylbenzenesulfonyl)ureido}-3-methyl-1-phenyl-(1H)-pyrazole

A solution of 5-{3-(4-methoxycarbonylbenzenesulfonyl)ureido}-3-methyl-1-phenyl-(1H)-pyrazole (130 mg, 0.314 mmol) in ammonia-methanol (10 ml) was heated at 10°C for 18 hours in an autoclave. The reaction was evaporated in vacuo. The residue was purified by thin layer chromatography (silica gel, methanol-chloroform 10:90) to yield 5-{3-(4-carbamoylbenzenesulfonyl)ureido}-3-methyl-1-phenyl-(1H)-pyrazole (82 mg, 77.0 %).
IR (KBr) 3460, 1680, 1610, 1558, 1503, 1407, 1380, 1345, 1170, 764, 677 cm⁻¹.

### Example 85

### Synthesis of 5-{3-(4-n-butylcarbamoyl-benzenesulfonyl)ureido}-3-methyl-1-phenyl-(1H)-pyrazole

According to a procedure substantially same as that in Example 82, 3-methyl-1-phenyl-5-{3-(4-carboxybenzenesulfonyl)ureido}-(1H)-pyrazole and n-butylamine were reacted to yield 5-{3-(4-n-butylcarbamoyl-benzenesulfonyl)ureido}-3-methyl-1-phenyl-(1H)-pyrazole was prepared starting from.
IR (KBr) 3352, 2930, 1643, 1558, 1502, 1453, 1340, 1171, 1092, 1026, 762, 694, 673, 614 cm⁻¹.

### Example 86

### Synthesis of 5-{3-(3-methylbenzenesulfonyl)ureido}-3-methyl-1-phenyl-(1H)-pyrazole

According to a procedure same or substantially same as that in Japanese Patent Publication (kokai) No. 258846/1988, reaction was performed starting from 3-methylbenzenesulfonamide [compound described in J. Org. Chem., 7022, 58, (1993)] to yield 3-methylbenzenesulfonyl isocyanate, which was then reacted with 5-amino-3-methyl-1-phenyl-(1H)-pyrazole [compound described in J. Org. Chem., 6155, 58, (1993)] according to a procedure substantially same as that in Preparation 1 to yield 5-{3-(3-methylbenzenesulfonyl)ureido}-3-methyl-1-phenyl-(1H)-pyrazole.
IR (KBr) 3332, 1729, 1631, 1598, 1540, 1501, 1385, 1349, 1279, 1237, 1137, 1100, 912, 758, 699, 638 cm⁻¹.

### Example 87

### Synthesis of 5-{3-(4-t-butylbenzenesulfonyl)ureido}-3-methyl-1-phenyl-(1H)-pyrazole

According to a procedure same or substantially same as that in Japanese Patent Publication (kokai) No. 258846/1988, reaction was performed starting from 4-t-butylbenzenesulfonamide to yield 4-t-butylbenzenesulfonyl isocyanate, which was then reacted with 5-amino-3-methyl-1-phenyl-(1H)-pyrazole [compound described in J. Org. Chem., 6155, 58, (1993)] according to a procedure substantially same as that in Preparation 1 to yield 5-{3-(4-t-butylbenzenesulfonyl)ureido}-3-methyl-1-phenyl-(1H)-pyrazole.
IR (KBr) 3523, 2964, 1733, 1597, 1564, 1503, 1477, 1333, 1147, 1112, 571 cm⁻¹.

### Example 88

### Synthesis of 5-{3-(3-chlorobenzenesulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and 3-chlorobenzenesulfonyl isocyanate[compound described in J. Org. Chem., 7022, 58, (1993)] were reacted to yield 5-{3-(3-chlorobenzenesulfonyl)ureido}-3-methyl-1-phenyl-(1H)-pyrazole.
IR (KBr) 3342, 1601, 1542, 1501, 1300, 1143, 592 cm⁻¹.

### Example 89

### Synthesis of 5-{3-(thiophen-2-yl-sulfonyl)ureido}-3-methyl-1-phenyl-(1H)-pyrazole

According to a procedure substantially same as that in Preparation 1, 5-amino-3-methyl-1-phenyl-(1H)-pyrazole [compound described in J. Org. Chem., 6155, 58, (1993)] and thiophen-2-yl-sulfonyl isocyanate [compound described in J. Org. Chem., 7022, 58, (1993)] were reacted to yield 5-{3-(thiophen-2-yl-sulfonyl)ureido}-3-methyl-1-phenyl-(1H)-pyrazole.
IR (KBr) 3424, 1601, 1542, 1501, 1299, 1132, 593 cm⁻¹.

### Example 90(CV-2401)

### Synthesis of 5-{3-(4-chlorobenzenesulfonyl)ureido}-4-cyano-3-ethoxycarbonylmethyl-1-phenyl-(1H)-pyrazole

According to a procedure substantially same as that in Preparation 1, 5-amino-4-cyano-3-ethoxycarbonylmethyl-1-phenyl-(1H)-pyrazole [compound described in J. Am. Chem. Soc., 2456, 81, (1959)] and 4-chlorobenzenesulfonyl isocyanate were reacted to yield 5-{3-(4-chlorobenzenesulfonyl)ureido}-4-cyano-3-ethoxycarbonylmethyl-1-phenyl-(1H)-pyrazole.
IR (KBr) 3416, 2234, 1737, 1611, 1505, 1396, 1265, 1148, 1077, 758, 630 cm⁻¹.

### Example 91

### Synthesis of 5-{3-(4-chlorobenzenesulfonyl)ureido}-4-cyano-3-carboxymethyl-1-phenyl-(1H)-pyrazole

According to a procedure substantially same as that in Example 35, 5-{3-(4-chlorobenzenesulfonyl)ureido}-4-cyano-3-carboxymethyl-1-phenyl-(1H)-pyrazole was prepared starting from 5-{3-(4-chlorobenzenesulfonyl)ureido}-4-cyano-3-ethoxycarbonylmethyl-1-phenyl-(1H)-pyrazole.
IR (KBr) 3272, 2237, 1726, 1596, 1502, 1159, 1092, 758 cm⁻¹.

### Example 92

### Synthesis of 5-{3-(4-chlorobenzenesulfonyl)ureido}-4-cyano-3-(2-hydroxyethyl)-1-phenyl-(1H)-pyrazole

According to a procedure substantially same as that in Example 83, 5-{3-(4-chlorobenzenesulfonyl)ureido}-4-cyano-3-(2-hydroxyethyl)-1-phenyl-(1H)-pyrazole was prepared starting from 5-{3-(4-chlorobenzenesulfonyl)ureido}-4-cyano-3-ethoxycarbonylmethyl-1-phenyl-(1H)-pyrazole.
IR (KBr) 3380, 3280, 2362, 1507, 1350, 1326, 1161, 1132, 806, 768, 680, 595, 490cm⁻¹.

### Example 93

### Synthesis of 5-{3-(4-n-propylbenzenesulfonyl)ureido}-3-methyl-1-phenyl-(1H)-pyrazole

According to a procedure same or substantially same as that in Japanese Patent Publication No. 258846/1988, reaction was performed starting from 4-n-propylbenzenesulfonamide [compound described in Bioorg. Chem., 387, 22, (1994)] to yield 4-n-propylbenzenesulfonyl isocyanate, which was then reacted with 5-amino-3-methyl-1-phenyl-(1H)-pyrazole [compound described in J. Org. Chem., 6155, 58, (1993)] according to a procedure substantially same as that in Preparation 1 to yield 5-{3-(4-n-propylbenzenesulfonyl)ureido}-3-methyl-1-phenyl-(1H)-pyrazole.
IR (KBr) 2961, 1732, 1599, 1556, 1502, 1458, 1384, 1349, 1297, 1246, 1140 cm⁻¹.

Structures of the compounds obtainable as shown in the above Preparations and Examples are described in the following tables:

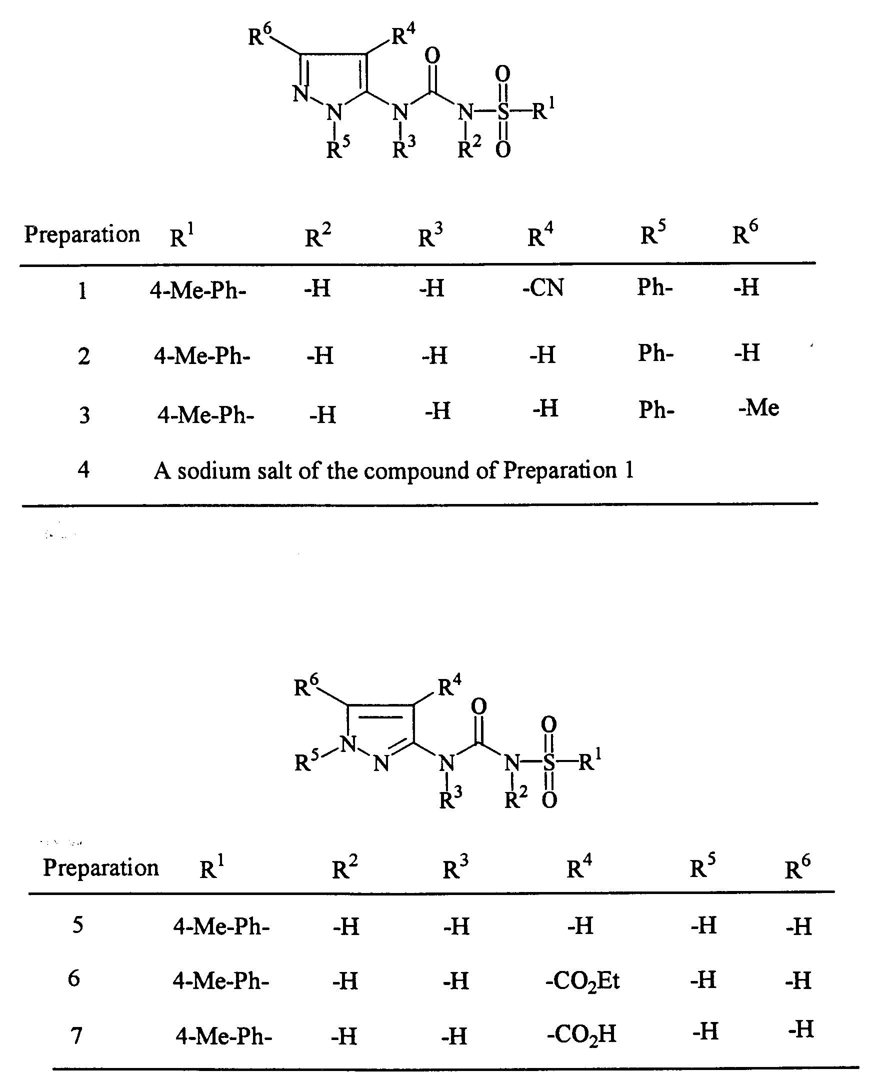

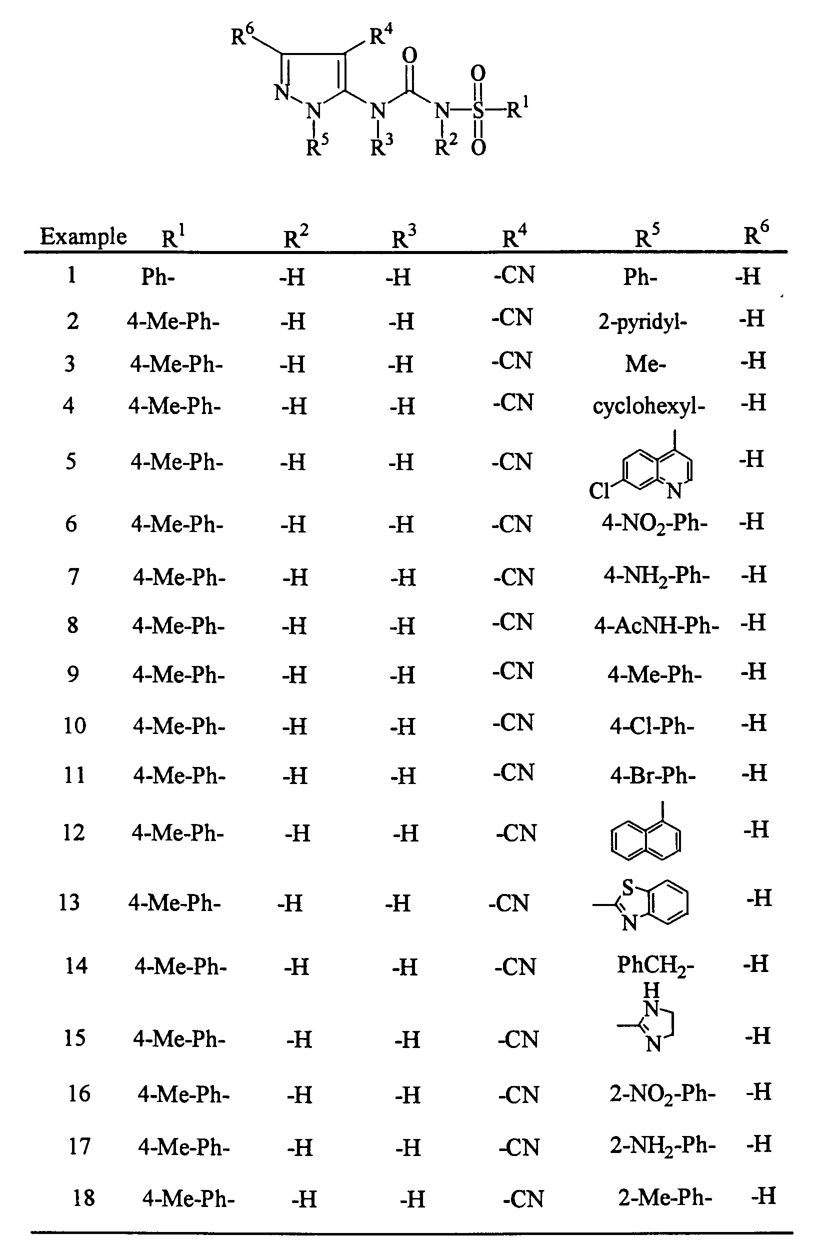

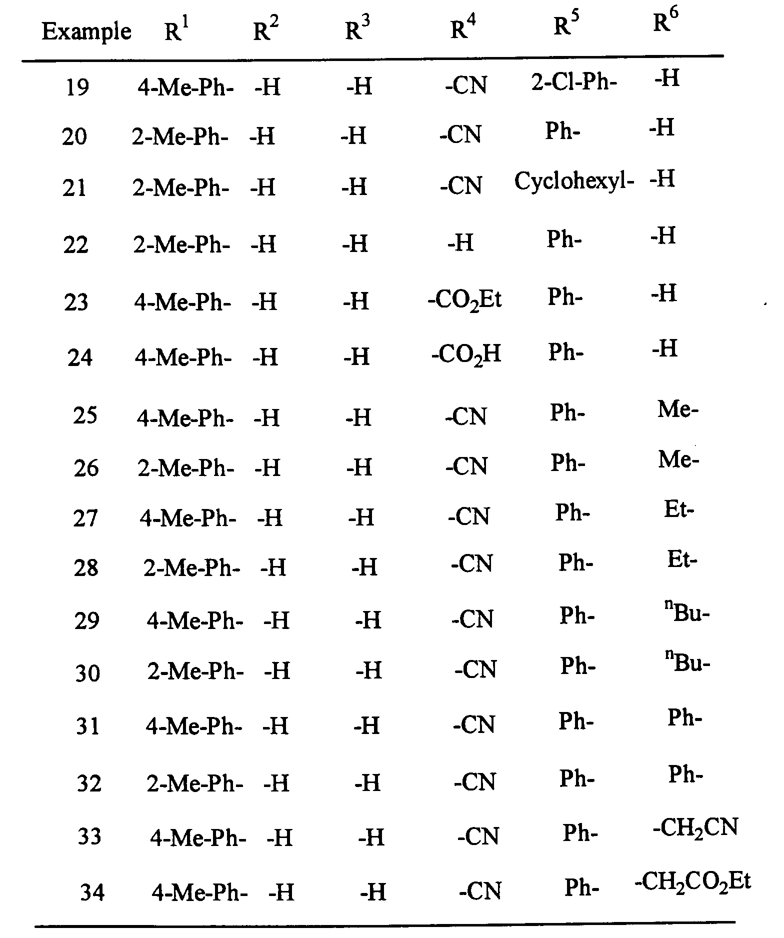

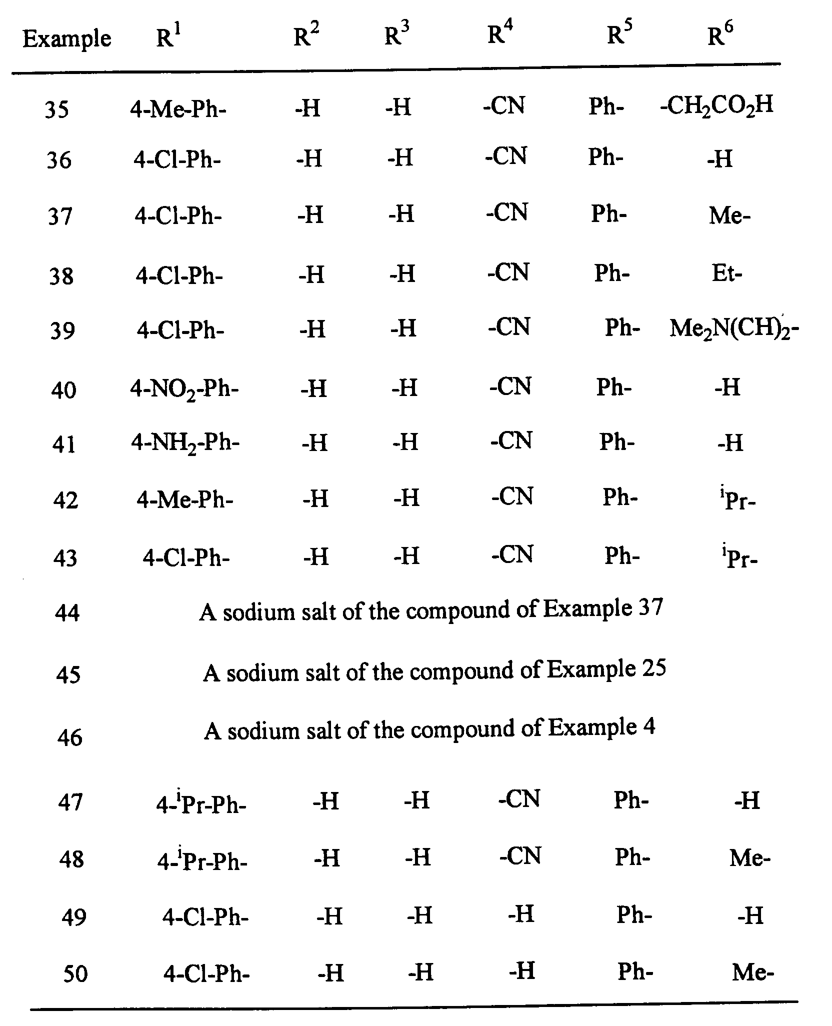

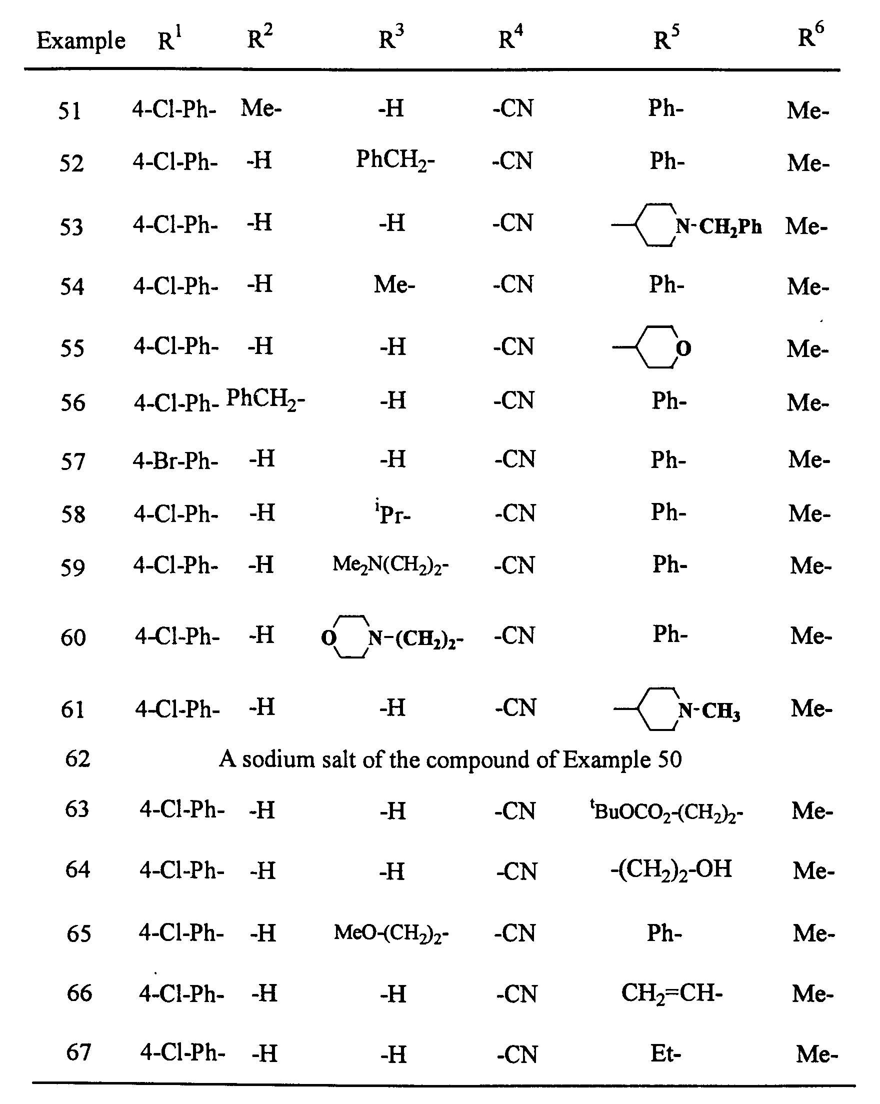

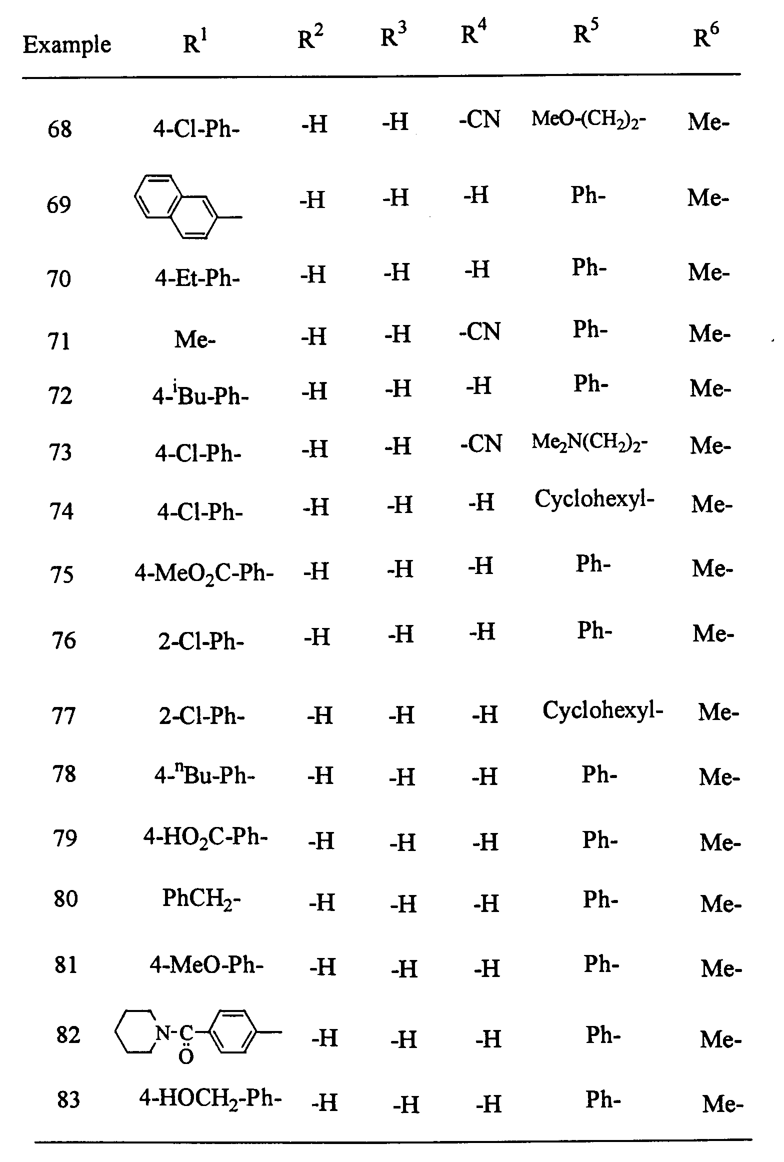

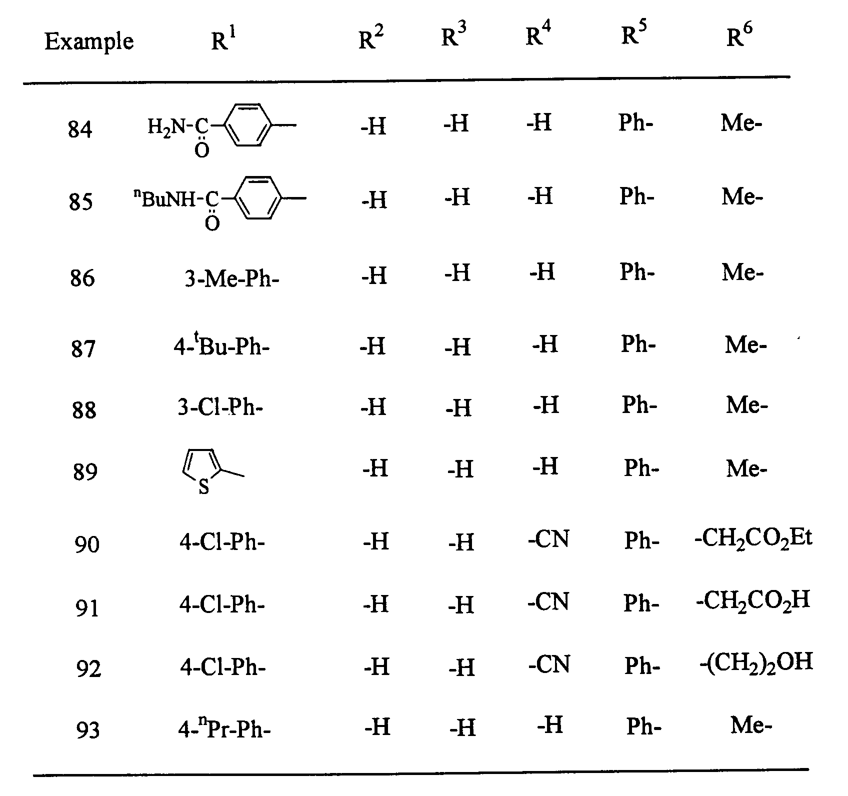

### EFFECT OF THE INVENTION

### Test 1 ECE inhibitory activities of 5-sulfonylureidopyrazole derivatives

### Methods

### Preparation of rat lung ECE and measurement of ECE inhibitory activity

Rat lung tissue was homogenized with a homogenizer in ice-cold 20 mM Tris-HCl buffer (pH7.5) containing 5 mM magnesium chloride, 1 mM phenylmethylsulfonyl fluoride (PMSF), 20 µM pepstatin A, and 20 µM leupeptin. The homogenate was centrifuged at 800 x G, and the supernatant was ultracentrifuged at 100,000 x G. Then, the procedures in which the resulting pellet was resuspended in the above buffer and ultracentrifuged as shown above, were repeated further twice so that plasma components and the like were removed. The resulting pellet was suspended, homogenized with a glass homogenizer, and ultracentrifuged. The resulting pellet was solubilized in 0.5% Triton X- 100. The solution was ultracentrifuged to obtain a supernatant, which can be used as the rat lung ECE. Each test compound and the rat lung ECE (10 µg) were preincubated at 37°C for 15 minutes in a 100mM Tris-HCl buffer (pH7.0) containing 1mM NEM, 100µM leupeptin, and 20µM pepstatin A, and then human big ET-1 (0.8µg) was added thereto (totally 200µl), and the mixture was incubated at 37°C for one hour.

The reaction was stopped by the addition of EDTA to give a final concentration of 1mM. ECE activities were determined to quantify the produced ET-1 by the ET-1 specific sandwich enzyme immunoassay method.

ECE inhibitory activities of the test compounds were evaluated by determination of the ECE activities in the presence or absence of the test compounds.

### Results

The sulfonylureidopyrazole derivatives was demonstrated to inhibit ECE, as shown in the following table.

### ECE inhibitory activies of sulfonylureidopyrazole derivatives

| The present compound | IC50(µM) |
|---|---|
| Preparation 1 | 4.6 |
| Example 4 | 0.29 |
| Example 37 | 0.045 |

### Test 2 Effect on the change in blood pressure induced by big ET-1 in rats

### Methods

Male SD rat (body weight 230∼280g) was anesthetized with sodium thiobarbiturate (65mg/kg, i.p.) and fixed on a warming surgical bed, and the right femoral artery and the vein were cannulated for measurement of arterial blood pressure and for injection of test compounds, respectively. The ganglia were blocked by intraperitoneal administration of pentolinium (10mg/kg). After stabilization of blood pressure for about 10 minutes, test compound or its vehicle (polyethylene glycol 400) was administered intravenously (0.5ml/kg). Fifteen minutes later, the big ET-1 (1nmol/kg) was administered intravenously. ECE inhibitory activities of the test compounds in vivo shown by an index of the change in blood pressure induced by big ET-1 were evaluated by inhibitory ratio to that of the vehicle group.

### Results

Big ET-1-induced increasing in blood pressure was apparently inhibited by administration of the compound of Example 37 (3 and 10mg/kg), as shown in the following table.

| Dose (mg/kg) | Increase in systolic pressure (mmHg) |
|---|---|
| 0 | 96 ± 4 |
| 3 | 65 ± 8 |
| 10 | 34 ± 6 |

### Referential Example

The inhibitory activity of the compound of Preparation 1 against metalloproteases other than ECE, such as endopeptidase, stromelissin, and the like, are under 20% at 10⁻⁵M, and therefore, the compound is highly specific to ECE.

As illustrated above, the compounds of the present invention have significant endothelin converting enzyme inhibitory activity, and are useful in treating and preventing various diseases which are induced or suspected to be induced by ET, such as for example, cardiac failure such as myocardial ischemia, congestive heart failure, arrhythmia, angina, cardiac hypertrophy, hypertension; tracheal constriction such as pulmonary hypertension, asthma; nervous disorder such as cerebral vasospasm, subarachnoid hemorrhage, stroke, cerebral infarction, Alzheimer's disease; parasecretion such as eclampsia; vascular disorder such as arteriosclerosis, Buerger's disease, Takayasu's arteritis, Raynaud's disease, complication of diabetes mellitus; ulcer such as gastric ulcer; cancer such as lung cancer; damage of gastric mucosa; endotoxin shock; sepsis; renal damage such as acute and chronic renal failure; and the like.

## Claims

1. A composition for inhibiting endothelin converting enzyme which comprises a compound of formula (1) or (2): 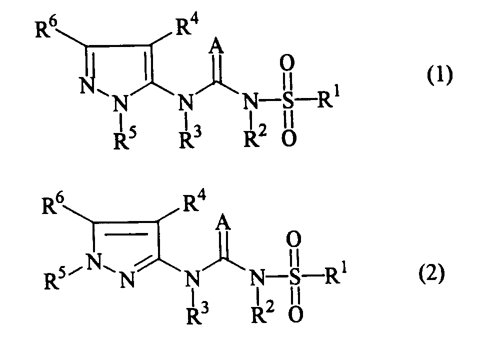 wherein:
A is an oxygen atom or a sulfur atom;
R¹ is an alkyl group, an alkenyl group, an alkynyl group, a cycloalkyl group, a cycloalkenyl group, an aryl group, a heteroarylalkyl group, a heterocyclic group, -OR⁷, -SR⁷, N(R⁷)R⁷¹, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heteroarylalkyl group, or a substituted heterocyclic group, or a group of formula (a): 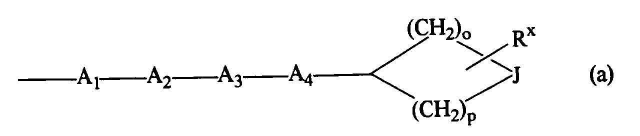 or formula (b): 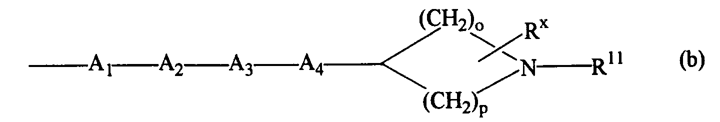
R² and R³, which may be the same or different, are each a hydrogen atom, an alkyl group, an alkenyl group, an alkynyl group, a cycloalkyl group, a cycloalkenyl group, an aryl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (a) or (b) as shown above;
R⁴ and R⁶, which may be the same or different, are each a hydrogen atom, a halogen atom, a cyano group, a nitro group, an alkyl group, an alkenyl group, an alkynyl group, a cycloalkyl group, a cycloalkenyl group, an aryl group, a heterocyclic group, a heteroarylalkyl group, -OR¹², -N(R¹²)R¹³, -CO-R¹², -CS-R¹², -CO₂-R¹², -CO-S-R¹², -CS₂-R¹², -CS-O-R¹², -O-CO-R¹², -O-CS-R¹², -S-CO-R¹², -S-CS-R¹², -CON(R¹²)R¹³, -CSN(R¹²)R¹³, -S(O)ₗ-R¹², -SO₂-N(R¹²)R¹³, -N(R¹²)-CO-R¹³, -OSO₂-R¹², a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heteroarylalkyl group, or a substituted heterocyclic group, or a group of formula (a) or (b) as shown above;
R⁵ is a hydrogen atom, an alkyl group, an alkenyl group, an alkynyl group, a cycloalkyl group, a cycloalkenyl group, an aryl group, a heterocyclic group, a heteroarylalkyl group, -CO-R¹², -CS-R¹², -CO₂-R¹², -CO-S-R¹², -CS₂-R¹², -CS-O-R¹², -CON(R¹²)R¹³, -CSN(R¹²)R¹³, -S(O)ₗ-R¹², or -SO₂-N(R¹²)R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (a) or (b) as shown above;
R⁷ and R⁷¹ are the same or different and are each a hydrogen atom, an alkyl group, an alkenyl group, an alkynyl group, a cycloalkyl group, a cycloalkenyl group, an aryl group, a heteroarylalkyl group, a heterocyclic group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group,
provided that in the case of -N(R⁷)R⁷¹, then R⁷ and R⁷¹ may be combined together with the nitrogen atom to which they are attached to form a saturated 3- to 8-membered ring which optionally contains other heteroatoms in the ring;
A₁, A₂, A₃, and A₄ are the same or different and are each a bond or -CH₂-, or any two adjacent groups of them are combined together to form -CH=CH-, or -C≡C-;
R^{X} may be absent, or one or more groups which replaces by a hydrogen atom attached to a ring carbon atom, and they are the same or different and are each a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, a heterocyclic group, or -A₅-A₆-A₇-A₈-R^{Y};
o and p are independently 0 or an integer of 1 to 3, provided that o and p are not simultaneously 0;
J is an oxygen atom, or -S(O)_{q}- in which q is 0, 1, or 2;
R¹¹ is a hydrogen atom, an alkyl group, an alkenyl group, an alkynyl group, a cycloalkyl group, a cycloalkenyl group, an aryl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
A₅, A₆, A₇, and A₈ are the same or different and are each a bond or -CH₂-, or any two adjacent groups of them are combined together to form -CH=CH-, or -C≡C-;
R^{Y} is -OR⁸, -N(R⁸)R⁹, -CO-R⁸, -CS-R⁸, -CO₂-R⁸, -CO-S-R⁸, -CS₂-R⁸, -CS-O-R⁸, -O-CO-R⁸, -O-CS-R⁸, -S-CO-R⁸, -S-CS-R⁸, -CON(R⁸)R⁹, -CSN(R⁸)R⁹, -S(O)ₗ-R⁸, -SO₂-N(R⁸)R⁹, -O-CO₂-R⁸, or -N(R⁸)-CO-R⁹;
l is 0, 1, or 2;
R⁸ and R⁹ are the same or different and are independently a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, or a heteroarylalkyl group,
provided that in the case of -N(R⁸)R⁹, -CON(R⁸)R⁹, -CSN(R⁸)R⁹, -SO₂-N(R⁸)R⁹, or -N(R⁸)-CO-R⁹, then R⁸ and R⁹ may be combined together with the nitrogen atom (and carbon atom) to which they are attached to form a saturated 3- to 8-membered ring which optionally contains other heteroatoms in the ring,
provided that R⁸ is not a hydrogen atom in the case of -O-CO-R⁸, -O-CS-R⁸, -S-CO-R⁸, -S-CS-R⁸, -SO-R⁸, or -SO₂-R⁸;
R¹² and R¹³ are the same or different and are each a hydrogen atom, an alkyl group, an alkenyl group, an alkynyl group, a cycloalkyl group, a cycloalkenyl group, an aryl group, a heteroarylalkyl group, a heterocyclic group, a substituted alkyl group, a substituted alkenyl group, a substituted alkynyl group, a substituted cycloalkyl group, a substituted cycloalkylalkyl group, a substituted cycloalkenyl group, a substituted cycloalkenylalkyl group, a substituted aryl group, a substituted aralkyl group, a substituted heteroarylalkyl group, or a substituted heterocyclic group,
provided that R¹² is not a hydrogen atom, in the case of -O-CO-R¹², -O-CS-R¹², -S-CO-R¹², -S-CS-R¹², -SO-R¹², or -SO₂-R¹²;
in the above R¹ to R¹³, such substituents on the substituted alkyl group, the substituted alkenyl group, and the substituted alkynyl group are the same or different and are each one or more selected from the group consisting of a halogen atom, a nitro group, a cyano group, a cycloalkyl group, a cycloalkenyl group, an aryl group, -A₅-A₆-A₇-A₈-R^{Y}, and a group of the formula: 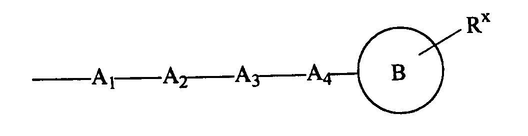 wherein A₁, A₂, A₃, A₄ and R^{X} are as defined above, B ring is a cycloalkyl group, a cycloalkenyl group, an aryl group, or a heterocyclic group; and
such substituents on the substituted cycloalkyl group, the substituted cycloalkenyl group, the substituted cycloalkylalkyl group, the substituted cycloalkenylalkyl group, the substituted aryl group, the substituted aralkyl group, the substituted heterocyclic group, or the substituted heteroarylalkyl group are the same or different and are each one or more selected from the group consisting of a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -A₅-A₆-A₇-A₈-R^{Y}, and a group of the formula: 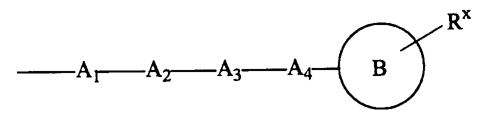 wherein A₁, A₂, A₃, A₄ and R^{X} are as defined above, B ring is a cycloalkyl group, a cycloalkenyl group, an aryl group, or a heterocyclic group; or
any substituents attached to the adjacent carbon atoms may be optionally combined together with the carbon atoms with which they are substituted to form a 4- to 8-membered ring optionally containing other heteroatoms;
or a pharmaceutically acceptable acid addition salt or alkali salt thereof.

2. A pharmaceutical composition for treating or preventing a cardiac failure, a tracheal constriction, a nervous disorder, a parasecretion, a vascular disorder, an ulcer, a cancer, a damage of gastric mucosa, an endotoxin shock, sepsis, or a renal damage, which comprises the compound of formula (1) or (2): 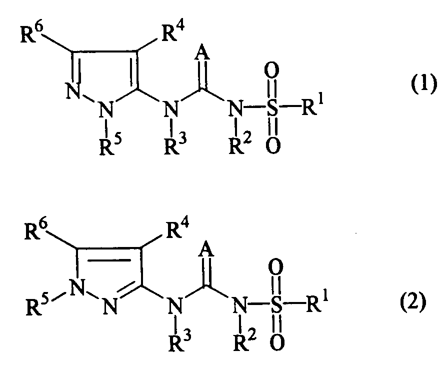 wherein A, R¹, R², R³, R⁴, R⁵ and R⁶ are as defined in Claim 1; or a pharmaceutically acceptable acid addition salt or alkali salt thereof.

3. The pharmaceutical composition as claimed in Claim 2, in which the composition is for treating or preventing myocardial ischemia, congestive heart failure, arrhythmia, angina, cardiac hypertrophy, hypertension, pulmonary hypertension, asthma, cerebral vasospasm, subarachnoid hemorrhage, stroke, cerebral infarction, Alzheimer's disease, eclampsia, arteriosclerosis, Buerger's disease, Takayasu's arteritis, Raynaud's disease, a complication of diabetes mellitus, lung cancer, gastric ulcer, a damage of gastric mucosa, an endotoxin shock, sepsis, or acute or chronic renal failure.

4. A 5-sulfonylureido-(1H)-pyrazole derivative of formula (1): 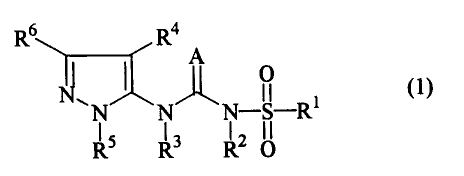 wherein A, R¹, R², R³, R⁴, R⁵ and R⁶ are as defined in Claim 1; provided that the following compounds are excluded:
i) a compound of the formula: 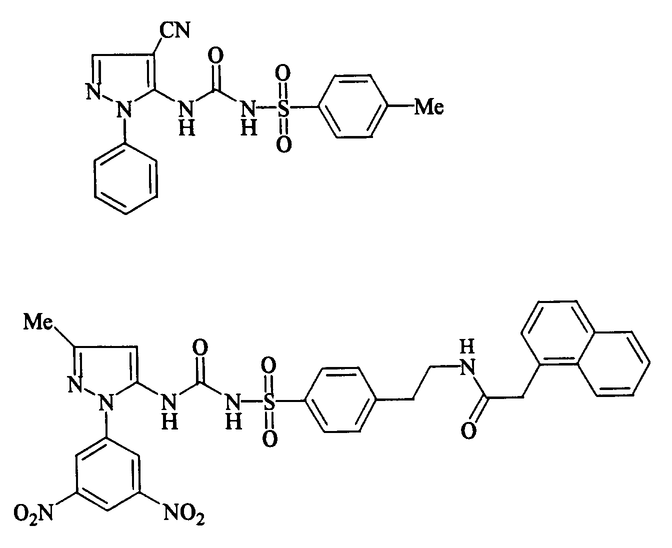 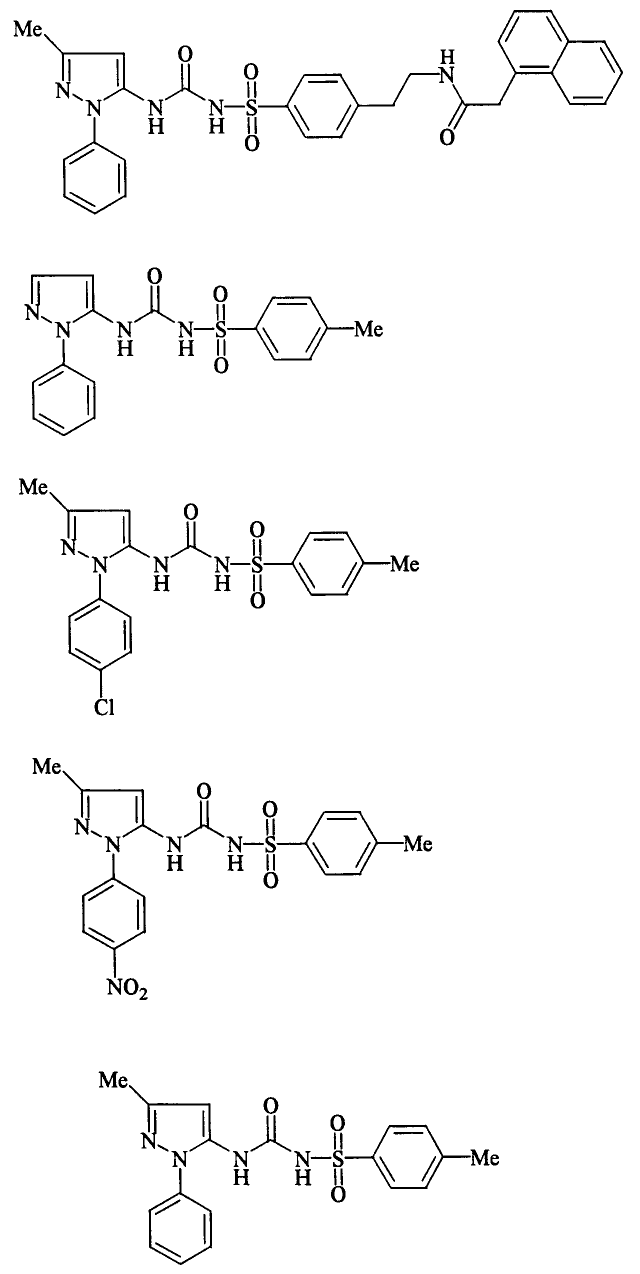 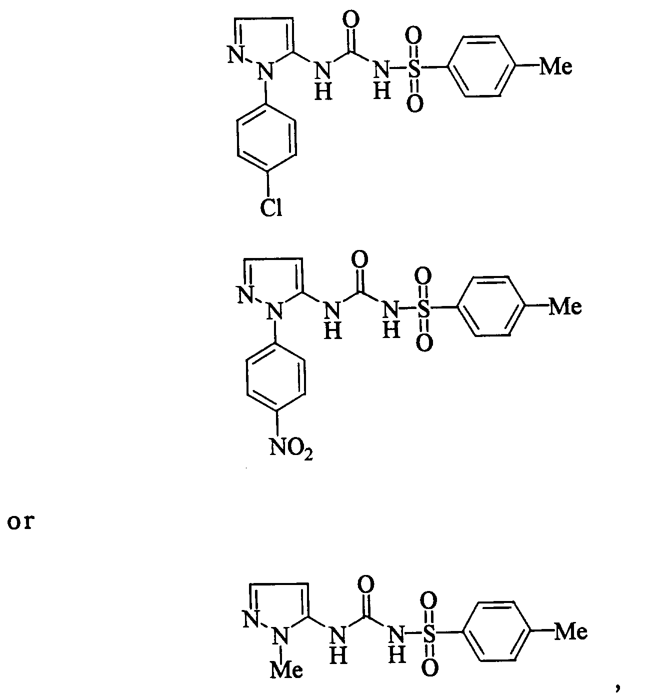
ii) a compound wherein R¹ is 2-methylphenyl group, and R⁵ is hydrogen or an alkyl group,
iii) a compound wherein R¹ is -N(R⁷)R⁷¹, and
iv) a compound wherein R¹ is a substituted heteroarylalkyl group
; or a pharmaceutically acceptable acid addition salt or alkali salt thereof.

5. A 3-sulfonylureido-(1H)-pyrazole derivative of formula (2): 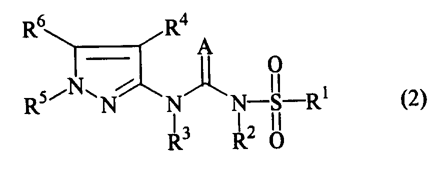 wherein A, R¹, R², R³, R⁴, R⁵ and R⁶ are as defined in Claim 1; provided that the following compounds are excluded:
i) a compound of the formula: 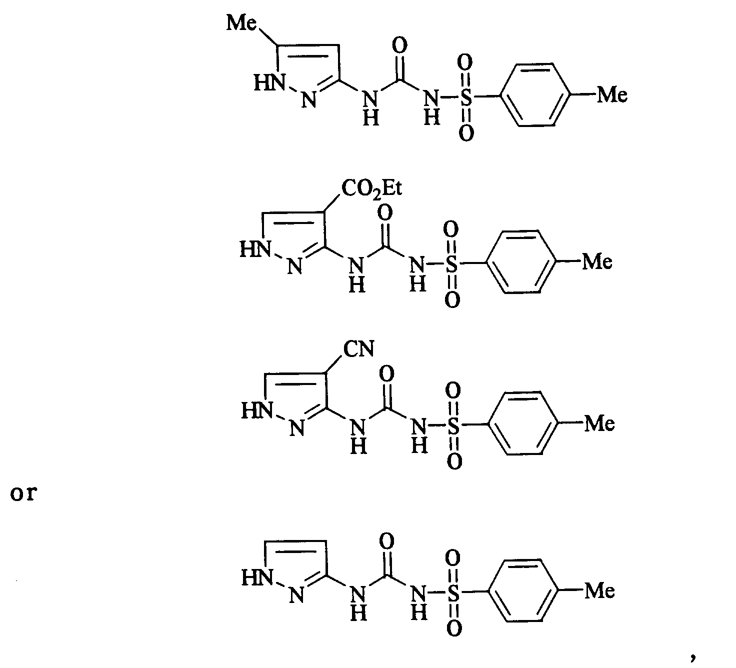
ii) a compound wherein R¹ is 2-methylphenyl group, and R⁵ is hydrogen,
iii) a compound wherein R¹ is a substituted or unsubstituted pyrazolyl,
iv) R¹ is a substituted heteroarylalkyl group
;or a pharmaceutically acceptable acid addition salt or alkali salt thereof.

6. The compound as claimed in Claim 5, wherein
R¹ is an aryl group, a substituted aryl group, an alkyl group, a substituted alkyl group, a cycloalkyl group, a substituted cycloalkyl group, or a substituted aralkyl group;
R² and R³, which may be the same or different, are each a hydrogen atom, an alkyl group, a substituted alkyl group, or an aralkyl group;
R⁴ is a hydrogen atom, or a cyano group;
R⁵ is an alkyl group, a cycloalkyl group, an aryl group, a substituted alkyl group, a substituted cycloalkyl group, a substituted aryl group, or a group of formula (a)
R⁶ is a hydrogen atom, an alkyl group, a substituted alkyl group, an aryl group, or a substituted aryl group;
or a pharmaceutically acceptable acid addition salt or alkali salt thereof.

7. A compound of formula (1): 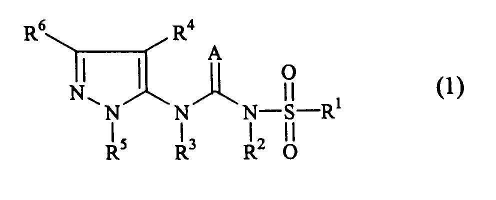 wherein:
A is an oxygen atom or a sulfur atom;
R¹ is an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁷, -SR⁷, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, or a substituted heteroarylalkyl group, or a group of formula (c): 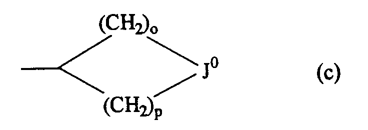 or formula (d): 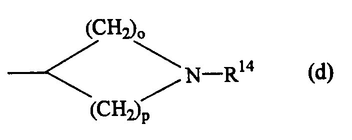
R² and R³, which may be the same or different, are each a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (c) or (d) as shown above;
R⁴ is a hydrogen atom, a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -OSO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (c) or (d) as shown above;
R⁵ is an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -S(O)ₗ-R¹², -CO₂-R¹², -CO-R¹², -CS-R¹², -CON(R¹²)R¹³, -SO₂-N(R¹²)R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
R⁶ is a hydrogen atom, a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -O-SO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
R⁷ is a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
o and p are independently 0 or an integer of 1 to 3, provided that o and p are not simultaneously 0;
J^{O} is an oxygen atom, or a sulfur atom;
R¹⁴ is a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
l is 0, 1, or 2;
R⁸ and R⁹ are the same or different and are independently a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, or a heteroarylalkyl group;
R¹² and R¹³ are the same or different and are each a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a substituted alkyl group, a substituted cycloalkyl group, a substituted cycloalkenyl group, a substituted aryl group, a substituted aralkyl group, a substituted heterocyclic group, or a substituted heteroarylalkyl group;
in the above R¹ to R¹³, such substituents on the substituted alkyl group, the substituted alkenyl group, and the substituted alkynyl group are the same or different and are each one or more selected from the group consisting of a halogen atom, a nitro group, a cyano group, a cycloalkyl group, a cycloalkenyl group, an aryl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹; and
such substituents on the substituted cycloalkyl group, the substituted cycloalkenyl group, the substituted cycloalkylalkyl group, the substituted cycloalkenylalkyl group, the substituted aryl group, the substituted aralkyl group, the substituted heterocyclic group, and the substituted heteroarylalkyl group are the same or different and are each one or more selected from the group consisting of a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹,
provided that i) when R⁴ is the hydrogen atom, then R¹ is 4-chlorophenyl or 2-methylphenyl, ii) when R⁵ is the alkyl group, then R⁴ is the cyano group, and iii) the compound of the formula: 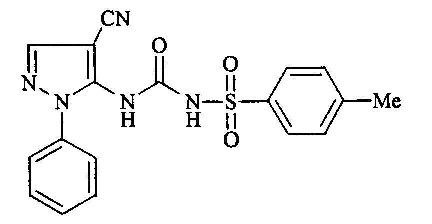 is excluded;
or a pharmaceutically acceptable acid addition salt or alkali salt thereof.

8. A compound of formula (2): 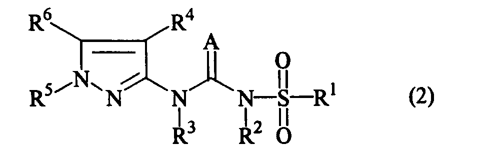 wherein:
A is an oxygen atom or a sulfur atom;
R¹ is an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁷, -SR⁷, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, or a substituted heteroarylalkyl group, or a group of formula (c): 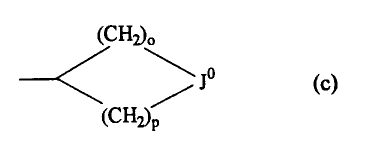 or formula (d): 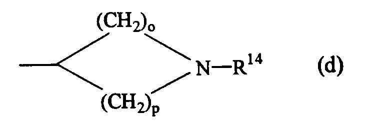
R² and R³, which may be the same or different, are each a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group, or a group of formula (c) or (d) as shown above;
R⁴ is a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -OSO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
R⁵ is an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -S(O)ₗ-R¹², -CO₂-R¹², -CO-R¹², -CS-R¹², -CON(R¹²)R¹³, -SO₂-N(R¹²)R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
R⁶ is a hydrogen atom, a halogen atom, a cyano group, a nitro group, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, -N(R¹²)R¹³, -OR¹², -S(O)ₗ-R¹², -CO₂-R¹², -CO-R¹², -CS-R¹², -O-CO-R¹², -CON(R¹²)R¹³, -O-SO₂-R¹², -SO₂-N(R¹²)R¹³, -N(R¹²)-CO-R¹³, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, a substituted heteroarylalkyl group, or a group of formula (c) or (d) as shown above;
R⁷ is a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
o and p are independently 0 or an integer of 1 to 3, provided that o and p are not simultaneously 0;
J^{O} is an oxygen atom, or a sulfur atom;
R¹⁴ is a hydrogen atom, an alkyl group, an alkenyl group, an alkynyl group, a cycloalkyl group, a cycloalkylalkyl group, a cycloalkenyl group, a cycloalkenylalkyl group, an aryl group, an aralkyl group, a heterocyclic group, a heteroarylalkyl group, a substituted alkyl group, a substituted alkenyl group, a substituted alkynyl group, a substituted cycloalkyl group, a substituted cycloalkenyl group, a substituted cycloalkylalkyl group, a substituted cycloalkenylalkyl group, a substituted aryl group, a substituted aralkyl group, a substituted heterocyclic group, or a substituted heteroarylalkyl group;
l is 0, 1, or 2;
R⁸ and R⁹ are the same or different and are independently a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, or a heteroarylalkyl group;
R¹² and R¹³ are the same or different and are each a hydrogen atom, an alkyl group, an alkenyl group, an alkynyl group, a cycloalkyl group, a cycloalkenyl group, a cycloalkylalkyl group, a cycloalkenylalkyl group, an aryl group, an aralkyl group, a heterocyclic group, a substituted alkyl group, a substituted cycloalkyl group, a substituted cycloalkenyl group, a substituted aryl group, a substituted aralkyl group, a substituted heterocyclic group, or a substituted heteroarylalkyl group;
in the above R¹ to R¹³, such substituents on the substituted alkyl group, the substituted alkenyl group, and the substituted alkynyl group are the same or different and are each one or more selected from the group consisting of a halogen atom, a nitro group, a cyano group, a cycloalkyl group, a cycloalkenyl group, an aryl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹; and
such substituents on the substituted cycloalkyl group, the substituted cycloalkenyl group, the substituted cycloalkylalkyl group, the substituted cycloalkenylalkyl group, the substituted aryl group, the substituted aralkyl group, the substituted heterocyclic group, and the substituted heteroarylalkyl group are the same or different and are each one or more selected from the group consisting of a halogen atom, a nitro group, a cyano group, an alkyl group, an alkenyl group, an alkynyl group, a cycloalkyl group, a cycloalkylalkyl group, a cycloalkenyl group, a cycloalkenylalkyl group, an aryl group, an aralkyl group, a heteroarylalkyl group, -OR⁸, -N(R⁸)R⁹, -CO-R⁸, -CS-R⁸, -CO₂-R⁸, -O-CO-R⁸, -CONR⁸R⁹, -S(O)ₗ-R⁸, -SO₂-N(R⁸)R⁹, and -N(R⁸)-CO-R⁹;
or a pharmaceutically acceptable acid addition salt or alkali salt thereof.

9. The 5-sulfonylureido-(1H)-pyrazole derivative as claimed in Claim 4 wherein R¹ is an aryl group, a substituted aryl group, an alkyl group, a substituted alkyl group, a cycloalkyl group, a substituted cycloalkyl group, or a substituted aralkyl group; or a pharmaceutically acceptable acid addition salt or alkali salt thereof.

10. The 5-sulfonylureido-(1H)-pyrazole derivative as claimed in Claim 4 wherein at least one of R² and R³ is a hydrogen atom, an alkyl group, a substituted alkyl group, or an aralkyl group; or a pharmaceutically acceptable acid addition salt or alkali salt thereof.

11. The 5-sulfonylureido-(1H)-pyrazole derivative as claimed in Claim 4 wherein R⁴ is hydrogen atom, cyano group, methyl group, or ethyl group; or a pharmaceutically acceptable acid addition salt or alkali salt thereof.

12. The 5-sulfonylureido-(1H)-pyrazole derivative as claimed in Claim 4 wherein R⁵ is an alkyl group, a cycloalkyl group, an aryl group, a substituted alkyl group, a substituted cycloalkyl group, a substituted aryl group, or a group of formula (a): 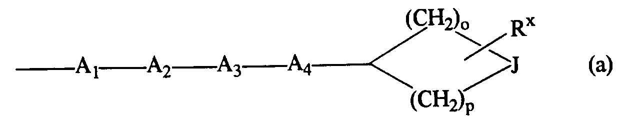 wherein A₁, A₂, A₃, A₄, R^{X}, J, o and p are as defined in Claim 1; or a pharmaceutically acceptable acid addition salt or alkali salt thereof.

13. The 5-sulfonylureido-(1H)-pyrazole derivative as claimed in Claim 4 wherein R⁶ is a hydrogen atom, an alkyl group, a substituted alkyl group, an aryl group, or a substituted aryl group; or a pharmaceutically acceptable acid addition salt or alkali salt thereof.

14. The 5-sulfonylureido-(1H)-pyrazole derivative as claimed in Claim 4 wherein R¹ is cyclohexyl group, phenyl group, 2-naphthyl group, 3-naphthyl group, 3-tolyl group, 4-tolyl group, 3-ethylphenyl group, 4-ethylphenyl group, 3-n-propylphenyl group, 4-n-propylphenyl group, 3-isopropylphenyl group, 4-isopropylphenyl group, 3-n-butylphenyl group, 4-n-butylphenyl group, 3-isobutylphenyl group, 4-isobutylphenyl group, 3-methoxyphenyl group, 4-methoxyphenyl group, 3-chlorophenyl group, 4-chlorophenyl group, 3-bromophenyl group, 4-bromophenyl group or benzyl group; or a pharmaceutically acceptable acid addition salt or alkali salt thereof.

15. The 5-sulfonylureido-(1H)-pyrazole derivative as claimed in Claim 4 wherein R² is hydrogen atom, methyl group or benzyl group, R³ is hydrogen atom, methyl group, 2-methoxyethyl group or benzyl group; or a pharmaceutically acceptable acid addition salt or alkali salt thereof.

16. The 5-sulfonylureido-(1H)-pyrazole derivative as claimed in Claim 4 wherein R⁵ is vinyl group, ethyl group, n-propyl group, isopropyl group, cyclohexyl group, cyclopentyl group, phenyl group, thiophen-2-yl, thiophen-3-yl, furan-2-yl, furan-3-yl or tetrahydro-(4H)-pyran-4-yl; or a pharmaceutically acceptable acid addition salt or alkali salt thereof.

17. The 5-sulfonylureido-(1H)-pyrazole derivative as claimed in Claim 4 wherein R⁶ is hydrogen atom, methyl group, ethyl group, n-propyl group, n-butyl group, isopropyl group, cyanomethyl group, methoxycarbonylmethyl group or ethoxycarbonylmethyl group; or a pharmaceutically acceptable acid addition salt or alkali salt thereof.

18. The 5-sulfonylureido-(1H)-pyrazole derivative as claimed in Claim 4 wherein R¹ is an aryl group, a substituted aryl group, an alkyl group, a substituted alkyl group, a cycloalkyl group, a substituted cycloalkyl group, or a substituted aralkyl group;
R² and R³ are the same or different and are each a hydrogen atom, an alkyl group, a substituted alkyl group, or an aralkyl group;
R⁴ is a hydrogen atom, or a cyano group;
R⁵ is an alkyl group, a cycloalkyl group, an aryl group, a substituted alkyl group, a substituted cycloalkyl group, a substituted aryl group or a group of foumula (a);
R⁶ is a hydrogen atom, an alkyl group, a substituted alkyl group, an aryl group or a substituted aryl group; or a pharmaceutically acceptable acid addition salt or alkali salt thereof.

19. The 5-sulfonylureido-(1H)-pyrazole derivative as claimed in Claim 4, which is selected from the group consisting of:
(1) 5-{3-(4-toluenesulfonyl)ureido}-4-cyano-1-cyclohexyl-(1H)-pyrazole;
(2) 5-(3-benzenesulfonylureido)-4-cyano-1-phenyl-(1H)-pyrazole;
(3) 5-{3-(4-toluenesulfonyl)ureido}-4-cyano-3-methyl-1-phenyl-(1H)-pyrazole;
(4) 5-{3-(4-toluenesulfonyl)ureido}-4-cyano-3-ethyl-1-phenyl-(1H)-pyrazole;
(5) 5-{3-(4-toluenesulfonyl)ureido}-4-cyano-3-n-butyl-1-phenyl-(1H)-pyrazole;
(6) 5-{3-(4-chlorobenzenesulfonyl)ureido}-4-cyano-1-phenyl-(1H)-pyrazole;
(7) 5-{3-(4-chlorobenzenesulfonyl)ureido}-4-cyan-3-methyl-1-phenyl-(1H)-pyrazole;
(8) 5-{3-(4-chlorobenzenesulfonyl)ureido}-4-cyano-3-ethyl-1-phenyl-(1H)-pyrazole;
(9) 5-{3-(4-toluenesulfonyl)ureido}-4-cyano-3-cyanomethyl-1-phenyl-(1H)-pyrazole;
(10) 5-{3-(4-toluenesulfonyl)ureido}-4-cyano-3-ethoxycarbonylmethyl-1-phenyl-(1H)-pyrazole;
(11) 5-{3-(4-toluenesulfonyl)ureido}-4-cyano-3-isopropyl-1-phenyl-(1H)-pyrazole;
(12) 5-{3-(4-chlorobenzenesulfonyl)ureido}-4-cyano-3-isopropyl-1-phenyl-(1H)-pyrazole;
(13) 5-{3-(4-chlorobenzenesulfonyl)ureido}-1-phenyl-(1H)-pyrazole;
(14) 5-{3-(4-chlorobenzenesulfonyl)ureido}-3-methyl-1-phenyl-(1H)-pyrazole;
(15) 5-{3-(4-isopropylbenzenesulfonyl)ureido}-4-cyano-1-phenyl-(1H)-pyrazole;
(16) 5-{3-(4-isopropylbenzenesulfonyl)ureido}-4-cyano-3-methyl-1-phenyl-(1H)-pyrazole;
(17) 5-{3-(4-chlorobenzenesulfonyl)-1-benzylureido}-4-cyano-3-methyl-1-phenyl-(1H)-pyrazole;
(18) 5-{3-(4-chlorobenzenesulfonyl)ureido}-4-cyano-3-methyl-1-{tetrahydro-(4H)-pyran-4-yl}-(1H)-pyrazole;
(19) 5-{3-benzyl-3-(4-chlorobenzenesulfonyl)-ureido}-4-cyano-3-methyl-1-phenyl-(1H)-pyrazole;
(20) 5-{3-(4-bromobenzenesulfonyl)ureido}-4-cyano-3-methyl-1-phenyl-(1H)-pyrazole;
(21) 5-{3-(2-naphthylsulfonyl)ureido}-3-methyl-1-phenyl-(1H)-pyrazole;
(22) 5-{3-(4-ethylbenzenesulfonyl)ureido}-3-methyl-1-phenyl-(1H)-pyrazole;
(23) 5-{3-(4-isobutylbenzenesulfonyl)ureido}-3-methyl-1-phenyl-(1H)-pyrazole;
(24) 5-{3-(4-chlorobenzenesulfonyl)ureido}-3-methyl-1-cyclohexyl-(1H)-pyrazole;
(25) 5-{3-(4-n-butylbenzenesulfonyl)ureido}-3-methyl-1-phenyl-(1H)-pyrazole; and
(26) 5-{3-(4-methoxybenzenesulfonyl)ureido}-3-methyl-1-phenyl-(1H)-pyrazole;
or a pharmaceutically acceptable acid addition salt or alkali salt thereof.
